# EUROPEAN PATENT APPLICATION

(11) **EP 2 319 509 A1**
(43) Date of publication of application: **11.05.2011**
(21) Application number: 11153508.4
(22) Date of filing: 24.09.2004
(51) Int. Cl.: A61K 31/265, A61K 31/16, A61K 31/165, A61P 3/06, A61P 9/10, A61K 31/137, A61K 31/341, A61K 31/506, A61K 31/415, A61K 31/4245, A61K 31/196, A61K 31/4196, A61K 31/42, A61K 31/421, A61K 31/425, A61K 31/426, A61K 31/433, A61K 31/4402, A61K 31/4406

(54) **Method of Inhibiting remnant lipoprotein production**

(30) Priority: 26.09.2003 JP 2003373453; 23.07.2004 US 590811 P
(62) Divisional of application: 04773516.2
(71) Applicant: Japan Tobacco, Inc., Tokyo 105-8422 (JP)
(72) Inventor: The designation of the inventor has not yet been filed
(74) Representative: Vossius & Partner

(57) **Abstract**

The present invention aims at provision of a method for inhibiting remnant lipoprotein production and a remnant lipoprotein production inhibitor, which includes administering a compound having a CETP inhibitory activity to an administration subject. The remnant lipoprotein production inhibitor of the present invention contains a compound having a CETP inhibitory activity as an active ingredient.

## Description

### Technical Field

The present invention relates to a method of inhibiting remnant lipoprotein production, which comprises administering a compound having a CETP (cholesteryl ester transfer protein) inhibitory activity to an administration subject, and a remnant lipoprotein production inhibitor comprising a compound having a CETP inhibitory activity as an active ingredient. The present invention further relates to a prophylactic or therapeutic agent for hyperlipidemia, arteriosclerosis or hyper-remnant lipoproteinemia, which comprises such compound having a CETP inhibitory activity as an active ingredient.

### Background Art

The fat components in blood are present in lipoproteins that are bound with apolipoprotein. These serum lipoproteins are classified into chylomicron (CM), chylomicron remnant, very low density lipoprotein (VLDL), intermediate-density lipoprotein (remnant lipoprotein; IDL), low density lipoprotein (LDL) and high density lipoprotein (HDL), based on the densities thereof.

The remnant lipoprotein is a lipoprotein as an intermediate form produced by metabolism of CM or VLDL (triglyceride (TG)-rich lipoproteins) in blood due to enzymes such as lipoprotein lipase (LPL) and the like. For example, an intermediate metabolite on conversion of VLDL to LDL is VLDL remnant, and similarly, a metabolite of CM is CM remnant.

For production of remnant lipoproteins, two main routes can be mentioned. First, CM that transports exogenous fat is produced in the small intestine, flows through mesenteric lymph node and flows into blood from the thoracic duct. The CM in blood flow is subject to the action of LPL, and TG in CM particle is hydrolyzed to produce CM remnant.

On the other hand, VLDL that transports endogenous fat is produced in the liver and directly released into the blood from the liver. Thereafter, like CM, TG is dissociated from VLDL due to the action of LPL, and VLDL remnant is produced.

These remnant lipoproteins are clinically important as arteriosclerosis-promoting lipoproteins. Hyperlipidemia of the type associated with increased remnant lipoprotein in fasting serum in the early morning is called hyper-remnant lipoproteinemia.

The hyper-remnant lipoproteinemia includes hereditary hyper-remnant lipoproteinemia such as familial dysbetalipidemia, familial lipase deficiency, familial combined hyperlipidemia and the like, and secondary hyper-remnant lipoproteinemia that expresses subsequent to diabetic hypertriglyceridemia, hypothyroidism, nephritic syndrome and the like.

Many lipoproteins are considered to be capable of causing arteriosclerosis, intraarterial thrombosis, coronary artery diseases and the like. Particularly, remnant lipoprotein is considered to be deeply involved in the formation of arteriosclerotic lesion, because, unlike LDL etc., it is englobed by macrophage and promotes foaming, without undergoing further modification.

In contrast, it is known that CETP is involved in the metabolism of lipoproteins in plasma physiologically, and of those, particularly involved strongly in the metabolism of HDL. Inasmuch as HDL prevents accumulation of cholesterol in peripheral cells through the actions of cholesterol uptake and antioxidation, it is considered to be a lipoprotein that acts defensively against arteriosclerosis. In fact, a number of epidemiological researches have shown that a decrease in CE (cholesteryl ester) of HDL in blood is one of the risk factors of coronary artery diseases. It has been also clarified that the CETP activity varies among animal species, wherein arteriosclerosis due to cholesterol-loading is hardly induced in animals with lower CETP activity, and in reverse, easily induced in animals with higher CETP activity, and that hyper-HDL-cholesterolemia and hypo-LDL (low density lipoprotein)-cholesterolemia are induced in the case of CETP deficiency, thus rendering the development of arteriosclerosis difficult, which in turn led to the recognition of the significance of blood HDL, as well as significance of CETP as a risk factor of arteriosclerosis.

The remnant lipoprotein is known to have, besides macrophage foaming-promoting action, plural actions leading to the onset and progression of arteriosclerosis, such as platelet aggregation-promoting action, inhibitory action on endothelium-dependent vascular relaxing response and the like. In fact, increase in the remnant lipoprotein is reported to increase coronary artery events and sudden cardiac death. Meanwhile, blood concentration of remnant lipoprotein is known to show a strong positive correlation with blood concentration of small dense LDL, which is said to be a very bad lipoprotein, and it is widely recognized that lowering blood concentration of remnant lipoprotein is highly useful for the prophylaxis or treatment of arteriosclerosis. However, while involvement of LPL in the remnant lipoprotein production has been pointed out, nothing else is known.

Since, when the fat compositions of CM/VLDL and remnant lipoprotein are compared, remnant lipoproteins show increased CE content and decreased TG content as compared to CM/VLDL, there is a possibility that transfer of CE in HDL to CM/VLDL due to CETP and transfer of TG in CM/VLDL to HDL may lead to the production of remnant lipoproteins (see NIPPONRINSHO vol. 59, extra number 2 (2001), pp. 545-549).

However, transfer of CE in HDL to CM/VLDL due to CETP, which leads to the production of remnant lipoprotein, has not been demonstrated, and there is no experimental proof of this action.

### Disclosure of the Invention

An object of the present invention is to provide a method of inhibiting remnant lipoprotein production, which comprises administering a compound having a CETP inhibitory activity to an administration subject, and a remnant lipoprotein production inhibitor comprising a compound having a CETP inhibitory activity as an active ingredient. A further object is to provide a prophylactic or therapeutic agent for hyperlipidemia, arteriosclerosis, hyper-remnant lipoproteinemia and the like, which comprises such compound having a CETP inhibitory activity as an active ingredient.

The present inventors have conducted intensive studies to achieve the above-mentioned objects and actually found that CETP transfers CE in HDL to CM and/or VLDL to promote of remnant lipoprotein production. Moreover, they have found that a CETP inhibitor inhibits transfer of CE in HDL to CM and/or VLDL and inhibits of remnant lipoprotein production, which resulted in the completion of the present invention.

That is, the present invention provides the following.
[1] A method for inhibiting remnant lipoprotein production, which comprises administering a compound having a CETP inhibitory activity to an administration subject.
[2] A remnant lipoprotein production inhibitor comprising a compound having a CETP inhibitory activity as an active ingredient.
[3] A method for inhibiting transfer of cholesteryl ester in HDL to chylomicron and/or VLDL, which comprises administering a compound having a CETP inhibitory activity to an administration subject.
[4] An inhibitor of transfer of cholesteryl ester in HDL to chylomicron and/or VLDL, which comprises a compound having a CETP inhibitory activity as an active ingredient.
[5] A CETP inhibitor having an inhibitory activity on the transfer of cholesteryl ester in HDL to chylomicron and/or VLDL.
[6] The method of [1], wherein the inhibition of remnant lipoprotein production is achieved by inhibiting the transfer of cholesteryl ester in HDL to chylomicron and/or VLDL due to a CETP inhibitory activity.
[7] The inhibitor of [2], wherein the compound having a CETP inhibitory activity inhibits transfer of cholesteryl ester in HDL to chylomicron and/or VLDL due to a CETP inhibitory activity thereof.
[8] A CETP inhibitor that inhibits transfer of cholesteryl ester in HDL to chylomicron and/or VLDL and has a remnant lipoprotein production inhibitory activity.
[9] The method of [1] or [6], wherein the remnant lipoprotein is a chylomicron remnant.
[10] The inhibitor of [2], [7] or [8], wherein the remnant lipoprotein is a chylomicron remnant.
[11] The method of [1] or [6], wherein the remnant lipoprotein is a VLDL remnant.
[12] The inhibitor of [2], [7] or [8], wherein the remnant lipoprotein is a VLDL remnant.
[13] The method of [1], [3] or [6], wherein the compound having a CETP inhibitory activity is S-{2-([[1-(2-ethylbutyl)cyclohexyl]carbonyl]amino)phenyl} 2-methylpropanethioate.
[14] The inhibitor of [2], [4] or [7], wherein the compound having a CETP inhibitory activity is S-{2-([[1-(2-ethylbutyl)cyclohexyl]carbonyl]amino)phenyl} 2-methylpropanethioate.
[15] The inhibitor of [5] or [8], which is S-{2-([[1-(2-ethylbutyl)cyclohexyl]carbonyl]amino)phenyl} 2-methylpropanethioate.
[16] The method of [1], [3] or [6], wherein the compound having a CETP inhibitory activity is ethyl (2R,4S)-4-[[3,5-bis(trifluoromethyl)benzyl](methoxycarbonly)amino]-2-ethyl-6-(trifluoromethyl)-3,4-dihydroquinoline-l(2H)-carboxylic acid ester, an anhydride thereof, an ethanolate thereof, or a crystal thereof.
[17] The inhibitor of [2], [4] or [7], wherein the compound having a CETP inhibitory activity is ethyl (2R,4S)-4-[[3,5-bis(trifluoromethyl)benzyl](methoxycarbonyl)amino]-2-ethyl-6-(trifluoromethyl)-3,4-dihydroquinoline-1(2H)-carboxylic acid ester, an anhydride thereof, an ethanolate thereof, or a crystal thereof.
[18] The inhibitor of [5] or [8], which is ethyl (2R,4S)-4-[[3,5-bis(trifluoromethyl)benzyl](methoxycarbonyl)amino]-2-ethyl-6-(trifluoromethyl)-3,4-dihydroquinoline-1(2H)-carboxylic acid ester, an anhydride thereof, an ethanolate thereof, or a crystal thereof.
[19] The method of [1], [3] or [6], wherein the compound having a CETP inhibitory activity is methyl (3-cyano-5-trifluoromethylbenzyl)-[6-(N-cyclopentylmethyl-N-ethylamino)indan-5-ylmethyl]-carbamate.
[20] The inhibitor of [2], [4] or [7], wherein the compound having a CETP inhibitory activity is methyl (3-cyano-5-trifluoromethylbenzyl)-[6-(N-cyclopentylmethyl-N-ethylamino)indan-5-ylmethyl]-carbamate.
[21] The inhibitor of [5] or [8], which is methyl (3-cyano-5-trifluoromethylbenzyl)-[6-(N-cyclopentylmethyl-N-ethylamino)indan-5-ylmethyl]-carbamate.
[22] The method of [1], [3] or [6], wherein the compound having a CETP inhibitory activity is trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate.
[23] The inhibitor of [2], [4] or [7], wherein the compound having a CETP inhibitory activity is trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate.
[24] The inhibitor of [5] or [8], which is trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methy}cyclohexyl)acetic acid methanesulfonate.
[25] The method of [1], [3] or [6], wherein the compound having a CETP inhibitory activity is a compound represented by the formula (VII) 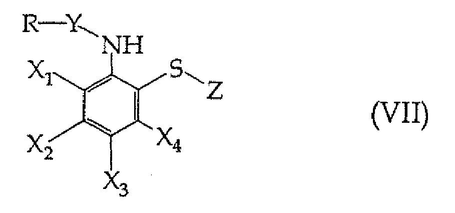 wherein
   - R: is a straight chain or branched C₁₋₁₀ alkyl group, a straight chain or branched C₂₋₁₀ alkenyl group, a halogenated C₁₋₄ lower alkyl group, an optionally substituted C₃₋₁₀ cycloalkyl group, an optionally substituted C₅₋₈ cycloalkenyl group, an optionally substituted C₃₋₁₀ cycloalkyl C₁₋₁₀ alkyl group, an optionally substituted aryl group, an optionally substituted aralkyl group or an optionally substituted 5- or 6-membered heterocyclic group having 1 to 3 from nitrogen atom, oxygen atom and sulfur atom;
   - X₁, X₂, X₃ and X₄: are the same or different and each is a hydrogen atom, a halogen atom, a C₁₋₄ lower alkyl group, a halogenated C₁₋₄ lower alkyl group, a C₁₋₄ lower alkoxy group, a cyano group, a nitro group, an acyl group or an aryl group;
   - Y: is -CO- or -SO₂-; and
   - Z: is a hydrogen atom or a mercapto protecting group,
   a prodrug compound thereof, a pharmaceutically acceptable salt thereof, or a hydrate thereof or a solvate thereof.
[26] The inhibitor of [2], [4] or [7], wherein the compound having a CETP inhibitory activity is the compound of the formula (VII) of [25], a prodrug compound thereof, a pharmaceutically acceptable salt thereof, or a hydrate thereof or a solvate thereof.
[27] The inhibitor of [5] or [8], which is the compound of the formula (VII) of [25], a prodrug compound thereof, a pharmaceutically acceptable salt thereof, or a hydrate thereof or a solvate thereof.
[28] The method of [1], [3] or [6], wherein the compound having a CETP inhibitory activity is the compound represented by the formula (VIII) 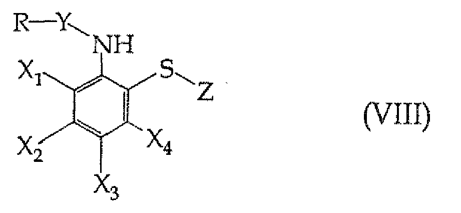 wherein
   - R: is a straight chain or branched C₁₋₁₀ alkyl group, a straight chain or branched C₂₋₁₀ alkenyl group, a halogenated C₁₋₄ lower alkyl group, a C₃₋₁₀ cycloalkyl group, a C₅₋₈ cycloalkenyl group, a C₃₋₁₀ cycloalkyl, C₁₋₁₀ alkyl group (wherein these cycloalkyl group, cycloalkenyl group and cycloalkylalkyl, group are each optionally substituted by a group selected from straight chain or branched C₁₋₁₀ alkyl group, straight chain or branched C₂₋₁₀ alkenyl group, C₃₋₁₀ cycloalkyl group, C₅₋₈ cycloalkenyl group, C₃₋₁₀ cycloalkyl, C₁₋₁₀ alkyl group, aryl group, amino group, C₁₋₄ lower alkylamino group, acylamino group, oxo group, aralkyl group and arylalkenyl group), an aryl group, an aralkyl group or a 5- or 6-membered heterocyclic group having 1 to 3 from nitrogen atom, oxygen atom and sulfur atom (wherein these aryl group, aralkyl group and heterocyclic group are each optionally substituted by a group selected from straight chain or branched C₁₋₁₀ alkyl group, straight chain or branched C₂₋₁₀ alkenyl group, halogen atom, nitro group, amino group optionally substituted by C₁₋₄ lower alkyl group or acyl group, hydroxyl group, C₁₋₄ lower alkoxy group, C₁₋₄ lower alkylthio group, halogenated C₁₋₄ lower alkyl group, acyl group and oxo group);
   - X₁, X₂, X₃ and X₄: may be the same or different and each is a hydrogen atom, a halogen atom, a C₁₋₄ lower alkyl group, a halogenated C₁₋₄ lower alkyl group, a C₁₋₄ lower alkoxy group, a cyano group, a nitro group, an acyl group or an aryl group;
   - Y: is -CO- or -SO₂-;
   - Z: is a hydrogen atom or a mercapto-protecting group selected from a group that forms a disulfide form, which is a dimer, C₁₋₄ lower alkoxymethyl group, C₁₋₄ lower alkylthiomethyl group, aralkyloxymethyl group, aralkylthiomethyl group, C₃₋₁₀ cycloalkyloxymethyl group, C₅₋₈ cycloalkenyloxymethyl group, C₃₋₁₀ cycloalkyl C₁₋₁₀ alkoxymethyl group, aryloxymethyl group, arylthiomethyl group, acyl group, acyloxy group, aminocarbonyloxymethyl group, thiocarbonyl group and thio group,
   a prodrug compound thereof, a pharmaceutically acceptable salt thereof, or a hydrate thereof or a solvate thereof.
[29] The inhibitor of [2], [4] or [7], wherein the compound having a CETP inhibitory activity is a compound of the formula (VIII) of [28], a prodrug compound thereof, a pharmaceutically acceptable salt thereof, or a hydrate thereof or a solvate thereof.
[30] The inhibitor of [5] or [8], which is a compound of the formula (VIII) of [28], a prodrug compound thereof, a pharmaceutically acceptable salt thereof, or a hydrate thereof or a solvate thereof.
[31] The method of [28], wherein the compound of the formula (VIII) is a compound selected from a group consisting of bis-[2-(pivaloylamino)phenyl]disulfide; bis-[2-(2-propylpentanoylamino)phenyl]disulfide; bis-[2-(1-methylcyclohexanecarbonylamino)phenyl]disulfide; bis-[2-(1-isopentylcyclopentanecarbonylamino)phenyl]disulfide; bis-[2-(1-isopentylcyclohexanecarbonylamino)phenyl]disulfide; N-(2-mercaptophenyl)-2,2-dimethylpropionamide; N-(2-mercaptophenyl)-1-isopentylcyclohexanecarboxamide; N-(2-mercaptophenyl)-1-methylcyclohexanecarboxamide; N-(2-mercaptophenyl)-1-isopentylcyclopentanecarboxamide; N-(2-mercaptophenyl)-1-isopropylcyclohexanecarboxamide; N-(4,5-dichloro-2-mercaptophenyl)-1-isopentylcyclohexanecarboxamide; N-(4,5-dichloro-2-mercaptophenyl)-1-isopentylcyclopentanecarboxamide; N-(2-mercapto-5-methylphenyl)-1-isopentylcyclohexanecarboxamide; N-(2-mercapto-4-methylpheny)-1-isopentylcyclohexanecarboxamide; thioacetic acid S-[2-(1-isopentylcyclohexanecarbonylamino)phenyl] ester; 2,2-dimethylthiopropionic acid S-[2-(1-methylcyclohexanecarbonylamino)phenyl] ester; phenylthioacetic acid S-[2-(pivaloylamino)phenyl] ester; 2,2-dimethylthiopropionic acid S-[2-(1-isopentylcyclohexanecarbonylamino)phenyl] ester; 2-acetylamino-3-phenylthiopropionic acid S-[2-(1-isopentylcyclohexanecarbonylamino)phenyl] ester; 3-pyridinethiocarboxylic acid S-[2-(1-isopentylcyclohexanecarbonylamino)phenyl] ester; chlorothioacetic acid S-[2-(1-isopentylcyclohexanecarbonylamino)phenyl] ester; methoxythioacetic acid S-[2-(1-isopentylcyclohexanecarbonylamino)phenyl] ester; thiopropionic acid S-[2-(1-isopentylcyclohexanecarbonylamino)phenyl] ester; phenoxythioacetic acid S-[2-(1-isopentylcyclohexanecarbonylamino)phenyl] ester; 2-methylthiopropionic acid S-[2-(1-isopentylcyclohexanecarbonylamino)phenyl] ester; 4-chlorophenoxythioacetic acid S-[2-(1-isopentylcyclohexanecarbonylamino)phenyl] ester; cyclopropanethiocarboxylic acid S-[2-(1-isopentylcyclohexanecarbonylamino)phenyl] ester; 2-acetylamino-4-carbamoylthiobutyric acid S-[2-(1-isopentylcyclohexanecarbonylamino)phenyl] ester; 2-hydroxy-2-methylthiopropionic acid 8-[2-(1-isopentylcyclohexanecarbonylamino)phenyl] ester; 2,2-dimethylthiopropionic acid S-[2-(1-isopentylcyclopentanecarbonylamino)phenyl] ester; thioacetic acid S-[2-(1-isopentylcyclopentanecarbonylamino)phenyl] ester; 2,2-dimethylthiopropionic acid S-[4,5-dichloro-2-(1-isopentylcyclohexanecarbonylamino)phenyl] ester; 2,2-dimethylthiopropionic acid S-[4,5-dichloro-2-(1-isopentylcyclopentanecarbonylamino)phenyl] ester; 2,2-dimethylthiopropionic acid S-[2-(1-isopentylcyclohexanecarbonylamino)-4-trifluoromethylphenyl] ester; thiocarbonic acid O-methyl ester S-[2-(1-isopentylcyclohexanecarbonylamino)phenyl] ester; dithiocarbonic acid S-[2-(1-methylcyclohexanecarbonylamino)phenyl] ester S-phenyl ester; N-phenylthiocarbamic acid S-[2-(1-isopentylcyclohexanecarbonylamino)phenyl] ester; 2,2-dimethylthiopropionic acid S-[2-(pivaloylamino)-4-trifluoromethylphenyl] ester; 2,2-dimethylthiopropionic acid S-[4,5-dichloro-2-(1-cyclopropylcyclohexanecarbonylamino)phenyl] ester; 2,2-dimethylthiopropionic acid S-[4,5-dichloro-2-(2-cyclohexylpropionylamino)phenyl] ester; 2,2-dimethylthiopropionic acid S-[4,5-dichloro-2-(1-pentylcyclohexanecarbonylamino)phenyl] ester; 2,2-dimethylthiopropionic acid S-[4,5-dichloro-2-(1-cyclopropylmethylcyclohexanecarbonylamino)phenyl] ester; 2,2-dimethylthiopropionic acid S-[4,5-dichloro-2-(1-cyclohexylmethylcyclohexanecarbonylamino)phenyl] ester; 2,2-dimethylthiopropionic acid S-[4,5-dichloro-2-(1-isopropylcyclohexanecarbonylamino)phenyl] ester; 2,2-dimethylthiopropionic acid S-[4,5-dichloro-2-(1-isopentylcycloheptanecarbonylamino)phenyl] ester; 2,2-dimethylthiopropionic acid S-[4,5-dichloro-2-(1-isopentylcyclobutanecarbonylamino)phenyl] ester; 2,2-dimethylthiopropionic acid S-[2-(1-isopentylcyclohexanecarbonylamino)-4-nitrophenyl] ester; 2,2-dimethylthiopropionic acid S-[4-cyano-2-(1-isopentylcyclohexanecarbonylamino)phenyl] ester; 2,2-dimethylthiopropionic acid S-[4-chloro-2-(1-isopentylcyclohexanecarbonylamino)phenyl] ester; 2,2-dimethylthiopropionic acid S-[5-chloro-2-(1-isopentylcyclohexanecarbonylamino)phenyl] ester; 2,2-dimethylthiopropionic acid S-[4-fluoro-2-(1-isopentylcyclohexanecarbonylamino)phenyl] ester; 2,2-dimethylthiopropionic acid S-[4,5-difluoro-2-(1-isopentylcyclohexanecarbonylamino)phenyl] ester; 2,2-dimethylthiopropionic acid S-[5-fluoro-2-(1-isopentylcyclohexanecarbonylamino)phenyl] ester; bis-[4,5-dichloro-2-(1-isopentylcyclohexanecarbonylamino)phenyl] disulfide; 2-tetrahydrofurylmethyl 2-(1-isopentylcyclohexanecarbonylamino)phenyl disulfide; N- (2-mercaptophenyl)-1-ethylcyclohexanecarboxamide; N-(2-mercaptophenyl)-1-propylcyclohexanecarboxamide; N-(2-mercaptophenyl)-1-butylcyclohexanecarboxamide; N-(2-mercaptophenyl)-1-isobutylcyclohexanecarboxamide; cyclohexanethiocarboxylic acid S-[2-(1-isopentylcyclohexanecarbonylamino)phenyl] ester; thiobenzoic acid S-[2-(1-isopentylcyclohexanecarbonylamino)phenyl] ester; 5-carboxythiopentanoic acid S-[2-(1-isopentylcyclohexanecarbonylamino)phenyl] ester; thioacetic acid S-[2-(1-isopentylcyclohexanecarbonylamino)-4-methylphenyl] ester; bis-[2-[1-(2-ethylbutyl)cyclohexanecarbonylamino]phenyl] disulfide; N-(2-mercaptophenyl)-1-(2-ethylbutyl)cyclohexanecarboxamide; 2-methylthiopropionic acid S-[2-[1-(2-ethylbutyl)cyclohexanecarbonylamino]phenyl] ester; 2-methylthiopropionic acid S-[2-(1-isobutylcyclohexanecarbonylamino)phenyl] ester; 1-acetylpiperidine-4-thiocarboxylic acid S-[2-[1-(2-ethylbutyl)cyclohexanecarbonylamino]phenyl] ester; thioacetic acid S-[2-[1-(2-ethylbutyl)cyclohexanecarbonylamino]phenyl] ester; 2,2-dimethylthiopropionic acid S-[2-[1-(2-ethylbutyl)cyclohexanecarbonylamino]phenyl] ester; methoxythioacetic acid S-[2-[1-(2-ethylbutyl)cyclohexanecarbonylamino]phenyl] ester; 2-hydroxy-2-methylthiopropionic acid S-[2-[1-(2-ethylbutyl)cyclohexanecarbonylamino]phenyl] ester; 4-chlorophenoxythioacetic acid S-[2-[1-(2-ethylbutyl)cyclohexanecarbonylamino]phenyl] ester; 4-chlorophenoxythioacetic acid S-[2-(1-isobutylcyclohexanecarbonylamino)phenyl] ester; and 1-acetylpiperidine-4-thiocarboxylic acid S-[2-(1-isobutylcyclohexanecarbonylamino)phenyl] ester, a prodrug compound thereof, a pharmaceutically acceptable salt thereof, or a hydrate thereof or a solvate thereof.
[32] The inhibitor of [29] or [30], wherein the compound of the formula (VIII) is a compound selected from a group consisting of bis-[2-(pivaloylamino)phenyl] disulfide; bis-[2-(2-propylpentanoylamino)phenyl] disulfide; bis-[2-(1-methylcyclohexanecarbonylamino)phenyl] disulfide; bis-[2-(1-isopentylcyclopentanecarbonylamino)phenyl] disulfide; bis-[2-(1-isopentylcyclohexanecarbonylamino)phenyl] disulfide; N-(2-mercaptophenyl)-2,2-dimethylpropionamide; N-(2-mercaptophenyl)-1-isopentylcyclohexanecarboxamide; N-(2-mercaptophenyl)-1-methylcyclohexanecarboxamide; N-(2-mercaptophenyl)-1-isopentylcyclopentanecarboxamide; N-(2-mercaptophenyl)-1-isopropylcyclohexanecarboxamide; N-(4,5-dichloro-2-mercaptophenyl)-1-isopentylcyclohexanecarboxamide; N-(4,5-dichloro-2-mercaptophenyl)-1-isopentylcyclopentanecarboxamide; N-(2-mercapto-5-methylphenyl)-1-isopentylcyclohexanecarboxamide; N-(2-mercapto-4-methylphenyl)-1-isopentylcyclohexanecarboxamide; thioacetic acid S-[2-(1-isopentylcyclohexanecarbonylamino)phenyl] ester; 2,2-dimethylthiopropionic acid S-[2-(1-methylcyclohexanecarbonylamino)phenyl] ester; phenylthioacetic acid S-[2-(pivaloylamino)phenyl] ester; 2,2-dimethylthiopropionic acid S-[2-(1-isopentylcyclohexanecarbonylamino)phenyl] ester; 2-acetylamino-3-phenylthiopropionic acid S-[2-(1-isopentylcyclohexanecarbonylamino)phenyl] ester; 3-pyridinethiocarboxylic acid S-[2-(1-isopentylcyclohexanecarbonylamino)phenyl] ester; chlorothioacetic acid S-[2-(1-isopentylcyclohexanecarbonylamino)phenyl] ester; methoxythioacetic acid S-[2-(1-isopentylcyclohexanecarbonylamino)phenyl] ester; thiopropionic acid S-[2-(1-isopentylcyclohexanecarbonylamino)phenyl] ester; phenoxythioacetic acid S-[2-(1-isopentylcyclohexanecarbonylamino)phenyl] ester; 2-methylthiopropionic acid S-[2-(1-isopentylcyclohexanecarbonylamino)phenyl] ester; 4-chlorophenoxythioacetic acid S-[2-(1-isopentylcyclohexanecarbonylamino)phenyl] ester; cyclopropanethiocarboxylic acid S-[2-(1-isopentylcyclohexanecarbonylamino)phenyl] ester; 2-acetylamino-4-carbamoylthiobutyric acid S-[2-(1-isopentylcyclohexanecarbonylamino)phenyl] ester; 2-hydroxy-2-methylthiopropionic acid S-[2-(1-isopentylcyclohexanecarbonylamino)phenyl] ester; 2,2-dimethylthiopropionic acid S-[2-(1-isopentylcyclopentanecarbonylamino)phenyl] ester; thioacetic acid S-[2-(1-isopentylcyclopentanecarbonylamino)phenyl] ester; 2,2-dimethylthiopropionic acid S-[4,5-dichloro-2-(1-isopentylcyclohexanecarbonylamino)phenyl] ester; 2,2-dimethylthiopropionic acid S-[4,5-dichloro-2-(1-isopentylcyclopentanecarbonylamino)phenyl] ester; 2,2-dimethylthiopropionic acid S-[2-(1-isopentylcyclohexanecarbonylamino)-4-trifluoromethylphenyl] ester; thiocarbonic acid O-methyl ester S-[2-(1-isopentylcyclohexanecarbonylamino)phenyl] ester; dithiocarbonic acid S-[2-(1-methylcyclohexanecarbonylamino)phenyl] ester S-phenyl ester; N-phenylthiocarbamic acid S-[2-(1-isopentylcyclohexanecarbonylamino)phenyl] ester; 2,2-dimethylthiopropionic acid S-[2-(pivaloylamino)-4-trifluoromethylphenyl] ester; 2,2-dimethylthiopropionic acid S-[4,5-dichloro-2-(1-cyclopropylcyclohexanecarbonylamino)phenyl] ester; 2,2-dimethylthiopropionic acid S-[4,5-dichloro-2-(2-cyclohexylpropionylamino)phenyl] ester; 2,2-dimethylthiopropionic acid S-[4,5-dichloro-2-(1-pentylcyclohexanecarbonylamino)phenyl] ester; 2,2-dimethylthiopropionic acid S-[4,5-dichloro-2-(1-cyclopropylmethylcyclohexanecarbonylamino)phenyl] ester; 2,2-dimethylthiopropionic acid S-[4,5-dichloro-2-(1-cyclohexylmethylcyclohexanecarbonylamino)phenyl] ester; 2,2-dimethylthiopropionic acid S-[4,5-dichloro-2-(1-isopropylcyclohexanecarbonylamino)phenyl] ester; 2,2-dimethylthiopropionic acid S-[4,5-dichloro-2-(1-isopentylcycloheptanecarbonylamino)phenyl] ester; 2,2-dimethylthiopropionic acid S-[4,5-dichloro-2-(1-isopentylcyclobutanecarbonylamino)phenyl] ester; 2,2-dimethylthiopropionic acid S-[2-(1-isopentylcyclohexanecarbonylamino)-4-nitrophenyl] ester; 2,2-dimethylthiopropionic acid S-[4-cyano-2-(1-isopentylcyclohexanecarbonylamino)pheriyl] ester; 2,2-dimethylthiopropionic acid S-[4-chloro-2-(1-isopentylcyclohexanecarbonylamino)phenyl] ester; 2,2-dimethylthiopropionic acid S-[5-chloro-2-(1-isopentylcyclohexanecarbonylamino)phenyl] ester; 2,2-dimethylthiopropionic acid S-[4-fluoro-2-(1-isopentylcyclohexanecarbonylamino)phenyl] ester; 2,2-dimethylthiopropionic acid S-[4,5-difluoro-2-(1-isopentylcyclohexanecarbonylamino)phenyl] ester; 2,2-dimethylthiopropionic acid S-[5-fluoro-2-(1-isopentylcyclohexanecarbonylamino)phenyl] ester; bis-[4,5-dichloro-2-(1-isopentylcyclohexanecarbonylamino)phenyl] disulfide; 2-tetrahydrofurylmethyl 2-(1-isopentylcyclohexanecarbonylamino)phenyl disulfide; N-(2-mercaptophenyl)-1-ethylcyclohexanecarboxamide; N-(2-mercaptophenyl)-1-propylcyclohexanecarboxamide; N-(2-mercaptophenyl)-1-butylcyclohexanecarboxamide; N-(2-mercaptophenyl)-1-isobutylcyclohexanecarboxamide; cyclohexanethiocarboxylic acid S-[2-(1-isopentylcyclohexanecarbonylamino)phenyl] ester; thiobenzoic acid S-[2-(1-isopentylcyclohexanecarbonylamino)phenyl] ester; 5-carboxythiopentanoic acid S-[2-(1-isopentylcyclohexanecarbonylamino)phenyl] ester; thioacetic acid S-[2-(1-isopentylcyclohexanecarbonylamino)-4-methylphenyl] ester; bis-[2-[1-(2-ethylbutyl)cyclohexanecarbonylamino]phenyl] disulfide; N-(2-mercaptophenyl)-1-(2-ethylbutyl)cyclohexanecarboxamide; 2-methylthiopropionic acid S-[2-[1-(2-ethylbutyl)cyclohexanecarbonylamino]phenyl] ester; 2-methylthiopropionic acid S-[2-(1-isobutylcyclohexanecarbonylamino)phenyl] ester; 1-acetylpiperidine-4-thiocarboxylic acid S-[2-[1-(2-ethylbutyl)cyclohexanecarbonylamino]phenyl] ester; thioacetic acid S-[2-[1-(2-ethylbutyl)cyclohexanecarbonylamino]phenyl] ester; 2,2-dimethylthiopropionic acid S-[2-[1-(2-ethylbutyl)cyclohexanecarbonylamino]phenyl] ester; methoxythioacetic acid S-[2-[1-(2-ethylbutyl)cyclohexanecarbonylamino]phenyl] ester; 2-hydroxy-2-methylthiopropionic acid S-[2-[1-(2-ethylbutyl)cyclohexanecarbonylamino]phenyl] ester; 4-chlorophenoxythioacetic acid S-[2-[1-(2-ethylbutyl)cyclohexanecarbonylamino]phenyl] ester; 4-chlorophenoxythioacetic acid S-[2-(1-isobutylcyclohexanecarbonylamino)phenyl] ester; and 1-acetylpiperidine-4-thiocarboxylic acid S-[2-(1-isobutylcyclohexanecarbonylamino)phenyl] ester, a prodrug compound thereof, a pharmaceutically acceptable salt thereof, or a hydrate thereof or a solvate thereof.
[33] The method of [1], [3] or [6], wherein the compound having a CETP inhibitory activity is a dibenzylamine compound represented by the formula (IX) 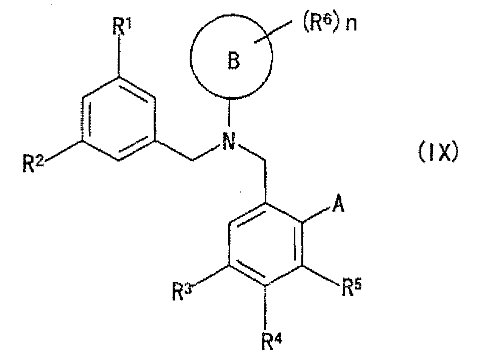 wherein
   - R¹ and R²: are the same or different and each is a halogen atom, a nitro group, a cyano group or a C₁₋₆ alkyl group optionally substituted by halogen atom;
   - R³, R⁴ and R⁵: are the same or different and each is a hydrogen atom, a halogen atom, a C₁₋₆ alkyl group optionally substituted by halogen atom, a C₁₋₆ alkylthio group optionally substituted by halogen atom or a C₁₋₆ alkoxy group optionally substituted by halogen atom, or R³ and R⁴ or R⁴ and R⁵ may form, together with a carbon atom they are bonded to, a homocyclic ring optionally having substituent(s) or a heterocyclic ring optionally having substituent(s);
   - A: is -N(R⁷)(R⁸) (wherein R⁷ and R⁸ are the same or different and each is a hydrogen atom, a C₁₋₆ alkyl group (wherein C₁₋₆ alkyl group is optionally substituted by phenyl group or -(CH₂)ₘ-COOR⁹ (wherein R⁹ is a hydrogen atom or a C₁₋₆ alkyl group and m is 0 or an integer of 1 to 5)) or a C₄₋₁₀ cycloalkylalkyl, group (wherein C₄₋₁₀ cycloalkylalkyl group is optionally substituted by 1 to 3 substituents from halogen atom, nitro group, amino group, hydroxyl group, cyano group, acyl group, C₁₋₆ alkoxy group, C₁₋₆ alkyl group (wherein C₁₋₆ alkyl group is optionally substituted by hydroxyl group, C₁₋₆ alkoxy group or phosphono group), -(CH₂)_{q}-CON(R²⁰)(R²¹) (wherein R²⁰ and R²¹ are the same or different and each is hydrogen atom or C₁₋₆ alkyl group and q is 0 or an integer of 1 to 5) or -(CH₂)ᵣ-COOR¹⁰ (wherein R¹⁰ is hydrogen atom or C₁₋₆ alkyl group and r is 0 or an integer of 1 to 5)), -C(R¹¹)(R¹²)(R¹³) (wherein R¹¹, R¹² and R¹³ are the same or different and each is a hydrogen atom, a C₁₋₆ alkyl group (wherein C₁₋₆ alkyl group is optionally substituted by phenyl group or -COOR⁹ (wherein R⁹ is as defined above)) or a C₄₋₁₀ cycloalkylalkyl, group (wherein C₉₋₁₀ cycloalkylalkyl group is optionally substituted by 1 to 3 substituents from halogen atom, nitro group, amino group, hydroxyl group, cyano group, acyl group, C₁₋₆ alkoxy group, C₁₋₆ alkyl group (wherein C₁₋₆ alkyl group is optionally substituted by hydroxyl group, C₁₋₆ alkoxy group or phosphono group),- (CH₂)_{q}-CON(R²⁰)(R²¹) (wherein R²⁰, R²¹ and q are as defined above) or -(CH₂)ᵣ-COOR¹⁰ (wherein R¹⁰ and r are as defined above))) or -O-C(R¹¹)(R¹²)(R¹³) (wherein R¹¹, R¹² and R¹³ are as defined above);
   - ring B: is an aryl group or a heterocyclic residue;
   - R⁶: is a hydrogen atom, a halogen atom, a nitro group, an amino group, a hydroxyl group, a cyano group, an acyl group, a C₁₋₆ alkoxy group, a C₂₋₆ alkenyl group or a C₁₋₆ alkyl group (wherein C₁₋₆ alkyl group is optionally substituted by hydroxyl group or -COOR¹⁴ (wherein R¹⁴ is a hydrogen atom or a C₁₋₆ alkyl group)); and
   - n: is an integer of 1 to 3
   or a prodrug thereof or a pharmaceutically acceptable salt thereof.
[34] The inhibitor of [2], [4] or [7], wherein the compound having a CETP inhibitory activity is a compound of the formula (IX) of [33] or a prodrug thereof, or a pharmaceutically acceptable salt thereof.
[35] The inhibitor of [5] or [8], which is a compound of the formula (IX) of [33] or a prodrug thereof, or a pharmaceutically acceptable salt thereof.
[36] The method of [33], wherein the compound of the formula (IX) is a compound selected from a group consisting of
   N-[3-(N'-cyclopentylmethyl-N'-ethylamino)-5,6,7,8-tetrahydronaphthalen-2-ylmethyl]-N-[3,5-bis(trifluoromethyl)benzyl]-(2-methyl-2H-tetrazol-5-yl)amine,
   3-([N-[3-(N'-cyclopentylmethyl-N'-ethylamino)-5,6,7,8-tetrahydronaphthalen-2-ylmethyl]-N-(2-methyl-2H-tetrazol-5-yl)amino]methyl)-5-trifluoromethylbenzonitrile,
   N-[3,5-bis(trifluoromethyl)benzyl]-N-[6-(N'-cyclopentylmethyl-N'-ethylamino)indan-5-ylmethyl]-(2-methyl-2H-tetrazol-5-yl)amine,
   N-[3,5-bis(trifluoromethyl)benzyl]-N-[6-(N'-cyclopentylmethyl-N'-ethylamino)indan-5-ylmethyl]-(2-methyl-2H-tetrazol-5-yl)amine hydrochloride,
   N-[6-(N'-cyclopentylmethyl-N'-ethylamino)indan-5-ylmethyl]-N-(2H-tetrazol-5-yl)-[3,5-bis(trifluoromethyl)benzyl]amine,
   N-[6-(N'-cyclopentylmethyl-N'-ethylamino)indan-5-ylmethyl]-N-[3,5-bis(trifluoromethyl)benzyl]-(pyrimidin-2-yl)amine hydrochloride,
   N-[6-(N'-cyclopentylmethyl-N'-ethylamino)indan-5-ylmethyl]-N-[3,5-bis(trifluoromethyl)benzyl]-(5-methyl-1H-pyrazol-3-yl)amine,
   5-{N-(6-{[N'-[3,5-bis(trifluoromethyl)benzyl]-N'-(5-methyl-[1,2,4]oxadiazol-3-yl)amino]methyl}indan-5-yl)-N-ethylamino}pentanoic acid hydrochloride,
   methyl trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylate,
   3-{[N-[6-(N'-cyclopentylmethyl-N'-ethylamino)indan-5-ylmethyl]-N-(2-methyl-2H-tetrazol-5-yl)amino]methyl}-5-trifluoromethylbenzonitrile,
   N-[3,5-bis(trifluoromethyl)benzyl]-N-[6-(N'-cyclopentylmethyl-N'-ethylamino)indan-5-ylmethyl]-(2-ethyl-2H-tetrazol-5-yl)amine,
   N-[3,5-bis(trifluoromethyl)benzyl]-N-[6-(N'-cyclopentylmethyl-N'-ethylamino)indan-5-ylmethyl]-(1-methyl-1H-[1,2,4]triazol-3-yl)amine,
   3-({N-6-(N'-cyclopentylmethyl-N'-ethylamino)indan-5-ylmethyl]-N-phenylamino}methyl)-5-trifluoromethylbenzonitrile,
   3-{[N-[6-(N'-cyclopentylmethyl-N'-ethylamino)indan-5-ylmethyl]-N-(4,5-dimethylthiazol-2-yl)amino]methyl}-5-trifluoromethylbenzonitrile,
   N-[3,5-bis(trifluoromethyl)benzyl]-N-[6-(N'-cyclopentylmethyl-N'-ethylamino)indan-5-ylmethyl]-(thiazol-2-y1)amine hydrochloride,
   3-({N-[6-(N'-cyclopentylmethyl-N'-ethylamino)indan-5-ylmethyl]-N-(thiazol-2-yl)amino}methyl)-5-trifluoromethylbenzonitrile hydrochloride,
   N-[3,5-bis(trifluoromethyl)benzyl]-N-[6-(N'-cyclopentylmethyl-N'-ethylamino)indan-5-ylmethyl]-(oxazol-2-yl)amine hydrochloride,
   N-[3,5-bis(trifluoromethyl)benzyl]-N-[6-(N'-cyclopentylmethyl-N'-ethylamino)indan-5-ylmethyl]-(5-methylthiazol-2-yl)amine hydrochloride,
   N-[3,5-bis(trifluoromethyl)benzyl]-N-[6-(N'-cyclopentylmethyl-N'-ethylamino)indan-5-ylmethyl]-(4-methylthiazol-2-yl)amine hydrochloride,
   N-[3,5-bis(trifluoromethyl)benzyl]-N-[6-(N'-cyclopentylmethyl-N'-ethylamino)indan-5-ylmethyl]-(4,5-dimethylthiazol-2-yl)amine hydrochloride,
   3-([N-[6-(N'-cyclopentylmethyl-N'-ethylamino)indan-5-ylmethyl]-N-(5-methylthiazol-2-yl)amino]methyl)-5-trifluoromethylbenzonitrile hydrochloride,
   3-{[N-[6-(N'-cyclopentylmethyl-N'-ethylamino)indan-5-ylmethyl]-N-(4-methylthiazol-2-yl)amino]methyl}-5-trifluoromethylbenzonitrile hydrochloride,
   N-[3,5-bis(trifluoromethyl)benzyl]-N-[6-(N'-cyclopentylmethyl-N'-ethylamino)indan-5-ylmethyl]-(4-methyloxazol-2-yl)amine hydrochloride,
   N-[3,5-bis(trifluoromethyl)benzyl]-N-[6-(N'-cyclopentylmethyl-N'-ethylamino)indan-5-ylmethyl]-(3-methylisothiazol-5-yl)amine hydrochloride,
   N-[3,5-bis(trifluoromethyl)benzyl]-N-[6-(N'-cyclopentylmethyl-N'-ethylamino)indan-5-ylmethyl]-(5-methylisoxazol-3-yl)amine hydrochloride,
   N-[3,5-bis(trifluoromethyl)benzyl]-N-[6-(N'-cyclopentylmethyl-N'-ethylamino)indan-5-ylmethyl]-(3-methylisoxazol-5-yl)amine hydrochloride,
   N-[3,5-bis(trifluoromethyl)benzyl]-N-[6-(N'-cyclopentylmethyl-N'-ethylamino)indan-5-ylmethyl]-(1-methyl-1H-pyrazol-3-yl)amine hydrochloride,
   N-[3,5-bis(trifluoromethyl)benzyl]-N-[6-(N'-cyclopentylmethyl-N'-ethylamino)indan-5-ylmethyl]-(1-methyl-1H-pyrazol-4-yl)amine hydrochloride,
   N-[3,5-bis(trifluoromethyl)benzy]-N-[6-(N'-cyclopentylmethyl-N'-ethylamino)indan-5-ylmethyl]-(5-methyl- [1,3,4]thiadiazol-2-yl)amine hydrochloride,
   N-[3,5-bis(trifluoromethyl)benzyl]-N-[6-(N'-cyclopentylmethyl-N'-ethylamino)indan-5-ylmethyl]-(5-methyl-[1,3,4]oxadiazol-2-yl)amine hydrochloride,
   N-[3,5-bis(trifluoromethyl)benzyl]-N-[6-(N'-cyclopentylmethyl-N'-ethylamino)indan-5-ylmethyl]-pyridin-3-ylamine hydrochloride,
   N-[3,5-bis(trifluoromethyl)benzyl]-N-[6-(N'-cyclopentylmethyl-N'-ethylamino)indan-5-ylmethyl]-pyridin-2-ylamine hydrochloride,
   N-[3,5-bis(trifluoromethyl)benzyl]-N-[2-(N'-cyclopentylmethyl-N'-ethylamino)-5-trifluoromethylbenzyl]-(2-methyl-2H-tetrazol-5-yl)amine hydrochloride,
   3-{[N-[2-(N'-cyclopentylmethyl-N'-ethylamino)-5-trifluoromethylbenzyl]-N-(2-methyl-2H-tetrazol-5-yl)amino]methyl}-5-trifluoromethylbenzonitrile hydrochloride,
   methyl 5-[N-(6-{[N-[3,5-bis(trifluoromethyl)benzyl]-N'-(2-methyl-2H-tetrazol-5-yl)amino]methyl}indan-5-yl)-N-ethylamino]pentanoate hydrochloride,
   methyl 5-[N-(6-{[N'-(3-cyano-5-trifluoromethylbenzyl)-N'-(2-methyl-2H-tetrazol-5-yl)amino]methyl}indan-5-ylmethyl)-N-ethylamino]pentanoate hydrochloride,
   methyl 5-[N-(6-{[N'-[3,5-bis(trifluoromethyl)benzyl]-N'-(1-methyl-1H-pyrazol-3-yl)amino]methyl}indan-5-yl)-N-ethylamino]pentanoate hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylamino]pentanoic acid hydrochloride,
   5-[N-(6-{[N'-(3-cyano-5-trifluoromethylbenzyl)-N'-(2-methyl-2H-tetrazol-5-yl)amino]methyl}indan-5-yl)-N-ethylamino]pentanoic acid hydrochloride,
   5-[N-(6-{[N'-[3,5-bis(trifluoromethyl)benzyl]-N'-(3-methylisoxazol-5-yl)amino]methyl}indan-5-yl)-N-ethylamino]pentanoic acid hydrochloride,
   5-[N-(6-{[N'-[3,5-bis(trifluoromethyl)benzyl]-N'-(1-methyl-1H-pyrazol-3-yl)amino]methyl}indan-5-yl)-N-ethylamino]pentanoic acid hydrochloride,
   methyl trans-4-{[N-(3-{[N'-[3,5-bis(trifluoromethyl) benzyl]-N'-(1-methyl-1H-pyrazol-3-yl)amino]methyl}-5,6,7,8-tetrahydronaphthalen-2-yl)-N-ethylamino]methyl}cyclohexanecarboxylate hydrochloride,
   methyl trans-4-{[N-(3-{[N'-(3-cyano-5-trifluoromethylbenzyl)-N'-(l-methyl-1H-pyrazol-3-yl)amino]methyl}-5,6,7,8-tetrahydronaphthalen-2-yl)-N-ethylamino]methyl}cyclohexanecarboxylate hydrochloride,
   trans-4-{[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]methyl}cyclohexanecarboxylic acid hydrochloride,
   N-[3,5-bis(trifluoromethyl)benzyl]-N-[6-(N'-cyclopentylmethyl-N'-ethylamino)indan-5-ylmethyl]-(5-methyl-[1,2,4]oxadiazol-3-yl)amine hydrochloride,
   methyl trans-4-{[N-(6-{[N'-[3,5-bis(trifluoromethyl)benzyl]-N'-(1-methyl-1H-pyrazol-3-yl)amino]methyl}indan-5-yl)-N-ethylamino]methyl)cyclohexanecarboxylate hydrochloride,
   trans-4-{[N-(6-{[N'-[3,5-bis(trifluoromethyl)benzyl]-N'-(1-methyl-1H-pyrazol-3-yl)amino]methyl}indan-5-yl)-N-ethylamino]methyl}cyclohexanecarboxylic acid hydrochloride,
   methyl trans-4-{[N-(6-{[N'-(3-cyano-5-trifluoromethylbenzyl)-N'-(1-methyl-1H-pyrazol-3-yl)amino]methyl}indan-5-yl)-N-ethylamino]methyl}cyclohexanecaxanecarboxylate,
   trans-4-{[N-(6-{[N'-(3-cyano-5-trifluoromethylbenzyl)-N'-1-methyl-1H-pyrazol-3-yl)amino]methyl}indan-5-yl)-N-ethylamino]methyl}cyclohexanecarboxylic acid hydrochloride,
   trans-4-{[N-(6-{[N'-[3,5-bis(trifluoromethyl)benzyl]-N'-(2-methyl-2H-tetrazol-5-yl)amino]methyl}indan-5-yl)-N-ethylamino]methyl}cyclohexanecarboxylic acid hydrochloride,
   trans-4-{[N-(6-"{[N'-(3-cyano-5-trifluoromethylbenzyl)-N'-(2-methyl-2H-tetrazol-5-yl)amino]methyl}indan-5-yl)-N-ethylamino]methyl}cyclohexanecarboxylic acid hydrochloride,
   trams-4-{[N-(6-{[N'-[3,5-bis(trifluoromethyl)benzyl]-N'-(3-methylisoxazol-5-yl)amino]methyl}indan-5-yl)-N-ethylamino]methyl}cyclohexanecarboxylic acid hydrochloride,
   trans-4-{[N-(6-{[N'-(3-cyano-5-trifluoromethylbenzyl)-N'-(3-methylisoxazol-5-yl)amino]methyl}indan-5-yl)-N-ethylamino]methyl}cyclohexanecarboxylic acid hydrochloride,
   methyl 5-[N-(6-{[N'-(3-cyano-5-trifluoromethylbenzyl)-N'-(1-methyl-1H-pyrazol-3-yl)amino]methyl}indan-5-yl)-N-ethylamino]pentanoate hydrochloride,
   5-[N-(6-{[N'-(3-cyano-5-trifluoromethylbenzyl)-N'-(3-methyl-isoxazol-5-yl)amino]methyl}indan-5-yl)-N-ethylamino]pentanoic acid hydrochloride,
   5-[N-(6-{[N'-(3-cyano-5-trifluoromethylbenzyl)-N'-(1-methyl-1H-pyrazol-3-yl)amino]methyl}indan-5-yl)-N-ethylamino]pentanoic acid hydrochloride,
   5-[N-(6-{[N'-[3,5-bis(trifluoromethyl)benzyl]-N'-(1-methyl-1H-[1,2,4]triazol-3-yl)amino]methyl}indan-5-yl)-N-ethylamino]pentanoic acid hydrochloride,
   trans-4-{[N-(6-{[N'-[3,5-bis(trifluoromethyl)benzyl]-N'-(1-methyl-1H-[1,2,4]triazol-3-yl)amino]methyl}indan-5-yl)-N-ethylamino]methyl}cyclohexanecarboxylic acid hydrochloride,
   trans-4-{[N-(6-{[N'-(3-cyano-5-trifluoromethylbenzyl)-N'-(1-methyl-1H-[1,2,4]triazol-3-yl)amino]methyl}indan-5-yl)-N-ethylamino]methyl}cyclohexanecarboxylic acid,
   N-[3,5-bis(trifluoromethyl)benzyl]-N-[6-(N'-cyclopentylmethyl-N'-ethylamino)indan-5-ylmethyl]-(3-methyl-[1,2,4]thiadiazol-5-yl)amine,
   5-[N-(6-{[N'-(3-cyano-5-trifluoromethylbenzyl)-N'-(1-methyl-1H-[1,2,4]triazol-3-yl)amino]methyl}indan-5-yl)-N-ethylamino]pentanoic acid hydrochloride,
   methyl 5-[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]pentanoate hydrochloride,
   methyl 5-[N-(3-{[N'-(3-cyano-5-trifluoromethylbenzyl)-N'-(1-methyl-1H-pyrazol-3-yl)amino]methyl}-5,6,7,8-tetrahydronaphthale-2-yl)-N-ethylamino]pentanoate hydrochloride,
   5-[N-{3-{[N'-[3,5-bis(trifluoromethyl)benzyl]-N'-(1-methyl-1H-pyrazol-3-yl)amino]methyl}-5,6,7,8-tetrahydronaphthalen-2-yl}-N-ethylamino]pentanoic acid hydrochloride,
   5-[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]pentanoic acid hydrochloride,
   trans-4-{[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(2-methyl-2H-tetrazol-5-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-[3,5-bis(trifluoromethyl)benzyl]-N'-(1-methyl-1H-[1,2,4]triazol-3-yl)aminolmethyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]methyl}cyclohexanecarboxylic acid,
   trans-4-{[N-(3-{[N'-[3,5-bis(trifluoromethyl)benzyl]-N'-(3-methylisoxazol-5-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(3-methylisoxazol-5-yl)amino]methyl}-5,6,7,8-tetrahydronaphthalen-2-yl)-N-ethylamino]methyl}cyclohexanecarboxylic acid hydrochloride,
   2-(5-{N-[3,5-bis(trifluoromethyl)benzyl]-N-[6-(N'-cyclopentylmethyl-N'-ethylamino)indan-5-ylmethyl]amino}tetrazol-2-yl)ethanol hydrochloride,
   methyl 5-[N-(3-{[N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]pentanoate hydrochloride,
   methyl 5-[N-(3-{([N'-(3-cyano-5-trifluoromethylbenzyl)-N'-(1-methyl-1H-[1,2,4]triazo1-3-yl)amino]methyl)-5,6,7,8-tetrahydronaphthalen-2-yl)-N-ethylamino]pentanoate hydrochloride,
   5-[N-(3-{[N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]pentanoic acid hydrochloride,
   5-[N-(3-{[N'-[3,5-bis(trifluoromethyl)benzyl]-N'-(3-methylisoxazol-5-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(3-methylisoxazol-5-yl)amino]methyl}-5,6,7,8-tetrahydronaphthalen-2-yl)-N-ethylamino]pentanoic acid hydrochloride,
   5-[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(2-methyl-2H-tetrazol-5-yl)amino]methyl}-5,6,7,8-tetrahydronaphthalen-2-yl)-N-ethylamino]pentanoic acid hydrochloride,
   trans-4-{[N-(6-{[N'-[3,5-bis(trifluoromethyl)benzyl]-N'-(5-methyl-[1,2,4]oxadiazol-3-yl)amino]methyl}indan-5-yl)-N-ethylamino]methyl}cyclohexanecarboxylic acid,
   5-[N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]pentanoic acid hydrochloride,
   5-[N-(2-{[N'-[3,5-bis(trifluoromethyl)benzyl]-N'-(1-methyl-1H-pyrazol-3-yl)amino]methyl}-4-trifluoromethylphenyl)-N-ethylamino]pentanoic acid hydrochloride,
   5-[N-(2-{[N'-(3-cyano-5-trifluoromethylbenzyl)-N'-(1-methyl-1H-pyrazol-3-yl)amino]methyl}-4-trifluoromethylphenyl)-N-ethylamino]pentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]pentanoic acid hydrochloride,
   5-[N-(6-{[N'-[3,5-bis(trifluoromethyl)benzyl]-N'-(2-ethyl-2H-tetrazol-5-yl)amino]methyl}indan-5-yl)-N-ethylamino]pentanoic acid hydrochloride,
   5-{N-[6-({N'-[3,5-bis(trifluoromethyl)benzyl]-N'-[2-(2-hydroxyethyl)-2H-tetrazol-5-yl]amino}methyl)indan-5-yl]-N-ethylamino}pentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]pentanoic acid hydrochloride,
   5-[N-(6-([N'-[3,5-bis(trifluoromethyl)benzyl]-N'-(2-methyl-2H-tetrazol-5-yl)amino]methyl)indan-5-yl)-N-ethylaminol-2,2-dimethylpentanoic acid hydrochloride,
   6-[N-(6-{[N'-(3,5-bis(trifluoromethyl)benzyl]-N'-(2-methyl-2H-tetrazol-5-yl)amino]methyl}indan-5-yl)-N-ethylamino]-hexanoic acid hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylamino]-3,3-dimethylpentanoic acid hydrochloride,
   trans-4-{[N-(6-{[N'-[3,5-bis(trifluoromethyl)benzyl]-N'-(2-ethyl-2H-tetrazol-5-yl)amino]methyl}indan-5-yl)-N-ethylamino]methyl}cyclohexanecarboxylic acid hydrochloride, (1-{2-[N-(6-([N'-[3,5-bis(trifluoromethyl)benzyl]-N'-(2-methyl-2H-tetrazol-5-yl)amino]methyl)indan-5-yl)-N-ethylamino]ethyl}cyclopentyl)acetic acid hydrochloride,
   trans-4-({N-[6-({N'-[3,5-bis(trifluoromethyl)benzyl]-N'-[2-(2-hydroxyethyl)-2H-tetrazol-5-yl]amino}methyl)indan-5-yl]-N-ethylamino}methyl)cyclohexanecarboxylic acid,
   trans-4-{[N-(2-{[N'-[3,5-bis(trifluoromethyl)benzyl]-N'-(2-methyl-2H-tetrazol-5-yl)amino]methyl}-4-trifluoromethylphenyl)-N-ethylamino]methyl}cyclohexanecarboxylic acid hydrochloride, (1-{2-[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]ethyl}cyclopentyl)acetic acid,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-(3-{[N'-[3,5-bis(trifluoromethyl)benzyl]-N'-(2-ethyl-2H-tetrazol-5-yl)amino]methyl}-5,6,7,8-tetrahydronaphthalen-2-yl)-N-ethylamino]methyl}cyclohexanecarboxylic acid hydrochloride,
   trans-4-({N-[3-({N'-[3,5-bis(trifluoromethyl)benzyl]-N'-[2-(2-hydroxyethyl)-2H-tetrazol-5-yl]amino}methyl)-5,6,7,8-tetrahydronaphthalen-2-yl]-N-ethylamino}methyl)cyclohexanecarboxylic acid hydrochloride,
   1-{3-[N-(6-{[N'-[3,5-bis(trifluoromethyl)benzyl]-N'-(2-methyl-2H-tetrazol-5-yl)amino]methyl}indan-5-yl)-N-ethylamino]propyl}cyclohexanecarboxylic acid hydrochloride,
   5-[N-(6-{[N'-[3,5-bis(trifluoromethyl)benzyl]-N'-(1-methyl-1H-pyrazol-3-yl)amino}methyl)indan-5-yl)-N-ethylamino]-3,3-dimethylpentanoic acid,
   5-[N-(6-{[N'-[3,5-bis(trifluoromethyl)benzyl]-N'-(5-methyl-[1,2,4]oxadiazol-3-yl)amino]methyl}indan-5-yl)-N-ethylamino]-3,3-dimethylpentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]-3,3-dimethylpentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]-3,3-dimethylpentanoic acid hydrochloride,
   trans-4-{ [N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]methyl}cyclohexanecarboxylic acid hydrochloride,
   5-[N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]-3,3-dimethylpentanoic acid hydrochloride,
   5-[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]-3,3-dimethylpentanoic acid hydrochloride,
   trans-4-{[N-(2-([N'-[3,5-bis(trifluoromethyl)benzyl]-N'-(5-methyl-[1,2,4]oxadiazol-3-yl)aminolmethyl}-4-trifluoromethoxyphenyl)-N-ethylamino]methyl}cyclohexanecarboxylic acid hydrochloride,
   6-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]hexanoic acid hydrochloride,
   trans-(4-{[N-(6-{[N'-[3,5-bis(trifluoromethyl)benzyl]-N'-(2-methyl-2H-tetrazol-5-yl)amino]methyl}indan-5-yl)-N-ethylamino]methyl}cyclohexyl)acetic acid hydrochloride,
   6-[N-(6-{[N'-[3,5-bis(trifluoromethyl)benzyl]-N'-(2-methyl-2H-tetrazol-5-yl)amino]methyl}indan-5-yl)-N-ethylamino]-4,4-dimethylhexanoic acid hydrochloride,
   6-[N-(6-{[N'-[3,5-bis(trifluoromethyl)benzyl]-N'-(2-methyl-2H-tetrazol-5-yl)amino]methyl}indan-5-yl)-N-ethylamino]-3,3-dimethylhexanoic acid hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylamino]-4,4-dimethylpentanoic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylarmino]methyl}cyclohexyl)acetic acid hydrochloride,
   6-[N-(2-{[N,-[3,5-bis(trifluoromethyl)benzyl]-N'-(2-methyl-2H-tetrazol-5-yl)amino]methyl}-4-trifluoromethoxyphenyl)-N-ethylamino]-4,4-dimethylhexanoic acid hydrochloride,
   6-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]-4,4-dimethylhexanoic acid hydrochloride,
   trans-(4-{[N-(2-{[N'-[3,5-bis(trifliaoromethyl)benzyl]-N'-(2-methyl-2H-tetrazol-5-yl)amino]methyl}-4-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)methanol hydrochloride,
   6-[N-(6-{[N'-[3,5-bis(trifluoromethyl)benzyl]-N'-(2-methyl-2H-tetrazol-5-yl)amino]methyl}indan-5-yl)-N-ethylaminol-5,5-dimethylhexanoic acid hydrochloride,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-propylamino]methyl}cyclohexanecarboxylic acid hydrochloride,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-isobutylamino]methyl}cyclohexanecarboxylic acid hydrochloride,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid amide,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid methylamide,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid dimethylamide,
   trans-4-{[N-(2-{[N'-[3,5-bis(trifluoromethyl)benzyl]-N'-(4-chlorophenyl)amino]methyl}-4-trifluoromethoxyphenyl)-N-ethylamino]methyl}cyclohexanecarboxylic acid hydrochloride,
   trans-4-{[N-(2-{[N'-[3,5-bis(trifluoromethyl)benzyl]-N'-(p-tolyl)amino]methyl}-4-trifluoromethoxyphenyl)-N-ethylamino]methyl}cyclohexanecarboxylic acid hydrochloride,
   trans-4-{[N-(2-{[N'-[3,5-bis(trifluoromethyl)benzyl]-N'-(m-tolyl)amino]methyl}-4-trifluoromethoxyphenyl)"N-ethylamino]methyl}cyclohexanecarboxylic acid hydrochloride,
   trans-4-{[N-(2-{[N'-(3,5-dichlorobenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-ethyl-N-(2-{[N'-(2-methyl-2H-tetrazol-5-yl)-N'-(3-methyl-5-trifluoromethylbenzyl)amino]methyl}-4-trifluoromethoxyphenyl)amino]methyl}cyclohexanecarboxylic acid hydrochloride,
   trans-4-{[N-(2-{[N'-(3-chloro-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-ethyl-N-(2-{[N'-(2-methyl-2H-tetrazol-5-yl)-N'-(3-nitro-5-trifluoromethylbenzyl)amino]methyl}-4-trifluoromethoxyphenyl)amino]methyl}cyclohexanecarboxylic acid hydrochloride,
   trans-(4-{[N-(6-{[N'-[3,5-bis(trifluoromethyl)benzyl]-N'-(2-methyl-2H-tetrazol-5-yl)amino]methyl}-2,2-difluorobenzo[1,3]dioxol-5-yl)-N-ethylamino]methyl}cyclohexyl)methanol hydrochloride,
   trans-(4-{[N"(6-{[N'-[3,5-bis(trifluoromethyl)benzyl]-N'-(2-methyl-2H-tetrazol-5-yl)amino]methyl}-2,2-difluorobenzo[1,3]dioxol-5-yl)-N-ethylamino]methyl}cyclohexyl)acetic acid hydrochloride,
   trans-3-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)propionic acid hydrochloride,
   trans-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)methanol hydrochloride,
   trans-(4-{[N-(7-{[N'-[3,5-bis(trifluoromethyl)benzyl]-N'-(2-methyl-2H-tetrazo1-5-yl)amino]methyl}-2,2,3,3-tetrafluoro-2,3-dihydrobenzo[1,4]dioxin-6-yl)-N-ethylaminojmethyl}cyclohexyl)acetic acid hydrochloride,
   trans-2-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)acetamide hydrochloride,
   trans-N-[3,5-bis(trifluoromethyl)benzyl]-N-{2-[N'-ethyl-N'-(4-(methoxymethyl)cyclohexylmethyl)amino]-5-trifluoromethoxybenzyl}-(2-methyl-2H-tetrazol-5-yl)amine hydrochloride,
   trans-2-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)ethanol hydrochloride,
   trans-(4-{[N-(2-{[N'-[3,5-bis(tri-fluoromethyl)benzyl]-N'-(2-methyl-2H-tetrazol-5-yl)amino]methyl}-4-methyl-5-trifluoromethylphenyl)-N-ethylamino]methyl}cyclohexyl)methanol hydrochloride,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexylmethyl)phosphonic acid,
   trans-4-{[N-(2-{[N'-(3-bromo-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-(4-{[N-(2-{[N'-[3,5-bis(trifluoromethyl)benzyl]-N'-(2-methyl-2H-tetrazol-5-yl)amino]methyl}-4-bromophenyl)-N-ethylamino]methyl}cyclohexyl)methanol hydrochloride,
   trans-(4-{[N-(2-{[N'-[3,5-bis(trifluoromethyl)benzyl]-N'-(2-methyl-2H-tetrazol-5-yl)amino]methyl}-4-bromophenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-chloro-5-ethylphenyl)-N-ethylamino]methyl}cyclohexyl)methanol hydrochloride,
   trans-4-{[N-(2-{[N'-[3,5-bis(trifluoromethyl)benzyl]-N'-(4-methoxyphenyl)amino]methyl}-4-trifluoromethoxyphenyl)-N-ethylamino]methyl}cyclohexanecarboxylic acid hydrochloride,
   trans-(4-{[N-(2-{[N'-[3,5-bis(trifluoromethyl)benzyl]-N'-(2-methyl-2H-tetrazol-5-yl)amino]methyl}-5-methoxy-4-methylphenyl)-N-ethylamino]methyl}cyclohexyl)methanol hydrochloride,
   trans-(4-{[N-(2-{[N'-[3,5-bis(trifluoromethyl)benzyl]-N'-(2-methyl-2H-tetrazol-5-yl)amino]methyl}-4,5-dimethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethylthiophenyl)-N-ethylaminolmethyl}cyclohexyl)acetic acid hydrochloride, trans-(4-{[N-(2-{[N'-[3,5-bis(trifluoromethyl)benzyl]-N'-(2-methyl-2H-tetrazol-5-yl)amino]methyl}-4-chloro-5-ethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethylphenyl)-N-propylamino]methyl}cyclohexyl)acetic acid hydrochloride,
   trans-(4-{[N-(2-{[N'-[3,5-bis(triflucromethyl)benzyl]-N'-(2-methyl-2H-tetrazol-5-yl)amino]methyl}-5-methoxy-4-methylphenyl)-N-ethylamino]methyl}cyclohexyl)acetic acid hydrochloride,
   trans-4-({N-[2-{[N'-[3,5-bis(trifluoromethyl)benzyl]-N'-(2-methyl-2H-tetrazol-5-yl)amino]methyl}-4-(2,2,2-trifluoroethyl)phenyl]-N-ethylamino}methyl)cyclohexanecarboxylic acid hydrochloride,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-propylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-propylamino]methyl}cyclohexyl)acetic acid hydrochloride,
   trans-4-{[N-(2-{[N'-[3,5-bis(trifluoromethyl)benzyl]-N'-(4-ethylphenyl)amino]methyl}-4-trifluoromethoxyphenyl)-N-ethylamino]methyl}cyclohexanecarboxylic acid hydrochloride,
   trans-4-{[N-(2-{[N'-(3-cyano-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-(2-{[N'-[3,5-bis(trifluoromethyl)benzyl]-N'-(4-isopropenylphenyl)amino]methyl}-4-trifluoromethoxyphenyl)-N-ethylamino]methyl}cyclohexanecarboxylic acid,
   trans-(4-{[N-(2-{[N'-[3,5-bis(trifluoromethyl)benzyl]-N'-(p-tolyl)amino]methyl}-4-trifluoromethoxyphenyl)-N-ethylamino]methyl}cyclohexyl)acetic acid dihydrochloride,
   trans-(4-{[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]methyl}cyclohexyl)acetic acid hydrochloride,
   trans-(4-{[N-(2-{[N'-[3,5-bis(trifluoromethyl)benzyl]-N'-(5-methyl-[1,2,4]oxadiazol-3-yl)amino]methyl}-5-methyl-4-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(5-methyl-[1,2,4]oxadiazol-3-yl)amino]methyl}-5-methyl-4-trifluoromethylphenyl)-N-propylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-propylamino]methyl}cyclohexyl)acetic acid,
   trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)acetic acid,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate,
   ethyl trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetate, and
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid, a prodrug thereof or a pharmaceutically acceptable salt thereof.
[37] The inhibitor of [34] or [35], wherein the compound of the formula (IX) is a compound selected from a group consisting of
   N-[3-(N'-cyclopentylmethyl-N'-ethylamino)-5,6,7,8-tetrahydronaphthalen-2-ylmethyl]-N-[3,5-bis(trifluoromethyl)benzyl]-(2-methyl-2H-tetrazol-5-yl)amine,
   3-{[N-[3-(N'-cyclopentylmethyl-N'-ethylamino)-5,6,7,8-tetrahydronaphthalen-2-ylmethyl]-N-(2-methyl-2H-tetrazol-5-yl)amino]methyl}-5-trifluoromethylbenzonitrile,
   N-[3,5-bis(trifluoromethyl)benzyl]-N-[6-(N'-cyclopentylmethyl-N'-ethylamino)indan-5-ylmethyl]-(2-methyl-2H-tetrazol-5-yl)amine,
   N-[3,5-bis(trifluoromethyl)benzyl]-N-[6-(N'-cyclopentylmethyl-N'-ethylamino)indan-5-ylmethyl]-(2-methyl-2H-tetrazol-5-yl)amine hydrochloride,
   N-[6-(N'-cyclopentylmethyl-N'-ethylamino)indan-5-ylmethyl]-N-(2H-tetrazol-5-yl)-[3,5-bis(trifluoromethyl)benzyl]amine,
   N-[6-(N'-cyclopentylmethyl-N'-ethylamino)indan-5-ylmethyl]-N-[3,5-bis(trifluoromethyl)benzyl]-(pyrimidin-2-yl)amine hydrochloride,
   N-[6-(N'-cyclopentylmethyl-N'-ethylamino)indan-5-ylmethyl]-N-[3,5-bis(trifluoromethyl)benzyl]-(5-methyl-1H-pyrazol-3-yl)amine,
   5-{N-(6-{[N'-[3,5-bis(trifluoromethyl)benzyl]-N'-(5-methyl-[1,2,4]oxadiazol-3-yl)amino]methyl}indan-5-yl)-N-ethylamino}pentanoic acid hydrochloride,
   methyl trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylate,
   3-{[N-[6-(N'-cyclopentylmethyl-N'-ethylamino)indan-5-ylmethyl]-N-(2-methyl-2H-tetrazol-5-yl)amino]methyl}-5-trifluoromethylbenzonitrile,
   N-[3,5-bis(trifluoromethyl)benzyl]-N-[6-(N'-cyclopentylmethyl-N'-ethylamino)indan-5-ylmethyl]-(2-ethyl-2H-tetrazol-5-yl)amine,
   N-[3,5-bis(trifluoromethyl)benzyl]-N-[6-(N'-cyclopentylmethyl-N'-ethylamino)indan-5-ylmethyl]-(1-methyl-1H-[1,2,4]triazol-3-yl)amine,
   3-({N-[6-(N'-cyclopentylmethyl-N'-ethylamino)indan-5-ylmethyl]-N-phenylamino}methyl)-5-trifluoromethylbenzonitrile,
   3-{[N-[6-(N'-cyclopentylmethyl-N'-ethylamino)indan-5-ylmethyl]-N-(4,5-dimethylthiazol-2-yl)amino]methyl}-5-trifluoromethylbenzonitrile,
   N-[3,5-bis(trifluoromethyl)benzyl]-N-[6-(N'-cyclopentylmethyl-N'-ethylamino)indan-5-ylmethyl]-(thiazol-2-yl)amine hydrochloride,
   3-({N-[6-(N'-cyclopentylmethyl-N'-ethylamino)indan-5-ylmethyl]-N-(thiazol-2-yl)amino}methyl)-5-trifluoromethylbenzonitrile hydrochloride,
   N-[3,5-bis(trifluoromethyl)benzyl]-N-[6-(N'-cyclopentylmethyl-N'-ethylamino)indan-5-ylmethyl]-(oxazol-2-yl)amine hydrochloride,
   N-[3,5-bis(trifluoromethyl)benzyl]-N-[6-(N'-cyclopentylmethyl-N'-ethylamino)indan-5-ylmethyl]-(5-methylthiazol-2-yl)amine hydrochloride,
   N-[3,5-bis(trifluoromethyl)benzyl]-N-[6-(N'-cyclopentylmethyl-N'-ethylamino)indan-5-ylmethyl]-(4-methylthiazol-2-yl)amine hydrochloride,
   N-[3,5-bis(trifluoromethyl)benzyl]-N-[6-(N'-cyclopentylmethyl-N'-ethylamino)indan-5-ylmethyl]-(4,5-dimethylthiazol-2-yl)amine hydrochloride,
   3-{[N-[6-(N'-cyclopentylmethyl-N'-ethylamino)indan-5-ylmethyl]-N-(5-methylthiazol-2-yl)amino]methyl}-5-trifluoromethylbenzonitrile hydrochloride,
   3-{[N-[6-(N'-cyclopentylmethyl-N'-ethylamino)indan-5-ylmethyl]-N-(4-methylthiazol-2-yl)amino]methyl}-5-trifluoromethylbenzonitrile hydrochloride,
   N-[3,5-bis(trifluoromethyl)benzyl]-N-[6-(N'-cyclopentylmethyl-N'-ethylamino)indan-5-ylmethyl]-(4-methyloxazol-2-yl)amine hydrochloride,
   N-[3,5-bis(trifluoromethyl)benzyl]-N-[6-(N'-cyclopentylmethyl-N'-ethylamino)indan-5-ylmethyl]-(3-methylisothiazol-5-yl)amine hydrochloride,
   N-[3,5-bis(trifluoromethyl)benzyl]-N-[6-(N'-cyclopentylmethyl-N'-ethylamino)indan-5-ylmethyl]-(5-methylisoxazol-3-yl)amine hydrochloride,
   N-[3,5-bis(trifluoromethyl)benzyl]-N-[6-(N'-cyclopentylmethyl-N'-ethylamino)indan-5-ylmethyl]-(3-methylisoxazol-5-yl)amine hydrochloride,
   N-[3,5-bis(trifluoromethyl)benzyl]-N-[6-(N'-cyclopentylmethyl-N'-ethylamino)indan-5-ylmethyl]-(1-methyl-1H-pyrazol-3-yl)amine hydrochloride,
   N-[3,5-bis(trifluoromethyl)benzyl]-N-[6-(N'-cyclopentylmethyl-N'-ethylamino)indan-5-ylmethyl]-(1-methyl-1H-pyrazol-4-yl)amine hydrochloride,
   N-[3,5-bis(trifluoromethyl)benzyl]-N-[6-(N'-cyclopentylmethyl-N'-ethylamino)indan-5-ylmethyl-(5-methyl-[1,3,4]thiadiazol-2-yl)amine hydrochloride,
   N-[3,5-bis(trifluoromethyl)benzyl]-N-[6-(N'-cyclopentylmethyl-N'-ethylamino)indan-5-ylmethyl]-(5-methyl-[1,3,4]oxadiazol-2-yl)amine hydrochloride,
   N-[3,5-bis(trifluoromethyl)benzyl]-N-[6-(N'-cyclopentylmethyl-N'-ethylamino)indan-5-ylmethyl]-pyridin-3-ylamine hydrochloride,
   N-[3,5-bis(trifluoromethyl)benzyl]-N-[6-(N'-cyclopentylmethyl-N'-ethylamino)indan-5-ylmethyl]-pyridin-2-ylamine hydrochloride,
   N-[3,5-bis(trifluoromethyl)benzyl]-N-[2-(N'-cyclopentylmethyl-N'-ethylamino)-5-trifluoromethylbenzyl]-(2-methyl-2H-tetrazol-5-yl)amine hydrochloride,
   3-{[N-[2-(N'-cyclopentylmethyl-N'-ethylamino)-5-trifluoromethylbenzyl]-N-(2-methyl-2H-tetrazol-5-yl)amino]methyl}-5-trifluoromethylbenzonitrile hydrochloride,
   methyl 5-[N-(6-{[N'-[3,5-bis(trifluoromethyl)benzyl]-N'-(2-methyl-2H-tetrazol-5-yl)amino]methyl}indan-5-yl)-N-ethylamino]pentanoate hydrochloride,
   methyl 5-[N-(6-{[N'-(3-cyano-5-trifluoromethylbenzyl)-N'-(2-methyl-2H-tetrazol-5-yl)amino]methyl}indan-5-ylmethyl)-N-ethylamino]pentanoate hydrochloride,
   methyl 5-[N-(6-{[N'-[3,5-bis(trifluoromethyl)benzyl]-N'-(1-methyl-1H-pyrazol-3-yl)amino]methyl}indan-5-yl)-N-ethylamino]pentanoate hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylamino]pentanoic acid hydrochloride,
   5-[N-(6-{[N'-(3-cyano-5-trifluoromethylbenzyl)-N'-(2-methyl-2H-tetrazol-5-yl)amino]methyl}indan-5-yl)-N-ethylamino]pentanoic acid hydrochloride,
   5-[N-(6-{[N'-[3,5-bis(trifluoromethyl)benzyl]-N'-(3-methylisoxazol-5-yl)amino]methyl}indan-5-yl)-N-ethylamino]pentanoic acid hydrochloride,
   5-[N-(6-{[N'-[3,5-bis(trifluoromethyl)benzyl]-N'-(1-methyl-1H-pyrazol-3-yl)amino]methyl}indan-5-yl)-N-ethylaminolpentanoic acid hydrochloride,
   methyl trans-4-{[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]methyl}cyclohexanecarboxylate hydrochloride,
   methyl trans-4-{[N-(3-{[N'-(3-cyano-5-trifluoromethylbenzyl)-N'-(1-methyl-1H-pyrasol-3-yl)amino]methyl}-5,6,7,8-tetrahydronaphthalen-2-yl)-N-ethylamino]methyl}cyclohexanecarboxylate hydrochloride,
   trans-4-{[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]methyl}cyclohexanecarboxylic acid hydrochloride,
   N-[3,5-bis(trifluoromethyl)benzyl]-N-[6-(N'-cyclopentylmethyl-N'-ethylamino)indan-5-ylmethyl]-(5-methyl-[1,2,4]oxadiazol-3-yl)amine hydrochloride,
   methyl trans-4-{[N-(6-{[N'-[3,5-bis(trifluoromethyl)benzyl]-N'-(1-methyl-1H-pyrazol-3-yl)amino]methyl}indan-5-yl)-N-ethylamino]methyl}cyclohexanecarboxylate hydrochloride,
   trans-4-{[N-(6-{[N'-[3,5-bis(trifluoromethyl)benzyl]-N'-(1-methyl-1H-pyrazol-3-yl)amino]methyl}indan-5-yl)-N-ethylamino]methyl}cyclohexanecarboxylic acid hydrochloride,
   methyl trans-4-{[N-(6-{[N'-(3-cyano-5-trifluoromethylbenzyl)-N'-(1-methyl-1H-pyrazol-3-yl)amino]methyl}indan-5-yl)-N-ethylamino]methyl}cyclohexanecarboxylate,
   trans-4-{[N-(6-{[N'-(3-cyano-5-trifluoromethylbenzyl)-N'-(1-methyl-1H-pyrazol-3-yl)amino]methyl}indan-5-yl)-N-ethylamino]methyl}cyclohexanecarboxylic acid hydrochloride,
   trans-4-{[N-(6-{[N'-[3,5-bis(trifluoromethyl)benzyl]-N'-(2-methyl-2H-tetrazol-5-yl)amino]methyl}indan-5-yl)-N-ethylamino]methyl}cyclohexanecarboxylic acid hydrochloride,
   trans-4-{[N-(6-{[N'-(3-cyano-5-trifluoromethylbenzyl)-N'-(2-methyl-2H-tetrazol-5-yl)amino]methyl}indan-5-yl)-N-ethylamino]methyl}cyclohexanecarboxylic acid hydrochloride,
   trans-4-{[N-(6-{[N'-[3,5-bis(trifluoromethyl)benzyl]-N'-(3-methylisoxazol-5-yl)amino]methyl}indan-5-yl)-N-ethylamino]methyl}cyclohexanecarboxylic acid hydrochloride,
   trans-4-{[N-(6-{[N'-(3-cyano-5-trifluoromethylbenzyl)-N'-(3-methylisoxazol-5-yl)amino]methyl}indan-5-yl)-N-ethylamino]methyl}cyclohexanecarboxylic acid hydrochloride,
   methyl 5-[N-(6-{[N'-(3-cyano-5-trifluoromethylbenzyl)-N'-(1-methyl-1H-pyrazol-3-yl)amino]methyl}indan-5-yl)-N-ethylamino]pentanoate hydrochloride,
   5-[N-(6-{[N'-(3-cyano-5-trifluoromethylbenzyl)-N'-(3-methyl-isoxazol-5-yl)amino]methyl}indan-5-yl)-N-ethylamino]pentanoic acid hydrochloride,
   5-[N-(6-{[N'-(3-cyano-5-trifluoromethylbenzyl)-N'-(1-methyl-1H-pyrazol-3-yl)amino]methyl}indan-5-yl)-N-ethylamino]pentanoic acid hydrochloride,
   5-[N-(6-{[N'-[3,5-bis(trifluoromethyl)benzyl]-N'-(1-methyl-1H-[1,2,4]triazol-3-yl)amino]methyl}indan-5-yl)-N-ethylamino]pentanoic acid hydrochloride,
   trans-4-{[N-(6-{[N'-[3,5-bis(trifluoromethyl)benzyl]-N'-(1-methyl-1H-[1,2,4]triazol-3-yl)amino]methyl}indan-5-yl)-N-ethylamino]methyl}cyclohexanecarboxylic acid hydrochloride,
   trans-4-{[N-(6-{[N'-(3-cyano-5-trifluoromethylbenzyl)-N'-(1-methyl-1H-[1,2,4]triazol-3-yl)amino]methyl}indan-5-yl)-N-ethylamino]methyl}cyclohexanecarboxylic acid,
   N-[3,5-bis(trifluoromethyl)benzyl]-N-[6-(N'-cyclopentylmethyl-N'-ethylamino)indan-5-ylmethyl]-(3-methyl-[1,2,4]thiadiazol-5-yl)amine,
   5-[N-(6-{[N'-(3-cyano-5-trifluoromethylbenzyl)-N'-(1-methyl-1H-[1,2,4]triazol-3-yl)amino]methyl}indan-5-yl)-N-ethylamino]pentanoic acid hydrochloride,
   methyl 5-[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]pentanoate hydrochloride,
   methyl 5-[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]pentanoate hydrochloride,
   5-[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]pentanoic acid hydrochloride,
   trans-4-{[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(2-methyl-2H-tetrazol-5-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]methyl}cyclohexanecarboxylic acid,
   trans-4-{[N-(3-{[N'-[3,5-bis(trifluoromethyl)benzyl]-N'-(3-methylisoxazol-5-yl)amino]methyl}-5,6,7,8-tetrahydronaphthalen-2-yl)-N-ethylamino]methyl}cyclohexanecarboxylic acid hydrochloride,
   trans-4-{[N-(3-{[N'-(3-cyano-5-trifluoromethylbenzyl)-N'-(3-methylisoxazol-5-yl)amino]methyl}-5,6,7,8-tetrahydronaphthalen-2-yl)-N-ethylamino]methyl}cyclohexanecarboxylic acid hydrochloride,
   2-(5-{N-[3,5-bis(trifluoromethyl)benzyl]-N-[6-(N'-cyclopentylmethyl-N'-ethylamino)indan-5-ylmethyl]amino}tetrazol-2-yl)ethanol hydrochloride,
   methyl 5-[N-(3-{[N'-[3,5-bis(trifluoromethyl)benzyl]-N'-(1-methyl-1H-[1,2,4]triazol-3-yl)amino]methyl}-5,6,7,8-tetrahydranaphthalen-2-yl)-N-ethylamino]pentanoate hydrochloride,
   methyl 5-[N-(3-{[N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]pentanoate hydrochloride,
   5-[N-(3-{[N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]pentanoic acid hydrochloride,
   5-[N-(3-{[N'-[3,5-bis(trifluoromethyl)benzyl]-N'-(3-methylisoxazol-5-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(3-methylisoxazol-5-yl)amino]methyl}-5,6,7,8-tetrahydronaphthalen-2-yl)-N-ethylamino]pentanoic acid hydrochloride,
   5-[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]pentanoic acid hydrochloride,
   5-[N-(3-{[N'-(3-cyano-5-trifluoromethylbenzyl)-N'-(2-methyl-2H-tetrazol-5-yl)amino]methyl}-5,6,7,8-tetrahydronaphthalen-2-yl)-N-ethylamino]pentanoic acid hydrochloride,
   trans-4-{[N-(6-{[N'-[3,5-bis(trifluoromethyl)benzyl]-N'-(5-methyl-[1,2,4]oxadiazol-3-yl)amino]methyl}indan-5-yl)-N-ethylamino]methyl}cyclohexanecarboxylic acid,
   5-[N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]pentanoic acid hydrochloride,
   5-[N-(2-{[N'-[3,5-bis(trifluoromethyl)benzyl]-N'-(1-methyl-1H-pyrazol-3-yl)amino]methyl}-4-trifluoromethylphenyl)-N-ethylamino]pentanoic acid hydrochloride,
   5-[N-(2-{[N'-(3-cyano-5-trifluoromethylbenzyl)-N'-(1-methyl-1H-pyrazol-3-yl)amino]methyl}-4-trifluoromethylphenyl)-N-ethylamino]pentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]pentanoic acid hydrochloride,
   5-[N-(6-{[N'-[3,5-bis(trifluoromethyl)benzyl]-N'-(2-ethyl-2H-tetrazol-5-yl)amino]methyl}indan-5-yl)-N-ethylamino]pentanoic acid hydrochloride,
   5-{N-[6-({N'-[3,5-bis(trifluoromethyl)benzyl]-N'-[2-(2-hydroxyethyl)-2H-tetrazol-5-yl]amino}methyl)indan-5-yl]-N-ethylamino}pentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]pentanoic acid hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylaminol]-2,2-dimethylpentanoic acid hydrochloride,
   6-[N-(6-{[N'-(3,5-bis(trifluoromethyl)benzyl]-N'-(2-methyl-2H-tetrazol-5-yl)amino]methyl}indan-5-yl)-N-ethylamino]-hexanoic acid hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylamino]-3,3-dimethylpentanoic acid hydrochloride,
   trans-4-{[N-(6-{[N'-[3,5-bis(trifluoromethyl)benzyl]-N'-(2-ethyl-2H-tetrazol-5-yl)amino]methyl}indan-5-yl)-N-ethylamino]methyl}cyclohexanecarboxylic acid hydrochloride,
   (1-{2-[N-(6-{[N'-[3,5-bis(trifluoromethyl)benzyl]-N'-(2-methyl-2H-tetrazol-5-yl)amino]methyl}indan-5-yl)-N-ethylamino]ethyl}cyclopentyl)acetic acid hydrochloride,
   trans-4-({N-[6-({N'-[3,5-bis(trifluoromethyl)benzyl]-N'-[2-(2-hydroxyethyl)-2H-tetrazol-5-yl]amino}methyl)indan-5-yl]-N-ethylamino}methyl)cyclohexanecarboxylic acid,
   trans-4-{[N-(2-{[N'-[3,5-bis(trifluoromethyl)benzyl]-N'-(2-methyl-2H--tetrazol-5-yl)amino]methyl}-4-trifluoromethylphenyl)-N-ethylamino]methyl}cyclohexanecarboxylic acid hydrochloride,
   (1-{2-[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]ethyl}cyclopentyl)acetic acid,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-(3-{[N'-[3,5-bis(trifluoromethyl)benzyl]-N'-(2-ethyl-2H-tetrazol-5-yl)amino]methyl}-5,6,7,8-tetrahydronaphthalen-2-yl)-N-ethylamino]methyl}cyclohexanecarboxylic acid hydrochloride,
   trans-4-({N-[3-({N'-[3,5-bis(trifluoromethyl)benzyl]-N'-[2-(2-hydroxyethyl)-2H-tetrazol-5-yl]amino}methyl)-5,6,7,8-tetrahydronaphthalen-2-yl]-N-ethylamino}methyl)cyclohexanecarboxylic acid hydrochloride,
   1-{3-[N-(6-{[N'-[3,5-bis(trifluoromethyl)benzyl]-N'-(2-methyl-2H-tetrazol-5-yl)amino]methyl}indan-5-yl)-N-ethylamino]propyl}cyclohexanecarboxylic acid hydrochloride,
   5-[N-(6-{[N'-[3,5-bis(trifluoromethyl)benzyl]-N'-(1-methyl-1H-pyrazol-3-yl)amino]methyl}indan-5-yl)-N-ethylamino]-3,3-dimethylpentanoic acid,
   5-[N-(6-{[H'-[3,5-bis(trifluoromethyl)benzyl]-N'-(5-methyl-[1,2,4]oxadiazol-3-yl)amino]methyl}indan-5-yl)-N-ethylamino]-3,3-dimethylpentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]-3,3-dimethylpentanoic acid hydrochloride,
   5-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]-3,3-dimethylpentanoic acid hydrochloride,
   trans-4-{[N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]methyl}cyclohexanecarboxylic acid hydrochloride,
   5-[N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]-3,3-dimethylpentanoic acid hydrochloride,
   5-[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]-3,3-dimethylpentanoic acid hydrochloride,
   trans-4-{[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]methyl}cyclohexanecarboxylic acid hydrochloride,
   6-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]hexanoic acid hydrochloride,
   trans-(4-{[N-(6-{[N'-[3,5-bis(trifluoromethyl)benzyl]-N'-(2-methyl-2H-tetrazol-5-yl)amino]methyl}indan-5-yl)-N-ethylamino]methyl}cyclohexyl)acetic acid hydrochloride,
   6-[N-(6-{[N'-[3,5-bis(trifluoromethyl)benzyl]-N'-(2-methyl-2H-tetrazol-5-yl)amino]methyl}indan-5-yl)-N-ethylamino]-4,4-dimethylhexanoic acid hydrochloride,
   6-[N-(6-{[N'-[3,5-bis(trifluoromethyl)benzyl]-N'-(2-methyl-2H-tetrazol-5-yl)amino]methyl}indan-5-yl)-N-ethylamino]-3,3-dimethylhexanoic acid hydrochloride,
   5-[N-(6-{[N'-[3,5-bis(trifluoromethyl)benzyl]-N'-(2-methyl-2H-tetrazol-5-yl)amino]methyl}indan-5-yl)-N-ethylamino]-4,4-dimethylpentanoic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)acetic acid hydrochloride,
   6-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]-4,4-dimethylhexanoic acid hydrochloride,
   6-[N-{2-{[N'-[3,5-bis(trifluoromethyl)benzyl]-N'-(2-methyl-2H-tetrazol-5-yl)amino]methyl}-4-trifluoromethylphenyl)-N-ethylamino]-4,4-dimethylhexanoic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethylphenyl)-N-ethylamino]methyl}cyclohexyl)acetic acid hydrochloride,
   trans-(4-{[N-(2-{[N'-[3,5-bisftrifluoromethyl)benzyl]-N'-(2-methyl-2H-tetrazo1-5-yl)amino]methyl}-4-trifluoromethoxyphenyl)-N-ethylamino]methyl}cyclohexyl)methanol hydrochloride,
   6-[N-(6-{[N'-[3,5-bis(trifluoromethyl)benzyl]-N'-(2-methyl-2H-tetrazol-5-yl)amino]methyl}indan-5-yl)-N-ethylamino]-5,5-dimethylhexanoic acid hydrochloride,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-propylamino]methyl}cyclohexanecarboxylic acid hydrochloride,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-isobutylamino]methyl}cyclohexanecarboxylic acid hydrochloride,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid amide,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid methylamide,
   trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid dimethylamide,
   trans-4-{[N-(2-{[N'-[3,5-bis(trifluoromethyl)benzyl]-N'-(4-chlorophenyl)amino]methyl}-4-trifluoromethoxyphenyl)-N-ethylamino]methyl}cyclohexanecarboxylic acid hydrochloride,
   trans-4-{[N-(2-{[N'-[3,5-bis(trifluoromethyl)benzyl]-N'-(p-tolyl)amino]methyl}-4-trifluoromethoxyphenyl)-N-ethylamino]methyl}cyclohexanecarboxylic acid hydrochloride,
   trans-4-{[N-(2-{[N'-[3,5-bis(trifluoromethyl)benzyl]-N'-(m-tolyl)amino]methyl}-4-trifluoromethoxyphenyl)-N-ethylamino]methyl}cyclohexanecarboxylic acid hydrochloride,
   trans-4-{[N-(2-{[N'-(3,5-dichlorobenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-ethyl-N-(2-{[N'-(2-methyl-2H-tetrazol-5-yl)-N'-(3-methyl-5-trifluoromethylbenzyl)amino]methyl}-4-trifluoromethoxyphenyl)amino]methyl}cyclohexanecarboxylic acid hydrochloride,
   trans-4-{[N-(2-{[N'-(3-chloro-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-ethyl-N-(2-{[N'-(2-methyl-2H-tetrazol-5-yl)-N'-(3-nitro-5-trifluoromethylbenzyl)amino]methyl}-4-trifluoromethoxyphenyl)amino]methyl}cyclohexanecarboxylic acid hydrochloride,
   trans-(4-{[N-(6-{[N'-[3,5-bis(trifluoromethyl)benzyl]-N'-(2-methyl-2H-tetrazol-5-yl)amino]methyl}-2,2-difluorobenzo[1,3]dioxol-5-yl)-N-ethylamino]methyl}cyclohexyl)methanol hydrochloride,
   trans-(4-{[N-(6-{[N'-[3,5-bis(trifluoromethyl)benzyl]-N'-(2-methyl-2H-tetrazol-5-yl)amino]methyl}-2,2-difluorobenzo[1,3]dioxol-5-yl)-N-ethylamino]methyl}cyclohexyl)acetic acid hydrochloride,
   trans-3-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)propionic acid hydrochloride,
   trans-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)methanol hydrochloride,
   trans-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)acetic acid hydrochloride,
   trans-2-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)acetamide hydrochloride,
   trans-N-[3,5-bis(trifluoromethyl)benzyl]-N-{2-[N'-ethyl-N'-(4-(methoxymethyl)cyclohexylmethyl)amino]-5-trifluoromethoxybenzyl}-(2-methyl-2H-tetrazol-5-yl)amine hydrochloride,
   trans-2-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)ethanol hydrochloride,
   trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)methanol hydrochloride,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexylmethyl)phosphonic acid,
   trans-4-{[N-(2-{[N'-(3-bromo-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-(4-{[N-(2-{[N'-[3,5-bis(trifluoromethyl)benzyl]-N'-(2-methyl-2H-tetrazol-5-yl)amino]methyl}-4-bromophenyl)-N-ethylamino]methyl}cyclohexyl)methanol hydrochloride,
   trans-(4-{[N-(2-{[N'-[3,5-bis(trifluoromethyl)benzyl]-N'-(2-methyl-2H-tetrazol-5-yl)amino]methyl}-4-bromophenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-chloro-5-ethylphenyl)-N-ethylamino]methyl}cyolohexyl)methanol hydrochloride,
   trans-4-{[N-(2-{[N'-[3,5-bis(trifluoromethyl)benzyl]-N'-(4-methoxyphenyl)amino]methyl}-4-trifluoromethoxyphenyl)-N-ethylamino]methyl}cyclohexanecarboxylic acid hydrochloride,
   trans-(4-{[N-(2-{[N'-[3,5-bis(trifluoromethyl)benzyl]-N'-(2-methyl-2H-tetrazol-5-yl)amino]methyl}-5-methoxy-4-methylphenyl)-N-ethylamino]methyl}cyclohexyl)methanol hydrochloride,
   trans-(4-{[N-(2-{[N'-[3,5-bis(trifluoromethyl)benzyl]-N'-(2-methyl-2H-tetrazol-5-yl)amino]methyl}-4,5-dimethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethylthiophenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-chloro-5-ethylphenyl)-N-ethylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethylphenyl)-N-propylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-5-methoxy-4-methylphenyl)-N-ethylamino]methyl}cyclohexyl)acetic acid hydrochloride,
   trans-4-({N-[2-{[N'-[3,5-bis(trifluoromethyl)benzyl]-N'-(2-methyl-2H-tetrazol-5-yl)amino]methyl}-4-(2,2,2-trifluoroethyl)phenyl]-N-ethylamino}methyl)cyclohexanecarboxylic acid hydrochloride,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-propylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-propylamino]methyl}cyclohexyl)acetic acid hydrochloride,
   trans-4-{[N-(2-{[N'-[3,5-bis(trifluoromethyl)benzyl]-N'-(4-ethylphenyl)amino]methyl}-4-trifluoromethoxyphenyl)-N-ethylamino]methyl}cyclohexanecarboxylic acid hydrochloride,
   trans-4-{[N-(2-{[N'-(3-cyano-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
   trans-4-{[N-(2-{[N'-[3,5-bis(trifluoromethyl)benzyl]-N'-(4-isopropenylphenyl)amino]methyl}-4-trifluoromethoxyphenyl)-N-ethylamino]methyl}cyclohexanecarboxylic acid,
   trans-(4-{[N-(2-{[N'-[3,5-bis(trifluoromethyl)benzyl]-N'-(p-tolyl)amino]methyl}-4-trifluoromethoxyphenyl)-N-ethylamino]methyl}cyclohexyl)acetic acid dihydrochloride,
   trans-(4-{[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]methyl}cyclohexyl)acetic acid hydrochloride,
   trans-(4-{[N-(2-{[N'-[3,5-bis(trifluoromethyl)benzyl]-N'-(5-methyl-[1,2,4]oxadiazol-3-yl)amino]methyl}-5-methyl-4-trifluoromethylphenyl)-N-ethylamino]methyl}cyclohexyl)acetic acid hydrochloride,
   trans-(4-{[N-(2-{[N'-[3,5-bis(trifluoromethyl)benzyl]-N'-(5-methyl-[1,2,4]oxadiazol-3-yl)amino]methyl}-5-methyl-4-trifluoromethylphenyl)-N-propylamino]methyl}cyclohexyl)acetic acid hydrochloride,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-propylamino]methyl}cyclohexyl)acetic acid,
   trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)acetic acid,
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate, ethyl trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetate, and
   trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid, a prodrug thereof or a pharmaceutically acceptable salt thereof.
[38] The method of [1], [3] or [6], wherein the compound having a CETP inhibitory activity is a compound represented by the formula (X) 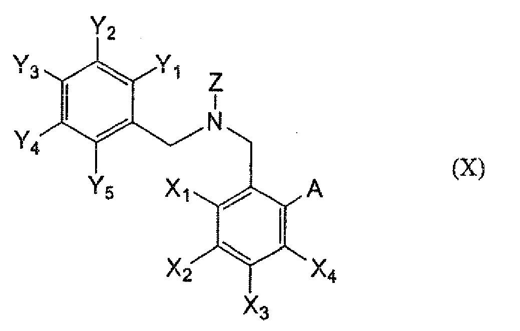 wherein X₁-X₄ and Y₁-Y₅ are the same or different and each is a hydrogen atom or any substituent, Z is the formula (XI); -Z₁-Z₂ wherein Z₁ is -CO-, -CS- or -SO₂-, Z₂ is a hydrogen atom, an optionally substituted hydrocarbon group having 1 to 20 carbon atoms, an optionally substituted amino group, an optionally substituted alkoxy group or an optionally substituted alkylthio group, A is a disubstituted amino group, an optionally substituted alkoxy group, an optionally substituted alkylthio group or an optionally substituted hydrocarbon group having 1 to 20 carbon atoms, or X₂ and X₃, X₃ and X₄ or X₄ and A may be taken together to form a homocyclic ring group optionally having substituents or a heterocyclic group optionally having substituents, or a salt thereof.
[39] The inhibitor of [2], [4] or [7], wherein the compound having a CETP inhibitory activity is a compound of the formula (X) of [38], or a salt thereof.
[40] The inhibitor of [5] or [8], which is a compound of the formula (X) of [38], or a salt thereof.
[41] The method of [38], wherein the compound of the formula (X) is a compound selected from the group consisting of methyl (3-cyano-5-trifluoromethylbenzyl)-[6-(N-cyclopentylmethyl-N-ethylamino)indan-5-ylmethyl]-carbamate, methyl [6-(N-butyl-N-ethylamino)indan-5-ylmethyl]-(3-cyano-5-trifluoromethylbenzyl)-carbamate, methyl (3-nitro-5-trifluoromethylbenzyl)-[6-(N-cyclopentylmethyl-N-ethylamino)indan-5-ylmethyl]-carbamate, methyl [6-(N-butyl-N-ethylamino)indan-5-ylmethyl]-(3,5-dichlorobenzyl)-carbamate, methyl [6-(N-butyl-N-ethylamino)indan-5-ylmethyl]-(3-chloro-5-trifluoromethylbenzyl)-carbamate, methyl (3-bromo-5-trifluoromethylbenzyl)-[6-(N-butyl-N-ethylamino)indan-5-ylmethyl]-carbamate, methyl [6-(N-butyl-N-ethylamino)indan-5-ylmethyl]-(3-methyl-5-trifluoromethylbenzyl)-carbamate, methyl [6-(N-butyl-N-ethylamino)indan-5-ylmethyl]-(3-nitro-5-trifluoromethylbenzyl)-carbamate, methyl [6-(N-cyclopentylmethyl-N-ethylamino)tetralin-5-ylmethyl]-(3-methylsulfonyl-5-trifluoromethylbenzyl)-carbamate, methyl (3-cyano-5-trifluoromethylbenzyl)-[6-(N-cyclopentylmethyl-N-ethylamino)tetralin-5-ylmethyl]-carbamate, methyl (3-cyano-5-trifluoromethylbenzyl)-[6-(N-cyclobutylmethyl-N-ethylamino)tetralin-5-ylmethyl]-carbamate, methyl (3-cyano-5-trifluoromethylbenzyl)-[6-(N-cyclopropylmethyl-N-ethylamino)tetralin-5-ylmethyl]-carbamate, methyl (3-cyano-5-trifluoromethylbenzyl)-[6-(N-cyclopropylethyl-N-ethylamino)tetralin-5-ylmethyl]-carbamate, methyl (3-cyano-5-trifluoromethylbenzyl)-[6-(N-2-methylpropyl-N-ethylamino)tetralin-5-ylmethyl]-carbamate, methyl (3-cyano-5-trifluoromethylbenzyl)-[6-(N-cyclopentylmethyl-N-ethylamino)-6,7,8,9-tetrahydro-5H-benzocycloheptan-2-ylmethyl]-carbamate, methyl (3,5-bistrifluoromethylbenzyl)-[2,2-dimethyl-6-(N-butyl-N-ethylamino)indan-5-ylmethyl]-carbamate, methyl (3,5-bistrifluoromethylbenzyl)-[6-(N-propyl-N-ethylamino)indan-5-ylmethyl]-carbamate, methyl (3,5-bistrifluoromethylbenzyl)-[6-(N-butyl-N-ethylamino)indan-5-ylmethyl]-carbamate, methyl (3,5-bistrifluoromethylbenzyl)-[6-(N,N-dipropylamino)indan-5-ylmethyl]-carbamate, methyl (3,5-bistrifluoromethylbenzyl)-[6-(N-butyl-N-propylamino)indan-5-ylmethyl]-carbamate, methyl (3,5-bistrifluoromethylbenzyl)-[6-(N-3-methylbutyl-N-ethylamino)indan-5-ylmethyl]-carbamate, methyl (3,5-bistrifluoromethylbenzyl)-[6-(N-3,3-dimethylbutyl-N-ethylamino)indan-5-ylmethyl]-carbamate, methyl (3,5-bistrifluoromethylbenzyl)-[6-(N-cyclopentylmethyl-N-ethylamino)indan-5-ylmethyl]-carbamate, methyl (3,5-bistrifluoromethylbenzyl)-[6-(N-3-butylenyl-N-ethylamino)indan-5-ylmethyl]-carbamate, methyl (3,5-bistrifluoromethylbenzyl)-[6-(N-benzyl-N-propylamino)indan-5-ylmethyl]-carbamate, methyl (3,5-bistrifluoromethylbenzyl)-[6-(N-1-methylethyl-N-propylamino)indan-5-ylmethyl]-carbamate, methyl (3,5-bistrifluoromethylbenzyl)-(6-piperidylindan-5-ylmethyl)-carbamate, methyl benzyl-[6-(N,N-dipropylamino)indan-5-ylmethyl]-carbamate, methyl [6-(N,N-dipropylamino)indan-5-ylmethyl]-(3-trifluoromethylbenzyl)-carbamate, methyl [6-(N,N-dipropylamino)indan-5-ylmethyl]-(3-methylbenzyl)-carbamate, methyl (3-chlorobenzyl)-[6-(N,N-dipropylamino)indan-5-ylmethyl]-carbamate, methyl [6-(N,N-dzpropylamino)indan-5-ylmethylj-(3-nitrobenzyl)-carbamate, methyl (3-cyanobenzyl)-[6-(N,N-dipropylamino)indan-5-ylmethyl]-oarbamate, methyl [6-(N,N-dipropylamino)indan-5-ylmethyl]-(3-methoxycarbonylbenzyl)-carbamate, methyl (3,5-dinitrobenzyl)-[6-(N,N-dipropylamino)indan-5-ylmethyl]-carbamate, methyl (3,5-bistrifluoromethylbenzyl)-(2-dipropylamino-5-methylbenzyl)-carbamate, methyl (3,5-bistrifluoromethylbenzyl)-(2-dipropylamino-5-chlorobenzyl)-carbamate, methyl (3,5-bistrifluoromethylbenzyl)-(2-dipropylamino-5-methoxybenzyl)-carbamate, methyl (3,5-bistrifluoromethylbenzyl)-(2-dipropylamino-5-trifluoromethyloxybenzyl)-carbamate, methyl [2-(N-butyl-N-ethylamino)-5-trifluoromethyloxybenzyl]-(3-nitro-5-trifluoromethylbenzyl)-carbamate, methyl (3-cyano-5-trifluoromethylbenzyl)-[2-(N-butyl-N-ethylamino)-5-trifluoromethyloxybenzyl]-carbamate, methyl (3,5-bistrifluoromethylbenzyl)-(2-dipropylamino-4,5-dimethylbenzyl)-carbamate, (3,5-bistrifluoromethylbenzyl)-[6-(N-dipropylamino)indan-5-ylmethyl]-acetamide, (3,5-bistrifluoromethylbenzyl)-[6-(N-dipropylamino)indan-5-ylmethyl]-urea, (3,5-bistrifluoromethylbenzyl)-[6-(N-dipropylamino)indan-5-ylmethyl]-formamide, methyl (3,5-bistrifluoromethylbenzyl)-(2-dipropylamino-5-trifluoromethylbenzyl)-carbamate, methyl (3,5-bistrifluoromethylbenzyl)-(2-dipropylaminobenzyl)-carbamate, methyl (3,5-bistrifluoromethylbenzyl)-(2-dipropylamino-4,5-dichlorobenzyl)-carbamate, methyl (3,5-bistrifluoromethylbenzyl)-(2-dipropylamino-4-chloro-5-trifluoromethyloxybenzyl)-carbamate, methyl [3-(N-butyl-N-ethylamino)naphthalen-2-ylmethyl]-(3-cyano-5-trifluoromethylbenzyl)-carbamate, methyl (3,5-bistrifluoromethylbenzyl)-(2-dipropylamino-4-methyloxybenzyl)-carbamate, methyl (3,5-bistrifluoromethylbenzyl)-[6-(N-propionyl-N-propylamino)indan-5-ylmethyl]carbamate, methyl (3,5-bistrifluoromethylbenzyl)-[6-(N-ethoxycarbonyl-N-propylamino)indan-5-ylmethyl]carbamate, methyl (3,5-bistrifluoromethylbenzyl)-[6-(1-propylbutoxy)indan-5-ylmethyl]carbamate, methyl (3,5-bistrifluoromethylbenzyl)-[6-(1-ethylpropyloxy)indan-5-ylmethyl]carbamate, methyl (3,5-bistrifluoromethylbenzyl)-[4,5-dimethyl-2-(1-propylbutyl)benzyl]carbamate and methyl (3-cyano-5-trifluoromethylbenzyl)-[6-(1-propylbutyl)indan-5-ylmethyl]-carbamate, or a salt thereof.
[42] The inhibitor of [39] or [40], wherein the compound of the formula (X) is a compound selected from the group consisting of methyl (3-cyano-5-trifluoromethylbenzyl)-[6-(N-cyclopentylmethyl-N-ethylamino)indan-5-ylmethyl]-carbamate, methyl [6-(N-butyl-N-ethylamino)indan-5-ylmethyl]-(3-cyano-5-trifluoromethylbenzyl)-carbamate, methyl (3-nitro-5-trifluoromethylbenzyl)-[6-(N-cyclopentylmethyl-N-ethylamino)indan-5-ylmethyl]-carbamate, methyl [6-(N-butyl-N-ethylamino)indan-5-ylmethyl]-(3,5-dichlorobenzyl)-carbamate, methyl [6-(N-butyl-N-ethylamino)indan-5-ylmethyl]-(3-chloro-5-trifluoromethylbenzyl)-carbamate, methyl (3-bromo-5-trifluoromethylbenzyl)-[6-(N-butyl-N-ethylamino)indan-5-ylmethyl]-carbamate, methyl [6-(N-butyl-N-ethylamino)indan-5-ylmethyl]-(3-methyl-5-trifluoromethylbenzyl)-carbamate, methyl [6-(N-butyl-N-ethylamino)indan-5-ylmethyl]-(3-nitro-5-trifluoromethylbenzyl)-carbamate, methyl [6-(N-cyclopentylmethyl-N-ethylamino)tetralin-5-ylmethyl]-(3-methylsulfonyl-5-trifluoromethylbenzyl)-carbamate, methyl (3-cyano-5-trifluoromethylbenzyl)-[6-(N-cyclopentylmethyl-N-ethylamino)tetralin-5-ylmethyl]-carbamate, methyl (3-cyano-5-trifluoromethylbenzyl)-[6-(N-cyclobutylmethyl-N-ethylamino)tetralin-5-ylmethyl]-carbamate, methyl (3-cyano-5-trifluoromethylbenzyl)-[6-(N-cyclopropylmethyl-N-ethylamino)tetralin-5-ylmethyl]-carbamate, methyl (3-cyano-5-trifluoromethylbenzyl)-[6-(N-cyclopropylethyl-N-ethylamino)tetralin-5-ylmethyl]-carbamate, methyl (3-cyano-5-trifluoromethylbenzyl)-[6-(N-2-methylpropyl-N-ethylamino)tetralin-5-ylmethyl]-carbamate, methyl (3-cyano-5-trifluoromethylbenzyl)-[6-(N-cyclopentylmethyl-N-ethylamino)-6,7,8,9-tetrahydro-5H-benzocycloheptan-2-ylmethyl]-carbamate, methyl (3,5-bistrifluoromethylbenzyl)-[2,2-dimethyl-6-(N-butyl-N-ethylamino)indan-5-ylmethyl]-carbamate, methyl (3,5-bistrifluoromethylbenzyl)-[6-(N-propyl-N-ethylamino)indan-5-ylmethyl]-carbamate, methyl (3,5-bistrifluoromethylbenzyl)-[6-(N-butyl-N-ethylamino)indan-5-ylmethyl]-carbamate, methyl (3,5-bistrifluoromethylbenzyl)-[6-(N,N-dipropylamino)indan-5-ylmethyl]-carbamate, methyl (3,5-bistrifluoromethylbenzyl)-[6-(N-butyl-N-propylamino)indan-5-ylmethyl]-carbamate, methyl (3,5-bistrifluoromothylbenzyl)-[6-(N-3-methylbutyl-N-ethylamino)indan-5-ylmethyl]-carbamate, methyl (3,5-bistrifluoromethylbenzyl)-[6-(N-3,3-dimethylbutyl-N-ethylamino)indan-5-ylmethyl]-carbamate, methyl (3,5-bistrifluoromethylbenzyl)-[6-(N-cyclopentylmethyl-N-ethylamino)indan-5-ylmethyl]-carbamate, methyl (3,5-bistrifluoromethylbenzyl)-[6-(N-3-butylenyl-N-ethylamino)indan-5-ylmethyl]-carbamate, methyl (3,5-bistrifluoromethylbenzyl)-[6-(N-benzyl-N-propylamino)indan-5-ylmethyl]-carbamate, methyl (3,5-bistrifluoromethylbenzyl)-[6-(N-1-methylethyl-N-propylamino)indan-5-ylmethyl]-carbamate, methyl (3,5-bistrifluoromethylbenzyl)-(6-piperidylindan-5-ylmethyl)-carbamate, methyl benzyl-[6-(N,N-dipropylamino)indan-5-ylmethyl]-carbamate, methyl [6-(N,N-dipropylamino)indan-5-ylmethyl]-(3-trifluoromethylbenzyl)-carbamate, methyl [6-(N,N-dipropylamino)indan-5-ylmethyl]-(3-methylbenzyl)-carbamate, methyl (3-chlorobenzyl)-[6-(N,N-dipropylamino)indan-5-ylmethyl]-carbamate, methyl [6-(N,N-dipropylamino)indan-5-ylmethyl]-(3-nitrobenzyl)-carbamate, methyl (3-cyanobenzyl)-[6-(N,N-dipropylamino)indan-5-ylmethyl]-carbamate, methyl [6-(N,N-dipropylamino)indan-5-ylmethyl]-(3-methoxycarbonylbenzyl)-carbamate, methyl (3,5-dinitrobenzyl)-[6-(N,N-dipropylamino)indan-5-ylmethyl]-carbamate, methyl (3,5-bistrifluoromethylbenzyl)-(2-dipropylamino-5-methylbenzyl)-carbamate, methyl (3,5-bistrifluoromethylbenzyl)-(2-dipropylamino-5-chlorobenzyl)-carbamate, methyl (3,5-bistrifluoromethylbenzyl)-(2-dipropylamino-5-methoxybenzyl)-carbamate, methyl (3,5-bistrifluoromethylbenzyl)-(2-dipropylamino-5-trifluoromethyloxybenzyl)-carbamate, methyl [2-(N-butyl-N-ethylamino)-5-trifluoromethyloxybenzyl]-(3-nitro-5-trifluoromethylbenzyl)-carbamate, methyl (3-cyano-5-trifluoromethylbenzyl)-[2-(N-butyl-N-ethylamino)-5-trifluoromethyloxybenzyl]-carbamate, methyl (3,5-bistrifluoromethylbenzyl)-(2-dipropylamino-4,5-dimethylbenzyl)-carbamate, (3,5-bistrifluoromethylbenzyl)-[6-(N-dipropylamino)indan-5-ylmethyl]-acetamide, (3,5-bistrifluoromethylbenzyl)-[6-(N-dipropylamino)indan-5-ylmethyl]-urea, (3,5-bistrifluoromethylbenzyl)-[6-(N-dipropylamino)indan-5-ylmethyl]-formamide, methyl (3,5-bistrifluoromethylbenzyl)-(2-dipropylamino-5-trifluoromethylbenzyl)-carbamate, methyl (3,5-bistrifluoromethylbenzyl)-(2-dipropylaminobenzyl)-carbamate, methyl (3,5-bistrifluoromethylbenzyl)-(2-dipropylamino-4,5-dichlorobenzyl)-carbamate, methyl (3,5-bistrifluoromethylbenzyl)-(2-dipropylamino-4-chloro-5-trifluoromethyloxybenzyl)-carbamate, methyl [3-(N-butyl-N-ethylamino)naphthalen-2-ylmethyl]-(3-cyano-5-trifluoromethylbenzyl)-carbamate, methyl (3,5-bistrifluoromethylbenzyl)-(2-dipropylamino-4-methyloxybenzyl)-carbamate, methyl (3,5-bistrifluoromethylbenzyl)-[6-(N-propionyl-N-propylamino)indan-5-ylmethyl]carbamate, methyl (3,5-bistrifluoromethylbenzyl)-[6-(N-ethoxycarbonyl-N-propylamino)indan-5-ylmethyl]carbamate, methyl (3,5-bistrifluoromethylbenzyl)-[6-(1-propylbutoxy)indan-5-ylmethyl]carbamate, methyl (3,5-bistrifluoromethylbenzyl)-[6-(1-ethylpropyloxy)indan-5-ylmethyl]carbamate, methyl (3,5-bistrifluoromethylbenzyl)-[4,5-dimethyl-2-(1-propylbutyl)benzyl]carbamate and methyl (3-cyano-5-trifluoromethylbenzyl)-[6-(1-propylbutyl)indan-5-ylmethyl]-carbamate, or a salt thereof.
[43] The method of [1], [3] or [6], wherein the compound having a CETP inhibitory activity is a compound of the formula (I) 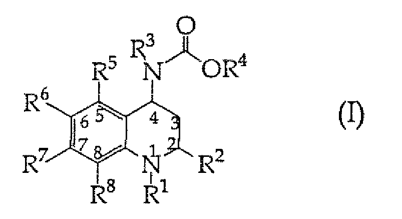 wherein
   - R¹: is a hydrogen, Y, W-X or W-Y;
   - W: is carbonyl, thiocarbonyl, sulfinyl or sulfonyl;
   - X: is -O-Y, -S-Y, -N(H)-Y or -N-(Y)₂; wherein Y in each case is independently Z or a fully saturated, partially unsaturated or fully unsaturated 1- to 10-membered straight chain or branched carbon chain, wherein the carbons other than a connecting carbons may be replaced with 1 or 2 heteroatoms independently selected from oxygen, sulfur and nitrogen, and the carbon may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon chain may be mono-substituted by Z;
   Z is a partially saturated, fully saturated or fully unsaturated 3- to 8-membered ring optionally having 1 to 4 heteroatoms independently selected from oxygen, sulfur and nitrogen, or a bicyclic ring consisting of two fused partially saturated, fully saturated or fully unsaturated 3- to 6-membered rings, taken independently, which may have 1 to 4 heteroatoms independently selected from oxygen, sulfur and nitrogen; the Z substituent may be independently mono-, di-or tri-substituted by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, wherein the (C₁-C₆)alkyl substituent may be independently mono-, di- or tri-substituted by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino and the (C₁-C₆)alkyl substituent may be substituted by 1 to 9 fluorines;
   - R²: is a partially saturated, fully saturated or fully unsaturated 1- to 6-membered straight chain or branched carbon chain, wherein the carbons other than a connecting carbons may be replaced with 1 or 2 heteroatoms independently selected from oxygen, sulfur and nitrogen, the carbon atom may be independently mono-, di- or tri-substituted by halo, the carbon may be mono-substituted by oxo, the carbon may be mono-substituted by hydroxy, the sulfur may be mono or di-substituted by oxo, the nitrogen may be mono or di-substituted by oxo; or R² is a partially saturated, fully saturated or fully unsaturated 3- to 7-membered ring optionally having 1 or 2 heteroatoms independently selected from oxygen, sulfur and nitrogen, wherein the R² ring may be bonded via (C₁-C₄)alkyl; wherein the R² ring may be independently mono-, di- or tri-substituted by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent may be independently mono-, di- or tri-substituted by halo, hydroxy, (C₁-C₆)alkoxy, (C₁-C₄)alkylthio, oxo or (C₁-C₆)alkyloxycarbonyl; provided that R² is not methyl;
   - R³: is a hydrogen or Q; wherein Q is a fully saturated, partially unsaturated or fully unsaturated 1- to 6-membered straight chain or branched carbon chain, wherein the carbons other than a connecting carbons may be replaced with one heteroatom selected from oxygen, sulfur and nitrogen, the carbon may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon chain may be mono-substituted by V; V is a partially saturated, fully saturated or fully unsaturated 3- to 8-membered ring optionally having 1 to 4 heteroatoms independently selected from oxygen, sulfur and nitrogen or a bicyclic ring consisting of two fused partially saturated, fully saturated or fully unsaturated 3- to 6-membered rings, taken independently, which may have 1 to 4 heteroatoms independently selected from oxygen, sulfur and nitrogen; wherein the V substituent may be independently mono-, di- or tri-substituted by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl, mono-N- or di-N,N-(C₁-C₆)alkylamino, wherein the (C₁-C₆)alkyl or (C₂-C₆)alkenyl substituent may be independently mono-, di- or tri-substituted by hydroxy, (C₁-C₆)alkoxy, (C₁-C₄)alkylthio, amino, nitro, cyano, oxo, carboxy. (C₁-C₆)alkyloxycarbonyl, mono-N- or di-N,N-(C₁-C₆)alkylamino, and the (C₁-C₆)alkyl substituent or (C₂-C₆)alkenyl substituent may be substituted by 1 to 9 fluorines;
   - R⁴: is Q¹ or V¹; wherein Q¹ is a fully saturated, partially unsaturated or fully unsaturated 1- to 6-membered straight chain or branched carbon chain, wherein the carbons other than a connecting carbons may be replaced with one heteroatom selected from oxygen, sulfur and nitrogen, the carbon may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon chain may be mono-substituted by V¹; V¹ is a partially saturated, fully saturated or fully unsaturated 3- to 6-membered ring optionally having 1 or 2 heteroatoms independently selected from oxygen, sulfur and nitrogen; V¹ substituent may be independently mono-, di-, tri- or tetra-substituted independently by halo, (C₁-C₆)alkyl, (C₁-C₆)alkoxy, amino, nitro, cyano, (C₁-C₆)alkyloxycarbonyl, mono-N- or di-N,N-(C₁-C₆)alkylamino wherein the (C₁-C₆)alkyl substituent is optionally mono-substituted independently by oxo or the (C₁-C₆)alkyl substituent is optionally substituted by 1 to 9 fluorines; wherein any R³ should contain V, or R⁴ should contain V¹; and
   - R⁵, R⁶, R⁷ and R⁸: are each independently a hydrogen, a bond, a nitro or a halo, wherein the bond is substituted by T or a partially saturated, fully saturated or fully unsaturated (C₁-C₁₂) straight chain or branched carbon chain wherein the carbons may be replaced with 1 or 2 heteroatoms independently selected from oxygen, sulfur and nitrogen, wherein the carbon atom may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon may be mono-substituted by T; T is a partially saturated, fully saturated or fully unsaturated 3- to 8-membered ring optionally having 1 to 4 heteroatoms independently selected from oxygen, sulfur and nitrogen, or a bicyclic ring consisting of two fused partially saturated, fully saturated or fully unsaturated 3- to 6-membered rings, taken independently, which may have 1 to 4 heteroatoms independently selected from oxygen, sulfur and nitrogen; wherein the T substituent is independently mono-, di- or tri-substituted by halo, (C₁-C₆)alkyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent is optionally substituted by 1 to 9 fluorines; and
   - R⁵ and R⁶, or R⁶ and R⁷, and/or R⁷ and R⁸: may be linked to form a partially saturated or fully unsaturated 4- to 8-membered ring optionally having 1 to 3, at least 1, heteroatom independently selected from nitrogen, sulfur and oxygen; wherein the ring (one or plural) formed by R⁵ and R⁶, or R⁶ and R⁷, and/or R⁷ and R⁸ may be independently mono-, di- or tri-substituted by halo, (C₁-C₆)alkyl, (C₁-C₄)alkylsulfonyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁- C₆)alkylamino, the (C₁-C₆)alkyl substituent may be independently mono-, di- or tri-substituted by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent may be substituted by 1 to 9 fluorines; provided that when R² is carboxyl or (C₁-C₄)alkylcarboxyl, R¹ is not a hydrogen,
   a prodrug thereof or a pharmaceutically acceptable salt of the compound or the prodrug.
[44] The inhibitor of [2], [4] or [7], wherein the compound having a CETP inhibitory activity is a compound of the formula (I) of [43], a prodrug thereof or a pharmaceutically acceptable salt of the compound or the prodrug.
[45] The inhibitor of [5] or [8], which is a compound of the formula (I) of [43], a prodrug thereof or a pharmaceutically acceptable salt of the compound or the prodrug.
[46] The method of [43], wherein the compound of the formula (I) is a compound selected from a group consisting of
   [2S,4S]4-[(3,5-bis-trifluoromethyl-benzyl)-methoxycarbonyl-amino]-2-isopropyl-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-6-chloro-2-cyclopropyl-3,4-dihydro-2H-quinoline-1-carboxylic acid isopropyl ester;
   [2S,4S]2-cyclopropyl-4-[(3,5-dichloro-benzyl)-methoxycarbonyl-amino]-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid tert-butyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-2-cyclobutyl-6-trifluoromethyl-3,4-dihydro-2H-quinoline-1-carboxylic acid isopropyl ester;
   [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid isopropyl ester;
   [2S,4S]4-[(3,5-bis-trifluoromethyl-benzyl)-methoxycaxbonyl-amino]-2-methoxymethyl-6-trifluoromethyl-3,4-dihydro-2H-quinoline-1-carboxylic acid isopropyl ester;
   [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid 2-hydroxy-ethyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid ethyl ester;
   [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid propyl ester; and [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid propyl ester, a prodrug thereof or a pharmaceutically acceptable salt of the compound or the prodrug.
[47] The inhibitor of [44] or [45], wherein the compound of the formula (I) is a compound selected from a group consisting of
   [2S,4S]4-[(3,5-bis-trifluoromethyl-benzyl)-methoxycarbonyl-amino]-2-isopropyl-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-6-chloro-2-cyclopropyl-3,4-dihydro-2H-quinoline-1-carboxylic acid isopropyl ester;
   [2S,4S]2-cyclopropyl-4-[(3,5-dichloro-benzyl)-methoxycarbonyl-amino]-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid tert-butyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-2-cyclobutyl-6-trifluoromethyl-3,4-dihydro-2H-quinoline-1-carboxylic acid isopropyl ester;
   [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid isopropyl ester;
   [2S,4S]4-[(3,5-bis-trifluoromethyl-benzyl)-methoxycarbonyl-amino]-2-methoxymethyl-6-trifluoromethyl-3,4-dihydro-2H-quinoline-1-carboxylic acid isopropyl ester;
   [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid 2-hydroxy-ethyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid ethyl ester;
   [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester;
   [2S,4S]4-[(3,5-bis-trifluoromethyl-benzyl)-methoxycarbonyl-amino]-2-cyclopropyl-6-trifluoromethyl-3,4-dihydro-2H-quinoline-1-carboxylic acid propyl ester; and [2R,4S]4-[(3,5-bis-trifluoromethyl-benzyl)-methoxycarbonyl-amino]-2-ethyl-6-trifluoromethyl-3,4-dihydro-2H-quinoline-1-carboxylic acid propyl ester, a prodrug thereof or a pharmaceutically acceptable salt of the compound or the prodrug.
[48] The method of [1], [3] or [6], wherein the compound having a CETP inhibitory activity is a compound of the formula (II): 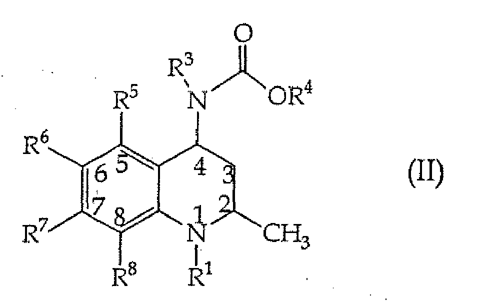
   wherein R¹ is hydrogen, Y, W-X, W-Y;
   W is carbonyl, thiocarbonyl, sulfinyl or sulfonyl;
   X is -O-Y, -S-Y, -N(H)-Y or -N-(Y)₂;
   Y in each case is independently, Z or a fully saturated, partially unsaturated or fully unsaturated 1- to 10-membered straight or branched carbon chain wherein the carbons other than the connecting carbon, may be replaced with 1 or 2 heteroatoms selected independently from oxygen, sulfur and nitrogen and aforementioned carbon is optionally mono-, di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon chain is optionally mono-substituted by Z;
   Z is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein the aforementioned Z substituent is optionally mono-, di- or tri-substituted independently by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the aforementioned (C₁-C₆)alkyl substituent is optionally mono-, di- or tri-substituted independently by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the aforementioned (C₁-C₆)alkyl is optionally substituted by 1 to 9 fluorines;
   R³ is hydrogen or Q;
   wherein Q is a fully saturated, partially unsaturated or fully unsaturated 1- to 6-membered straight or branched carbon chain wherein the carbons other than the connecting carbon, may be replaced with one heteroatom selected from oxygen, sulfur and nitrogen, the aforementioned carbon is optionally mono-, di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon chain is optionally mono-substituted by V;
   wherein V is a partially saturated, fully saturated or fully unsaturated 3- to 12-membered ring optionally having 1 to 4 heteroatoms selected independently from oxygen, sulfur and nitrogen or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   the aforementioned V substituent is optionally mono-, di-, tri- or tetra-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl, mono-N- or di-N,N-(C₁-C₆)alkylamino wherein the aforementioned (C₁-C₆)alkyl or (C₂-C₆)alkenyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino or the aforementioned (C₁-C₆)alkyl or (C₂-C₆)alkenyl substituent is optionally substituted by 1 to 9 fluorines;
   R⁴ is Q¹ or V¹;
   wherein Q¹ is a fully saturated, partially unsaturated or fully unsaturated 1- to 6-membered straight or branched carbon chain wherein the carbons other than the connecting carbon, may be replaced with one heteroatom selected from oxygen, sulfur and nitrogen and the aforementioned carbon is optionally mono-, di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon chain is optionally mono-substituted by V¹;
   wherein V¹ is a partially saturated, fully saturated or fully unsaturated 3- to 6-membered ring optionally having 1 or 2 heteroatoms independently selected from oxygen, sulfur and nitrogen;
   wherein the aforementioned V¹ substituent is optionally mono-, di-, tri- or tetra-substituted independently by halo, (C₁-C₆)alkyl, (C₁-C₆)alkoxy, amino, nitro, cyano, (C₁-C₆)alkyloxycarbonyl, mono-N- or di-N,N-(C₁-C₆)alkylamino, the aforementioned (C₁-C₆)alkyl substituent is optionally mono-substituted by oxo, the aforementioned (C₁-C₆)alkyl substituent is optionally substituted by 1 to 9 fluorines; wherein either R³ should contain V or R⁴ should contain V¹; and R⁵, R⁶, R⁷ and R⁸ are each independently hydrogen, a bond, nitro or halo wherein the aforementioned bond is substituted by T or a partially saturated, fully saturated or fully unsaturated straight or branched carbon chain having 1 to 12 carbon atoms wherein the carbons may be replaced with 1 or 2 heteroatoms selected independently from oxygen, sulfur and nitrogen wherein the aforementioned carbon atom is optionally mono- di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon is optionally mono-substituted by T;
   wherein T is a partially saturated, fully saturated or fully unsaturated 3- to 12-membered ring optionally having 1 to 4 heteroatoms selected independently from oxygen, sulfur and nitrogen or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein the aforementioned T substituent is optionally mono-, di- or tri-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the aforementioned (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the aforementioned (C₁-C₆)alkyl substituent is optionally substituted by 1 to 9 fluorines; provided that at least one of substituents R⁵, R⁶, R⁷ and R⁸ is not hydrogen and is not linked to the quinoline moiety through oxy, a prodrug thereof or a pharmaceutically acceptable salt of the compound or the prodrug.
[49] The inhibitor of [2], [4] or [7], wherein the compound having a CETP inhibitory activity is a compound of the formula (I) of [48], a prodrug thereof or a pharmaceutically acceptable salt of the compound or the prodrug.
[50] The inhibitor of [5] or [8], which is a compound of the formula (II) of [48], a prodrug thereof or a pharmaceutically acceptable salt of the compound or the prodrug.
[51] The method of [48], wherein the compound of the formula (II) is a compound selected from a group consisting of
   [2R,4S]4-[(3,5-bis-trifluoromethyl-benzyl)-methoxycarbonyl-amino]-2-methyl-7-trifluoromethyl-3,4-dihydro-2H-quinoline-1-carboxylic acid ethyl ester;
   [2R,4S]4-[(3,5-bis-trifluoromethyl-benzyl)-methoxycarbonyl-amino]-7-chloro-2-methyl-3,4-dihydro-2H-quinoline-1-carboxylic acid ethyl ester;
   [2R,4S]4-[(3,5-bis-trifluoromethyl-benzyl)-methoxycarbonyl-amino]-6-chloro-2-methyl-3,4-dihydro-2H-quinoline-1-carboxylic acid ethyl ester;
   [2R,4S]4-[(3,5-bis-trifluoromethyl-benzyl)-methoxycarbonyl-amino]-2,6,7-trimethyl-3,4-dihydro-2H-quinoline-1-carboxylic acid ethyl ester;
   [2R,4S]4-[(3,5-bis-trifluoromethyl-benzyl)-methoxycarbonyl-amino]-6,7-diethyl-2-methyl-3,4-dihydro-2H-quinoline-1-carboxylic acid ethyl ester;
   [2R,4S]4-[(3,5-bis-trifluoromethyl-benzyl)-methoxycarbonyl-amino]-6-ethyl-2-methyl-3,4-dihydro-2H-quinoline-1-carboxylic acid ethyl ester;
   [2R,4S]4-[(3,5-bis-trifluoromethyl-benzyl)-methoxycarbonyl-amino]-2-methyl-6-trifluoromethyl-3,4-dihydro-2H-quinoline-1-carboxylic acid ethyl ester; and
   [2R,4S]4-[(3,5-bis-trifluoromethyl-benzyl)-methoxycarbonyl-amino]-2-methyl-6-trifluoromethyl-3,4-dihydro-2H-quinoline-1-carboxylic acid isopropyl ester, a prodrug thereof or a pharmaceutically acceptable salt of the compound or the prodrug.
[52] The inhibitor of [49] or [50], wherein the compound of the formula (II) is a compound selected from a group consisting of
   [2R,4S]4-[(3,5-bis-trifluoromethyl-benzyl)-methoxycarbonyl-amino]-2-methyl-7-trifluoromethyl-3,4-dihydro-2H-quinoline-1-carboxylic acid ethyl ester;
   [2R,4S]4-[(3,5-bis-trifluoromethyl-benzyl)-methoxycarbonyl-amino]-7-chloro-2-methyl-3,4-dihydro-2H-quinoline-1-carboxylic acid ethyl ester;
   [2R,4S]4-[(3,5-bis-trifluoromethyl-benzyl)-methoxycarbonyl-amino]-6-chloro-2-methyl-3,4-dihydro-2H-quinoline-1-carboxylic acid ethyl ester;
   [2R,4S]4-[(3,5-bis-trifluoromethyl-benzyl)-methoxycarbonyl-amino]-2,6,7-trimethyl-3,4-dihydro-2H-quinoline-1-carboxylic acid ethyl ester;
   [2R,4S]4-[(3,5-bis-trifluoromethyl-benzyl)-methoxycarbonyl-amino]-6,7-diethyl-2-methyl-3,4-dihydro-2H-quinoline-1-carboxylic acid ethyl ester;
   [2R,4S]4-[(3,5-bis-trifluoromethyl-benzyl)-methoxycarbonyl-amino]-6-ethyl-2-methyl-3,4-dihydro-2H-quinoline-1-carboxylic acid ethyl ester;
   [2R,4S]4-[(3,5-bis-trifluoromethyl-benzyl)-methoxycarbonyl-amino]-2-methyl-6-trifluoromethyl-3,4-dihydro-2H-quinoline-1-carboxylic acid ethyl ester; and
   [2R,4S]4-[(3,5-bis-trifluoromethyl-benzyl)-methoxycarbonyl-amino]-2-methyl-6-trifluoromethyl-3,4-dihydro-2H-quinoline-1-carboxylic acid isopropyl ester, a prodrug thereof or a pharmaceutically acceptable salt of the compound or the prodrug.
[53] The method of [1], [3] or [6], wherein the compound having a CETP inhibitory activity is a compound of the formula (III) 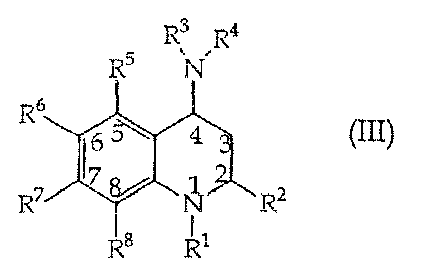
   wherein R¹ is Y, W-X or W-Y;
   wherein W is a carbonyl, thiocarbonyl, sulfinyl or sulfonyl;
   X is -O-Y, -S-Y, -N(H)-Y or -N-(Y)₂;
   Y in each case is independently Z or fully saturated, partially unsaturated or fully unsaturated 1- to 10-membered straight chain or branched carbon chain wherein the carbons other than the connecting carbon, may be replaced with 1 or 2 heteroatoms selected independently from oxygen, sulfur and nitrogen and the carbon may be mono-, di- or tri-substituted independently by halo, the carbon is optionally mono-substituted by hydroxy, the carbon is optionally mono-substituted by oxo, the sulfur is optionally mono or di-substituted by oxo, the nitrogen is optionally mono or di-substituted by oxo, and the carbon chain is optionally mono-substituted by Z;
   wherein Z is a partially saturated, fully saturated or fully unsaturated 3- to 8-membered ring optionally having 1 to 4 heteroatoms independently selected from oxygen, sulfur and nitrogen, or a bicyclic ring consisting of two fused partially saturated, fully saturated or fully unsaturated 3- to 6-membered rings, taken independently, optionally having 1 to 4 heteroatoms from nitrogen, sulfur and oxygen; wherein the Z substituent is optionally mono-, di- or tri-substituted independently by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl substituent is optionally substituted by 1 to 9 fluorines;
   R² is a partially saturated, fully saturated or fully unsaturated 1- to 6-membered straight chain or branched carbon chain wherein the carbons other than the connecting carbon, may be replaced with 1 or 2 heteroatoms selected independently from oxygen, sulfur and nitrogen wherein the carbon atom is optionally mono-, di- or tri-substituted independently by halo, the carbon is optionally mono-substituted by oxo, the carbon is optionally mono-substituted by hydroxy, the sulfur is optionally mono or di-substituted by oxo, the nitrogen is optionally mono or di-substituted by oxo; or the R² is a partially saturated, fully saturated or fully unsaturated 3- to 7-membered ring optionally having 1 or 2 heteroatoms selected independently from oxygen, sulfur and nitrogen, wherein the R² ring is optionally bonded via (C₁-C₄)alkyl;
   wherein the R² ring is optionally mono-, di- or tri-substituted by halo, (C₂-C₆)alkenyl, (C₁-C₆₎alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by halo, hydroxy, (C₁-C₆)alkoxy, (C₁-C₄)alkylthio, oxo or (C₁-C₆)alkyloxycarbonyl;
   R³ is hydrogen or Q;
   wherein Q is a fully saturated, partially unsaturated or fully unsaturated 1- to 6-membered straight chain or branched carbon chain wherein the carbons, other than connecting carbon, may be replaced with one heteroatom selected from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is mono or di-substituted by oxo, the nitrogen is optionally mono or di-substituted by oxo, and the carbon chain is optionally mono-substituted by V;
   wherein V is a partially saturated, fully saturated or fully unsaturated 3- to 8-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein the V substituent is optionally mono, di, tri- or tetra-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl, mono-N- or di-N,N-(C₁-C₆)alkylamino wherein the (C₁-C₆)alkyl substituent or (C₂-C₆)alkenyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl substituent or (C₂-C₆)alkenyl substituent is also optionally substituted by 1 to 9 fluorines;
   R⁴ is cyano, formyl, W¹Q¹, W¹V¹, (C₁-C₄)alkylene V¹ or V²; wherein W¹ is carbonyl, thiocarbonyl, SO or SO₂; Q¹ is a fully saturated, partially unsaturated or fully unsaturated 1- to 6-membered straight chain or branched carbon chain wherein the carbons may be replaced with one heteroatom selected from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is optionally mono or di-substituted by oxo, the nitrogen is optionally mono or di-substituted by oxo, and the carbon chain is optionally mono-substituted by V¹;
   wherein V¹ is a partially saturated, fully saturated or fully unsaturated 3- to 6-membered ring optionally having 1 or 2 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein the V¹ substituent is optionally mono, di, tri- or tetra-substituted independently by halo, (C₁-C₆)alkyl, (C₁-c₆)alkoxy, hydroxy, oxo, amino, nitro, cyano, (C₁-C₆)alkyloxycarbonyl, mono-N- or di-N,N-(C₁-C₆)alkylamino wherein the (C₁-C₆)alkyl substituent is optionally mono-substituted by oxo, the (C₁-C₆)alkyl substituent is also optionally substituted by 1 to 9 fluorines; wherein V² is a partially saturated, fully saturated or fully unsaturated 5- to 7-membered ring containing 1 to 4 heteroatoms independently from oxygen, sulfur and nitrogen; wherein the V² substituent is optionally mono-, di- or tri-substituted independently by halo, (C₁-C₂)alkyl, (C₁-C₂)alkoxy, hydroxy or oxo, wherein the (C₁-C₂)alkyl optionally has 1 to 5 fluorines; and
   wherein R⁴ does not include oxycarbonyl linked directly to the C⁴ nitrogen;
   wherein either R³ should contain V or R⁴ should contain V¹; R⁵, R⁶, R⁷ and R⁸ are independently hydrogen, a bond, nitro or halo wherein the bond is saturated with T or a partially saturated, fully saturated or fully unsaturated straight chain or branched carbon chain having 1 to 12 carbon atoms wherein the carbons may be replaced with 1 or 2 heteroatoms selected independently from oxygen, sulfur and nitrogen, wherein the carbon atom is optionally mono-, di- or tri-substituted independently by halo, the carbon is optionally mono-substituted by hydroxy, the carbon is optionally mono-substituted by oxo, the sulfur is optionally mono or di-substituted by oxo, the nitrogen is optionally mono or di-substituted by oxo, and the carbon chain is optionally mono-substituted by T;
   wherein T is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein the T substituent is optionally mono-, di- or tri-substituted by halo, (C₁-C₆)alkyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent also optionally has 1 to 9 fluorines;
   wherein. R⁵ and R⁶, or R⁶ and R⁷, and/or R⁷ and R⁸ may also be linked to form at least one ring that is a partially saturated or fully unsaturated 4- to 8-membered ring optionally having 1 to 3 heteroatoms independently selected from nitrogen, sulfur and oxygen;
   wherein the ring formed by R⁵ and R⁶, or R⁶ and R⁷, and/or R⁷ and R⁸ are optionally mono-, di- or tri-substituted independently by halo, (C₁-C₆)alkyl, (C₁-C₄)alkylsulfonyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent also optionally has 1 to 9 fluorines, a prodrug thereof or a pharmaceutically acceptable salt of the compound or the prodrug.
[54] The inhibitor of [2], [4] or [7], wherein the compound having a CETP inhibitory activity is a compound of the formula (III) of [53], a prodrug thereof or a pharmaceutically acceptable salt of the compound or the prodrug.
[55] The inhibitor of [5] or [8], which is a compound of the formula (III) of [53], a prodrug thereof or a pharmaceutically acceptable salt of the compound or the prodrug.
[56] The method of [53], wherein the compound of the formula (III) is a compound selected from a group consisting of
   [2S,4S]4-[(3,5-bistrifluoromethylbenzyl)-formylamino]-2-cyclopropyl-6-trifluoromethyl-3,4-dihydro-2H-quinoline-1-carboxylic acid isopropyl ester;
   [2S,4S]4-[(3,5-bistrifluoromethylbenzyl)-formylamino]-2-cyclopropyl-6-trifluoromethyl-3,4-dihydro-2H-quinoline-1-carboxylic acid propyl ester;
   [2S,4S]4-[acetyl-(3,5-bistrifluoromethylbenzyl)-aminol-2-cyclopropyl-6-trifluoromethyl-3,4-dihydro-2H-quinoline-1-carboxylic acid tert-butyl ester;
   [2R,4S]4-[acetyl-(3,5-bistrifluoromethylbenzyl)-amino]-2-ethyl-6-trifluoromethyl-3,4-dihydro-2H-quinoline-1-carboxylic acid isopropyl ester;
   [2R,4S]4-[acetyl-(3,5-bistrifluoromethylbenzyl)-amino]-2-methyl-6-trifluoromethyl-3,4-dihydro-2H-quinoline-1-carboxylic acid ethyl ester;
   [2S,4S]4-[1-(3,5-bistrifluoromethylbenzyl)-ureido]-2-cyclopropyl-6-trifluoromethyl-3,4-dihydro-2H-quinoline-1-carboxylic acid isopropyl ester;
   [2R,4S]4-[acetyl-(3,5-bistrifluoromethylbenzyl)-amino]-2-ethyl-6-trifluoromethyl-3,4-dihydro-2H-quinoline-1-carboxylic acid ethyl ester;
   [2S,4S]4-[acetyl-(3,5-bistrifluoromethylbenzyl)-amino]-2-methoxymethyl-6-trifluoromethyl-3,4-dihydro-2H-quinoline-1-carboxylic acid isopropyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid propyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid ethyl ester;
   [2R,4S]4-[(3,5-bistrifluoromethylbenzyl)-formylamino]-2-ethyl-6-trifluoromethyl-3,4-dihydro-2H-quinoline-1-carboxylic acid isopropyl ester;
   [2R,4S]4-[(3,5-bistrifluoromethylbenzyl)-formylamino]-2-methyl-6-trifluoromethyl-3,4-dihydro-2H-quinoline-1-carboxylic acid ethyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid isopropyl ester;
   [2R,4S]4-[(3,5-bistrifluoromethylbenzyl)-formylamino]-2-ethyl-6-trifluoromethyl-3,4-dihydro-2H-quinoline-1-carboxylic acid ethyl ester;
   [2S,4S]4-[(3,5-bistrifluoromethylbenzyl)-formylamino]-2-cyclopropyl-6-trifluoromethyl-3,4-dihydro-2H-quinoline-1-carboxylic acid ethyl ester;
   [2R,4S]4-[(3,5-bistrifluoromethylbenzyl)-formylamino]-2-methyl-6-trifluoromethyl-3,4-dihydro-2H-quinoline-1-carboxylic acid isopropyl ester;
   [2R,4S]4-[acetyl-(3,5-bistrifluoromethylbenzyl)-amino]-2-methyl-6-trifluoromethyl-3,4-dihydro-2H-quinoline-1-carboxylic acid isopropyl ester;
   [2R,4S]4-(3,5-bistrifluoromethylbenzylamino)-2-methyl-6-trifluoromethyl-3,4-dihydro-2H-quinoline-1-carboxylic acid ethyl ester;
   [2R,4S]4-(3,5-bistrifluoromethylbenzylamino)-2-methyl-6-trifluoromethyl-3,4-dihydro-2H-quinoline-1-carboxylic acid propyl ester;
   [2R,4S]4-(3,5-bistrifluoromethylbenzylamino)-2-methyl-6-trifluoromethyl-3,4-dihydro-2H-quinoline-1-carboxylic acid isopropyl ester;
   [2R,4S]4-(3,5-bistrifluoromethylbenzylamino)-2-ethyl-6-trifluoromethyl-3,4-dihydro-2H-quinoline-1-carboxylic acid ethyl ester;
   [2R,4S]4-(3,5-bistrifluoromethylbenzylamino)-2-ethyl-6-trifluoromethyl-3,4-dihydro-2H-quinoline-1-carboxylic acid propyl ester;
   [2R,4S]4-(3,5-bistrifluoromethylbenzylamino)-2-ethyl-6-trifluoromethyl-3,4-dihydro-2H-quinoline-1-carboxylic acid isopropyl ester;
   [2S,4S]4-(3,5-bistrifluoromethylbenzylamino)-2-cyclopropyl-6-trifluoromethyl-3,4-dihydro-2H-quinoline-1-carboxylic acid ethyl ester;
   [2S,4S]4-(3,5-bistrifluoromethylbenzylamino)-2-cyclopropyl-6-trifluoromethyl-3,4-dihydro-2H-quinoline-1-carboxylic acid propyl ester; and [2S,4S]4-(3,5-bistrifluoromethylbenzylamino)-2-cyclopropyl-6-trifluoromethyl-3,4-dihydro-2H-quinoline-1-carboxylic acid isopropyl ester, a prodrug thereof or a pharmaceutically acceptable salt of the compound or the prodrug.
[57] The inhibitor of [54] or [55], wherein the compound of the formula (III) is a compound selected from a group consisting of
   [2S,4S]4-[(3,5-bistrifluoromethylbenzyl)-formylamino]-2-cyclopxopyl-6-trifluoromethyl-3,4-dihydro-2H-quinoline-1-carboxylic acid isopropyl ester;
   [2S,4S]4-[(3,5-bistrifluoromethylbenzyl)-formylamino]-2-cyclopropyl-6-trifluoromethyl-3,4-dihydro-2H-quinoline-1-carboxylic acid propyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid tert-butyl ester;
   [2R,4S]4-[acetyl-(3,5-bistrifluoromethylbenzyl)-amino]-2-ethyl-6-trifluoromethyl-3,4-dihydro-2H-quinoline-1-carboxylic acid isopropyl ester;
   [2R,4S]4-[acetyl-(3,5-bistrifluoromethylbenzyl)-amino]-2-methyl-6-trifluoromethyl-3,4-dihydro-2H-quinoline-1-carboxylic acid ethyl ester;
   [2S,4S]4-[1-(3,5-bistrifluoromethylbenzyl)-ureido]-2-cyclopropyl-6-trifluoromethyl-3,4-dihydro-2H-quinoline-1-carboxylic acid isopropyl ester;
   [2R,4S]4-[acetyl-(3,5-bistrifluoromethylbenzyl)-amino]-2-ethyl-6-trifluoromethyl-3,4-dihydro-2H-quinoline-1-carboxylic acid ethyl ester;
   [2S,4S]4-[acetyl-(3,5-bistrifluoromethylbenzyl)-amino]-2-methoxymethyl-6-trifluoromethyl-3,4-dihydro-2H-quinoline-1-carboxylic acid isopropyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid propyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid ethyl ester;
   [2R,4S]4-[(3,5-bistrifluoromethylbenzyl)-formylamino]-2-ethyl-6-trifluoromethyl-3,4-dihydro-2H-quinoline-1-carboxylic acid isopropyl ester;
   [2R,4S]4-[(3,5-bistrifluoromethylbenzyl)-formylamino]-2-methyl-6-trifluoromethyl-3,4-dihydro-2H-quinoline-1-carboxylic acid ethyl ester;
   [2S,4S]4-[acetyl-(3,5-bistrifluoromethylbenzyl)-amino]-2-cyclopropyl-6-trifluoromethyl-3,4-dihydro-2H-quinoline-1-carboxylic acid isopropyl ester;
   [2R,4S]4-[(3,5-bistrifluoromethylbenzyl)-formylamino]-2-ethyl-6-trifluoromethyl-3,4-dihydro-2H-quinoline-1-carboxylic acid ethyl ester;
   [2S,4S]4-[(3,5-bistrifluoromethylbenzyl)-formylamino]-2-cyclopropyl-6-trifluoromethyl-3,4-dihydro-2H-quinoline-1-carboxylic acid ethyl ester;
   [2R,4S]4-[(3,5-bistrifluoromethylbenzyl)-formylamino]-2-methyl-6-trifluoromethyl-3,4-dihydro-2H-quinoline-1-carboxylic acid isopropyl ester;
   [2R,4S]4-[acetyl-(3,5-bistrifluoromethylbenzyl)-amino]-2-methyl-6-trifluoromethyl-3,4-dihydro-2H-quinoline-1-carboxylic acid isopropyl ester;
   [2R,4S]4-(3,5-bistrifluoromethylbenzylamino)-2-methyl-6-trifluoromethyl-3,4-dihydro-2H-quinoline-1-carboxylic acid ethyl ester;
   [2R,4S]4-(3,5-bistrifluoromethylbenzylamino)-2-methyl-6-trifluoromethyl-3,4-dihydro-2H-quinoline-1-carboxylic acid propyl ester;
   [2R,4S]4-(3,5-bistrifluoromethylbenzylamino)-2-methyl-6-trifluoromethyl-3,4-dihydro-2H-quinoline-1-carboxylic acid isopropyl ester;
   [2R,4S]4-(3,5-bistrifluoromethylbenzylamino)-2-ethyl-6-trifluoromethyl-3,4-dihydro-2H-quinoline-1-carboxylic acid ethyl ester
   [2R,4S]4-(3,5-bistrifluoromethylbenzylamino)-2-ethyl-6-trifluoromethyl-3,4-dihydro-2H-quinoline-1-carboxylic acid propyl ester;
   [2R,4S]4-(3,5-bistrifluoromethylbenzylamino)-2-ethyl-6-trifluoromethyl-3,4-dihydro-2H-quinoline-1-carboxylic acid isopropyl ester;
   [2S,4S]4-(3,5-bistrifluoromethylbenzylamino)-2-cyclopropyl-6-trifluoromethyl-3,4-dihydro-2H-quinoline-1-carboxylic acid ethyl ester;
   [2S,4S]4-(3,5-bistrifluoromethylbenzylamino)-2-cyclopropyl-6-trifluoromethyl-3,4-dihydro-2H-quinoline-1-carboxylic acid propyl ester; and [2S,4S]4-(3,5-bistrifluoromethylbenzylamino)-2-cycloprapyl-6-trifluoromethyl-3,4-dihydro-2H-quinoline-1-carboxylic acid isopropyl ester, a prodrug thereof or a pharmaceutically acceptable salt of the compound or the prodrug.
[58] The method of [1], [3] or [6], wherein the compound having a CETP inhibitory activity is a compound of the formula (IV): 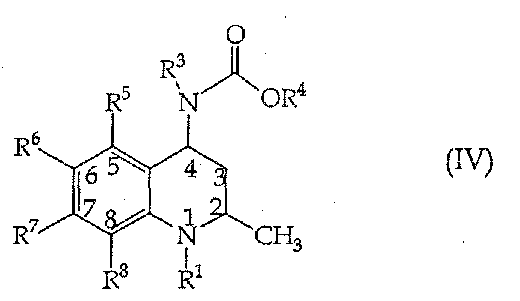
   wherein R¹ is hydrogen, Y, W-X, W-Y;
   wherein W is a carbonyl, thiocarbonyl, sulfinyl or sulfonyl;
   X is -O-Y, -S-Y, -N(H)-Y or -N-(Y)₂;
   Y for each occurrence is independently Z or a fully saturated, partially unsaturated or fully unsaturated 1- to 10-membered straight chain or branched carbon chain wherein the carbons other than the connecting carbon, may be replaced with 1 or 2 heteroatoms selected independently from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is optionally mono- or di-substituted by oxo, the nitrogen is optionally mono- or di-substituted by oxo, and the carbon chain is optionally mono-substituted by Z; wherein Z is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein Z substituent is optionally mono-, di- or tri-substituted independently by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein (C₁-C₆)alkyl substituent is optionally mono-, di- or tri-substituted independently by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl is optionally substituted by 1 to 9 fluorines;
   R³ is hydrogen or Q;
   wherein Q is a fully saturated, partially unsaturated or fully unsaturated 1- to 6-membered straight chain or branched carbon chain wherein the carbons other than the connecting carbon, may be replaced with one heteroatom independently selected from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is optionally mono- or di-substituted by oxo, the nitrogen is optionally mono- or di-substituted by oxo, the carbon chain is optionally mono-substituted by V;
   wherein V is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
   wherein the V substituent is optionally mono-, di-, tri- or tetra-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆ alkyloxycarbonyl, mono-N- or di-N,N-(C₁-C₆)alkylamino wherein the (C₁-C₆)alkyl or (C₂-C₆)alkenyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino or the (C₁-C₆)alkyl or (C₂-C₆)alkenyl is optionally substituted by 1 to 9 fluorines;
   R⁴ is Q¹ or V¹; wherein Q¹ is a fully saturated, partially unsaturated, or fully unsaturated 1- to 6-membered straight chain or branched carbon chain wherein the carbons other than the connecting carbon, may be replaced with one heteroatom selected independently from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is mono- or di-substituted by oxo, the nitrogen is optionally mono- or di-substituted by oxo, the carbon chain is optionally mono-substituted by V¹; wherein V¹ is a partially saturated, fully saturated or fully unsaturated 3- to 6-membered ring optionally having 1 or 2 heteroatoms selected independently from oxygen, sulfur and nitrogen; the V¹ substituent is optionally mono-, di-, tri- or tetra-substituted independently by halo, (C₁-C₆)alkyl, (C₁-C₆)alkoxy, amino, nitro, cyano, (C₁-C₆)alkyloxycarbonyl, mono-N- or di-N,N-(C₁-C₆)alkylamino wherein the (C₁-C₆)alkyl substituent is optionally mono-substituted by oxo, the (C₁-C₆)alkyl substituent optionally has 1 to 9 fluorines; either R³ should contain V or R⁴ should contain V¹; and
   R⁵ and R⁶, or R⁶ and R⁷, and/or R⁷ and R⁸ are linked to form at least one partially saturated or fully unsaturated 4- to 8-membered ring optionally having 1 to 3 heteroatoms independently selected from nitrogen, sulfur and oxygen; the rings (one or more) formed by R⁵ and R⁶, or R⁶ and R⁷, and/or R⁷ and R⁸ are optionally mono-, di- or tri-substituted independently by halo, (C₁-C₆)alkyl, (C₁-C₆)alkylsulfonyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent optionally has 1 to 9 fluorines; provided that when the R⁵, R⁶, R⁷ and/or R⁸ do not form at least one ring, they are each independently hydrogen, halo, (C₁-C₆)alkoxy or (C₁-C₆)alkyl, wherein the (C₁-C₆)alkyl optionally has 1 to 9 fluorines, a prodrug thereof or a pharmaceutically acceptable salt of the compound or the prodrug.
[59] The inhibitor of [2], [4] or [7], wherein the compound having a CETP inhibitory activity is a compound of the formula (IV) of [58], a prodrug thereof or a pharmaceutically acceptable salt of the compound or the prodrug.
[60] The inhibitor of [5] or [8], which is a compound of the formula (IV) of [58], a prodrug thereof or a pharmaceutically acceptable salt of the compound or the prodrug.
[61] The method of [58], wherein the compound of the formula (IV) is a compound selected from a group consisting of
   [2R,4S]4-[(3,5-bis-trifluoromethyl-benzyl)-methoxycarbonyl-amino]-2-methyl-2,3,4,6,7,8-hexahydro-cyclopenta[g]quinoline-1-carboxylic acid ethyl ester;
   [6R,8S]8-[(3,5-bis-trifluoromethyl-benzyl)-methoxycarbonyl-amino]-6-methyl-3,6,7,8-tetrahydro-1H-2-thia-5-aza-cyclopenta[b]naphthalene-5-carboxylic acid ethyl ester;
   [6R,8S]8-[(3,5-bis-trifluoromethyl-benzyl)-methoxycarbonyl-amino]-6-methyl-3,6,7,8-tetrahydro-2H-furo[2,3-g]quinoline-5-carboxylic acid ethyl ester; [2R,4S]4-[(3,5-bis-trifluoromethyl-benzyl)-methoxycarbonyl-amino]-2-methyl-3,4,6,8-tetrahydro-2H-furo[3,4-g]quinoline-1-carboxylic acid ethyl ester; and [2R,4S]4-[(3,5-bis-trifluoromethylbenzyl)-methoxycarbonyl-amino]-2-methyl-3,4,6,7,8,9-hexahydro-2H-benzo[g]quinoline-1-carboxylic acid propyl ester, a prodrug thereof or a pharmaceutically acceptable salt of the compound or the prodrug.
[62] The inhibitor of [59] or [60], wherein the compound of the formula (IV) is a compound selected from a group consisting of [2R,4S]4-[(3,5-bis-trifluoromethyl-benzyl)-methoxycarbonyl-amino]-2-methyl-2,3,4,6,7,8-hexahydro-cyclopenta[g]quinoline-1-carboxylic acid ethyl ester; [6R,8S]8-[(3,5-bis-trifluoromethyl-benzyl)-methoxycarbonyl-amino]-6-methyl-3,6,7,8-tetrahydro-1H-2-thia-5-aza-cyclopenta[b]naphthalene-5-carboxylic acid ethyl ester; [6R,8S]8-[(3,5-bis-trifluoromethyl-benzyl)-methoxycarbonyl-amino]-6-methyl-3,6,7,8-tetrahydro-2H-furo[2,3-g]quinoline-5-carboxylic acid ethyl ester; [2R,4S]4-[(3,5-bis-trifluoromethyl-benzyl)-methoxycarbonyl-amino]-2-methyl-3,4,6,8-tetrahydro-2H-furo[3,4-g]quinoline-1-carboxylic acid ethyl ester; and [2R,4S]4-[(3,5-bis-trifluoromethylbenzyl)-methoxycarbonyl-amino]-2-methyl-3,4,6,7,8,9-hexahydro-2H-benzo[g]quinoline-1-carboxylic acid propyl ester, a prodrug thereof or a pharmaceutically acceptable salt of the compound or the prodrug.
[63] The method of [1], [3] or [6], wherein the compound having a CETP inhibitory activity is a compound of the formula (V) 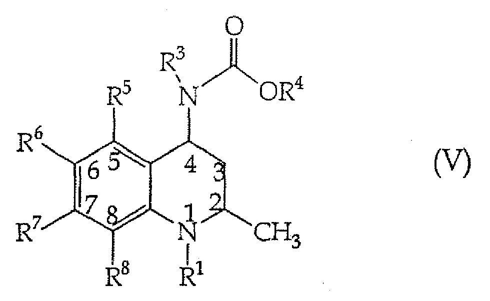 wherein
   - R¹: is a hydrogen, Y, W-X or W-Y;
   wherein W is carbonyl, thiocarbonyl, sulfinyl or sulfonyl;
   X is -O-Y, -S-Y, -N(H)-Y or -N-(Y)₂;
   Y for each bond is independently Z or a fully saturated, partially unsaturated or fully unsaturated 1- to 10-membered straight chain or branched carbon chain, wherein the carbons other than a connecting carbons may be replaced with 1 or 2 heteroatoms selected from oxygen, sulfur and nitrogen as desired, the aforementioned carbon is independently mono-substituted, di-substituted or tri-substituted by halogen as desired, the aforementioned carbon is mono-substituted by halogen, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted by Z as desired; wherein Z is a partially saturated, fully saturated or fully unsaturated 3- to 8-membered ring having 1 to 4 heteroatoms independently selected from oxygen, sulfur and nitrogen as desired, or a bicyclic ring consisting of two fused partially saturated, fully saturated or fully unsaturated 3- to 6-membered rings, taken independently, to have 1 to 4 heteroatoms independently selected from nitrogen, sulfur and oxygen as desired; the aforementioned Z substituent is independently mono-substituted, di-substituted or tri-substituted as desired by halogen, (C₂-C₆)alkenyl, (C₁-C₆)alkyl, hydroxy, (C₁-C₆)alkoxy, (C₁-C₄)alkylthio, amino, nitro, cyano, oxo, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent is independently mono-substituted, di-substituted or tri-substituted as desired by halogen, hydroxy, (C₁-C₆)alkoxy, (C₁-C₄)alkylthio, amino, nitro, cyano, oxo, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent is further substituted by 1 to 9 fluorines as desired;
   - R³: is a hydrogen or Q; wherein Q is a fully saturated, partially unsaturated or fully unsaturated 1- to 6-membered straight chain or branched carbon chain wherein the carbons, other than a connecting carbons may be replaced with one heteroatom selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted by V as desired; wherein V is a partially saturated, fully saturated or fully unsaturated 3- to 8-membered ring having 1 to 4 heteroatoms independently selected from oxygen, sulfur and nitrogen as desired, or a bicyclic ring consisting of two fused partially saturated, fully saturated or fully unsaturated 3- to 6-membered rings, taken independently, to have 1 to 4 heteroatoms independently selected from nitrogen, sulfur and oxygen as desired; the aforementioned V substituent is independently mono-substituted, di-substituted, tri-substituted or tetra-substituted as desired by halogen, (C₁-C₆)alkyl, (C₂-C₆)alkenyl, hydroxy, (C₁-C₆)alkoxy, (C₁-C₄)alkylthio, amino, nitro, cyano, oxo, carbamoyl, mono-N-(C₁-C₆)alkylcarbamoyl, di-N,N-(C₁-C₆)alkylcarbamoyl, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent or (C₂-C₆)alkenyl substituent is independently mono-substituted, di-substituted or tri-substituted as desired by hydroxy, (C₁-C₆)alkoxy, (C₁-C₄)alkylthio, amino, nitro, cyano, oxo, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent or (C₂-C₆)alkenyl substituent is further substituted by 1 to 9 fluorines as desired;
   - R⁴: is Q¹ or V¹; wherein Q¹ is a fully saturated, partially unsaturated or fully unsaturated 1- to 6-membered straight chain or branched carbon chain, wherein the carbons other than a connecting carbons may be replaced with one heteroatom selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted by V¹ as desired; wherein V¹ is a partially saturated, fully saturated or fully unsaturated 3- to 6-membered ring having, as desired, 1 or 2 heteroatoms independently selected from oxygen, sulfur and nitrogen; the aforementioned V¹ substituent is independently mono-substituted, di-substituted, tri-substituted or tetra-substituted as desired by halogen, (C₁-C₆alkyl, (C₁-C₆)alkoxy, amino, nitro, cyano, (C₁-C₆)alkyloxycarbonyl, mono-N-(C₁-C₆)alkylamino or di-N,N-(C₁-C₆)alkylamino, the aforementioned (C₁-C₆)alkyl substituent is mono-substituted as desired by oxo, and the aforementioned (C₁-C₆) alkyl substituent is further substituted by 1 to 9 fluorines as desired; R³ should contain V or R⁴ should contain V¹; and
   - R⁵, R⁶, R⁷, and R⁸: are each independently hydrogen, hydroxy or oxy, wherein the aforementioned oxy is substituted by T or a partially saturated, fully saturated or fully unsaturated 1- to 12-membered straight chain or branched carbon chain, the carbons other than a connecting carbons may be replaced with 1 or 2 heteroatoms selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted as desired by T; wherein T is a partially saturated, fully saturated or fully unsaturated 3- to 8-membered ring having 1 to 4 heteroatoms independently selected from oxygen, sulfur and nitrogen as desired, or a bicyclic ring consisting of two fused partially saturated, fully saturated or fully unsaturated 3- to 6-membered rings, taken independently, to have 1 to 4 heteroatoms independently selected from nitrogen, sulfur and oxygen as desired; the aforementioned T substituent is independently mono-substituted, di-substituted or tri-substituted as desired by halogen, (C₁-C₆)alky, (C₂-C₆)alkenyl, hydroxy, (C₁-C₆)alkoxy, (C₁-₄)alkylthio, amino, nitro, cyano, oxo, carboxy, (C₁-C₆)alkyloxycarbonyl, mono-N-(C₁-C₆)alkylamino or di-N,N- (C₁-C₆) al kyl amino, the aforementioned (C₁-C₆) alkyl substituent is independently mono-substituted, di-substituted or tri-substituted as desired by hydroxy, (C₁-C₆)alkoxy, (C₁-C₄)alkylthio, amino, nitro, cyano, oxo, carboxy, (C₁-C₆)alkyloxycarbonyl, mono-N-(C₁-C₆)alkylamino or di-N,N-(C₁-C₆)alkylamino, and the aforementioned (C₁-C₆)alkyl substituent is further substituted as desired by 1 to 9 fluorines,
   a prodrug thereof, a pharmaceutically acceptable salt of the compound or a pharmaceutically acceptable salt of the prodrug.
[64] The inhibitor of [2], [4] or [7], wherein the compound having a CETP inhibitory activity is a compound of the formula (V) of [63], a prodrug of the compound, a pharmaceutically acceptable salt of the compound or a pharmaceutically acceptable salt of the prodrug.
[65] The inhibitor of [5] or [8], which is a compound of the formula (V) of [63], a prodrug of the compound, a pharmaceutically acceptable salt of the compound or a pharmaceutically acceptable salt of the prodrug.
[66] The method of [63], wherein the compound of the formula (V) is a compound selected from a group consisting of [2R,4S]4-[(3,5-dichloro-benzyl)-methoxycarbonyl-amino]-6,7-dimethaxy-2-methyl-3,4-dihydro-2H-quinoline-1-carboxylic acid ethyl ester; [2R,4S]4-[(3,5-dinitro-benzyl)-methoxycarbonyl-amino]-6,7-dimethoxy-2-methyl-3,4-dihydro-2H-quinoline-1-caxboxylic acid ethyl ester; [2R,4S]4-[(2,6-dichloro-pyridin-4-ylmethyl)-methoxycarbonyl-amino]-6,7-dimethoxy-2-methyl-3,4-dihydro-2H-quinoline-1-carboxylic acid ethyl ester; [2R,4S]4-[(3,5-bis-trifluoromethylbenzyl)-methoxycarbonyl-amino]-6,7-dimethoxy-2-methyl-3,4-dihydro-2H-quinoine-1-carboxylic acid ethyl ester; [2R,4S]4-[(3,5-bis-trifluoromethyl-benzyl)-methoxycarbonyl-amino]-6-methoxy-2-methyl-3,4-dihydro-2H-quinoline-1-carboxylic acid ethyl ester; [2R,4S]4-[(3,5-bis-trifluoromethyl-benzyl)-methoxycarbonyl-amino]-7-methoxy-2-methyl-3,4-dihydro-2H-quinoline-1-carboxylic acid ethyl ester; [2R,4S]4-[(3,5-bis-trifluoromethyl-benzyl)-methoxycarbonyl-amino]-6,7-dimethoxy-2-methyl-3,4-dihydro-2H-quinoline-1-carboxylic acid isopropyl ester; [2R,4S]4-[(3,5-bis-trifluoromethyl-benzyl)-ethoxycarbonyl-amino]-6,7-dimethoxy-2-methyl-3,4-dihydro-2H-quinoline-1-carboxylic acid ethyl ester; [2R,4S]4-[(3,5-bis-trifluoromethyl-benzyl)-methoxycarbonyl-amino]-6,7-dimethoxy-2-methyl-3,4-dihydro-2H-quinoline-1-carboxylic acid 2,2,2-trifluoro-ethyl ester; [2R,4S]4-[(3,5-bis-trifluoromethyl-benzyl)-methoxycarbonyl-amino]-6,7-dimethoxy-2-methyl-3,4-dihydro-2H-quinoline-1-carboxylic acid propyl ester; [2R,4S]4-[(3,5-bis-trifluoromethylbenzyl)-methoxycarbonyl-amino]-6,7-dimethoxy-2-methyl-3,4-dihydro-2H-quinoline-1-carboxylic acid tert-butyl ester; and [2R,4S]4-[(3,5-bis-trifluoromethyl-benzyl)-methaxycarbonyl-amino]-2-methyl-6-trifluoromethoxy-3,4-dihydro-2H-quinoline-1-carboxylic acid ethyl ester, a prodrug thereof, a pharmaceutically acceptable salt thereof or a pharmaceutically acceptable salt of the prodrug.
[67] The inhibitor of [64] or [65], wherein the compound of the formula (V) is a compound selected from a group consisting of
   [2R,4S]4-[(3,5-dichloro-benzyl)-methoxycarbonyl-amino]-6,7-dimethoxy-2-methyl-3,4-dihydro-2H-quinoline-1-carboxylic acid ethyl ester; [2R,4S]4-[(3,5-dinitro-benzyl)-methoxycarbonyl-amino]-6,7-dimethoxy-2-methyl-3,4-dihydro-2H-quinoline-1-carboxylic acid ethyl ester; [2R,4S]4-[(2,6-dichloro-pyridin-4-ylmethyl)-methoxycarbonyl-amino]-6,7-dimethoxy-2-methyl-3,4-dihydro-2H-quinoline-1-carboxylic acid ethyl ester; [2R,4S]4-[(3,5-bis-trifluoromethylbenzyl)-methoxycarbonyl-amino]-6,7-dimethoxy-2-methyl-3,4-dihydro-2H-quinoline-1-carboxylic acid ethyl ester;
   [2R,4S]4-[(3,5-bis-trifluoromethyl-benzyl)-methoxycarbonyl-amino]-6-methoxy-2-methyl-3,4-dihydro-2H-quinoline-1-carboxylic acid ethyl ester; [2R,4S]4-[(3,5-bis-trifluoromethyl-benzyl)-methoxycarbonyl-amino]-7-methoxy-2-methyl-3,4-dihydro-2H-quinoline-1-carboxylic acid ethyl ester; [2R,4S]4-[(3,5-bis-trifluoromethyl-benzyl)-methoxycarbonyl-amino]-6,7-dimethoxy-2-methyl-3,4-dihydro-2H-quinoline-1-carboxylic acid isopropyl ester; [2R,4S]4-[(3,5-bis-trifluoromethyl-benzyl)-ethoxycarbonyl-amino]-6,7-dimethoxy-2-methyl-3,4-dihydro-2H-quinoline-1-carboxylic acid ethyl ester; [2R,4S]4-[(3,5-bis-trifluoromethyl-benzyl)-methoxycarbonyl-amino]-6,7-dimethoxy-2-methyl-3,4-dihydro-2H-quinoline-1-carboxylic acid 2,2,2-trifluoro-ethyl ester; [2R,4S]4-[(3,5-bis-trifluoromethyl-benzyl)-methoxycarbonyl-amino]-6,7-dimethoxy-2-methyl-3,4-dihydro-2H-quinoline-1-carboxylic acid propyl ester; [2R,4S]4-[(3,5-bis-trifluoromethyl-benzyl)-methoxycarbonyl-amino]-6,7-dimethoxy-2-methyl-3,4-dihydro-2H-quinoline-1-carbaxylic acid tert-butyl ester; and [2R,4S]4-[(3,5-bis-trifluoromethyl-benzyl)-methoxycarbonyl-amino]-2-methyl-6-trifluoromethoxy-3,4-dihydro-2H-quinoline-1-carboxylic acid ethyl ester, a prodrug thereof, a pharmaceutically acceptable salt thereof or a pharmaceutically acceptable salt of the prodrug.
[68] The method of [1], [3] or [6], wherein the compound having a CETP inhibitory activity is a compound having the formula (XII): 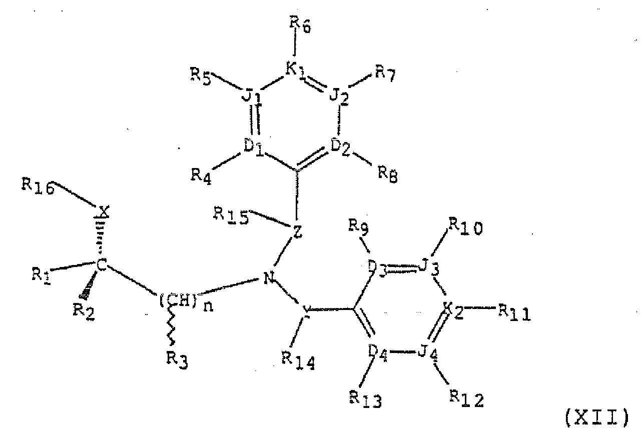
   or a pharmaceutically acceptable salt thereof,
      wherein
   n is an integer selected from 1 through 4:
   X is oxy;
   R¹ is selected from the group consisting of haloalkyl, haloalkenyl, haloalkoxymethyl, and haloalkenyloxymethyl with the proviso that R¹ has a higher Cahn-Ingold-Prelog stereochemical system ranking than both R² and (CHR₃)ₙ-N(A)Q wherein A is the formula (XIII) and Q is the formula (XIV); 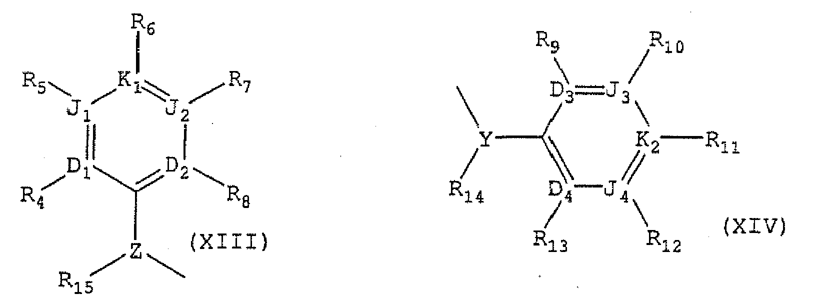
   R₁₆ is selected from the group consisting of hydrido; alkyl; acyl; aroyl; heteroaroyl; trialkylsilyl; and a spacer selected from the group consisting of a covalent single bond and a linear spacer moiety having a chain length of 1 to 4 atoms linked to the point of bonding of any aromatic substituent selected from the group consisting of R₄, R₈, R₉ and R₁₃ to form a heterocyclyl ring having from 5 through 10 continuous members:
   D₁, D₂, J₁, J₂ and K₁ are independently selected from the group consisting of C, N, O, S and a covalent bond with the proviso that no more than one of D₁, D₂, J₁, J₂ and K₁ is a covalent bond; no more than one of D₁, D₂, J₁, J₂ and K₁ is O; no more than one of D₁, D₂, J₁, J₂ and K₁ is S; one of D₁, D₂, J₁, J₂ and K₁ must be a covalent bond when two of D₁, D₂, J₁. J₂ and K₁ are O and S; and no more than four of D₁ , D₂, J₁, J₂ and K₁ are N;
   D₃, D₄, J₃, J₄ and K₂ are independently selected from the group consisting of C, N, O, S and a covalent bond with the proviso that no more than one of D₃, D₄, J₃, J₄ and K₂ is a covalent bond; no more than one of D₃, D₄, J₃, J₄ and K₂ is O; no more than one of D₃, D₄, J₃, J₄ and K₂ is S; no more than two of D₃, D₄, J₃, J₄ and K₂ are O and S; one of D₃, D₄, J₃, J₄ and K₂ must be a covalent bond when two of D₃, D₄, J₃, J₄ and K₂ are O and S; and no more than four of D₃, D₄, J₃, J₄ and K₂ are N;
   R₂ is selected from the group consisting of hydrido, aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl with the proviso that R₂ has a lower Cahn-Ingold-Prelog system ranking than both R₁ and (CHR₃)ₙ-N(A)Q;
   R₃ is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl with the provisos that (CHR₃)ₙ-N(A)Q has a lower Cahn Ingold-Prelog stereochemical system ranking than R₁ and a higher Cahn-Ingold-Prelog stereochemical system ranking than R₂;
   Y is selected from a group consisting of a covalent single bond, (C(R₁₄)₂)_{q} wherein q is an integer selected from 1 and 2 and (CH (R₁₄))_{g}-W-(CH(R₁₄₎)ₚ wherein g and p are integers independently selected from 0 and 1;
   R₁₄ is selected from the group consisting of hydrido, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl; Z is selected from the group consisting of a covalent single bond, (C(R₁₅)₂)_{q} wherein q is an integer selected from 1 and 2, and (CH(R₁₅))ⱼ-W-(CH(R₁₅))ₖ wherein j and k are integers independently selected from 0 and 1: W is selected from the group consisting of O, C(O), C(S), C(C)N(R₁₄), C(S)N(R₁₄), (R₁₄)NC(O), (R₁₄)NC(S), S, S(O), S (O)₂, S(O)₂N(R₁₄), (R₁₄)NC(O)₂, and N(R₁₄) with the proviso that R₁₄ is other than cyano;
   R₁₅ is selected from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl; R₄, R₅, R₆, R₇, R₈, R₉, R₁₀, R₁₁, R₁₂, and R₁₃ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl-amidosulfonyl, dialkyl-amidosulfonyl, monoarylamidosulfonyl, arylsulfonamido, diarylamidosulfonyl, monoalkyl-monoaryl-amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, heteroaralkyl, arylalkenyl, heteroarylalkenyl, carboxyalkyl, carboalkoxy, alkoxycarboxamido, alkylaminodocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that R₄, R₅, R₆, R₇, R₈, R₉, R₁₀, R₁₁, R₁₂, and R₁₃ are each independently selected to maintain the tetravalent nature of carbon, trivalent nature of nitrogen, the divalent nature of sulfur, and the divalent nature of oxygen;
   R₄ and R₅, R₅ and R₆, R₆ and R₇, R₇ and R₈, R₉ and R₁₀, R₁₀ and R₁₁, R₁₁ and R₁₂, and R₁₂ and R₁₃ are independently selected to form spacer pairs wherein a spacer pair is taken together to form a linear moiety having from 3 through 6 continuous atoms connecting the points of bonding of said spacer pair members to form a ring selected from the group consisting of a cycloalkenyl ring having 5 through 8 continuous members, a partially saturated heterocyclyl ring having 5 through 8 continuous members, a heteroaryl ring having 5 through 6 continuous members, and an aryl with the provisos that no more than one of the group consisting of spacer pairs R₄ and R₅, R₅ and R₆, R₆ and R₇, and R₇ and R₈, is used at the same time and that no more than one of the group consisting of spacer pairs R₉ and R₁₀, R₁₀ and
   R₁₁, R₁₁ and R₁₂, and R₁₂ and R₁₃ is used at the same time; R₄ and R₉, R₄ and R₁₃, R₈ and R₉, and R₈ and R₁₃ are independently selected to form a spacer pair wherein said spacer pair is taken together to form a linear moiety wherein said linear moiety forms a ring selected from the group consisting of a partially saturated heterocyclyl ring having from 5 through 8 continuous members and a heteroaryl ring having from 5 through 6 continuous members with the proviso that no more than one of the group consisting of spacer pairs R₄ and R₉, R₄ and R₁₃, R₈ and R₉, and R₈ and R₁₃ is used at the same time.
[69] The inhibitor of [2], [4] or [7], wherein the compound having a CETP inhibitory activity is a compound of the formula (XII) of [68] or a pharmaceutically acceptable salt thereof.
[70] The inhibitor of [5] or [8], which is a compound of the formula (XII) of [68] or a pharmaceutically acceptable salt thereof.
[71] The method of [68], wherein the compound of the formula (XII) is a compound selected from the group consisting of
   (2R)-3-[[3-(3-trifluoromethoxyphenoxy)phenyl][[3-(1,1,2,2-tetrafluoroethoxy)phenyl]methyl]amino]-1,1,1-trifluoro-2-propanol;
   (2R)-3-[[3-(3-isopropylphenoxy)phenyl][[3-(1,1,2,2-tetrafluoroethoxy)phenyl]methyl]amino]-1,1,1-trifluoro-2-propanol;
   (2R)-3-[[3-(3-cyclopropylphenoxy)phenyl][[3-(1,1,2,2-tetrafluoroethoxy)phenyl]methyl]amino]-1,1,1-trifluoro-2-propanol;
   (2R)-3-[[3-(3-(2-furyl)phenoxy)phenyl][[3-(1,1,2,2-tetrafluoroethoxy)phenyl]methyl]amino]-1,1,1-trifluoro-2-propanol;
   (2R)-3-[[3-(2,3-dichlorophenoxy)phenyl][[3-(1,1,2,2-tetrafluoroethoxy)phenyl]methyl]amino]-1,1,1-trifluoro-2-propanol;
   (2R)-3-[[3-(4-fluorophenoxy)phenyl][[3-(1,1,2,2-tetrafluoroethoxy)phenyl]methyl]amino]-1,1,1-trifluoro-2-propanol;
   (2R)-3-[[3-(4-methylphenoxy)phenyl][[3-(1,1,2,2-tetrafluoroethoxy)phenyl]methyl]amino]-1,1,1-trifluoro-2-propanol;
   (2R)-3-[[3-(2-fluoro-5-bromophenoxy)phenyl][[3-(1,1,2,2-tetrafluoroethoxy)phenyl]methyl]amino]-1,1,1-trifluoro-2-propanol;
   (2R)-3-[[3-(4-chloro-3-ethylphenoxy)phenyl][[3-(1,1,2,2-tetrafluoroethoxy)phenylJmethyl]amino]-1,1,1-trifluoro-2-propanol;
   (2R)-3-[[3-[3-(1,1,2,2-tetrafluoroethoxy)phenoxy]phenyl] [[3-(1,1,2,2-tetrafluoroethoxy)phenyl]methyl]amino]-1,1,1-trifluoro-2-propanol;
   (2R)-3-[[3-[3-(pentafluoroethyl)phenoxy]phenyl][[3-(1,1,2,2-tetrafluoroethoxy)phenyl]methyl]amino]-1,-,1-trifluoro-2-propanol;
   (2R)-3-[[3-(3,5-dimethylphenoxy)phenyl][[3-(1,1,2,2-tetrafluoroethoxy)phenyl]methyl]amino)-1,1,1-trifluoro-2-propanol;
   (2R)-3-[[3-(3-ethylphenoxy)phenyl][[3-(1,1,2,2-tetrafluoroethoxy)phenyl]methyl]amino]-1,1,1-trifluoro-2-propanol;
   (2R)-3-[[3-(3-t-butylphenoxy)phenyl][[3-(1,1,2,2-tetrafluoroethoxy)phenyl]methyl]amino]-1,1,1-trifluoro-2-propanol;
   (2R)-3-[[3-(3-methylphenoxy)phenyl][[3-(1,1,2,2-tetrafluoroethoxy)phenyl]methyl]amino]-1,1,1-trifluoro-2-propanol;
   (2R)-3-[[3-(5,6,7,8-tetrahydro-2-naphthoxy)phenyl][[3-(1,1,2,2-tetrafluoroethoxy)phenyl]methyl]amino]-1,1,1-trifluoro-2-propanol;
   (2R)-3-[[3-(phenoxy)phenyl][[3-(1,1,2,2-tetrafluoroethoxy)phenyl]methyl]amino]-1,1,1-trifluoro-2-propanol;
   (2R)-3-[[3-[3-(N,N-dimethylamino)phenoxy]phenyl][[3-(1,1,2, 2-tetrafluoroethoxy)phenyl]methyl]amino]-1,1,1-trifluoro-2-propanol;
   (2R)-3-[[[3-(1,1,2,2-tetrafluoroethoxy)phenyl]methyl][3-[[3-(trifluoromethoxy)phenyl]methoxy]phenyl]amino]-1,1,1,-trifluoro-2-propanol;
   (2R)-3-[[[3-(1,1,2,2-tetrafluoroethoxy)phenyl]methyl][3-[[3-(trifluoromethyl)phenyl]methoxy]phenyl]amino]-1,1,1-trifluoro-2-propanol;
   (2R)-3-[[[3-(1,1,2,2-tetrafluoroethoxy)phenyl]methyl][3-[[3,5-dimethylphenyl]methoxy]phenyl]amino]-1,1,1-trifluoro-2-propanol;
   (2R)-3-[[[3-(1,1,2,2-tetrafluoroethoxy)phenyl]methyl][3-[[3-(trifluoromethylthio)phenyl]methoxy]phenyl]amino]-1,1,1-trifluoro-2-propanol;
   (2R)-3-[[[3-(1,1,2,2-tetrafluoroethoxy)phenyl]methyl][3-[[3,5-difluorophenyl]methoxy]phenyl]amino]-1,1,1-trifluoro-2-propanol;
   3-[[[3-(1,1,2,2-tetrafluoroethoxy)phenyl]methyl](3-[cyclohexylmethoxy]phenyl]amino]-1,1,1-trifluoro-2-propanol;
   3-[[3-(2-difluoromethoxy)4-pyridyloxy)phenyl][[3-(1,1,2,2-tetrafluoroethoxy)phenyl]methyl]amino]-1,1,1-trifluoro-2-propanol;
   (2R)-3-[[3-(2-trifluoromethyl-4-pyridyloxy)phenyl][[3-(1,1,2,2-tetrafluoroethoxy)phenyl]methyl]amino]-1,1,1-trifluoro-2-propanol;
   (2R)-3-[[3-(3-difluoromethoxyphenoxy)phenyl][[3-(1,1,2,2-tetrafluoroethoxy)phenyl]methyl]amino]-1,1,1-trifluoro-2-propanol;
   (2R)-3-[[[3-(3-trifluoromethylthio)phenoxy]phenyl][[3-(1,1,2,2-tetrafluoroethoxy)phenyl]methyl]amino]-1,1,1-trifluoro-2-propanol;
   (2R)-3-[[3-(4-chloro-3-trifluoromethylphenoxy)phenyl][[3-(1,1,2,2-tetrafluoroethoxy)phenyl]methyl]amino]-1,1,1-tri fluoro-2-propanol;
   (2R)-3-[[3-(3-trifluoromethylphenoxy)phenyl][[3-(pentafluoroethyl)phenyl]methyl]amino]-1,1,1-trifluoro-2-propanol;
   (2R)-3-[[3-(3-isopropylphenoxy)phenyl][[3-(pentafluoroethyl)phenyl]methyl]amino]-1,1,1-trifluoro-2-propanol;
   (2R)-3-[[3-(3-cyclopropylphenoxy)phenyl][[3-(pentafluoroethyl)phenyl]methyl]amino]-1,1,1-trifluoro-2-propanol;
   (2R)-3-[[3-(3-(2-furyl)phenoxy)phenyl][[3-(pentafluoroethyl)phenyl]methyl]amino]-1,1,1-trifluoro-2-propanol;
   (2R)-3-[[3-(2,3-dichlorophenoxy)phenyl][[3-(pentafluoroethyl)phenyl]methyl]amino]-1,1,1-trifluoro-2-propanol;
   (2R)-3-[[3-(4-fluorophenoxy)phenyl][[3-(pentafluoroethyl)phenyl]methyl]amino]-1,1,1-trifluoro-2-propanol;
   (2R)-3-[[3-(4-methylphenoxy)phenyl][[3-(pentafluoroethyl)phenyl]methyl]amino]-1,1,1-trifluoro-2-propanol;
   (2R)-3-[[3-(2-fluoro-5-bromophenoxy)phenyl][[3-(pentafluoroethyl)phenyl]methyl]amino]-1,1,1-trifluoro-2-propanol;
   (2R)-3-[[3-(4-chloro-3-ethylphenoxy)phenyl][[3-(pentafluoroethyl)phenyl]methyl]amino]-1,1,1-trifluoro-2-propanol;
   (2R)-3-[[3-[3-(1,1,2,2-tetrafluoroethoxy)phenoxy]phenyl] [[3-(pentafluoroethyl)phenyl]methyllaminol-1,1,1-trifluoro-2-propanol;
   (2R)-3-[[3-[3-(pentafluoroethyl)phenoxy]phenyl][[3-(pentafluoroethyl)phenyl]methyl]amino]-1,1,1-trifluoro-2-propanol;
   (2R)-3-[[3-(3,5-dimethylphenoxy)phenyl][[3-(pentafluoroethyl)phenyl]methyl]amino]-1,1,1-trifluoro-2-propanol;
   (2R)-3-[[3-(3-ethylphenoxy)phenyl][[3-(pentafluoroethyl) phenyl]methyl]amina]-1,1,1-trifuoro-3-propanol;
   (2R)-3-[[3-(3-t-butylphenoxy)phenyl][[3-(pentafluoroethyl) phenyl]methyl]amino]-1,1,1-trifluoro-2-propanol;
   (2R)-3-[[3-(3-(methylphenoxy)phenyl)[[3-(pentafluoroethyl) phenyl]methyl]mino]-1,1,1-trifluora-2-propanol;
   (2R)-3-[[3-(5,6,7,8-tetrahydro-2-naphthoxy)phenyl][[3-(pentafluoroethyl)phenyl]methyl]amino]-1,1,1-trifluoro-2-propanol;
   (2R)-3-[[3-(phenoxy)phenyl][[3-(pentafluoroethyl)phenyl]methyl]amino]-1,1,1-trifluoro-2-propanol;
   (2R)-3-[[3-[3-(N,N-dimethylamino)phenoxy]phenyl][[3-(pentafluoroethyl)phenyl]methyl]amino]-1,1,1-trifluoro-2-propanol;
   (2R)-3-[[[3-(pentafluoroethyl)phenyl]methyl][3-[[3-(trifluoromethoxy)phenyl]methoxy]phenyl]amino]-1,1,1-trifluoro-2-propanol;
   (2R)-3-[[[3-(pentafluoroethyl)phenyl]methyl][3-[[3-(trifluoromethyl)phenyl]methoxy]phenyl]amino]-1,1,1-trifluoro-2-propanol;
   (2R)-3-[[[3-(pentafluoroethyl)phenyl]methyl][3-[[3,5-dimethylphenyl]methoxy]phenyl]amino]-1,1,1-trifluoro-2-propanol;
   (2R)-3-[[[3-(pentafluoroethyl)phenyl]methyl][3-[[3-(trifluoromethylthio)phenyl]methoxy]phenyl]amino]-1,1,1-trifluoro-2-propanol;
   (2R)-3-[[[3-(pentafluoroethyl)phenyl]methyl][3-[[3,5-difluorophenyl]methoxy]phenyl]amino)-1,1,1-trifluoro-2-propanol;
   (2R)-3-[[[3-(pentafluoroethyl)phehyl]methyl][3-[cyclohexylmethoxy]phenyl]amino]-1,1,1-trifluoro-2-propanol;
   (2R)-3-[[3-(2-difluoromethoxy-4-pyridyloxy)phenyl][[3-(pentafluoroethyl)phenyl]methyl]amino]-1,1,1-trifluoro-2-propanol;
   (2R)-3-[[3-(2-trifluoromethyl-4-pyridyloxy)phenyl][[3-(pentafluoroethyl)phenyl]methyl]amino]-1,1,1-trifluoro-2-propanol;
   (2R)-3-[[3-(3-difluoromethoxyphenoxy)phenyl][[3-(pentafluoroethyl)phenyl]methyl]amino]-1,1,1-trifluoro-2-propanol;
   (2R)-3-[[[3-(3-trifluoromethylthio)phenoxy]phenyl][[3-(pentafluoroethyl)phenyl]methyl]amino]-1,1,1-trifluoro-2-propanol;
   (2R)-3-[[3-(4-chloro-3-trifluoromethylphenoxy]phenyl][[3-(pentafluoroethyl)phenyl]methyl]amino]-1,1,1-trifluoro-2-propanol;
   (2R)-3-[[3-(3-trifluoromethoxyphenoxy)phenyl][[3-(pentafluoroethyl)phenyl]methyl]amino]-1,1,1-trifluoro-2-propanol;
   (2R)-3-[[3-(3-isopropylphenoxy)phenyl][[3-(heptafluoroethyl)phenyl]methyl]amino]-1,1,1-trifluoro-2-propanol;
   (2R)-3-[[3-(3-cyclopropylphenoxy)phenyl][[3-(heptafluoroethyl)phenyl]methyl]amino]-1,1,1-trifluoro-2-propanol;
   (2R)-3-[[3-(3-cyclopropylphenoxy)phenyl][[3-(heptafluoroethyl)phenyl]methyl]amino]-1,1,1-trifluoro-2-propanol;
   (2R)-3-[[3-(3-(2-furyl)phenoxy)phenyl][[3-(heptafluoroethyl)phenyl]methyl]amino]-1,1,1-trifluoro-2-propanol;
   (2R)-3-[[3-(2,3-dichlorophenoxy)phenyl][[3-(heptafluoroethyl)phenyl]methyl]amino]-1,1,1-trifluoro-2-propanol;
   (2R)-3-[[3-(4-fluorophenoxy)phenyl][[3-(heptafluoroethyl)phenyl]methyl]amino]-1,1,1-trifluoro-2-propanol;
   (2R)-3-[[3-(4-methylphenoxy)phenyl][[3-(heptafluoroethyl)phenyl]methyl]amino]-1,1,-trifluoro-2-propanol;
   (2R)-3-[[3-(2-fluoro-5-bromophenoxy)phenyl][[3-(heptafluoroethyl)phenyl]methyl]amino]-1,1,1-trifluoro-2-propanol;
   (2R)-3-[[3-(4-chloro-3-ethylphenoxy)phenyl][[3-(heptafluoroethyl)phenyl]methyl]amino]-1,1,1-trifluoro-2-propanol;
   (2R)-3-[[3-[3-(1,1,2,2-tetrafluoroethoxy)phenoxy]phenyl][[3-(heptafluoroethyl)phenyl]methyl]amino]-1,1,1-trifluoro-2-propanol;
   (2R)-3-[[3-[3-(pentafluoroethyl)phenoxy]phenyl][[3-(heptafluoroethyl)phenyl]methyl]amino]-1,1,1-trifluoro-2-propanol;
   (2R)-3-[[3-(3,5-dimethylphenoxy)phenyl][[3-(heptafluoroethyl)phenyl]methyl]amino]-1,1,1-trifluoro-2-propanol;
   (2R)-3-[[3-(3-ethylphenoxy)phenyl][[3-(heptafluoroethyl)phenyl]methyl]amino]-1,1,1-trifluoro-2-propanol;
   (2R)-3-[[3-(3-t-butylphenoxy)phenyl][[3-(heptaflubroethyl)phenyl]methyl]amino]-1,1,1-trifluoro-2-propanol;
   (2R)-3-[[3-(3-methylphenoxy)phenyl][[3-(heptafluoroethyl)phenyl]methyl]amino]-1,1,1-trifluoro-2-propanol;
   (2R)-3-[[3-(5,6,7,8-tetrahydro-2-naphthox)phenyl][[3-(heptafluoroethyl)phenyl]methyl]amino]-1,1,1-trifluoro-2-propanol;
   (2R)-3-[[3-(phenoxy)phenyl][[3-(heptafluoroethyl)phenyl] methyl]amino]-1,1,1-trifluoro-2-propanol:
   (2R)-3-[[3-[3-(N,N-dimethylamino)phenoxy]phenyl][[3-(heptafluoroethyl)phenyl]methyl]amino]-1,1,1-trifluoro-2-propanol;
   (2R)-3-[[[3-(heptafluoropropyl)phenyl]methyl][3-[[3-(trifluoromethoxy)phenyl]methoxy]phenyl]amino]-1,1,1-trifluoro-2-propanol;
   (2R)-3-[[[3-(heptafluoropropyl)phenyl]methyl][3-[[3-(trifluoromethyl)phenyl]methoxy]phenyl]amino]-1,1,1-trifluoro-2-propanol;
   (2R)-3-[[[3-(heptafluoropropyl)phenyl]methyl][3-[[3,5-dimethylphenyl]methoxy]phenyl]amino]-1,1,1-trifluoro-2-propanol;
   (2R)-3-[[[3-(heptafluoropropyl)phenyl]methyl][3-[[3-(trifluoromethylthio)phenyl]methoxy]phenyl]amino]-1,1,1-trifluoro-2-propanol;
   (2R)-3-[[[3-(heptafluoropropyl)phenyl]methyl][3-[[3,5-difluorophenyl]methoxy]phenyl]amino]-1,1,1-trifluoro-2-propanol;
   (2R)-3-[[[3-(heptafluoropropyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl][[3-(heptafluoropropyl)phenyl]methyl]amino]-1,1,1-trifluoro-2-propanol;
   (2R)-3-[[3-(3-difluoromethoxyphenoxy)phenyl][[3-(heptafluoropropyl)phenyllmethyl]aminol-1,1,1-trifluoro-2-propanol;
   (2R)-3-[[[3-(3-trifluoromethylthio)phenoxy]phenyl][[3-(heptafluoropropyl)phenyl]methyl]amino]-1,1,1-trifluoro-2-propanol;
   (2R)-3-[[3-(4-chloro-3-trifluoromethylphenoxy)phenyl][[3-(heptafluoropropyl)phenyl]methyl]amino]-1,1,1-trifluoro-2-propanol;
   (2R)-3-[[3-(3-trifluoromethoxyphenoxy)phenyl][[2-fluoro-5-(trifluoromethyl)phenyl]methyl]amino]-1,1,1-trifluoro-2-propanol;
   (2R)-3-[[3-(3-isopropylphenoxy)phenyl][[2-fluoro-5-(trifluoromethyl)phenyl]methyl]amino]-1,1,1-trifluoro-2-propanol;
   (2R)-3-[[3-(3--cyclopropylphenoxy)phenyl][[2-fluoro-5-(trifluoromethyl)phenyl]methyl]amino]-1,1,1-trifluoro-2-propanol;
   (2R)-3-[[3-(3-(2-furyl)phennxy)phenyl)pheny][[2-fluoro-5-(trifluoromethyl)phenyl]methyl]amino]-1,1,1-trifluoro-2-propanol;
   (2R)-3-[[3-(2,3-dichloropherioxy)phenyl][[2-fluoro-5-(trifluoromethyl)phenyl]methyl]amino]-1,1,1-trifluoro-2-propanol;
   (2R)-3-[[3-(4-fluorophenoxy)phenyl][[2-fluoro-5-(trifluoromethyl)phenyl]methyl]amino]-1,1,1-trifluoro-2-propanol;
   (2R)-3-[[3-(4-methylphenoxy)phenyl][[2-fluoro-5-(trifluoromethyl)phenyl]methyl]amino]-1,1,1-trifluoro-2-propanol;
   (2R)-3-[[3-(2-fluoro-5-bromophenoxy)phenyl][[2-fluoro-5-(trifluoromethyl)phenyl]methyl]amino]-1,1,1-trifluoro-2-propanol;
   (2R)-3-[[3-(4-chloro-3-ethylphenoxy)phenyl][[2-fluoro-5-(trifluoromethyl)phenyl]methyl]amino]-1,1,1-trifluoro-2-propanol;
   (2R)-3-[[3-[3-(1,1,2,2-tetrafluoroethoxy)phenoxy]phenyl][[2-fluoro-5-(trifluoromethyl)phenyl]methyl]aminol-1,1,1-trifluoro-2-propanol;
   (2R)-3-[[3-[3-(pentafluoroethyl)phenoxy]phenyl][[2-fluoro-5-(trifluoromethyl)phenyl]methyl]amino]-1,1,1-trifluoro-2-propanol;
   (2R)-3-[[3-(3,5-dimethylphenoxy)phenyl][2-fluoro-5-(trifluoromethyl)phenyl]methyl]amino]-1,1,1-trifluoro-2-propanol;
   (2R)-3-[[3-(3-ethylphenoxy)phenyl][[2-fluoro-5-(trifluoromethyl)phenyl]methyl]amino]-1,1,1-trifluoro-2-propanol;
   (2R)-3-[[3-(3-t-butylphenoxy)phenyl][[2-fluoro-5-(trifluoromethyl)phenyl]methyl]amino]-1,1,1-trifluoro-2-propanol;
   (2R)-3-[[3-(3-methylphenoxy)phenyl][[2-fluoro-5-(trifluoromethyl)phenyl]methyl]aminol-1,1,1-trifluoro-2-propanol;
   (2R)-3-[[3-(5,6,7,8-tetrahydro-2--naphthoxy)phenyl][[2-fluoro-5-(trifluoromethyl)phenyl]methyl]amino]-1,1,1-trifluoro-2-propanol;
   (2R)-3-[[3-(phenoxy)phenyl][[2-fluoro-5-(trifluoromethyl)phenyl]methyl]amino]-1,1,1-trifluoro-2-propanol;
   (2R)-3-[[3-[3-(N,N-dimethylamino)phenoxy]phenyl][[2-fluoro-5-(trifluoromethyl)phenyl]methyl]aminol-1,1,1-trifluoro-2-propanol;
   (2R)-3-[[[2-fluoro-5-(trifluoromethyl)phenyl]methyl][3-[[3-(trifluoromethoxy)phenyl]methoxy]phenyl]amino]-1,1,1-trifluoro-2-propanol;
   (2R)-3-[[[2-fluoro-5-(trifluoromethyl)phenyl]methyl][3-[[3-(trifluoromethyl)phenyl]methoxy]phenyl]amino]-1,1,1-trifluoro-2-propanol;
   (2R)-3-[[[2-fluoro-5-(trifluoromethyl)phenyl]methyl][3-[[3,5-dimethylphenyl]methoxy]phenyl]amino]-1,1,1-trifluoro-2-propanol;
   (2R)-3-[[[2-fluoro-5-(trifluoromethyl)phenyl]methyl][3-[[3-(trifluoromethylthio)phenyl]methoxy]phenyl]amino]-1,1,1-trifluoro-2-propanol;
   (2R)-3-[[[2-fluoro-5-(trifluoromethyl)phenyl]methyl][3-[[3,5-difluorophenyl]methoxy]phenyl]amino]-1,1,1-trifluoro-2-propanol;
   (2R)-3-[[[2-fluoro-5-(trifluoromethyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl][[2-fluoro-5-(trifluoromethyl)phenyl]methyl]amino]-1,1,1-trifluoro-2-propanol;
   (2R)-3-[[3-(2-trifluoromethyl-4-pyridyloxy)phenyl][[2-fluoro-5-(trifluoromethyl)phenyl]methyl]amino]-1,1,1-trifluoro-2-propanol;
   (2R)-3-[[3-(3-difluoromethoxyphenoxy)phenyl][[2-fluoro-5-(trifluoromethyl)phenyl]methyl]amino]-1,1,1-trifluoro-2-propanol;
   (2R)-3-[[[3-(3-trifluoromethylthio)phenoxy]phenyl][[2-fluoro-5-(trifluoromethyl)phenyl]methyl]amino]-1,1,1-trifluoro-2-propanol;
   (2R)-3-[[3-(4-chloro-3-trifluoromethylphenoxy)phenyl][2-fluoro-5-(trifluoromethyl)phenyl]methyl]amino]-1,1,1-trifluoro-2-propanol;
   (2R)-3-[[3-(3-trifluoromethoxyphenoxy)phenyl][[2-fluoro-4-(trifluoromethyl)phenyl]methyl]amino]-1,1,1-trifluoro-2-propanol;
   (2R)-3-[[3-(3-isopropylphenoxy)phenyl][2-fluoro-4-(trifluoromethyl)phenyl]methyl]amino]-1,1,1-trifluoro-2-propanol;
   (2R)-3-[[3-(3-cyclopropylphenoxy)phenyl][[2-fluoro-4-(trifluoromethyl)phenyl]methyl]amino]-1,1,1-trifluoro-2-propanol;
   (2R)-3-[[3-(3-(2-furyl)phenoxy)phenyl][2-fluoro-4-(trifluoromethyl)phenyl]methyl]amino]-1,1,1-trifluoro-2-propanol;
   (2R)-3-[[3-(2,3-dichlorophenoxy)phenyl][2-fluoro-4-(trifluoromethyl)phenyl]methyl]amino]-1,1,1-trifluoro-2-propanol;
   (2R)-3-[[3-(4-fluoxophenoxy)phenxy][2-fluoro-4-(trifluoromethyl)phenyl]methy]amino]-1,1,1-trifluoro-2-propanol;
   (2R)-3-[[3-(4-methylphenoxy)phenyl][2-fluoro-4-(trifluoromethyl)phenyl]methyl]amino]-1,1,1-trifluoro-2-propanol;
   (2R)-3-[[3-(2-fluoro-5-bromophenoxy)phenyl][2-fluoro-4-(trifluoromethyl)phenyl]methyl]amino]-1,1,1-trifiuoro-2-propanol;
   (2R)-3-[[3-(4-chloro-3-ethylphenoxy)phenyl][2-fluoro-4-(trifluoromethyl)phenyl]methyl]amino]-1,1,1-trifluoro-2-propanol;
   (2R)-3-[[3-[3-(1,1,2,2-tetrafluoroethoxy)phenoxy]phenyl][2-fluoro-4-(trifluoromethyl)phenyl]methyl]aminol-1,1,1-trifluoro-2-propanol;
   (2R)-3-[[3-[3-(pentafluoroethyl)phenoxy]phenyl][2-fluoro-4-(trifluoromethyl)phenyl]methyl]amino]-1,1,1-trifluoro-2-propanol;
   (2R)-3-[[3-[3,5-dimethylphenoxy)phenyl][2-fluoro-4-(trifluoromethyl)phenyl]methyl]amino]-1,1,1-trifluoro-2-propanol;
   (2R)-3-[[3-(3-ethylphenoxy)phenyl][2-fluoro-4-(trifluoromethyl)phenyl]methyl]amino]-1,1,1-trifluoro-2-propanol;
   (2R)-3-[[3-(3-t-butylphenoxy)phenyl][2-fluoro-4-(trifluoromethyl)phenyl]methyl]amino]-1,1,1-trifluoro-2-propanol;
   (2R)-3-[[3-(3-methylphenoxy)phenyl][2-fluoro-4-(trifluoromethyl)phenyl]methyl]amino]-1,1,1-trifluoro-2-propanol;
   (2R)-3-[[3-(5,6,7,8-tetrahydro-2-naphthoxy)phenyl][2-fluoro-4-(trifluoromethyl)phenyl]methyl]amino]-1,1,1-trifluoro-2-propanol;
   (2R)-3-[[3-(phenoxy)phenyl][2-fluoro-4-(trifluoromethyl) phenyl]methyl]amino]-1,1,1-trifluoro-2-propanol; (2R)-3-[[3-[3-(N,N-dimethylamino)phenoxy]phenyl][2-fluoro-4-(trifluoromethyl)phenyl]methyl]amino]-1,1,1-trifluoro-2-propanol;
   (2R)-3-[[[2-fluoro-4-(trifluoromethyl)phenyl]methyl][3-[[3-(trifluoromethoxy)phenyl]methoxy]phenyl]amino]-1,1,1-trifluoro-2-propanol;
   (2R)-3-[[[2-fluoro-4-(trifluoromethyl)phenyl]methyl][3-[[3,5-dimethylphenyl]methoxy]phenyl]amino]-1,1,1-trifluoro-2-propanol;
   (2R)-3-[[[2-fluoro-4-(trifluoromethyl)phenyl]methyl][3-[[3-(trifluoromethylthio)phenyl]methoxy]phenyl]amino]-1,1,1-trifluoro-2-propanol;
   (2R)-3-[[[2-fluoro-4-(trifluoromethyl)phenyl]methyl][3-[[3,5-difluorophenyl]methoxy]phenyl]amino]-1,1,1-trifluoro-2-propanol;
   (2R)-3-[[[2-fluoro-4-(trifluoromethyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl][2-fluoro-4-(trifluoromethyl)phenyl]methyl]amino]-1,1,1-trifluoro-2-propanol;
   (2R)-3-[[3-(2-trifluoromethyl-4-pyridyloxy)phenyl][2-fluoro-4-(trifluoromethyl)phenyl]methyl]amino]-1,1,1-trifluoro-2-propanol;
   (2R)-3-[[3-(3-difluoromethoxyphenoxy)phenyl][[2-fluoro-4-(trifluoromethyl)phenyl]methyl]amino]-1,1,1-trifluoro-2-propanol;
   (2R)-3-[[[3-(3-trifluoromethylthio)phenoxy]phenyl][2-fluoro-4-(trifluoromethyl)phenyl]methyl]amino]-1,1,1-trifluoro-2-propanol; and
   (2R)-3-[[3-(4-chloro-3-trifluoromethylphenoxy)phenyl][2-fluoro-4-(trifluoromethyl)phenyl]methyl]amino]-1,1,1-trifluoro-2-propanol, or a pharmaceutically acceptable salt thereof.
[72] The inhibitor of [69] or [70], wherein the compound of the formula (XII) is a compound selected from the group consisting of
   (2R)-3-[[3-(3-trifluoromethoxyphenoxy)phenyl][[3-(1,1,2,2-tetrafluoroethoxy)phenyl]methyl]amino]-1,1,1-trifluoro-2-propanol;
   (2R)-3-[[3-(3-isopropylphenoxy)phenyl][[3-(1,1,2,2-tetrafluoroethoxy)phenyl]methyl]amino]-1,1,1-trifluoro-2-propanol;
   (2R)-3-[[3-(3-cyclopropylphenoxy)phenyl][3-(1,1,2,2-tetrafluoroethoxy)phenyl]methyl]amino]-1,1,1-trifluoro-2-propanol;
   (2R)-3-[[3-(3-(2-furyl)phenoxy)phenyl][[3-(1,1,1-tetrafluoroethoxy)phenyl]methyl]amino]-1,1,1-trifluoro-2-propanol;
   (2R)-3-[[3-(2,3-dichlorophenoxy)phenyl][[3-(1,1,2,2-tetrafluoroethoxy)phenyl]methyl]amino]-1,1,1-trifluoro-2-propanol;
   (2R)-3-[[3-(4-fluorophenoxy)phenyl][[3-(1,1,2,2-tetrafluoroethoxy)phenyl]methyl]amino]-1,1,1-trifluoro-2-propanol;
   (2R)-3-[[3-(4-methylphenoxy)phenyl][[3-(1,1,2,2-tetrafluoroethoxy)phenyl]methyl]amino]-1,1,1-trifluoro-2-propanol;
   (2R)-3-[[3-(2-fluoro-5-bromophenoxy)phenyl][[3-(1,1,2,2-tetrafluoroethoxy)phenyl]methyl]amino]-1,1,1-trifluoro-2-propanol;
   (2R)-3-[[3-(4-chloro-3-ethylphenoxy)phenyl][[3-(1,1,2,2-tetrafluoroethoxy)phenyl]methyl]amino]-1,1,1-trifluoro-2-propanol;
   (2R)-3-[[3-[3-(1,1,2,2-tetrafluoroethoxy)phenoxy]phenyl] [[3-(1,1,2,2-tetrafluoroethoxy)phenyl]methyl]amino]-1,1,1-trifluoro-2-propanol;
   (2R)-3-[[3-[3-(pentafluoroethyl)phenoxy]phenyl][[3-(1,1,2,2-tetrafluoroethoxy)phenyl]methyl]amino]-1,1,1-trifluoro-2-propanol;
   (2R)-3-[[3-(3,5-dimethylphenoxy)phenyl][[3-(1,1,2,2-tetrafluoroethoxy)phenyl]methyl]amino)-1,1,1-trifluoro-2-propanol;
   (2R)-3-[[3-(3-ethylphenoxy)phenyl][[3-(1,1,2,2-tetrafluoroethoxy)phenyl]methyl]amino]-1,1,1-trifluoro-2-propanol;
   (2R)-3-[[3-(3-t-butylphenoxy)phenyl][[3-(1,1,2,2-tetrafluoroethoxy)phenyl]methyl]amino]-1,1,1-trifluoro-2-propanol;
   (2R)-3-[[3-(3-methylphenoxy)phenyl][[3-(1,1,2,2-tetrafluoroethoxy)phenyl]methyl]amino]-1,1,1-trifluoro-2-propanol;
   (2R)-3-[[3-(5,6,7,8-tetrahydro-2-naphthoxy)phenyl][[3-(1,1,2,2-tetrafluoroethoxy)phenyl]methyl]amino]-1,1,1-trifluoro-2-propanol;
   (2R)-3-[(3-(phenoxy)phenyl][[3-(1,1,2,2-tetrafluoroethoxy)phenyl]methyl]amino]-1,1,1-trifluoro-2-propanol;
   (2R)-3-[(3-[3-(N,N-dimethylamino)phenoxy]phenyl][[3-(1,1,2, 2-tetrafluoroethoxy)phenyl]methyl]amina]-1,1,1-trifluoro-2-propanol;
   (2R)-3-[[[3-(1,1,2,2-tetrafluoroethoxy)phenyl]methyl][3-[[3-(trifluoromethoxy)phenyl]methoxy]phenyl]amino]-1,1,1-trifluoro-2-propanol;
   (2R)-3-[[[3-(1,1,2,2-tetrafluoroethoxy)phenyl]methyl][3-[[3-(trifluoromethyl)phenyl]methoxy]phenyl]amino]-1,1,1-trifluoro-2-propanol;
   (2R)-3-[[[3-(1,1,2,2-tetrafluoroethoxy)phenyl]methyl][3-[[3,5-dimethylphenyl]methoxy]phenyl]amino]-1,1,1-trifluoro-2-propanol;
   (2R)-3-[[[3-(1,1,2,2-tetrafluoroethoxy)phenyl]methyl][3-[[3-(trifluoromethylthio)phenyl]methoxy]phenyl]amino]-1,1,1-trifluoro-2-propanol;
   (2R)-3-[[[3-(1,1,2,2-tetrafluoroethoxy)phenyl]methyl][3-[[3,5-difluorophenyl]methoxy]phenyl]amino]-1,1,1-trifluoro-2-propanol;
   3-[[[3-(1,1,2,2-tetrafluoroethoxy)phenyl]methyl][3-[cyclohexylmethoxy]phenyl]amino]-1,1,1-trifluoro-2-propanol;
   3-[[3-(2-difluoromethoxy-4-pyridyloxy)phenyl][[3,-(1,1,2,2-tetrafluoroethoxy)phenyl]methyl]amino]-1,1,1-trifluoro-2-propanol;
   (2R)-3-[[3-(2-trifluoromethyl-4-pyridyloxy)phenyl][[3-(1,1,2,2-tetrafluoroethoxy)phenyl]methyl]amino]-1,1,1-trifluoro-2-propanol;
   (2R)-3-[[3-(3-difluoromethoxyphenoxy)phenyl][[3-(1,1,2,2-tetrafluoroethoxy)phenyl]methyl]amino]-1,1,1-trifluoro-2-propanol;
   (2R)-3-[[[3-(3-trifluoromethylthio)phenoxy]phenyl][[3-(1,1,2,2-tetrafluoroethoxy)phenyl]methyl]amino]-1,1,1-trifluoro-2-propanol;
   (2R)-3-[[3-(4-chloro-3-trifluoromethylphenoxy)phenyl][[3-(1,1,2,2-tetrafluoroethoxy)phenyl]methyl]amino]-1,1,1-tri fluoro-2-propanol;
   (2R)-3-[[3-(3-trifluoromethylphenoxy)phenyl][[3-(pentafluoroethyl)phenyl]methyl]amino]-1,1,1-trifluoro-2-propanol;
   (2R)-3-[[3-(3-isopropylphenoxy)phenyl][[3-(pentafluoroethyl)phenyl]methyl]amino]-1,1,1-trifluoro-2-propanol;
   (2R)-3-[[3-(3-cyclopropylphenoxy)phenyl][[3-(pentafluoroethyl)phenyl]methyl]amino]-1,1,1-trifluoro-2-propanol;
   (2R)-3-[[3-(3-(2-furyl)phenoxy)phenyl][[3-(pentafluoroethyl)phenyl]methyl]amino]-1,1,1-trifluoro-2-propanol;
   (2R)-3-[[3-(2,3-dichlorophenoxy)phenyl][[3-(pentafluoroethyl)phenyl]methyl]amino]-1,1,trifluoro-2-propanol;
   (2R)-3-[[3-(4-fluorophenoxy)phenyl][[3-(pentafluoroethyl)phenyl]methyl]amino]-1,1,1-trifluoro-2-propanol;
   (2R)-3-[[3-(4-methylphenoxy)phenyl][[3-(pentafluoroethyl)phenyllmethyl]aminol-1,1,1-tri-fluoro-2-propanol;
   (2R)-3-[[3-(2-fluoro-5-bromophenoxy)phenyl][[3-(pentafluoroethyl)phenyl]methyl]amino]-1,1,1-trifluoro-2-propanol;
   (2R)-3-[[3-(4-chloro-3-ethylphenoxy)phenyl][[3-(pentafluoroethyl)phenyl]methyl]amino]-1,1,1-trifluoro-2-propanol;
   (2R)-3-[[3-[3-(1,1,2,2-tetrafluoroethoxy)phenoxy]phenyl] [[3-(pentafluoroethyl)phenyl]methyl]amino]-1,1,1-trifluoro-2-propanol;
   (2R)-3-[[3-[3-(pentafluoroethyl)phenoxy]phenyl][[3-(pentafluoroethyl)phenyl]methyl]amino]-1,1,1-trifluoro-2-propanol;
   (2R)-3-[[3-(3,5-dimethylphenoxy)phenyl][[3-(pentafluoroethyl)phenyl]methyl]amino]-1,1,1-trifluoro-2-propanol;
   (2R)-3-[[3-(3-ethylphenoxy)phenyl][[3-(pentafluoroethyl) phenyl]methyl]aminol-1,1,1-trifluoro-2-propanol;
   (2R)-3-[[3-(3-t-butylphenoxy)phenyl][[3-(pentafluoroethyl) phenyl]methyl]amino]-1,1,1-trifluoro-2-propanol;
   (2R)-3-[[3-(3-(methylphenoxy)phenyl)[[3-(pentafluoroethyl) phenyl]methyl]aminol-1,1,1-trifluoro-2-propanol;
   (2R)-3-[[3-(5,6,7,8-tetrahydro-2-naphthoxy)phenyl][[3-(pentafluoroethyl)phenyl]methyl]amino]-1,1,1-trifluoro-2-propanol;
   (2R)-3-[[3-(phenoxy)phenyl][3-(pentafluoroethyl)phenyl]methyl]amino]-1,1,1p-triflupro-2-propanol;
   (2R)-3-[[3-[3-(N,N-dimethylamino)phenoxy]phenyl][[3-(pentafluoroethyl)phenyl]methyl]amino]-1,1,1-trifluoro-2-propanol;
   (2R)-3-[[[3-(pentafluoroethyl)phenyl]methyl][3-[[3-(trifluoromethoxy)phenyl]methoxy]phenyl]amino]-1,1,1-trifluoro-2-propanol;
   (2R)-3-[[[3-(pentafluoroethyl)phenyl]methyl][3-[[3-(trifluoromethyl)phenyl]methoxy]phenyl]amino]-1,1,1-trifluoro-2-propanol;
   (2R)-3-[[[3-(pentafluoroethyl)phenyl]methyl][3-[[3,5-dimethylphenyl]methoxy]phenyl]amino]-1,1,1-trifluoro-2-propanol;
   (2R)-3-[[[3--(pentafluoroethyl)phenyl]methyl][3-[[3-(trifluoromethylthin)phenyl]methoxy]phenyl]amino]-1,1,1-trifluoro-2-propanol;
   (2R)-3-[[[3-(pentafluoroethyl)phenyl]methyl][3-[[3,5-difluorophenyl]methoxylphenyl]amino)-1,1,1-trifluoro-2-propanol;
   (2R)-3-[[[3-(pentafluoroethyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl][[3-(pentafluoroethyl)phenyl]methyl]amino]-1,1,1-trifluoro-2-propanol;
   (2R)-3-[[3-(2-trifluoromethyl-4-pyridyloxy)phenyl][[3-(pentafluoroethyl)phenyl]methyl]amino]-1,1,1-trifluoro-2-propanol;
   (2R)-3-[[3-(3-difluoromethoxyphenoxy)phenyl][[3-(pentafluoroethyl)phenyl]methyl]amino]-1,1,1-trifluoro-2-propanol;
   (2R)-3-[[[3-(3-trifluoromethylthio)phenoxy]phenyl][[3-(pentafluoroethyl)phenyl]methyl]amino]-1,1,1-trifluoro-2-propanol;
   (2R)-3-[[3-(4-chloro-3-trifluoromethylphenoxy]phenyl][[3-(pentafluoroethyl)phenyl]methyl]amino]-1,1,1-trifluoro-2-propanol;
   (2R)-3-[[3-(3-trifluoromethoxyphenoxy)phenyl][[3-(pentafluoroethyl)phenyl]mothyl]amino]-1,1,1-trifluoro-2-propanol;
   (2R)-3-[[3-(3-isopropylphenoxy)phenyl][[3-(heptafluoroethyl)phenyl]methyl]amino]-1,1,1-trifluoro-2-propanol;
   (2R)-3-[[3-(3-cyclopropylphenoxy)phenyl][[3-(heptafluoroethyl)phenyl]methyl]amino]-1,1,1-trifluoro-2-propanol;
   (2R)-3-[[3-(3-cyclopropylphenoxy)phenyl][[3-(heptafluoroethyl)phenyl]methyl]amino]-1,1,1-trifluoro-2-propanol;
   (2R)-3-[[3-(3-(2-furyl)phenoxy)phenyl][[3-(heptafluoroethyl)phenyl]methyl]amino]-1,1,1-trifluoro-2-propanol;
   (2R)-3-[[3-(2,3-dichlorophenoxy)phenyl][[3-(heptafluoroethyl)phenyl]methyl]amino]-1,1,1-trifluoro-2-propanol;
   (2R)-3-[[3-(4-fluorophenoxy)phenyl][[3-(heptafluoroethyl)phenyl]methyl]amino]-1,1,1-trifluoro-2-propanol;
   (2R)-3-[[3-(4-methylphenoxy)phenyl][[3-(heptafluoroethyl)phenyl]methyl]amino]-1,1,1-trifluoro-2-propanol;
   (2R)-3-[[3-(2-fluoro-5-bromophenoxy)phenyl][[3-(heptafluoroethyl)phenyl]methyl]amino]-1,1,1-trifluoro-2-propanol;
   (2R)-3-[[3-(4-chloro-3-ethylphenoxy)phenyl][[3-(heptafluoroethyl)phenyl]methyl]amino]-1,1,1-trifluoro-2-propanol;
   (2R)-3-[[3-[3-(1,1,2,2-tetrafluoroethoxy)phenoxy]phenyl][[3-(heptafluoroethyl)phenyl]methyl]amino]-1,1,1-trifluoro-2-propanol;
   (2R)-3-[[3-[3-(pentafluoroethyl)phenoxylphenyl][[3-(heptafluoroethyl)phenyl]methyl]amino]-1,1,1-trifluoro-2-propanol;
   (2R)-3-[[3-(3,5-dimethylphenoxy)phenyl][[3-(heptafluoroethyl)phenyl]methyl]amino]-1,1,1-trifluoro-2-propanol;
   (2R)-3-[[3-(3-ethylphenoxy)phenyl][[3-(heptafluoroethyl)phenyl]methyl]amino]-1,1,1-trifluoro-2-propanol;
   (2R)-3-[[3-(3-t-butylphenoxy)phenyl][[3-(heptafluoroethyl)phenyl]methyl]amino]-1,1,1-trifluoro-2-propanol;
   (2R)-3-[[3-(3-methylphenoxy)phenyl][[3-(heptafluoroethyl)phenyl]methyl]amino]-1,1,1-trifluoro-2-propanol;
   (2R)-3-[[3-(5,6,7,8-tetrahydro-2-naphthoxy)phenyl][[3-(heptafluoroethyl)phenyl]methyl]amino]-1,1,1-trifluoro-2-propanol;
   (2R)-3-[[3-(phenoxy)phenyl][[3-(heptafluoroethyl)phenyl] methyl]amino]-1,1,1-trifluoro-2-propanol:
   (2R)-3-[[3-[3-(N,N-dimethylamino)phenoxy]phenyl][[3-(heptafluoroethyl)phenyl]methyl]amino]-1,1,1-trifluoro-2-propanol;
   (2R)-3-[[[3-(heptafluoropropyl)phenyl]methyl][3-[[3-(trifluoromethoxy)phenyl]methoxy]phenyl]amino]-1,1,1-trifluoro-2-propanol;
   (2R)-3-[[[3-(heptafluoropropyl)phenyl]methyl][3-[[3-(trifluoromethyl)phenyl]methoxy]phenyl]amino]-1,1,1-trifluoro-2-propanol;
   (2R)-3-[[[3-(heptafluoropropyl)phenyl]methyl][3-[[3,5-dimethylphenyl]methoxy]phenyl]amino]-1,1,1-trifluoro-2-propanol;
   (2R)-3-[[[3-(heptafluoropropyl)phenyl]methyl][3-[[3-(trifluoromethylthio)phenyl]methoxy]phenyl]amino]-1,1,1-trifluoro-2-propanol;
   (2R)-3-[[[3-(heptafluoropropyl)phenyl]methyl][3-[[3,5-difluorophenyl]methoxy]phenyl]amino]-1,1.1-trifluoro-2-propanol;
   (2R)-3-[[[3-(heptafluoropropyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl][[3-(heptafluoropropyl)phenyl]methyl]amino]-1,1,1-trifluoro-2-propanol;
   (2R)-3-[[3-(3-difluoromethoxyphenoxy)phenyl][[3-(heptafluoropropyl)phenyl]methyl]amino]-1,1,1-trifluoro-2-propanol;
   (2R)-3-[[[3-(3-trifluoromethylthio)phenoxy]phenyl][[3-(heptafluoropropyl)phenyl]methyl]amino]-1,1,1-trifluoro-2-propanol;
   (2R)-3-[[3-(4-chloro-3-trifluoromethylphenoxy)phenyl][[3-(heptafluoropropyl)phenyl]methyl]amino]-1,1,1-trifluoro-2-propanol;
   (2R)-3-[[3-(3-trifluoromethoxyphenoxy)phenyl][[2-fluoro-5-(trifluoromethyl)phenyl]methyl]amino]-1,1,1-trifluoro-2-propanol;
   (2R)-3-[[3-(3-isopropylphenoxy)phenyl][[2-fluoro-5-(trifluoromethyl)phenyl]methyl]amino]-1,1,1-trifluoro-2-propanol;
   (2R)-3-[[3-(3-cyclopropylphenoxy)phenyl][2-fluoro-5-(trifluoromethyl)phenyl]methyl]amino]-1,1,1-trifluoro-2-propanol;
   (2R)-3-[[3-(3-(2-furyl)phenoxy)phenyl][[2-fluoro-5-(trifluoromethyl)phenyl]methyl]amino]-1,1,1-trifluoro-2-propanol;
   (2R)-3-[[3-(2,3-dichlorophenoxy)phenyl][[2-fluoro-5-(trifluoromethyl)phenyl]methyl]amino]-1,1,1-trifluoro-2-propanol;
   (2R)-3-[[3-(4-fluorophenoxy)phenyl][[2-fluoro-5-(trifluoromethyl)phenyl]methyl]amino]-1,1,1-trifluoro-2-propanol;
   (2R)-3-[[3-(4-methylphenoxy)phenyl][[2-fluoro-5-(trifluoromethyl)phenyl]methyl]amino]-1,1,1-trifluoro-2-propanol;
   (2R)-3-[[3-(2-fluoro-5-bromophenoxy)phenyl][[2-fluoro-5-(trifluoromethyl)phenyl]methyl]amino]-1,1,1-trifluoro-2-propanol;
   (2R)-3-[[3-(4-chloro-3-ethylphenoxy)phenyl][[2-fluoro-5-(trifluoromethyl)phenyl]methyl]amino]-1,1,1-trifluoro-2-propanol;
   (2R)-3-[[3-[3-(1,1,2,2-tetrafluoroethoxy)phenoxy]phenyl][[2-fluoro-5-(trifluoromethyl)phenyl]methyl]amino]-1,1,1-trifluoro-2-propanol;
   (2R)-3-[[3-[3(pentafluoroethyl)phenoxy]phenyl][[2-fluoro-5-(trifluoromethyl)phenyl]methyl]amino]-1,1,1-trifluoro-2-propanol;
   (2R)-3-[[3-(3,5-dimethylphenoxy)phenyl][[2-fluoro-5-(trifluoromethyl)phenyl]methyl]amino]-1,1,1-trifluoro-2-propanol;
   (2R)-3-[[3-(3-ethylphenoxy)phenyl][[2-fluoro-5-(trifluoromethyl)phenyl]methyl]amino]-1,1,1-trifluoro-2-propanol;
   (2R)-3-[[3-(3-t-butylphenoxy)phenyl][[2-fluoro-5-(trifluoromethyl)phenyl]methyl]amino]-1,1,1-trifluoro-2-propanol;
   (2R)-3-[[3-(3-methylphenoxy)phenyl][[2-fluoro-5-(trifluoromethyl)phenyl]methyl]amino]-1,1,1-trifluoro-2-propanol;
   (2R)-3-[[3-(5,6,7,8-tetrahydro-2-naphthoxy)phenyl][[2-fluoro-5-(trifluoromethyl)phenyl]methyl]amino]-1,1,1-trifluoro-2-propanol;
   (2R)-3-[[3-(phenoxy)phenyl][[2-fluoro-5-(trifluoromethyl)phenyl]methyl]amino]-1,1,1-trifluoro-2-propanol;
   (2R)-3-[[3-[3-(N,N-dimethylamino)phenoxy]phenyl][[2-fluoro-5-(trifluoromethyl)phenyl]methyl]amino]-1,1,1-trifluoro-2-propanol;
   (2R)-3-[[[2-fluoro-5-(trifluoromethyl)phenyl]methyl][3-[[3-(trifluoromethoxy)phenyl]methoxy]phenyl]amino]-1,1,1-trifluoro-2-propanol;
   (2R)-3-[[[2-fluoro-5-(trifluoromethyl)phenyl]methyl][3-[[3-(trifluoromethyl)phenyl]methoxy]phenyl]amino]-1,1,1-trifluoro-2-propanol;
   (2R)-3-[[[2-fluoro-5-(trifluoromethyl)phenyl]methyl][3-[[3,5-dimethylphenyl]methoxy]phenyl]amino]-1,1,1-trifluoro-2-propanol;
   (2R)-3-[[[2-fluoro-5-(trifluoromethyl)phenyl]methyl][3-[[3-(trifluoromethylthio)phenyl]methoxy]phenyl]amino]-1,1,1-trifluoro-2-propanol;
   (2R)-3-[[[2-fluoro-5-(trifluoromethyl)phenyl]methyl][3-[[3,5-difluorophenyl]mtethoxy]phenyl]amino]-1,1,1-trifluoro-2-propanol;
   (2R)-3-[[[2-fluoro-5-(trifluoromethyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl] [[2-fluoro-5-(trifluorormethyl)phenyl]methyl]amino]-1,1,1-trifluoro-2-propanol;
   (2R)-3-[[3-(2-trifluoromethyl-4-pyridyloxy)phenyl][[2-fluoro-5-(trifluoromethyl)phenyl]methyl]amino]-1,1,1-trifluoro-2-propanol;
   (2R)-3-[[3-(3-difluoromethoxyphenoxy)phenyl][[2-fluoro-5-(trifluoromethyl)phenyl]methyl]amino]-1,1,1-trifluoro-2-propanol;
   (2R)-3-[[[3-(3-trifluoromethylthio)phenoxy]phenyl][[2-fluoro-5-(trifluoromethyl)phenyl]methyl]amino]-1,1,1-trifluoro-2-propanol;
   (2R)-3-[[3-(4-chloro-3-trifluoromethylphenoxy)phenyl][2-fluoro-5-(trifluoromethyl)phenyl]methyl]amino]-1,1,1-trifluoro-2-propanol;
   (2R)-3-[[3-(3-trifluoromethoxyphenoxy)phenyl][[2-fluoro-4-(trifluoromethyl)phenyl]methyl]amino]-1,1,1-trifluoro-2-propanol;
   (2R)-3-[[3-(3-isopropylphenoxy)phenyl][2-fluoro-4-(trifluoromethyl)phenyl]methyl]amino]-1,1,1-trifluoro-2-propanol;
   (2R)-3-[[3-(3-cyclopropylphenoxy)phenyl][[2-fluoro-4-(trifluoromethyl)phenyl]methyl]amino]-1,1,1-trifluoro-2-propanol;
   (2R)-3-[[3-(3-(2-furyl)phenoxy)phenyl][2-fluoro-4-(trifluoromethyl)phenyl]methyl]amino]-1,1,1-trifluoro-2-propanol;
   (2R)-3-[[3-(2,3-dichlorophenoxy)phenyl][2-fluoro-4-(trifluoromethyl)phenyl]methyl]amino]-1,1,1-trifluoro-2-propanol;
   (2R)-3-[[3-(4-fluorophenoxy)phenyl][2-fluoro-4-(trifluoromethyl)phenyl]methyl]amino]-1,1,1-trifluoro-2-propanol;
   (2R)-3-[[3-(4-methylphenoxy)phenyl][2-fluoro-4-(trifluoromethyl)phenyl]methyl]amino]-1,1,1-trifluoro-2-propanol;
   (2R)-3-[[3-(2-fluoro-5-bromophenoxy)phenyl][2-fluoro-4-(trifluoromethyl)phenyl]methyl]amino]-1,1,1-trifluoro-2-propanol;
   (2R)-3-[[3-(4-chloro-3-ethylphenoxy)phenyl][2-fluoro-4-(trifluoromethyl)phenyl]methyl]amino]-1,1,1-trifluoro-2-propanol;
   (2R)-3-[[3-[3-(1,1,2,2-tetrafluoroethoxy)phenoxy]phenyl][2-fluoro-4-(trifluoromethyl)phenyl]methyl]amino]-1,1,1-trifluoro-2-propanol;
   (2R)-3-[[3-[3-(pentafluoroethyl)phenoxylphenyl][2-fluoro-4-(trifluoromethyl)phenyl]methyl]amino]-1,1,1-trifluoro-2-propanol;
   (2R)-3-[[3-(3,5-dimethylphenoxy)phenyl][2-fluoro-4-(trifluorormethyl)phenyl]methyl]amino]-1,1,1-trifluoro-2-propanol;
   (2R)-3-[[3-(3-ethylphenoxy)phenyl][2-fluoro-4-(trifluoromethyl)phenyl]methyl]amino]-1,1,1-trifluoro-2-propanol;
   (2R)-3-[[3-3-t-butylphenoxy)phenyl][2-fluoro-4-(trifluoromethyl)phenyl]methyl]amino]-1,1,1-trifluoro-2-propanol;
   (2R)-3-[[3-(3-methylphenoxy)phenyl][2-fluoro-4-(trifluoromethyl)phenyl]methyl]amino]-1,1,1-trifluoro-2-propanol;
   (2R)-3-[[3-(5,6,7,8-tetrahydro-2-naphthoxy)phenyl][2-fluoro-4-(trifluoromethyl)phenyl]methyl]amino]-1,1,1-trifluoro-2-propanol;
   (2R)-3-[[3-(phenoxy)phenyl][2-fluoro-4-(trifl uoromethyl) phenyl]methyl]amino]-1,1,1-trifluoro-2-propanol;
   (2R)-3-[[3-[3-(N,N-dimethylamino)phenoxy]phenyl][2-fluoro-4-(trifluoromethyl)phenyl]methyl]amino]-1,1,1-trifluoro-2-propanol;
   (2R)-3-[[[2-fluoro-4-(trifluoromethyl)phenyl]methyl][3-[[3-(trifluoromethoxy)phenyl]methoxyl]phenyl]amino]-1,1,1-trifluoro-2-propanol;
   (2R)-3-[[[2-fluoro-4-(trifluoromethyl)phenyl]methyl][3-[[3,5-dimethylphenyl]methoxy]phenyl]amino]-1,1,1-trifluoro-2-propanol;
   (2R)-3-[[[2-fluoro-4-(trifluoromethyl)phenyl]methyl][3-[[3-(trifluoromethylthio)phenyl]methoxy]phenyl]amino]-1,1,1-trifluoro-2-propanol;
   (2R)-3-[[[2-fluoro-4-(trifluoromethyl)phenyl]methyl][3-[[3,5-difluorophenyl]methoxy]phenyl]amino]-1,1,1-trifluoro-2-propanol;
   (2R)-3-[[[2-fluoro-4-(trifluoromethyl)phenyl]methyl][3-[cyclohexylmethoxy]phenyl]amino]-1,1,1-trifluoro-2-propanol;
   (2R)-3-[[3-(2-difluoromethoxy-4-pyridyloxy)phenyl][2-fluoro-4-(trifluoromethyl)phenyl]methyl]amino]-1,1,1-trifluoro-2-propanol;
   (2R)-3-[[3-(2-trifluoromethyl-4-pyridyloxy)phenyl][2-fluoro-4-(trifluoromethyl)phenyl]methyl]amino]-1,1,1-trifluoro-2-propanol;
   (2R)-3-[[3-(3-difluoromethoxyphenoxy)phenyl] [[2-fluoro-4-(trifluoromethyl)phenyl]methyl]amino]-1,1,1-trifluoro-2-propanol;
   (2R)-3-[[[3-(3-trifluoromethylthio)phenoxy]phenyl] [2-fluoro-4-(trifluoromethyl)phenyl]methyl]amino]-1,1,1-trifluoro-2-propanol; and
   (2R)-3-[[3-(4-chloro-3-trifluoromethylphenoxy)phenyl][2-fluoro-4-(trifluoromethyl)phenyl]methyl]amino]-1,1,1-trifluoro-2-propanol, or a pharmaceutically acceptable salt thereof.
[73] The method of [1], [3] or [6], wherein the compound having a CETP inhibitory activity is a compound represented by the formula (XV): 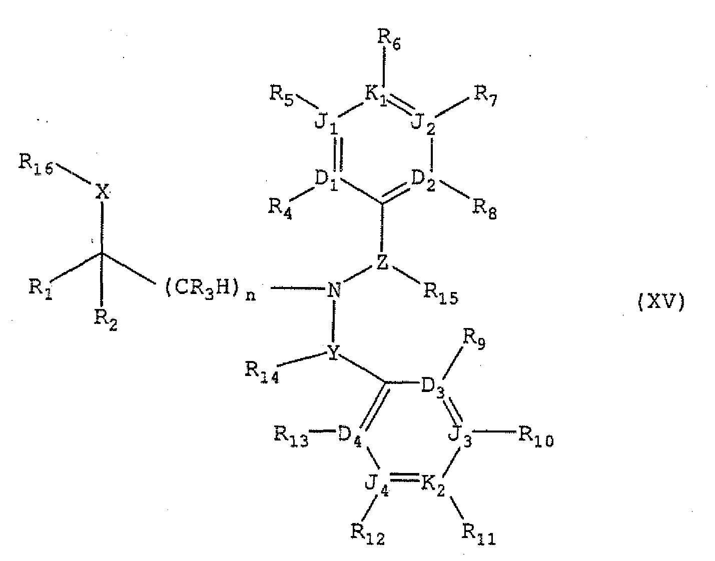 or a pharmaceutically acceptable salt thereof, wherein;
   n is an integer selected from 0 through 5;
   R₁ is selected from the group consisting of haloalkyl, haloalkenyl, haloalkoxyalkyl and haloalkenyloxyalkyl;
   X is selected from the group consisting of 0, H, F, S, S(O), NH, N(OH), N(alkyl), and N(alkoxy);
   R₁₆ is selected from the group consisting of hydrido, alkyl, alkenyl, alkynyl, aryl, aralkyl, aryloxyalkyl, alkoxyalkyl, alkenyloxyalkyl, alkylthioalkyl, arylthioalkyl, aralkoxyalkyl, heteroaralkoxyalkyl, alkylsulfinylalkyl, alkylsulfonylalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxyalkyl, haloalkenyloxyalkyl, halocycloalkoxyalkyl, halocycloalkenyloxyalkyl, perhaloaryl, perhaloaralkyl, perhaloaryloxyalkyl, heteroaryl, heteroarylalkyl, monocarboalkoxyalkyl, monocarboalkoxy, dicarboalkoxyalkyl, monocarboxamido, monocyanoalkyl, dicyanoalkyl, carboalkoxycyanoalkyl, acyl, aroyl, heteroaroyl, heteroaryloxyalkyl, dialkoxyphosphonoalkyl, trialklylsilyl, and a spacer selected from the group consisting of a covalent single bond and a linear spacer moiety having from 1 through 4 continuous atoms linked to the point of bonding of an aromatic substituent selected from the group consisting of R₄, R₈, R₉, and R₁₃ to form a heterocyclyl ring having from 5 through 10 continuous members with the provisos that said spacer moiety is other than a covalent single bond when R₂ is alkyl and there is no R₁₆ wherein X is H or F;
   D₁, D₂, J₁, J₂ and K₁ are independently selected from the group consisting of C, N, 0, S and a covalent bond with the provisos that no more than one of D₁, D₂, J₁, J₂ and K₁ is a covalent bond, no more than one of D₁, D₂, J₁, J₂ and K₁ is O, no more than one of D₁, D₂, J₁, J₂ and K₁ is S, one of D_{1,} D₂, J₁, J₂ and K₁ must be a covalent bond when two of D₁, D₂, J₁, J₂ and K₁ are O and S, and no more than four of D_{1,} D₂, J₁, J₂ and K₁ are N;
   D₃, D₄, J₃, J₄ and K₂ are independently selected from the group consisting of C, N, O, S and a covalent bond with the provisos that no more than one of D₃, D₄, J₃, J₄ and K₂ is a covalent bond, no more than one of D₃, D₄, J₃, J₄ and K₂ is O, no more than one of D₃, D₄, J₃, J₄ and K₂ is S, one of D₃, D₄, J₃, J₄ and K₂ must be a covalent bond when two of D₃, D₄, J₃, J₄ and K₂ are O and S, and no more than four of D₃, D₄, J₃, J₄ and K₂ are N;
   R₂ is independently selected from the group consisting of hydrido, hydroxy, hydroxyalkyl, amino, aminoalkyl, alkylamino, dialkylamino, alkyl, alkenyl, alkynyl, aryl, aralkyl, aralkoxyalkyl, aryloxyalkyl, alkoxyalkyl, heteroaryloxyalkyl, alkenyloxyalkyl, alkylthioalkyl, aralkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, alkylsulfinylalkyl, alkylsulfonylalkyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl and diaralkoxyphosphonoalkyl;
   R₂ and R₃ are taken together to form a linear spacer moiety selected from the group consisting of a covalent single bond and a moiety having from 1 through 6 continuous atoms to form a ring selected from the group consisting of a cycloalkyl having from 3 through 8 continuous members, a cycloalkenyl having from 5 through 8 continuous members, and a heterocyclyl having from 4 through 8 continuous members;
   R₃ is selected from the group consisting of hydrido, hydroxy, halo, cyano, aryloxy, hydroxyalkyl, amino, alkylamino, dialkylamino, acyl, sulfhydryl, acylamido, alkoxy, alkylthio, arylthio, alkyl, alkenyl, alkynyl, aryl, aralkyl, aryloxyalkyl, alkoxyalkyl, heteroarylthio, aralkylthio, aralkoxyalkyl, alkylsulfinylalkyl, alkylsulfonylalkyl, aroyl, heteroaroyl, aralkylthioalkyl, heteroaralkylthioalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl and diaralkoxyphosphonoalkyl;
   Y is selected from a group consisting of a covalent single bond, (C(R₁₄)₂)_{q} wherein q is an integer selected from 1 and 2, and (CH(R₁₄))_{g}-W-(CH(R₁₄))ₚ wherein g and p are integers independently selected from 0 and 1;
   R₁₄ is independently selected from the group consisting of hydrido, hydroxy, halo, cyano, aryloxy, amino, alkylamino, dialkylamino, hydroxyalkyl, acyl, aroyl, heteroaroyl, heteroaryloxyalkyl, sulfhydryl, acylamido, alkoxy, alkylthio, arylthio, alkyl, alkenyl, alkynyl, aryl, aralkyl, aryloxyalkyl, aralkoxyalkylalkoxy, alkylsulfinylalkyl, alkylsulfonylalkyl, aralkylthioalkyl, heteroaralkoxythioalkyl, alkoxyalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl, diaralkoxyphosphonoalkyl, a spacer selected from a moiety having a chain length of 3 to 6 atoms connected to the point of bonding selected from the group consisting of R₉ and R₁₃ to form a ring selected from the group consisting of a cycloalkenyl ring having from 5 through 8 continuous members and a heterocyclyl ring having from 5 through 8 continuous members, and a spacer selected from a moiety having a chain length of 2 to 5 atoms connected to the point of bonding selected from the group consisting of R₄ and R₈ to form a heterocyclyl having from 5 through 8 continuous members with the proviso that, when Y is a covalent bond, R₁₄ substituent is not attached to Y;
   R₁₄ and R₁₄, when bonded to the different atoms, are taken together to form a group selected from the group consisting of a covalent bond, alkylene, haloalkylene, and a spacer selected from a group consisting of a moiety having a chain length of 2 to 5 atoms connected to form a ring selected from the group of a saturated cycloalkyl having from 5 through 8 continuous members, a cycloalkenyl having from 5 through 8 continuous members, and a heterocyclyl having from 5 through 8 continuous members;
   R₁₄ and R₁₄ , when bonded to the same atom, are taken together to form a group selected from the group consisting of oxo, thiono, alkylene, haloalkylene, and a spacer selected from the group consisting of a moiety having a chain length of 3 to 7 atoms connected to form a ring selected from the group consisting of a cycloalkyl having from 4 through 8 continuous members, a cycloalkenyl having from 4 through 8 continuous members, and a heterocyclyl having from 4 through 8 continuous members;
   W is selected from the group consisting of O, C(O), C(S), C(O)N(R₁₄), C(S)N(R₁₄), (R₁₄)NC(O), (R₁₄)NC(S), S, S(O), S(O)₂, S(O)₂N(R₁₄), (R₁₄)NS(O)₂, and N(R₁₄) with the proviso that R₁₄ is selected from other than halo and cyano;
   Z is independently selected from a group consisting of a covalent single bond, (C(R₁₅)₂)_{q} wherein q is an integer selected from 1 and 2, and (CH(R₁₅))ⱼ-W-(CH(R₁₅))ₖ wherein j and k are integers independently selected from 0 and 1 with the proviso that, when Z is a covalent single bond, R₁₅ substituent is not attached to Z;
   R₁₅ is independently selected, when Z is (C(R₁₅)₂)_{q} wherein q is an integer selected from 1 and 2, from the group consisting of hydrido, hydroxy, halo, cyano, aryloxy, amino, alkylamino, dialkylamino, hydroxyalkyl, acyl, aroyl, heteroaroyl, heteroaryloxyalkyl, sulfhydryl, acylamido, alkoxy, alkylthio, arylthio, alkyl, alkenyl, alkynyl, aryl, aralkyl, aryloxyalkyl, aralkoxyalkyl, alkylsulfinylalkyl, alkylsulfonylalkyl, aralkylthioalkyl, heteroaralkylthioalkyl, alkoxyalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenlyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl, diaralkoxyphosphonoalkyl, a spacer selected from a moiety having a chain length of 3 to 6 atoms connected to the point of bonding selected from the group consisting of R₄ and R₈ to form a ring selected from the group consisting of a cycloalkenyl ring having from 5 through 8 continuous members and a heterocyclyl ring having from 5 through 8 continuous members, and a spacer selected from a moiety having a chain length of 2 to 5 atoms connected to the point of bonding selected from the group consisting of R₉ and R₁₃, to form a heterocyclyl having from 5 through 8 continuous members;
   R₁₅ and R₁₅, when bonded to the different atoms, are taken together to form a group selected from the group consisting of a covalent bond, alkylene, haloalkylene, and a spacer selected from a group consisting of a moiety having a chain length of 2 to 5 atoms connected to form a ring selected from the group consisting of a saturated cycloalkyl having from 5 through 8 continuous members, a cycloalkenyl having from 5 through 8 continuous members, and a heterocyclyl having from 5 through 8 continuous members;
   R₁₅ and R₁₅, when bonded to the same atom, are taken together to form a group selected from the group consisting of oxo, thiono, alkylene, haloalkylene, and a spacer selected from the group consisting of a moiety having a chain length of 3 to 7 atoms connected to form a ring selected from the group consisting of a cycloalkyl having from 4 through 8 continuous members, a cycloalkenyl having from 4 through 8 continuous members, and a heterocyclyl having from 4 through 8 continuous members;
   R₁₅ is independently selected, when Z is (CH(R₁₅))ⱼ-W-(CH(R₁₅))ₖ wherein j and k are integers independently selected from 0 and 1, from the group consisting of hydrido, halo, cyano, aryloxy, carboxyl, acyl, aroyl, heteroaroyl, hydroxyalkyl, heteroaryloxyalkyl, acylamido, alkoxy, alkylthio, arylthio, alkyl, alkenyl, alkynyl, aryl, aralkyl, aryloxyalkyl, alkoxyalkyl, heteroaryloxyalkyl, aralkoxyalkyl, heteroaralkoxyalkyl, alkylsulfonylalkyl, alkylsulfinylalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxyalkyl, carboalkoxy, carboxamide, carboxamidoalkyl, carboaralkoxy, dialkoxyphosphonoalkyl, diaralkoxyphosphonoalkyl, a spacer selected from a linear moiety having a chain length of 3 to 6 atoms connected to the point of bonding selected from the group consisting of R₄ and R₈ to form a ring selected from the group consisting of a cyclcalkenyl ring having from 5 through 8 continuous members and a heterocyclyl ring having from 5 through 8 continuous members, and a spacer selected from a linear moiety having a chain length of 2 to 5 atoms connected to the point of bonding selected from the group consisting of R₉ and R₁₃ to form a heterocyclyl ring having from 5 through 8 continuous members;
   R₄, R₅, R₆, R₇, R₈, R₉, R₁₀ R₁₁, R₁₂, and R₁₃ are independently selected from the group consisting of perhaloaryloxy, alkanoylalkyl, alkanoylalkoxy, alkanoyloxy, N-aryl-N-alkylamino, heterocyclylalkoxy, heterocyclylthio, hydroxyalkoxy, carboxamidoalkoxy, alkoxycarbonylalkoxy, alkoxycarbonylalkenyloxy, aralkanoylalkoxy, aralkenoyl, N-alkylcarboxamido, N-haloalkylcarboxamido, N-cycloalkylcarboxamido, N-arylcarboxamidoalkoxy, cycloalkylcarbonyl, cyanoalkoxy, heterocyclylcarbonyl, hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkylamidosulfonyl, dialkylamidosulfonyl, monoarylamidasulfonyl, arylsulfonamido, diarylamidosulfonyl, monoalkylmonoarylamidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloaralkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that there are one to five non-hydrido ring substituents R₄, R₅, R₆, R₇, and R₈ present, that there are one to five non-hydrido ring substituents R₉, R₁₀, R₁₁, R₁₂, and R₁₃ present, and R₄, R₅, R₆, R₇, R₈, R₉, R₁₀, R₁₁, R₁₂, and R₁₃ are each independently selected to maintain the tetravalent nature independently selected to maintain the tetravalent nature of carbon, the trivalent nature of nitrogen, the divalent nature of sulfur, and the divalent nature of oxygen;
   R₄ and R₅, R₅ and R₆, R₆ and R₇, R₇ and R₈, R₉ and T₁₀, R₁₀ and R₁₁, R₁₁ and R₁₂, and R₁₂ and R₁₃ are independently selected to form spacer pairs wherein a spacer pair is taken together to form a linear moiety having from 3 through 6 continuous atoms connecting the points of bonding of said spacer pair members to form a ring selected from the group consisting of a cycloalkenyl ring having 5 through 8 continuous members, a partially saturated heterocyclyl ring having 5 through 8 continuous members, a heteroaryl ring having 5 through 6 continuous members, and an aryl with the provisos that no more than one of the group consisting of spacer pairs R₄ and R₅, R₅ and R₆, R₆ and R₇, and R₇ and R₈, is used at the same time and that no more than one of the group consisting of spacer pairs R₉ and R₁₀, R₁₀ and R₁₁, R₁₁ and R₁₂, and R₁₂ and R₁₃ is used at the same time;
   R₄ and R₉, R₄ and R₁₃, R₈ and R₉, and R₈ and R₁₃ are independently selected to form a spacer pair wherein said spacer pair is taken together to form a linear moiety wherein said linear moiety forms a ring selected from the group consisting of a partially saturated heterocyclyl ring having from 5 through 8 continuous members and a heteroaryl ring having from 5 through 6 continuous members with the proviso that no more than one of the group consisting of spacer pairs R₄ and R₉, R₄ and R₁₃, R₈ and R₉, and R₈ and R₁₃ is used at the same time.
[74] The inhibitor of [2], [4] or [7], wherein the compound having a CETP inhibitory activity is a compound of the formula (XV) of [73] or a pharmaceutically acceptable salt thereof.
[75] The inhibitor of [5] or [8], which is a compound of the formula (XV) of [73] or a pharmaceutically acceptable salt thereof.
[76] The method of [73], wherein the compound of the formula (XV) is a compound selected from the group consisting of
   3-[[3-(3-trifluoromethoxyphenoxy)phenyl][[3-(1,1,2,2-tetrafluoroethoxy)-phenyl]methyl]amino]-1,1,1-trifluoro-2-propanol;
   3-[[3-(3-isopropylphenoxy)phenyl][[3-(1,1,2,2-tetrafluoroethoxy)phenyl]-methyl]amino]-1,1,1-trifluoro-2-propanol;
   3-[[3-(3-cyclopropylphenoxy)phenyl][[3-(1,1,2,2-tetrafluoroethoxy)phenyl]-methyl]amino]-1,1,1-trifluoro-2-propanol;
   3-[[3-(3-(2-furyl)phenoxy)phenyl][[3-(1,1,2,2-tetrafluoroethoxy)phenyl]-methyl]amino]-1,1,1-trifluoro-2-propanol;
   3-[[3-(2,3-dichlorophenoxy)phenyl][[3-(1,1,2,2-tetrafluoroethoxy)phenyl]-methyl]amino]-1,1,1-trifluoro-2-propanol;
   3-[[3-(4-fluorophenoxy)phenyl)[[3-(1,1,2,2-tetrafluoroethoxy)phenyl]-methyl]amino]-1,1,1-trifluoro-2-propanol;
   3-[[3-(4-methylphenoxy)phenyl][[3-(1,1,2,2-tetrafluoroethoxy)phenyl]-methyl]amino]-1,1,1-trifluoro-2-propanol;
   3-[[3-(2-fluoro-5-bromophenoxy)phenyl][[3-(1,1,2,2-tetrafluoroethoxy)phenyl]-methyl]amino]-1,1,1-trifluoro-2-propanol;
   3-[[3-(3-(4-chloro-3-ethylphenoxy)phenyl)[[3-(1,1,2,2-tetrafluoroethoxy)phenyl]-methyl]amino]-1,1,1-trifluoro-2-propanol;
   3-[[3-[3-(1,1,2,2-tetrafluoroethoxy)phenoxy]phenyl][[3-(1, 1,2,2-tetrafluoro-ethoxy)phenyl]methyl]amino]-1,1,1-trifluoro-2-propanol;
   3-[[3-[3-(pentafluoroethyl)phenoxy]phenyl][[3-(1,1,2,2-tetrafluoroethoxy)-phenyl]methyl]amino]-1,1,1-trifluoro-2-propanol;
   3-[[3-(3,5-dimethylphenoxy)phenyl][3-(1,1,2,2-tetrafluoroethoxy)phenyl]-methyl]amino]-1,1,1-trifluoro-2-propanol;
   3-[[3-(3-ethylphenoxy)phenyl][[3-(1,1,2,2-tetrafluoroethoxy)phenyl]-methyl]amino]-1,1,1-trifluoro-2-propanol;
   3-[[3-(3-t-butylphenoxy)phenyll[[3-(1,1,2,2-tetrafluoroethcxy)phenyl]-methyl]amino]-1,1,1-trifluoro-2-propanol;
   3-[[3-(3-methylphenoxy)phenyl][[3-(1,1,2,2-tetrafluoroethoxy)phenyl]-methyl]amino]-1,1,1-trifluoro-2-propanol;
   3-[[3-(5,6,7,8-tetrahydro-2-naphthoxy)phenyl][[3-(1,1,2,2-tetrafluoro-ethoxy)phenyl]methyl]amino]-1,1,1-trifluoro-2-propanol;
   3-[[3-(phenoxy)phenyl][[3-(1,1,2,2-tetrafluoroethoxy)phenyl]methyl]amino]-1,1,1-trifluoro-2-propanol;
   3-[[3-[3-(N,N-dimethylamino)phenoxy]phenyl][[3-(1,1,2,2-tetrafluoro-ethoxy)phenyl]methyl]amino]-1,1,1-trifluoro-2-propanol;
   3-[[[3-(1,1,2,2-tetrafluoroethoxy)phenyl]methyl][3-[[3-(trifluoromethoxy)-phenyl]methoxy]phenyl]amino]-1,1,1-trifluoro-2-propanol,
   3-[[[3-(1,1,2,2-tetrafluoroethoxy)phenyl]methyl][3-[[3-(trifluoromethyl)-phenyl]methoxy]phenyl]amino]-1,1,1-trifluoro-2-propanol;
   3-[[[3-(1,1,2,2-tetrafluoroethoxy)phenyl]methyl][3-[[3,5-dimethylphenyl]methoxy]phenyl]amino]-1,1,1-trifluoro-2-propanol;
   3-[[[3-(1,1,2,2-tetrafluoroethoxy)phenyl]methyl][3-[[3-(trifluoromethylthio)-phenyl]methoxy]phenyl]amino]-1,1,1-trifluoro-2-propanol;
   3-[[[3-(1,1,2,2-tetrafluoroethoxy)phenyl]methyl][3-[[3,5-difluorophenyl]methoxy]phenyl]amino]-1,1,1-trifluoro-2-propanol;
   3-[[[3-(1,1,2,2-tetrafluoroethoxy)phenyl]methyl][3-[cyclohexylmethoxy]phenyl]amino]-1,1,1-trifluoro-2-propanol;
   3-[[3-(2-difluoromethoxy-4-pyridyloxy)phenyl][[3-(1,1,2,2-tetrafluoroethoxy)-phenyl]methyl]amino]-1,1,1-trifluoro-2-propanol;
   3-[[3-(2-trifluoromethyl-4-pyridyloxy)phenyl][[3-(1,1,2,2-tetrafluoroethoxy)-phenyl]methyl]amino]-1,1,1-trifluoro-2-propanol;
   3-[[3-(3-difluoromethoxyphenoxy)phenyl][[3-(1,1,2,2-tetrafluoroethoxy)-phenyl]methyl]amino]-1,1,1-trifluoro-2-propanol;
   3-[[[3-(3-trifluoromethylthio)phenoxy]phenyl][[3-(1,1,2,2-tetrafluoroethoxy)-phenyl]methyl]amino]-1,1,1-trifluoro-2-propanol;
   3-[[3-(4-chloro-3-trifluoromethylphenoxy)phenyl][[3-(1,1,2,2-tetrafluoroethoxy)-phenyl]methyl]amino]-1,1,1-trifluoro-2-propanol;
   3-[[3-3-trifluoromethoxyphenoxy)phenyl][[3-(pentafluoroethyl)phenyl]-methyl]amino]-1,1,1-trifluoro-2-propanol;
   3-[[3-(3-isopropylphenoxy)phenyl][[3-(pentafluoroethyl)phenyl]methyl]-amino]-1,1,1-trifluoro-2-propanol;
   3-[[3-(3-cyclopropylphenoxy)phenyl][[3-(pentafluoroethyl)phenyl]methyl]-amino]-1,1-1-trifluoro-2-propanol;
   3-[[3-(3-(2-furyl)phenoxy)phenyl[[3-(pentafluoroethyl)phenyl]methyl]-amino]-1,1,1-trifluoro-2-propanol;
   3-[[3-(2,3-dichlorophenoxy)phenyl][[3-(pentafluoroethyl)phenyl]methyl]-amino]-1,1,1-trifluoro-2-propanol;
   3-[[3-(4-fluorophenoxy)phenyl][[3-(pentafluoroethyl)phenyl]methy]amino]-1,1,1-trifluoro-2-propanol;
   3-[[3-(4-methylphenoxy)phenyl][[3-(pentafluoroethyl)phenyl]methyl]amino]-1,1,1-trifluoro-2-propanol;
   3-[[3-(2-fluoro-5-bromophenoxy)phenyl][[3-(pentafluoroethyl)phenyl]methyl]-amino]-1,1,1-trifluoro-2-propanol;
   3-[[3-(4-chloro-3-ethylphenoxy)phenyl][[3-(pentafluoroethyl)phenyl]methyl]-amino]-1,1,1-trifluoro-2-propanol;
   3-[[3-[3-(1,1,2,2-tetrafluoroethoxy)phenoxy]phenyl][[3-(pentafluoroethyl)-phenyl]methyl]amino]-1,1,1-trifluoro-2-propanol;
   3-[[3-[3-(pentafluoroethyl)phenoxy]phenyl][[3-(pentafluoroethyl)phenyl]-methyl]amino]-1,1,1-trifluoro-2-propanol;
   3-[[3-(3,5-dimethylphenoxy)phenyl][[3-(pentafluoroethyl)phenyl]methyl]-amino1-1,1,1-trifluoro-2-propanol;
   3-[[3-(3-ethylphenoxy)phenyl][[3-(pentafluoroethyl)phenyl]methyl]amino]-1,1,1trifluoro-2-propanol;
   3-[[3-(3-t-butylphenoxy)phenyl][[3-(pentafluoroethyl)phenyl]methyl]amino]-1,1,1-trifluoro-2-propanol;
   3-[[3-[3-methylphenoxy]phenyl][[3-(pentafluoroethyl)phenyl]methyl]amino]-1,1,1-trifluoro-2-propanol;
   3-[[3-[3-(5,6,7,8-tetrahydro-2-naphthoxy)phenyl][[3-(pentafluoroethyl)phenyl]-methyl]amino-1,1,1-trifluoro-2-propanol;
   3-[[3-(phenoxy)phenyl][[3-(pentafluoroethyl)phenyl]methyl]amino]-1,1,1-trifluoro-2-propanol;
   3-[[3-[3-(N,N-dimethylamino)phenoxy]phenyl][[3-(pentafluoroethyl)phenyl]-methyl]amino]-1,1,1-trifluoro-2-propanol;
   3-[[[3-(pentafluoroethyl)phenyl]methyl][3-[[3-(trifluoromethoxy)phenyl]-methoxy]phenyl]amino]-1,1,1-trifluoro-2-propanol;
   3-[[[3-(pentafluoroethyl)phenyl]methyl][3-[[3-(trifluoromethyl)phenyl]-methoxy]phenyl]amino]-1,1,1-trifluoro-2-propanol;
   3-[[[3-(pentafluoroethyl)phenyl]methyl][3-[[3-5-dimethylphenyl]methoxyl-phenyl]amino]-1,1,1-trifluoro-2-propanol;
   3-[[[3-(pentafluoroethyl)phenyl]methyl]3-[[3-(trifluoromethylthio)phenyl]-methoxy]phenyl]amino]-1,1,1-trifluoro-2-propanol;
   3-[[[3-(pentafluoroethyl)phenyl]methyl][3-[[3,5-difluorophenyl]methoxy]-phenyl]amino]-1,1,1-trifluoro-2-propanol;
   3-[[[3-(pentafluoroethyl)phenyl]methyl][3-[cyclohexylmethoxy]phenyl]-amino]-1,1,1-trifluoro-2-propanol;
   3-[[3-(2-difluoromethoxy-4-pyridyloxy)phenyl][[3-(pentafluoroethyl)phenyl]-methyl]amino]-1,1,1-trifluoro-2-propanol;
   3-[[3-(2-trifluoromethyl-4-pyridyloxy)phenyl][[3-(pentafluoroethyl)phenyl]-methyl]amino]-1,1,1-trifluoro-2-propanol;
   3-[[3-(3-difluoromethoxyphenoxy)phenyl][[3-(pentafluoroethyl)phenyl]-methyl]amino]-1,1,1-trifluoro-2-propanol;
   3-[[[3-(3-trifluoromethylthio)phenoxy]phenyl][[3-(pentafluoroethyl)phenyl]-methyl]amino]-1,1,1-trifluoro-2-propanol;
   3-[[3-(4-chloro-3-trifluoromethylphenoxy)phenyl][[3-(pentafluoroethyl)-phenyl]methyl]amino]-1,1,1-trifluoro-2-propanol;
   3-[[3-(3-trifluoromethoxyphenoxy)phenyl][[3-(heptafluoropropyl)phenyl]-methyl]amino]-1,1,1-trifluoro-2-propanol;
   3-[[3-(3-isopropylphenoxy)phenyl][[3-(heptafluoropropyl)phenyl]methyl]-amino]-1,1,1-trifluoro-2-propanol;
   3-[[3-(3-cyclopropylphenoxy)phenyl][[3-(heptafluoropropyl)phenyl]methyl]-amino]-1,1,1-trifluoro-2-propanol;
   3-[[3-(3-(2-furyl)phenoxy)phenyl][[3-(heptafluoropropyl)phenyl]methyl]-amino]-1,1,1-trifluoro-2-propanol;
   3-[[3-(2,3-dichlorophenoxy)phenyl][[3-(heptafluoropropyl)phenyl]methyl]-amino]-1,1,1-trifluoro-2-propanol;
   3-[[3-(4-fluorophenoxy)phenyl][[3-(heptafluoropropyl)phenyl]methyl]amino]-1,1,1-trifluoro-2-propanol;
   3-[[3-(4-methylphenoxy)phenyl][[3-heptafluoropropyl]phenyl]methyl]amino]-1,1,1-trifluoro-2-propanol;
   3-[[3-(2-fluoro-5-bromophenoxy)phenyl][[3-(heptafluoropropyl)phenyl]-methyl]amino]-1,1,1-trifluoro-2-propanol;
   3-[[3-(4-chloro-3-ethylphenoxy)phenyl][[3-(heptafluoropropyl)phenyl]methyl]-amino]-1,1,1-trifluoro-2-propanol;
   3-[[3-(1,1,2,2-tetrafluoroethoxy)phenoxy]phenyl][[3-(heptafluoropropyl)-phenyl]methyl]amino]-1,1,1-trifluoro-2-propanol;
   3-[[3-[3-(pentafluoroethyl)phenoxy]phenyl][[3-(heptafluoropropyl)phenyl]-methyl]amino]-1,1,1-trifluoro-2-propanol;
   3-[[3-(3,5-dimethylphenoxy)phenyl][[3-(heptafluoropropyl)phenyl]methyl]-amino]-1,1,1-trifluoro-2-propanol;
   3-[[3-(3-ethylphenoxy)phenyl][[3-(heptafluoropropyl)phenyl]methyl]amino]-1,1,1-trifluoro-2-propanol;
   3-[[3-(3-t-butylphenoxy)phenyl][[3-(heptafluoropropyl)phenyl]methyl]amino]-1,1,1-trifluoro-2-propanol;
   3-[[3-(3-methylphenoxy)phenyl][[3-(heptafluoropropyl)phenyl]methyl]amino]-1,1,1-trifluoro-2-propanol;
   3-[[3-(5,6,7,8-tetrahydro-2-naphthoxy)phenyl][[3-(heptafluoropropyl)phenyl]methyl]amino]-1,1,1-trifluoro-2-propanol;
   3-[[3-(phenoxy)phenyl][[3-(heptafluoropropyl)phenyl]methyl]amino]-1,1,1-trifluoro-2-propanol;
   3-[[3-[3-(N,N-dimethylamino)phenoxy]phenyl][[3-(heptafluoropropyl)phenyl]-methyl]amino]-1,1,1-trifluoro-2-propanol;
   3-[[[3-(heptafluoropropyl)phenyl]methyl][3-[[3-(trifluoromethoxy)phenyl]-methoxy]phenyl]amino]-1,1,1-trifluoro-2-propanol:
   3-[[[3-(heptafluoropropyl)phenyl]methyl][3-[[3-(trifluoromethyl)phenyl]-methoxy]phenyl]amino]-1,1,1-trifluoro-2-propanol;
   3-[[[3-(heptafluoropropyl)phenyl]methyl][3-[[3,5-dimethylphenyl]methoxy]-phenyl]amino]-1,1,1-trifluoro-2-propanol;
   3-[[[3-(heptafluoropropyl)phenyl]methyl][3-[[3-(trifluoromethylthio)phenyl]-methoxy]phenyl]amino]-1,1,1-trifluoro-2-propanol;
   3-[[[3-(heptafluoropropyl)phenyl]methyl][3-[[3,5-difluorophenyl]methoxy]-phenyl]amino]-1,1,1-trifluoro-2-propanol;
   3-[[[3-(heptafluoropropyl)phenyl]methyl][3-[cyclohexylmethoxy]phenyl]-amino]-1,1,1-trifluoro-2-propanol;
   3-[[3-(2-difluoromethoxy-4-pyridyloxy)phenyl][[3-(heptafluoropropyl)phenyl]-methyl]amino]-1,1,1-trifluoro-2-propanol;
   3-[[3-(2-trifluoromethyl-4-pyridyloxy)phenyl][[3-(heptafluoropropyl)phenyl]-methyl]amino]-1,1,1-trifluoro-2-propanol;
   3-[[3-(3-difluoromethoxyphenoxy)phenyl][[3-(heptafluoropropyl)phenyl]-methyl]amino]-1,1,1-trifluoro-2-propanol;
   3-[[[3-(3-trifluoromethylthio)phenoxylphenyl][[3-(heptafluoropropyl)phenyl]-methyl]amino]-1,1,1-trifluoro-2-propanol:
   3-[[3-(4-chloro-3-trifluoromethylphenoxy)phenyl][[3-(heptafluoropropyl)-phenyl]methyl]amino]-1,1,1-trifluoro-2-propanol;
   3-[[3-(3-trifluoromethoxyphenoxy)phenyl][[2-fluoro-5-(trifluoromethyl)-phenyl]methyl]amino]-1,1,1-trifluoro-2-propahol;
   3-[[3-(3-isopropylphenoxy)phenyl][[2-fluoro-5-(trifluoromethyl)phenyl]-methyl]amino]-1,1,1-trifluoro-2-propanol;
   3-[[3-(3-cyclopropylphenoxy)phenyl] [[2-fluoro-5-(trifluoromethyl)phenyl]-methyl]amino]-1,1,1-trifluoro-2-propanol;
   3-[[3-(3-(2-furyl)phenoxy)phenyl][[2-fluoro-5-(trifluoromethyl)phenyl]-methyl]amino]-1,1,1-trifluoro-2-propanol;
   3-[[3-(2,3-dichlorophenoxy)phenyl][[2-fluoro-5-(trifluorometyl)phenyl]-methyl]amino]-1,1,1-trifluoro-2-propanol;
   3-[[3-(4-fluorophenoxy)phenyl][[2-fluoro-5-(trifluoromethyl)phenyl]-methyl]amino]-1,1,1-trifluoro-2-propanol;
   3-[[3-(4-methylphenoxy)phenyl][[2-fluoro-5-(trifluoromethyl)phenyl]methyl]amino]-1,1,1-trifluoro-2-propanol;
   3-[[3-(2-fluoro-5-bromophenoxy)phenyl][[2-fluoro-5-(trifluoromethyl)-phenyl]methyl]amino]-1,1,1-trifluoro-2-propanol;
   3-[[3-(4-chloro-3-ethylphenoxy)phenyl][[2-fluoro-5-(trifluoromethyl)-phenyl]methyl]amino]-1,1,1-trifluoro-2-propanol;
   3-[[3-[3-(1,1,2,2-tetrafluoroethoxy)phenoxy]phenyl][[2-fluoro-5-(trifluoro-methyl)-phenyl]methyl]amino]-1,1,1-trifluoro-2-propanol;
   3-[[3-[3-(pentafluoroethyl)phenoxy]phenyl][[2-fluoro-5-(trifluoromethyl)-phenyl]methyl]amino]-1,1,1-trifluoro-2-propanol:
   3-[[3-(3,5-dimethylphenoxy)phenyl][[2-fluoro-5-(trifluoromethyl)phenyl]-methyl]amino]-1,1,1-trifluoro-2-propanol;
   3-[[3-(3-ethylphenoxy)phenyl][[2-fluoro-5-(trifluoromethyl)phenyl]methyl]-amino]-1,1,1-trifluoro-2-propanol;
   3-[[3-(3-t-butylphenoxy)phenyl][[2-fluoro-5-(trifluoromethyl)phenyl]methyl]-amino]-1,1,1-trifluoro-2-propanol;
   3-[[3-(3-methylphenoxy)phenyl][[2-fluoro-5-(trifluoromethyl)phenyl]methyl]-amino]-1,1,1-trifluoro-2-propanol;
   3-[[3-(5,6,7,8-tetrahydro-2-naphthoxy)phenyl][[2-fluoro-5-(trifluoromethyl)-phenyl]methyl]amino]-1,1,1-trifluoro-2-propanol;
   3-[[3-(phenoxy)phenyl][[2-fluoro-5-(trifluoromethyl)phenyl]methyl]mino]-1,1,1-trifluoro-2-propanol;
   3-[[3-[3-(N,N-dimethylamino)phenoxy]phenyl][[2-fluoro-5-(trifluoromethyl)-phenyllmethyllamino]-1,1,1-trifluoro-2-propanol;
   3-[[[2-fluoro-5-(trifluoromethyl)phenyl]methyl][3-[[3-(trifluoromethoxy)-phenyl]methoxy]phenyl]amino]-1,1,1-trifluoro-2-propanol;
   3-[[[2-fluoro-5-(trifluoromethyl)phenyl]methyl][3-[[3-(trifluoromethyl)-phenyl]methoxy]phenyl]amino]-1,1,1-trifluoro-2-propanol;
   3-[[[2-fluoro-5-(trifluoromethyl)phenyl]methyl][3-[[3,5-dimethylphenyl]-methoxy]phenyl]amino]-1,1,1-trifluoro-2-propanol:
   3-[[[2-fluoro-5-(trifluoromethyl)phenyl]methyl][3-[[3-(trifluoromethylthio)-phenyl]methoxy]phenyl]amino]-1,1,1-trifluoro-2-propanol;
   3-[[[2-fluoro-5-(trifluoromethyl)phenyl]methyl][3-[[3,5-difluorophenyl]-methoxy]phenyl]amino]-1,1,1-trifluoro-2-propanol;
   3-[[[2-fluoro-5-(trifluoromethyl)phenyl]methyl][3-[cyclohexylmethoxy]-phenyl]amino]-1,1,1-trifluoro-2-propanol;
   3-[[3-(2-difluoromethoxy-4-pyridyloxy)phenyl][[2-fluoro-5-(trifluoromethyl)-phenyl]methyl]amino]-1,1,1-trifluoro-2-propanol;
   3-[[3-(2-trifluoromethyl-4-pyridyloxy)phenyl [[2-fluoro-5-(trifluoromethyl)-phenyl]methyl]amino]-1,1,1-trifluoro-2-propanol;
   3-[[3-(3-difluoromethoxyphenoxy)phenyl][[2-fluoro-5-(trifluoromethyl)-phenyl]methyl]amino]-1,1,1-trifluoro-2-propanol;
   3-[[[3-(3-trifluoromethylthio)phenoxy]phenyl][[2-fluoro-5-(trifluoromethyl)-phenyl]methyl]amino]-1,1-1-trifluoro-2-propanol;
   3-[[3-(4-chloro-3-trifluoromethylphenoxy)phenyl][[2-fluoro-5-(trifluoromethyl)phenyl]methyl]amino]-1,1,1-trifluoro-2-propanol;
   3-[[3-(3-trifluoromethoxyphenoxy)phenyl][[2-fluoro-4-(trifluoromethyl)-phenyl]methyl]amino]-1,1,1-trifluoro-2-propanol;
   3-[[3-(3-isopropylphenoxy)phenyl][[2-fluoro-4-(trifluoromethyl)phenyl]-methyl]amino]-1,1,1-trifluoro-2-propanol;
   3-[[3-(3-cyclopropylphenoxy)phenyl][[2-fluoro-4-(trifluoromethyl)phenyl]-methyl]amino]-1,1,1-trifluoro-2-propanol;
   3-[[3-(3-(2-furyl)phenoxy)phenyl][[2-fluoro-4-(trifluoromethyl)phenyl]-methyl]amino]-1,1,1-trifluoro-2-propanol;
   3-[[3-(2,3-dichlorophenoxy)phenyl][[2-fluoro-4-(trifluoromethyl)phenyl]-methyl]amino]-1,1,1-trifluoro-2-propanol;
   3-[[3-(4-fluorophenoxy)phenyl][[2-fluoro-4-trifluoromethyl]phenyl]-methyl]amino]-1,1,1-trifluoro-2-propanol;
   3-[[3-(4-methylphenoxy)phenyl][[2-fluoro-4-(trifluoromethyl)phenyl]-methyl]amino]-1,1,1-trifluoro-2-propanol;
   3-[[3-(2-fluoro-5-bromophenoxy)phenyl][[2-fluoro-4-(trifluoromethyl)-phenyl]methyl]amino]-1,1,1-trifluoro-2-propanol;
   3-[[3-(4-chloro-3-ethylphenoxy)phenyl][[2-fluoro-4-(trifluoromethyl)-phenyl]methyl]amino]-1,1,1-trifluoro-2-propanol;
   3-[[3-[3-(1,1,2,2-tetrafluoroethoxy)phenoxy]phenyl][[2-fluoro-4-(trifluoro-methyl)phenyl]methyl]amino]-1,1,1-trifluoro-2-propanol;
   3-[[3-[3-(pentafluoroethyl)phenoxy]phenyl][[2-fluoro-4-(trifluoromethyl)-phenyl]methyl]amino]-1,1,1-triflucro-2-propanol;
   3-[[3-(3,5-dimethylphenoxy)phenyl][[2-fluoro-4-(trifluoromethyl)phenyl]-methyl]amino]-1,1,1-trifluoro-2-propanol;
   3-[[3-(3-ethylphenoxy)phenyl][[2-fluoro-4-(trifluoromethyl)phenyl]methyl]-amino]-1,1,1-trifluoro-2-propanol;
   3-[[3-(3-t-butylphenoxy)phenyl] [[2-fluoro-4-(trifluoromethyl)phenyl]methyl]-amino]-1,1,1-trifluoro-2-propanol;
   3-[[3-(3-methylphenoxy)phenyl][[2-fluoro-4-(trifluoromethyl)phenyl]methyl]-amino]-1,1,1-trifluoro-2-propanol;
   3-[[3-(5,6,7,8-tetrahydro-2-naphthoxy)phenyl][[2-fluoro-4-(trifluoromethyl)-phenyl]methyl]amino]-1,1,1-trifluoro-2-propanol;
   3-[[3-(phenoxy)phenyl][[2-fluoro-4-(trifluoromethyl)phenyl]methyl]amino]-1,1,1-trifluoro-2-propanol;
   3-[[3-3-(N,N-dimethylamino)phenoxy]phenyl][[2-fluoro-4-(trifluoromethyl)-phenyl]methyl]amino]-1,1,1-trifluoro-2-propanol;
   3-[[[2-fluoro-4-(trifluoromethyl)phenyl]methyl][3-[[3-(trifluoromethoxy)-phenyl]methoxy]phenyl]amino]-1,1,1-trifluoro-2-propanol;
   3-[[[2-fluoro-4-(trifluoromethyl)phenyl]methyl][3-[[3-(trifluoromethyl)-phenyl]methoxy]phenyl]amino]-1,1,1-trifluoro-2-propanol;
   3-[[[2-fluoro-4-(trifluoromethyl)phenyl]methyl][3-[[3,5-dimethylphenyl]methoxy]phenyl]amino]-1,1,1-trifluoro-2-propanol;
   3-[[[2-fluoro-4-(trifluoromethyl)phenl]methyl][3-[[3-(trifluoromethylthio)-phenyl]methoxy]phenyl]amino]-1,1,1-trifluoro-2-propanol;
   3-[[[2-fluoro-4-(trifluoromethyl)phenyl]methyl][3-[[3,5-difluorophenyl]-methoxy]phenyl]amino]-1,1,1-trifluoro-2-propanol;
   3-[[[2-fluoro-4-(trifluoromethyl)phenyl]methyl] [3-[3-[cyclohexylmethoxy]-phenyl]amino]-1,1,1-trifluoro-2-propanol;
   3-[[3-(2-difluoromethoxy-4-pyridyloxy)phenyl][[2-fluoro-4-(trifluoromethyl)-phenyl]methyl]amino]-1,1,1-trifluoro-2-propanol;
   3-[[3-(2-trifluoromethyl-4-pyridyloxy)phenyl][[2-fluoro-4-(trifluoromethyl)-phenyl]methyl]amino]-1,1,1-trifluoro-2-propanol;
   3-[[3-3-difluoromethoxyphenoxy)phenyl][[2-fluoro-4-(trifluoromethyl)-phenyl]methyl]amino]-1,1,1-trifluoro-2-propanol;
   3-[[[3-(3-trifluoromethylthio)phenoxy]phenyl][[2-fluoro-4-(trifluoromethyl)-phenyl]methyl]amino]-1,1,1-trifluoro-2-propanol; and
   3-[[3-(4-chloro-3-trifluoromethylphenoxy)phenyl][[2-fluoro-4-(trifluoromethyl)phenyl]methyl]amino]-1,1,1-trifluoro-2-propanol, or a pharmaceutically acceptable salt thereof.
[77] The inhibitor of [74] or [75], wherein the compound of the formula (XV) is a compound selected from the group consisting of
   3-[[3-(3-trifluoromethoxyphenoxy)phenyl][[3-(1,1,2,2-tetrafluoroethoxy)-phenyl]methyl]amino]-1,1,1-trifluoro-2-propanol;
   3-[[3-(3-isopropylphenoxy)phenyl][[3-(1,1,2,2-tetrafluoroethoxy)phenyl]-methyl]amino]-1,1,1-trifluoro-2-propanol;
   3-[[3-(3-cyclopropylphenoxy)phenyl][[3-(1,1,2,2-tetrafluoroethoxy)phenyl]-methyl]amino]-1,1,1-trifluoro-2-propanol;
   3-[[3-(3-(2-furyl)phenoxy)phenyl][[3-(1,1,2,2-tetrafluoroethoxy)phenyl]-methyl]amino]-1,1,1trifluoro-2-propanol;
   3-[[3-(2,3-dichlorophenoxy)phenyl][[3-(1,1,2,2-tetrafluoroethoxy)phenyl]-methyl]amino]-1,1,1-trifluoro-2-propanol;
   3-[[3-(4-fluorophenoxy)phenyl][[3-(1,1,2,2-tetrafluoroethoxy)phenyl]-methyl]amino]-1,1,1-trifluoro-2-propanol;
   3-[[3-(4-methylphenoxy)phenyl][[3-(1,1,2,2-tetrafluoroethoxy)phenyl]-methyl]amino]-1,1,1-trifluoro-2-propanol;
   3-[[3-(2-fluoro-5-bromophenoxy)phenyl][[3-(1,1,2,2-tetrafluoroethoxy)phenyl]-methyl]amino]-1,1,1-trifluoro-2-propanol;
   3-[[3-(3-(4-chloro-3-ethylphenoxy)phenyl)[[3-(1,1,2,2-tetrafluoroethoxy)phenyl]-methyl]amino]-1,1,1-trifluoro-2-propanol;
   3-[[3-[3-(1,1,2,2-tetrafluoroethoxy)phenoxy]phenyl][[3-(1, 1,2,2-tetrafluoro-ethoxy)phenyl]methyl]ainino]-1,1,1-trifluoro-2-propanol;
   3-[[3-[3-(pentafluoroethyl)phenoxy]phenyl][[3-(1,1,2,2-tetrafluoroethoxy)-phenyl]methyl]amino]-1,1,1-trifluoro-2-propanol;
   3-[[3-(3,5-dimethylphenoxy)phenyl][[3-(1,1,2,2-tetrafluoroethoxy)phenyl]-methyl]amino]-1,1,1-trifluoro-2-propanol;
   3-[[3-(3-ethylphenoxy)phenyl][[3-(1,1,2,2-tetrafluoroethoxy)phenyl]-methyl]amino]-1,1,1-trifluoro-2-propanol;
   3-[[3-(3-t-butylphenoxy)phenyl][[3-(1,1,2,2-tetrafluoroethoxy)phenyll-methyllaminol-1,1,1-trifluoro-2-propanol;
   3-[[3-(3-methylphenoxy)phenyl][[3-(1,1,2,2-tetrafluoroethoxy)phenyl]-methyl]amino]-1,1,1-trifluoro-2-propanol;
   3-[[3-(5,6,7,8-tetrahydro-2-naphthoxy)phenyl][[3-(1,1,2,2-tetrafluoro-ethoxy)phenyl]methyl]amino]-1,1,1-trifluoro-2-propanol;
   3-[[3-(phenoxy)phenyl][[3-(1,1,2,2-tetrafluoroethoxy)phenyl]methyl]amino]-1,1,1-trifluoro-2-propanol;
   3-[[3-[3-(N,N-dimethylamino)phenoxy]phenyl][[3-(1,1,2,2-tetrafluoro-ethoxy)phenyl]methyl]amino]-1,1,1-trifluoro-2-propanol;
   3-[[[3-(1,1,2,2-tetrafluoroethoxy)phenyl]methyl][3-[[3-(trifluoromethoxy)-phenyl]methoxy]phenyl]amino]-1,1,1-trifluoro-2-propanol,
   3-[[[3-(1,1,2,2-tetrafluoroethoxy)phenyl]methyl][3-[[3-(trifluoromethyl)-phenyl]methoxy]phenyl]amino]-1,1,1-trifluoro-2-propanol;
   3-[[[3-(1,1,2,2-tetrafluoroethoxy)phenyl]methyl][3-[[3,5-dimethylphenyl]methoxy]phenyl]amino]-1,1,1-trifluoro-2-propanol;
   3-[[[3-(1,1,2,2-tetrafluoroethoxy)phenyl]methyl][3-[[3-(trifluoromethylthio)-phenyl]methoxy]phenyl]aminol-1,1,1-trifluoro-2-propanol;
   3-[[[3-(1,1,2,2-tetrafluoroethoxy)phenyl]methyl][3-[[3,5-difluorophenyl]methoxy]phenyl]amino]-1,1,1-trifluoro-2-propanol;
   3-[[[3-(1,1,2,2-tetrafluoroethoxy)phenyl]methyl][3-[cyclohexylmethoxy]phenyl]amino]-1,1,1-trifluoro-2-propanol;
   3-[[3-(2-difluoromethoxy-4-pyridyloxy)phenyl][[3-(1,1,2,2-tetrafluoroethoxy)-phenyl]methyl]amino]-1,1,1-trifluoro-2-propanol;
   3-[[3-(2-trifluoromethyl-4-pyridyloxy)phenyl][[3-(1,1,2,2-tetrafluoroethoxy)-phenyl]methyl]amino]-1,1,1-trifluoro-2-propanol;
   3-[[3-(3-difluoromethoxyphenoxy)phenyl][[3-(1,1,2,2-tetrafluoroethoxy)-phenyl]methyl]amino]-1,1,1-trifluoro-2-propanol;
   3-[[[3-(3-trifluoromethylthio)phenoxylphenyl][[3-(1,1,2,2-tetrafluoroethoxy)-phenyl]methyl]amino]-1,1,1-trifluoro-2-propanol;
   3-[[3-(4-chloro-3-trifluoromethylphenoxy)phenyl][[3-(1,1,2,2-tetrafluoroethoxy)-phenyl]methyl]amino]-1,1,1-trifluoro-2-propanol;
   3-[[3-(3-trifluoromethoxyphenoxy)phenyl][[3-(pentafluoroethyl)phenyl]-methyl]amino]-1,1,1-trifluoro-2-propanol;
   3-[[3-(3-isopropylphenoxy)phenyl][[3-(pentafluoroethyl)phenyl]methyl]-amino]-1,1,1-trifluoro-2-propanol;
   3-[[3-(3-cyclopropylphenoxy)phenyl][[3-(pentafluoroethyl)phenyl]methyl]-aminol-1,1,1-trifluoro-2-propanol;
   3-[[3-(3-(2-furyl)phenoxy)phenyl[[3-(pentafluoroethyl)phenyl]methyl]-amino]-1,1,1-trifluoro-2-propanol;
   3-[[3-(2,3-dichlorophenoxy)phenyl][[3-(pentafluoroethyl)phenyl]methyl]-amino]-1,1,1-trifluoro-2-propanol;
   3-[[3-(4-fluorophenoxy)phenyl][[3-(pentafluoroethyl)phenyl]methyl]amino]-1,1,1-trifluoro-2-propanol;
   3-[[3-(4-methylphenoxy)phenyl][[3-(pentafluoroethyl)phenyl]methyl]amino]-1,1,1-trifluoro-2-propanol;
   3-[[3-(2-fluoro-5-bromophenoxy)phenyl][[3-(pentafluoroethyl)phenyl]methyl]-amino]-1,1,1-trifluoro-2-propanol;
   3-[[3-(4-chloro-3-ethylphenoxy)phenyl][[3-(pentafluoroethyl)phenyl]methyl]-amino]-1,1,1-trifluoro-2-propanol;
   3-[[3-[3-(1,1,2,2-tetrafluoroethoxy)phenoxy]phenyl][[3-(pentafluoroethyl)-phenyl]methyl]amino]-1,1,1-trifluoro-2-propanol;
   3-[[3-[3-(pentafluoroethyl)phenoxy]phenyl][[3-(pentafluoroethyl)phenyl]-methyl]amino]-1,1,1-trifluoro-2-propanol;
   3-[[3-(3,5-dimethylphenoxy)phenyl][[3-(pentafluoroethyl)phenyl]methyl]-amino]-1,1,1-trifluoro-2-propanol;
   3-[[3-(3-ethylphenoxy)phenyl][[3-(pentafluoroethyl)phenyl]methyl]amino]-1,1,1-trifluoro-2-propanol;
   3-[[3-(3-t-butylphenoxy)phenyl][[3-(pentafluoroethyl)phenyl]methyl]amino]-1,1,1-trifluoro-2-propanol;
   3-[[3-[3-methylphenoxy]phenyl][[3-(pentafluoroethyl)phenyl]methyl]amino]-1,1,1-trifluoro-2-propanol;
   3-[[3-[3-(5,6,7,8-tetrahydro-2-naphthoxy)phenyl][[3-(pentafluoroethyl)phenyl]-methyl]amino-1,1,1-trifluoro-2-propanol;
   3-[[3-(phenoxy)phenyl][[3-(pentafluoroethyl)phenyl]methyl]amino]-1,1,1-trifluoro-2-propanol;
   3-[[3-[3-(N,N-dimethylamino)phenoxy]phenyl][[3-(pentafluoroethyl)phenyl]-methyl]amino]-1,1,1-trifluoro-2-propanol;
   3-[[[3-(pentafluoroethyl)phenyl]methyl][3-[[3-(trifluoromethoxy)phenyl]-methoxy]phenyl]amino]-1,1,1-trifluoro-2-propanol;
   3-[[[3-(pentafluoroethyl)phenyl]methyl][3-[[3-(trifluoromethyl)phenyl]-methoxy]phenyl]amino]-1,1,1-trifluoro-2-propanol;
   3-[[[3-(pentafluoroethyl)phenyl]methyl][3-[[3-5-dimethylphenyl]methoxy]-phenyl]amino]-1,1,1-trifluoro-2-propanol;
   3-[[[3-(pentafluoroethyl)phenyl]methyl][3-[[3-(trifluoromethylthio)phenyl]-methoxy]phenyl]amino]-1,1,1-trifluoro-2-propanol;
   3-[[[3-(pentafluoroethyl)phenyl]methyl][3-[[3,5-difluorophenyl]methoxy]-phenyl]amino]-1,1,1-trifluoro-2-propanol;
   3-[[[3-(pentafluoroethyl)phenyl]methyl][3-[cyclohexylmethoxy]phenyl]-amino]-1,1,1-trifluoro-2-propanol;
   3-[[3-(2-difluoromethoxy-4-pyridyloxy)phenyl][[3-(pentafluoroethyl)phenyl]-methyl]amino]-1,1,1-trifluoro-2-propanol;
   3-[[3-(2-trifluoromethyl-4-pyridyloxy)phenyl][[3-(pentafluoroethyl)phenyl]-methyl]amino]-1,1,1-trifluoro-2-propanol;
   3-[[3-(3-difluoromethoxyphenoxy)phenyl][[3-(pentafluoroethyl)phenyl]-methyl]amino]-1,1,1-trifluoro-2-propanol;
   3-[[[3-(3-trifluoromethylthio)phenoxy]phenyl][[3-(pentafluoroethyl)phenyl]-methyl]amino]-1,1,1-trifluoro-2-propanol;
   3-[[3-(4-chloro-3-trifluoromethylphenoxy)phenyl][[3-(pentafluoroethyl)-phenyllmethyljamino]-1,1,1-trifluoro-2-propanol;
   3-[[3-(3-trifluoromethoxyphenoxy)phenyl][[3-(heptafluoropropyl)phenyl]-methyl]amino]-1,1,1-trifluoro-2-propanol;
   3-[[3-(3-isopropylphenoxy)phenyl][[3-(heptafluoropropyl)phenyl]methyl]-amino]-1,1,1-trifluoro-2-propanol;
   3-[[3-(3-cyclopropylphenoxy)phenyl][[3-(heptafluoropropyl)phenyl]methyl]-amino]-1,1,1-trifluoro-2-propanol;
   3-[[3-(3-(2-furyl)phenoxy)phenyl][[3-(heptafluoropropyl)phenyl]methyl]-amino]-1,1,1-trifluoro-2-propanol;
   3-[[3-(2,3-dichlorophenoxy)phenyl]-[[3-(heptafluoropropyl)phenyl]methyl]-amino]-1,1,1-trifluoro-2-propanol;
   3-[[3-(4-fluorophenoxy)phenyl][3-(heptafluoropropyl)phenyl]methyl]amino]-1,1,1-trifluoro-2-propanol;
   3-[[3-(4-methylphenoxy)phenyl][[3-heptafluoropropyl]phenyl]methyl]amino]-1,1,1-trifluoro-2-propanol;
   3-[[3-(2-fluoro-5-bromophenoxy)phenyl][[3-(heptafluoropropyl)phenyl]-methyl]amino]-1,1,1-trifluoro-2-propanol;
   3-[[3-(4-chloro-3-ethylphenoxy)phenyl][[3-(heptafluoropropyl)phenyl]methyl]-amino]-1,1,1-trifluoro-2-propanol;
   3-[[3-(1,1,2,2-tetrafluoroethoxy)phenoxy]phenyl][[3-(heptafluoropropyl)-phenyl]methyl]amino]-1,1,1-trifluoro-2-propanol;
   3-[[3-[3-(pentafluoroethyl)phenoxy]phenyl][[3-(heptafluoropropyl)phenyl]-methyl]amino]-1,1,1-trifluoro-2-propanol;
   3-[[3-(3,5-dimethylphenoxy)phenyl][[3-(heptafluoropropyl)phenyl]methyl]-amino]-1,1,1-trifluoro-2-propanol;
   3-[[3-(3-ethylphenoxy)phenyl][[3-(heptafluoropropyl)phenyl]methyl]amino]-1,1,1-trifluoro-2-propanol;
   3-[[3-(3-t-butylphenoxy)phenyl][[3-(heptafluoropropyl)phenyl]methyl]amino]-1,1,1-trifluoro-2-propanol;
   3-[[3-(3-methylphenoxy)phenyl][3-(heptafluoropropyl)phenyl]methyl]amino]-1,1,1-trifluoro-2-propanol;
   3-[[3-(5,6,7,8-tetrahydro-2-naphthoxy)phenyl][[3-(heptafluoropropyl)phenyl]methyl]amino]-1,1,1-trifluoro-2-propanol;
   3-[[3-(phenoxy)phenyl][[3-(heptafluoropropyl)phenyl]methyl]amino]-1,1,1-trifluoro-2-propanol;
   3-[[3-[3-(N,N-dimethylamino)phenoxy]phenyl][[3-(heptafluoropropyl)phenyl]-methyl]amino]-1,1,1-trifluoro-2-propanol;
   3-[[[3-(heptafluoropropyl)phenyl]methyl][3-[[3-(trifluoromethoxy)phenyl]-methoxy]-phenyl]amino]-1,1,1-trifluoro-2-propanol:
   3-[[[3-(heptafluoropropyl)phenyl]methyl][3-[[3-(trifluoromethyl)phenyl]-methoxy]phenyl]amino]-1,1,1-trifluoro-2-propanol;
   3-[[[3-(heptafluoropropyl)phenyl]methyl][3-[[3,5-dimethylphenyl]methoxy]-phenyl]amino]-1,1,1-trifluoro-2-propanol;
   3-[[[3-(heptafluoropropyl)phenyl]methyl][3-[[3-(trifluoromethylthio)phenyl]-methoxy]phenyl]amino]-1,1,1-trifluoro-2-propanol;
   3-[[[3-(heptafluoropropyl)phenyl]methyl][3-[[3,5-difluorophenyl]methoxy]-phenyl]amino]-1,1,1-trifluoro-2-propanol;
   3-[[[3-(heptafluoropropyl)phenyl]methyl][3-[cyclohexylmethoxy]phenyl]-amino]-1,1,1-trifluoro-2-propanol;
   3-[[3-(2-difluoromethoxy-4-pyridyloxy)phenyl][[3-(heptafluoropropyl)phenyl]-methyl]amino]-1,1,1-trifluoro-2-propanol;
   3-[[3-(2-trifluoromethyl-4-pyridyloxy)phenyl][[3-(heptafluoropropyl)phenyl]-methyl]amino]-1,1,1-trifluoro-2-propanol;
   3-[[3-(3-difluoromethoxyphenoxy)phenyl][[3-(heptafluoropropyl)phenyl]-methyl]amino]-1,1,1-trifluoro-2-propanol;
   3-[[[3-(3-trifluoromethylthio)phenoxy]phenyl][[3-(heptafluoropropyl)phenyl]-methyl]amino]-1,1,1-trifluoro-2-propanol:
   3-[[3-(4-chloro-3-trifluoromethylphenoxy)phenyl][[3-(heptafluoropropyl)-phenyl]methyl]amino]-1,1,1-trifluoro-2-propanol;
   3-[[3-(3-trifluoromethoxyphenoxy)phenyl][[2-fluoro-5-(trifluoromethyl)-phenyl]methyl]amino]-1,1,1-trifluoro-2-propanol;
   3-[[3-(3-isopropylphenoxy)phenyl][[2-fluoro-5-(trifluoromethyl)phenyl]-methyl]amino]-1,1,1-trifluoro-2-propanol;
   3-[[3-(3-cyclopropylphenoxy)phenyl][[2-fluoro-5-(trifluoromethyl)phenyl]-methyl]amino]-1,1,1-trifluoro-2-propanol;
   3-[[3-(3-(2-furyl)phenoxy)phenyl][[2-fluoro-5-(trifluoromethyl)phenyl]-methyl]amino]-1,1,1-trifluoro-2-propanol;
   3-[[3-(2,3-dichlorophenoxy)phenyl][[2-fluoro-5-(trifluorometyl)phenyl]-methyl]amino]-1,1,1-trifluoro-2-propanol;
   3-[[3-(4-fluorophenoxy)phenyl][[2-fluoro-5-(trifluoromethyl)phenyl]-methyl]amino]-1,1,1-trifluoro-2-propanol;
   3-[[3-(4-methylphenoxy)phenyl][[2-fluoro-5-(trifluoromethyl)phenyl]methyl]amino]-1,1,1-trifluoro-2-propanol;
   3-[[3-(2-fluoro-5-bromophenoxy)phenyl][[2-fluoro-5-(trifluoromethyl)-phenyl]methyl]aminol-1,1,1-trifluoro-2-propanol;
   3-[[3-(4-chloro-3-ethylphenoxy)phenyl][[2-fluoro-5-(trifluoromethyl)-phenyl]methyl]amino]-1,1,1-trifluoro-2-propanol;
   3-[[3-[3-(1,1,2,2-tetrafluoroethoxy)phenoxy]phenyl][[2-fluoro-5-(trifluoro-methyl)-phenyl]methyl]amino]-1,1,1-trifluoro-2-propanol;
   3-[[3-[3-[pentafluoroethyl)phenoxy]phenyl][[2-fluoro-5-(trifluoromethyl)-phenyl]methyl]amino]-1,1,1-trifluoro-2-propanol:
   3-[[3-(3,5-dimethylphenoxy)phenyl][[2-fluoro-5-(trifluoromethyl)phenyl]-methyl]amino]-1,1,1-trifluoro-2-propanol;
   3-[[3-(3-ethylphenoxy)phenyl][[2-fluoro-5-(trifluoromethyl)phenyl]methyl]-amino]-1,1,1-trifluoro-2-propanol;
   3-[[3-(3-t-butylphenoxy)phenyl][[2-fluoro-5-(trifluoromethyl)phenyl]methyl]-amino]-1,1,1-trifluoro-2-propanol;
   3-[[3-(3-methylphenoxy)phenyl][[2-fluoro-5-(trifluoromethyl)phenyl]methyl]-amino]-1,1,1-trifluoro-2-propanol;
   3-[[3-(5,6,7,8-tetrahydro-2-naphthoxy)phenyl][[2-fluoro-5-(trifluoromethyl)-phenyl]methyl]amino]-1,1,1-trifluoro-2-propanol;
   3-[[3-(phenoxy)phenyl][[2-fluoro-5-(trifluoromethyl)phenyl]methyl]amino]-1,1,1-trifluoro-2-propanol;
   3-[[3-[3-(N,N-dimethylamino)phenoxy]phenyl][[2-fluoro-5-(trifluoromethyl)-phenyl]methyl]amino]-1,1,1-trifluoro-2-propanol;
   3-[[[2-fluoro-5-(trifluoromethyl)phenyl]methyl][3-[[3-(trifluoromethoxy)-phenyl]methoxy]phenyl]amino]-1,1,1-trifluoro-2-propanol;
   3-[[[2-fluoro-5-(trifluoromethyl)phenyl]methyl][3-[[3-(trifluoromethyl)-phenyl]methoxy]phenyl]amino]-1,1,1-trifluoro-2-propanol;
   3-[[[2-fluoro-5-(trifluoromethyl)phenyl]methyl][3-[[3,5-dimethylphenyl]-methoxy]phenyl]amino]-1,1,1-trifluoro-2-propanol:
   3-[[[2-fluoro-5-(trifluoromethyl)phenyl]methyl][3-[[3-(trifluoromethylthio)-phenyl]methoxy]phenyl]amino]-1,1,1-trifluoro-2-propanol;
   3-[[[2-fluoro-5-(trifluoromethyl)phenyl]methyl][3-[[3,5-difluorophenyl]-methoxy]phenyl]amino]-1,1,1-trifluoro-2-propanol;
   3-[[[2-fluoro-5-(trifluoromethyl)phenyl]methyl][3-[cyclohexylmethoxy]-phenyl]amino]-1,1,1-trifluoro-2-propanol;
   3-[[3-(2-difluoromethoxy-4-pyridyloxy)phenyl][[2-fluoro-5-(trifluoromethyl)-phenyl]methyl]amino]-1,1,1-trifluoro-2-propanol;
   3-[[3-(2-trifluoromethyl-4-pyridyloxy)phenyl][[2-fluoro-5-(trifluoromethyl)-phenyl]methyl]amino]-1,1,1-trifluoro-2-propanol;
   3-[[3-(3-difluoromethoxyphenoxy)phenyl][[2-fluoro-5-(trifluoromethyl)-phenyl]methyl]amino]-1,1,1-trifluoro-2-propanol;
   3-[[[3-(3-trifluoromethylthio)phenoxy]phenyl][[2-fluoro-5-(trifluoromethyl)-phenyl]methyl]amino]-1,1,1-trifluoro-2-propanol;
   3-[[3-(4-chloro-3-trifluoromethylphenoxy)phenyl][[2-fluoro-5-(trifluoromethyl)phenyl]methyl]amino]-1,1,1-trifluoro-2-propanol;
   3-[[3-(3-trifluoromethoxyphenoxy)phenyl][[2-fluoro-4-(trifluoromethyl)-phenyl]methyl]amino]-1,1,1-trifluoro-2-propanol;
   3-[[3-(3-isopropylphenoxy)phenyl][[2-fluoro-4-(trifluoromethyl)phenyl]-methyl]amino]-1,1,1-trifluoro-2-propanol;
   3-[[3-(3-cyclopropylphenoxy)phenyl][[2-fluoro-4-(trifluoromethyl)phenyl]-methyl]amino]-1,1,1-trifluoro-2-propanol;
   3-[[3-(3-(2-furyl)phenoxy)phenyl][[2-fluoro-4-(trifluoromethyl)phenyl]-methyl]amino]-1,1,1-trifluoro-2-propanol;
   3-[[3-(2,3-dichlorophenoxy)phenyl][[2-fluoro-4-(trifluoromethyl)phenyl]-methyl]amino]-1,1,1-trifluoro-2-propanol;
   3-[[3-(4-fluorophenoxy)phenyl][[2-fluoro-4-trifluoromethyl]phenyl]-methyl]amino]-1,1,1-trifluoro-2-propanol;
   3-[[3-(4-methylphenoxy)phenyl][[2-fluoro-4-(trifluoromethyl)phenyl]-methyl]amino]-1,1,1-trifluoro-2-propanol;
   3-[[3-(2-fluoro-5-bromophenoxy)phenyl][[2-fluoro-4-(trifluoromethyl)-phenyl]methyl]amino]-1,1,1-trifluoro-2-propanol;
   3-[[3-(4-chloro-3-ethylphenoxy)phenyl][[2-fluoro-4-(trifluoromethyl)-phenyl]methyl]amino]-1,1,1-trifluoro-2-propanol;
   3-[[3-[3-(1,1,2,2-tetrafluoroethoxy)phenoxy]phenyl][[2-fluoro-4-(trifluoro-methyl)phenyl]methyl]amino]-1,1,1-trifluoro-2-propanol;
   3-[[3-[3-(pentafluoroethyl)phenoxy]phenyl][[2-fluoro-4-(trifluoromethyl)-phenyl]methyl]amino]-1,1,1-trifluoro-2-propanol;
   3-[[3-(3,5-dimethylphenoxy)phenyl][[2-fluoro-4-(trifluoromethyl)phenyl]-methyl]amino]-1,1,1-trifluoro-2-propanol;
   3-[[3-(3-ethylphenoxy)phenyl][[2-fluoro-4-(trifluoromethyl)phenyl]methyl]-amino]-1,1,1-trifluoro-2-propanol;
   3-[[3-(3-t-butylphenoxy)phenyl][[2-fluoro-4-(trifluoromethyl)phenyl]methyl]-amino]-1,1,1-trifluoro-2-propanol;
   3-[[3-(3-methylphenoxy)phenyl][[2-fluoro-4-(trifluoromethyl)phenyl]methyl]-amino]-1,1,1-trifluoro-2-propanol;
   3-[[3-(5,6,7,8-tetrahydro-2-naphthoxy)phenyl][[2-fluoro-4-(trifluoromethyl)-phenyl]methyl]amino]-1,1,1-trifluoro-2-propanol;
   3-[[3-(phenoxy)phenyl][[2-fluoro-4-(trifluoromethyl)phenyl]methyl]amino]-1,1,1-trifluoro-2-propanol;
   3-[[3-[3-(N,N-dimethylamino)phenoxy]phenyl][[2-fluoro-4-(trifluoromethyl)-phenyl]methyl]amino]-1,1,1-trifluoro-2-propanol;
   3-[[[2-fluoro-4-(trifluoromethyl)phenyl]methyl][3-[[3-(trifluoromethoxy)-phenyl]methoxy]phenyl]amino]-1,1,1-trifluoro-2-propanol;
   3-[[[2-fluoro-4-(trifluoromethyl)phenyl]methyl][3-[[3-(trifluoromethyl)-phenyl]methoxy]phenyl]amino]-1,1,1-trifluoro-2-propanol;
   3-[[[2-fluoro-4-(trifluoromethyl)phenyl]methyl][3-[[3,5-dimethylphenyl]methoxy]phenyl]amino]-1,1,1-trifluoro-2-propanol;
   3-[[[2-fluoro-4-(trifluoromethyl)phenl]methyl][3-[[3-(trifluoromethylthio)-phenyl]methoxy]phenyl]amino]-1,1,1-trifluoro-2-propanol;
   3-[[[2-fluoro-4-(trifluoromethyl)phenyl]methyl][3-[[3,5-difluorophenyl]-methoxy]phenyl]amino]-1,1,1-trifluoro-2-propanol;
   3-[[[2-fluoro-4-(trifluoromethyl)phenyl]methyl][3-[3-[cyclohexylmethoxy]-phenyl]amino]-1,1,1-trifluoro-2-propanol;
   3-[[3-(2-difluoromethoxy-4-pyridyloxy)phenyl][[2-fluoro-4-(trifluoromethyl)-phenyl]methyl]amino]-1,1,1-trifluoro-2-propanol;
   3-[[3-(2-trifluoromethyl-4-pyridyloxy)phenyl][[2-fluoro-4-(trifluoromethyl)-phenyl]methyl]amino]-1,1,1-trifluoro-2-propanol;
   3-[[3-(3-difluoromethoxyphenoxy)phenyl][[2-fluoro-4-(trifluoromethyl)-phenyl]methyl]amino]-1,1,1-trifluoro-2-propanol;
   3-[[[3-(3-trifluoromethylthio)phenoxy]phenyl][[2-fluoro-4-(trifluoromethyl)-phenyl]methyl]amino]-1,1,1-trifluoro-2-propanol; and
   3-[[3-(4-chloro-3-trifluoromethylphenoxy)phenyl][[2-fluoro-4-(trifluoromethyl)phenyl]methyl]amino]-1,1,1-trifluoro-2-propanol, or a pharmaceutically acceptable salt thereof.
[78] The method of [1], [3] or [6], wherein the compound having a CETP inhibitory activity is a compound having the formula (XVI): 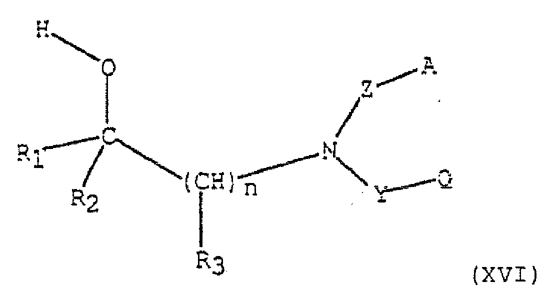 or a pharmaceutically acceptable salt thereof,
   wherein
   n is an integer selected from 1 through 2;
   A and Q are independently selected from the group consisting of -CH₂(CR₃₇R₃₈)ᵥ-(CR₃₃R₃₄)ᵤ-T-(CR₃₅R₃₆)_{w}-H, 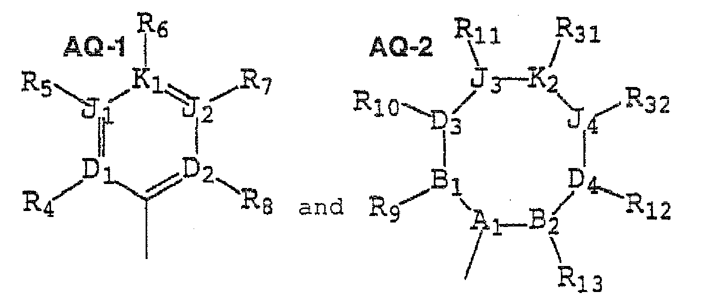
   with the provisos that one of A and Q must be AQ-1 and that one of A and Q must be selected from the group consisting of AQ-2 and -CH₂(CR₃₇R₃₈)ᵥ-(CR₃₃R₃₄)ᵤ-T-(CR₃₅R₃₆)_{w}-H;
   T is selected from the group consisting of a single covalent bond, O, S , S(O), S(O)₂, C(R₃₃)=C(R₃₅), and C≡C;
   v is an integer selected from 0 through 1 with the proviso that v is 1 when any one of R₃₃, R₃₄, R₃₅, and R₃₆ is aryl or heteroaryl;
   u and w are integers independently selected from 0 through 6;
   A₁ is C(R₃₀);
   D₁, D₂, J₁, J₂ and K₁ are independently selected from the group consisting of C, N, O, S and a covalent bond with the provisos that no more than one of D₁, D_{2,} J₁, J₂ and K₁ is a covalent bond, no more than one of D₁,D₂, J₁, J₂ and K₁ is O, no more than one of D₁, D₂, J₁, J₂ and K₁ is S, one of D₁, D₂, J₁, J₂ and K₁ must be a covalent bond when two of D₁, D₂, J₁, J₂ and K₁ are O and S, and no more than four of D₁, D₂, J₁, J₂ and K₁ are N;
   B₁, B₂, D₃, D₄, J₃, J₄ and K₂ are independently selected from the group consisting of C, C(R₃₀), N, O, S and a covalent bond with the provisos that no more than 5 of B₁, B₂, D₃, D₄, J₃, J₄ and K₂ are a covalent bond, no more than two of B₁, B₂, D₃, D₄, J₃, J₄ and K₂ are O, no more than two of B₁, B₂, D₃, D₄, J₃, J₄ and K₂ are S, no more than two of B₁, B₂, D₃, D₄, J₃, J₄ and K₂ are simultaneously O and S, and no more than two of B₁, B₂ D₃, D₄, J₃, J₄ and K₂ are N; B₁ and D₃, D₃ and J₃, J₃ and K₂, K₂ and J₄, J₄ and D₄, and D₄ and B₂ are independently selected to form an in-ring spacer pair wherein said spacer pair is selected from the group consisting of C(R₃₃)=C(R₃₅) and N=N with the provisos that AQ-2 must be a ring of at least five continuous members, that no more than two of the group of said spacer pairs are simultaneously C(R₃₃)=C(R₃₅), and that no more than one of the group of said spacer pairs are N=N unless the other spacer pairs is other than C(R₃₃)=C(R₃₅), O, N, and S;
   R₁ is selected from the group consisting of haloalkyl and haloalkoxymethyl;
   R₂ is selected from the group consisting of hydrido, aryl, alkyl, alkenyl, haloalkyl, haloalkoxy, haloalkoxyalkyl, perhaloaryl, perhaloaralkyl, perhaloaryloxyalkyl and heteroaryl;
   R₃ is selected from the group consisting of hydrido, aryl, alkyl, alkenyl, haloalkyl and haloalkoxyalkyl; Y is selected from a group consisting of a covalent single bond, (CH₂)_{q} wherein q is an integer selected from 1 through 2, and (CH₂)ⱼ-O-(CH₂)ₖ wherein j and k are integers independently selected from 0 through 1;
   Z is selected from the group consisting of a covalent single bond, (CH₂) wherein q is an integer selected from 1 through 2, and (CH₂)ⱼ-O-(CH₂)ₖ wherein j and k are integers independently selected from 0 through 1;
   R₄, R₈, R₉ and R₁₃ are independently selected from the group consisting of hydrido, halo, haloalkyl and alkyl; R₃₀ is selected from the group consisting of hydrido, alkoxy, alkoxyalkyl, halo, haloalkyl, alkylamino, alkylthio, alkylthioalkyl, alkyl, alkenyl, haloalkoxy, and haloalkoxyalkyl with the proviso that R₃₀ is selected to maintain the tetravalent nature of carbon, trivalent nature of nitrogen, the divalent nature of sulfur, and the divalent nature of oxygen;
   R₃₀, when bonded to A₁, is taken together to form an intra-ring linear spacer connecting the A₁-carbon at the point of attachment of R₃₀ to the point of bonding of a group selected from the group consisting of R₁₀, R₁₁, R₁₂, R₃₁, and R₃₂ wherein said intra-ring linear spacer is selected from the group consisting of a covalent single bond and a spacer moiety having from 1 through 6 continuous atoms to form a ring selected from the group consisting of a cycloalkyl having from 3 through 10 continuous members, a cycloalkenyl having from 5 through 10 continuous members, and a heterocyclyl having from 5 through 10 continuous members; R₃₀, when bonded to A₁, is taken together to form an intra-ring branched spacer connecting the A₁-carbon at the point of attachment of R₃₀ to the points of bonding of each member of any one of substituent pairs selected from the group consisting of subsitituent pairs R₁₀ and R₁₁, R₁₀ and R₃₁, R₁₀ and R₃₂, R₁₀ and R₁₂, R₁₁ and R₃₁, R₁₁ and R₃₂, R₁₁ and R₁₂, R₃₁ and R₃₂, R₃₁ and R₁₂, and R₃₂ and R₁₂ and wherein said intra-ring branched spacer is selected to form two rings selected from the group consisting of cycloalkyl having from 3 through 10 continuous members, cycloalkenyl having from 5 through 10 continuous members, and heterocyclyl having from 5 through 10 continuous members;
   R₄, R₅, R₆, R₇, R₈, R₉, R₁₀, R₁₁, R₁₂, R₁₃, R₃₁, R₃₂, R₃₃, R₃₄, R₃₅, and R₃₆ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroaxylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl-monoaryl-amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the provisos that R₄, R₅, R₆, R₇, R₈, R₉, R₁₀, R₁₁, R₁₂, R₁₃, R₃₁, R₃₂, R₃₃, R₃₄, R₃₅, and R₃₆ are each independently selected to maintain the tetravalent nature of carbon, the trivalent nature of nitrogen, the divalent nature of sulfur, and the divalent nature of oxygen, that no more than three of the R₃₃ and R₃₄ substituents are simultaneously selected from other than the group consisting of of hydrido and halo, and that no more than three of the R₃₅ and R₃₅ substituents are simultaneously selected from other than the group consisting of of hydrido and halo: R₉, R₁₀, R₁₁,R₁₂, R₁₃, R₃₁, and R₃₂ are independently selected to be oxo with the provisos that B₁, B₂, D₃, D₄, J₃, J₄ and K₂ are independently selected from the group consisting of C and S, no more than two of R₉, R₁₀, R₁₁, R₁₂, R₁₃, R₃₁, and R₃₂ are simultaneously oxo, and that R₉, R₁₀, R₁₁, R₁₂, R₁₃, R₃₁, and R₃₂ are each independently selected to maintain the tetravalent nature of carbon, the trivalent nature of nitrogen, the divalent nature of sulfur, and the divalent nature of oxygen;
   R₄ and R₅, R₅ and R₆, R₆ and R₇, R₇ and R₈, R₉ and R₁₀, R₁₀ and R₁₁, R₁₁ and R₃₁, R₃₁ and R₃₂, R₃₂ and R₁₂, and R₁₂ and R₁₃ are independently selected to form spacer pairs wherein a spacer pair is taken together to form a linear moiety having from 3 through 6 continuous atoms connecting the points of bonding of said spacer pair members to form a ring selected from the group consisting of a cycloalkenyl ring having 5 through 8 continuous members, a partially saturated heterocyclyl ring having 5 through 8 continuous members, a heteroaryl ring having 5 through 6 continuous members, and an aryl with the provisos that no more than one of the group consisting of spacer pairs R₄ and R₅, R₅ and R₆, R₆ and R₇, and R₇ and R₈, are used at the same time and that no more than one of the group consisting of spacer pairs R₉ and R₁₀, R₁₀ and R₁₁, R₁₁ and R₃₁, R₃₁ and R₃₂, R₃₂ and R₁₂, and R₁₂ and R₁₃ are used at the same time;
   R₉ and R₁₁, R₉ and R₁₂, R₉ and R₁₃, R₉ and R₃₁, R₉ and R₃₂, R₁₀ and R₁₂, R₁₀ and R₁₃, R₁₀ and R₃₁, R₁₀ and R₃₂, R₁₁ and R₁₂, R₁₁ and R₁₃, R₁₁ and R₃₂, R₁₂ and R₃₁, R₁₃ and R₃₁, and R₁₃ and R₃₂ are independently selected to form a spacer pair wherein said spacer pair is taken together to form a linear spacer moiety selected-from the group consisting of a covalent single bond and a moiety having from 1 through 3 continuous atoms to form a ring selected from the group consisting of a cycloalkyl having from 3 through 8 continuous members, a cycloalkenyl having from 5 through 8 continuous members, a saturated heterocyclyl having from 5 through 8 continuous members and a partially saturated heterocyclyl having from 5 through 8 continuous members with the provisos that no more than one of said group of spacer pairs is used at the same time;
   R₃₇ and R₃₈ are independently selected from the group consisting of hydrido, alkoxy, alkoxyalkyl, hydroxy, amino, thio, halo, haloalkyl, alkylamino, alkylthio, alkylthioalkyl, cyano, alkyl, alkenyl, haloalkoxy, and haloalkoxyalkyl.
[79] The inhibitor of [2], [4] or [7], wherein the compound having a CETP inhibitory activity is a compound of the formula (XVI) of [78] or a pharmaceutically acceptable salt thereof.
[80] The inhibitor of [5] or [8], which is a compound of the formula (XVI) of [78] or a pharmaceutically acceptable salt thereof.
[81] The method of [78], wherein the compound of the formula (XVI) is a compound selected from the group consisting of
   3-[[3-(4-chloro-3-ethylphenoxy)phenyl](cyclohexylmethyl)amino]-1,1,1-trifluoro-2-propanol;
   3-[[3-(4-chloro-3-ethylphenoxy)phenyl](cyclopentylmethyl)amino]-1,1,1-trifluoro-2-propanol;
   3-[[3-(4-chloro-3-ethylphenoxy)phenyl](cyclopropylmethyl)amino]-1,1,1-trifluoro-2-propanol;
   3-[[3-(4-chloro-3-ethylphenoxy)phenyl][(3-trifluoromethyl)cyclohexylmethyl]amino]-1,1,1-trifluoro-2-propanol;
   3-[[3-(4-chloro-3-ethylphenoxy)phenyl][(3-pentafluoroethyl)cyclohexylmethyl]amino]-1,1,1-trifluoro-2-propanol;
   3-[[3-(4-chloro-3-ethylphenoxy)phenyl)[(3-trifluoromethoxy)cyclohexylmethyl]amino]-1,1,1-trifluoro-2-propanol;
   3-[[3-(4-chloro-3-ethylphenoxy)phenyl][[3-(1,1,2,2-tetrafluoroethoxy)cyclohexylmethyl]amino]-1,1,1-trifluoro-2-propanol;
   3-[[3-(3-trifluoromethoxyphenoxy)phenyl][(cyclohexylmethyl)amino]-1,1-trifluoro-2-propanol;
   3-[[3-(3-trifluoromethoxyphenoxy)phenyl](cyclopentylmethyl)amino]-1,1,1-trifluoro-2-propanol;
   3-[[3-(3-trifluoromethoxyphenoxy)phenyl](cyclopropylmethyl)amino]-1,1,1]-trifluoro-2-propanol;
   3-[[3-(3-trifluoromethoxyphenoxy)phenyl][(3-trifluoromethyl)cyclohexylmethyl]amino]-1,1,1-trifluoro-2-propanol;
   3-[[3-(3-trifluoromethoxyphenoxy)phenyl](3-pentafluoroethyl)cyclohexylmethyl]amino]-1,1,1-trifluoro-2-propanol;
   3-[[3-(3-trifluoromethoxyphenoxy)phenyl][3-trifluoromethoxy)cyclohexylmethyl]amino]-1,1,1-trifluoro-2-propanol;
   3-[[3-(3-trifluoromethoxyphenoxy)phenyl][[3-(1,1,2,2-tetrafluoroethoxy)cyclohexylmethyl]amino]-1,1,1-trifluoro-2-propanol;
   3-[[3-(3-isopropylphenoxy)phenyl](cyclohexylmethyl)amino]-1,1,1-trifluoro-2-propanol;
   3-[[3-(3-isopropylphenoxy)phenyl](cyclopentylmethyl)amino]-1,1,1-trifluoro-2-propanol;
   3-[[3-(3-isopropylphenoxy)phenyl](cyclopropylmethyl)amino]-1,1,1-trifluoro-2-propanol;
   3-[[3-(3-isopropylphenoxy)phenyl][(3-trifluoromethyl)cyclohexylmethyl]amino]-1,1,1-trifluoro-2-propanol;
   3-[[3-(3-isopropylphenoxy)phenyl](3-pentafluoroethyl)cyclohexylmethyl]amino]-1,1,1-trifluoro-2-propanol;
   3-[[3-(3-isopropylphenoxy)phenyl][(3-trifluoromethoxy)cyclohexylmethyl]amino]-1,1,1-trifluoro-2-propanol;
   3-[[3-(3-isopropylphenoxy)phenyl][3-(1,1,2,2-tetrafluoroethoxy)cyclohexyl]methyl]amino]-1,1,1-trifluoro-2-propanol;
   3-[[3-(2,3-dichlorophenoxy)phenyl](cyclohexylmethyl)amino]-1,1,1-trifluoro-2-propanol;
   3-[[3-(2,3-dichlorophenoxy)phenyl](cyclopentylmethyl)aminol-1,1,1-trifluoro-2-propanol;
   3-[[3-(2,3-dichlorophenoxy)phenyl](cyclopropylmethyl)aminol-1,1,1-trifluoro-2-propanol;
   3-[[3-(2,3-dichlorophenoxy)phenyl][(3-trifluoromethyl)cyclohexylmethyl)amino]-1,1,1-trifluoro-2-propanol;
   3-[[3-(2,3-dichlorophenoxy)phenyl][(3-pentafluoroethyl)cyclohexylmethyl]amino]-1,1,1-trifluoro-2 propanol;
   3-[[3-(2,3-dichlorophenoxy)phenyl][(3-trifluoromethoxy)cyclohexylmethyl]amino]-1,1,1-trifluoro-2-propanol;
   3-[[3-(2,3-dichlorophenoxy)phenyl][[3-(1,1,2,2-tetrafluoroethoxy)cyclohexylmethyl]amino]-1,1,1-trifluoro-2-propanol;
   3-[[3-(4-fluorophenoxy)phenyl](cyclohexylmethyl)amino]-1,1,1-trifluoro-2-propanol;
   3-[[3-(4-fluorophenoxy)phenyl](cyclopentylmethyl)amino-1,1,1-trifluoro-2-propanol;
   3-[[3-(4-fluorophenoxy)phenyl](cyclopropylmethyl)amino]-1,1,1-trifluoro-2-propanol;
   3-[[3-(4-fluorophenoxy)phenyl][(3-trifluoromethyl)cyclohexylmethyl]ainino]-I,1,1-trifluoro-2-propanol;
   3-[[3-(4-fluorophenoxy)phenyl][(3-pentafluoroethyl)cyclohexylmethyl]amino]-1,1,1-trifluoro-2-propanol;
   3-[[3-(4-fluorophenoxy)phenyl][(3-trifluoromethoxy)cyclohexylmethyl]amino]-1,1,1-trifluoro-2-propanol,
   3-[[3-(4-fluorophenoxy)phenyl][3-(1,1,2,2-tetrafluoroethoxy)cyclohexylmethyl]amino]-1,1,1-trifluoro-2-propanol;
   3-[[3-(3-trifluoromethoxybenzyloxy)phenyl](cyclohexylmethyl)amino]-1,1,1-trifluoro-2-propanol;
   3-[[3-(3-trifluoromethoxybenzyloxy)phenyl](cyclopentylmethyl)amino]-1,1,1-trifluoro-2-propanol;
   3-[[3-(3-trifluoromethoxybenzyloxy)phenyl](cyclopropylmethyl)amino]-1,1,1-trifluoro-2-propanol;
   3-[[3-(3-trifluoromethoxybenzyloxy)phenyl][(3-trifluoromethyl)cyclohexylmethyl]amino]-1,1,1-trifluoro-2-propanol;
   3-[[3-(3-trifluoromethoxybenzyloxy)phenyl][(3-pentafluoroethyl)cyclohexylmethyl]amino]-1,1,1-trifluoro-2-propanol;
   3-[[3-(3-trifluoromethoxybenzyloxy)phenyl][(3-trifluoromethoxy)cyclohexylmethyl]amino]-1,1,1-trifluoro-2-propanol;
   3-[[3-(3-trifluoromethoxybenzyloxy)phenyl][[3-(1,1,2,2-tetrafluoroethoxy)cyclohexylmethyl]amino]-1,1,1-trifluoro-2-propanol;
   3-[[3-(3-trifluoromethylbenzyloxy)phenyl](cyclohexylmethyl)amino]-1,1,1-trifluoro-2-propanol;
   3-[[3-3-trifluoromethylbenzyloxy)phenyl](cyclopentylmethyl)amino]-1,1,1-trifluoro-2-propanol;
   3-[[3-(3-trifluoromethylbenzyloxy)phenyl](cyclopropylmethyl)amino]-1,1,1-trifluoro-2-propanol;
   3-[[3-(3-trifluoromethylbenzyloxy)phenyl][(3-trifluoromethyl)cyclohexylmethyl]amino]-1,1,1-trifluoro-2-propanol;
   3-[[3-(3-trifluoromethylbenzyloxy)phenyl][(3-pentafiuoroethyl)cyclohexylmethyl]amino]-1,1,1-trifluoro-2-propanol;
   3-[[3-(3-trifluoromethylbenzyloxy)phenyl][[(3-trifluoromethoxy)cyclohexylmethyl]amino]-1,1,1-trifluoro-2-propanol;
   3-[[3-(3-trifluoromethylbenzyloxy)phenyl][3-(1,1,2,2-tetrafluoroethoxy)cyclohexylmethyl]amino]-1,1,1-trifluoro-2-propanol;
   3-[[[(3-trifluoromethyl)phenyl]methyl](cyclohexyl)amino]-1,1,1-trifluoro-2-propanol;
   3-[[[(3-pentafluoroethyl)phenyl]methyl](cyclohexyl)amino]-1,1,1-trifluoro-2-propanol;
   3-[[[(3-trifluoromethoxy)phenyl]methyl](cyclohexyl)amino]-1,1,1-trifluoro-2-propanol;
   3-[[[3-(1,1,2,2-tetrafluoroethoxy)phenyl]methyl](cyclohexyl)amino]-1,1,1-trifluoro-2-propanol;
   3-[[[(3-trifluoromethyl)phenyl]methyl](4-methylcyclohexyl)amino]-1,1,1-trifluoro-2-propanol;
   3-[[[(3-pentafluoroethyl)phenyl]methyl][(4-methylcyclohexyl)amino]-1,1,1-trifluoro-2-propanol;
   3-[[[(3-trifluoromethoxy)phenyl]methyl][[4-methylcyclohexyl)amino]-1,1,1-trifluoro-2-propanol;
   3-[[[3-(1,1,2,2-tetrafluoroethoxy)phenyl]methyl](4-methylcyclohexyl)amino]-1,1,1-trifluoro-2-propanol;
   3-[[[(3-trifluoromethyl)phenyl]methyl] (3-trifluoromethylcyclohexyl)amino]-1,1,1-trifluoro-2-propanol;
   3-[[[(3-pentafluoroethyl)phenyl]methyl](3-trifluoromethylcyclohexyl)amino]-1,1,1-trifluoro-2-propanol;
   3-[[[(3-trifluoromethoxy)phenyl]methyl](3-trifluoromethylcyclohexyl)amino]-1,1,1-trifluoro-2-propanol;
   3-[[[3-(1,1,2,2-tetrafluoroethoxy)phenyl]methyl](3-trifluoromethylcyclohexyl)amino]-1,1,1-trifluoro-2-propanol;
   3-[[[(3-trifluoromethyl)phenyl]methyl][3-(4-chloro-3-ethylphenoxy)cyclohexyl]aminol-1,1,1-triflucro-2-propanol;
   3-[[[(3-pentafluoroethyl)phenyl]methyl][3-(4-chloro-3-ethylphenoxy)cyclohexyl]amino]-1,1,1-trifluoro-2-propanol;
   3-[[[(3-trifluoromethoxy)phenyl]methyl][3-(4-chloro-3-ethylphenoxy)cyclohexyl]amino]-1,1,1-trifluoro-2-propanol;
   3-[[[3-(1,1,2,2-tetrafluoroethoxy)phenyl]methyl][3-(4-chloro-3-ethylphenoxy)cyclohexyl]amino]-1,1,1-trifluoro-2-propanol;
   3-[[[(3-trifluoromethyl)phenyl]methyl](3-phenoxycyclohexyl)amino]-1,1,1-trifluoro-2-propanol;
   3-[[[(3-pentafluoroethyl)phenyl]methyl](3-phenoxycyclohexyl)amino]-1,1,1-trifluoro-2-propanol;
   3-[[[(3-trifluoromethoxy)phenyl]methyl](3-phenoxycyclohexyl)amino]-1,1,1-trifluoro-2-propanol;
   3-[[[3-(1,1,2,2-tetrafluoroethoxy)phenyl]methyl](3-phenoxycyclohexyl)amino]-1,1,1-trifluoro-2-propanol;
   3-[[[(3-trifluoromethyl)phenyl]methyl](3-isopropoxycyclohexyl)amino]-1,1,1-trifluoro-2-propanol;
   3-[[[(3-pentafluoroethyl)phenyl]methyl](3-isopropoxycyclohexyl)amino]-1,1,1-trifluoro-2-propanol;
   3-[[[(3-trifluoromethoxy)phenyl]methyl](3-isopropoxycyclohexyl)amino]-1,1,1-trifluoro-2-propanol;
   3-[[[3-(1,1,2,2-tetrafluoroethoxy)phenyl]methyl](3-isopropoxycyclohexyl)amino]-1,1,1-trifluoro-2-propanol;
   3-[[[(3-trifluoromethyl)phenyl]methyl](3-cyclopentyloxycyclohexyl)amino]-1,1,1-trifluoro-2-propanol;
   3-[[[(3-pentafluoroethyl)phenyl]methyl](3-cyclopentyloxycyclohexyl)amino]-1,1,1-trifluoro-2-propanol;
   3-[[[(3-trifluoromethoxy)phenyl]methyl](3-cyclopentyloxycyclohexyl)amino]-1,1,1-trifluoro-2-propanol;
   3-[[[3-(1,1,2,2-tetrafluoroethoxy)phenyl]methyl](3-cyclopentyloxycyclohexyl)amino]-1,1,1-trifluoro-2-propanol;
   3-[[[(2-trifluoromethyl)pyrid-6-yl]methyl](3-isopropoxycyclohexyl)amino]-1,1,1-trifluoro-2-propanol;
   3-[[[(2-trifluoromethyl)pyrid--6-yl]methyl](3-cyclopentyloxycyclohexyl)amino]-1,1,1-trifluoro-2-propanol;
   3-[[[(2-trifluoromethyl)pyrid-6-yl]methyl](3-phenoxycyclohexyl)amino]-1,1,1-trifluoro-2-propanol;
   3-[[[(2-trifluoromethyl)pyrid-6-yl]methyl](3-trifluoromethylcyclohexyl)amino]-1,1,1-trifluoro-2-propanol;
   3-[[[(2-trifluoromethyl)pyrid-6-yl]methyl][3-(4-chloro-3-ethylphenoxy)cyclohexyl]amino]-1,1,1-trifluoro-2-propanol;
   3-[[[(2-trifluoromethyl)pyrid-6-yl]methyl][3-(1,2,2-tetrafluoroethoxy)cyclohexyl]amino]-1,1,1-trifluoro-2-propanol;
   3-[[[(2-trifluoromethyl)pyrid-6-yl]methyl](3-pentafluoroethylcyclohexyl)amino]-1,1,1-trifluoro-2-propanol;
   3-[[[(2-trifluoromethyl)pyrid-6-yl]methyl](3-trifluoromethoxycyclobexyl)amino]-1,1,1-trifluoro-2-propanol;
   3-[[[(3-trifluoromethyl)phenyl]methyl][3-(4-chloro-3-ethylphenoxy)propyl]amino]-1,1,1-trifluoro-2-propanol;
   3-[[[(3-pentafluoroethyl)phenyl]methyl][3-(4-chloro-3-ethylphenoxy)propyl]amino]-1,1,1-trifluoro-2-propanol;
   3-[[[(3-trifluoromethoxy)phenyl]methyl][3-(4-chloro-3-ethylphenoxy)propyl]amino]-1,1,1-trifluoro-2-propanol;
   3-[[[3-(1,1,2,2-tetrafluoroethoxy)phenyl]methyl][3-(4-chloro-3-ethylphenoxy)propyl]amino]-1,1,1-trifluoro-2-propanol;
   3-[[[(3-trifluoromethyl)phenyl]methyl][3-(4-chloro-3-ethylphenoxy)-2,2-di-fluoropropyl]amino]-1,1,1-trifluoro-2-propanol;
   3-[[[(3-pentafluoroethyl)phenyl]methyl][3-(4-chloro-3-ethylphenoxy)-2,2-difluoropropyl]amino]-1,1,1-trifluoro-2-propanol;
   3-[[[(3-trifluoromethoxy)phenyl]methyl][3-(4-chloro-3-ethylphenoxy)-2,2-difluoropropyl]amino]-1,1,1-trifluoro-2-propanol;
   3-[[[(3-(1,1,2,2-tetrafluoroethoxy)phenyl]methyl][3-(4-chloro-3-ethylphenoxy)-2,2-difluoropropyl]amino]-1,1,1-trifluoro-2-propanol;
   3-[[[(3-trifluoromethyl)phenyl]methyl][3-(isopropoxy)propyl]amino]-1,1,1-trifluoro-2-propanol;
   3-[[[(3-pentafluoroethyl)phenyl]methyl][3-(isopropoxy)propyl]amino]-1,1,1-trifluoro-2-propanol;
   3-[[[(3-trifluoromethoxy)phenyl]methyl][3-(isopropoxy)propyl]amino]-1,1,1-trifluoro-2-propanol;
   3-[[[3-(1,1,2,2-tetrafluoroethoxy)phenyl]methyl][3-(isopropoxy)propyl]amino]-1,1,1-trifluoro-2-propanol; and
   3-[[[3-(1,1,2,2-tetrafluoroethoxy)phenyl]methyl][3-(phenoxy)propyl]amino]-1,1,1-trifluoro-2-propanol, or a pharmaceutically acceptable salt thereof.
[82] The inhibitor of [79] or [80], wherein the compound of the formula (XVI) is a compound selected from the group consisting of
   3-[[3-(4-chloro-3-ethylphenoxy)phenyl](cyclohexylmethyl)amino]-1,1,1-trifluoro-2-propanol;
   3-[[3-(4-chloro-3-ethylphenoxy)phenyl](cyclopentylmethyl)amino]-1,1,1-trifluoro-2-propanol;
   3-[[3-(4-chloro-3-ethylphenoxy)phenyl](cyclopropylmethyl)amino]-1,1,1-trifluoro-2-propanol;
   3-[[3-(4-chloro-3-ethylphenoxy)phenyl][(3-trifluoromethyl)cyclohexylmethyl]amino]-1,1,1-trifluoro-2-propanol;
   3-[[3-(4-chloro-3-ethylphenoxy)phenyl][(3-pentafluoroethyl)cyclohexylmethyl]amino]-1,1,1-trifluoro-2-propanol;
   3-[[3-(4-chloro-3-ethylphenoxy)phenyl][(3-trifluoromethoxy)cyclohexylmethyl]amino]-1,1,1-trifluoro-2-propanol;
   3-[[3-(4-chloro-3-ethylphenoxy)phenyl][[3-(1,1,2,2-tetrafluoroethoxy)cyclohexylmethyl]amino]-1,1,1-trifluoro-2-propanol;
   3-[[3-(3-trifluoromethoxyphenoxy)phenyl][(cyclohexylmethyl)amino]-1,1,1-trifluoro-2-propanol;
   3-[[3-(3-trifluoromethoxyphenoxy)phenyl](cyclopentylmethyl)amino]-1,1,1-trifluoro-2-propanol;
   3-[[3-(3-trifluoromethoxyphenoxy)phenyl](cyclopropylmethyl)amino]-1,1,1]-triflucro-2-propanol;
   3-[[3-(3-trifluoromethoxyphenoxy)phenyl][(3-trifluoromethyl)cyclohexylmethyl]amino]-1,1,1-trifluoro-2-propanol;
   3-[[3-(3-trifluoromethoxyphenoxy)phenyl](3-pentafluoroethyl)cyclohexylmethyl]amino]-1,1,1-trifluoro-2-propanol;
   3-[[3-(3-trifluoromethoxyphenoxy)phenyl][(3-trifluoromethoxy)cyclohexylmethyl]amino]-1,1,1-trifluoro-2-propanol;
   3-[[3-(3-trifluoromethoxyphenoxy)phenyl][[3-(1,1,2,2-tetrafluoroethoxy)cyclohexylmethyl]amino]-1,1,1-trifluoro-2-propanol;
   3-[[3-(3-isopropylphenoxy)phenyl](cyclohexylmethyl)amino]-1,1,1-trifluoro-2-propanol;
   3-[[3-(3-isopropylphenoxy)phenyl](cyclopentylmethyl)amino]-1,1,1-trifluoro-2-propanol;
   3-[[3-(3-isopropylphenoxy)phenyl](cyclopropylmethyl)amino]-1,1,1-trifluoro-2-propanol;
   3-[[3-(3-isopropylphenoxy)phenyl][(3-trifluoromethyl)cyclohexylmethyl]amino]-1,1,1-trifluoro-2-propanol;
   3-[[3-(3-isopropylphenoxy)phenyl][(3-pentafluoroethyl)cyclohexylmethyl]amino]-1,1,1-trifluoro-2-propanol;
   3-[[3-(3-isopropylphenoxy)phenyl][(3-trifluoromethoxy)cyclohexylmethyl]amino]-1,1,1-trifluoro-2-propanol;
   3-[[3-(3-isopropylphenoxy)phenyl][3-(1,1,2,2-tetrafluoroethoxy)cyclohexyl]methyl]amino]-1,1,1-trifluoro-2-propanol;
   3-[[3-(2,3-dichlorophenoxy)phenyl](cyclohexylmethyl)amino]-1,1,1-trifluoro-2-propanol;
   3-[[3-(2,3-dichlorophenoxy)phenyl](cyclopentylmethyl)amino]-1,1,1-trifluoro-2-propanol;
   3-[[3-(2,3-dichlorophenoxy)phenyl](cyclopropylmethyl)amino]-1,1,1-trifluoro-2-propanol;
   3-[[3-(2,3-dichlorophenoxy)phenyl][(3-trifluoromethyl)cyclohexylmethyl)amino]-1,1,1-trifluoro-2-propanol;
   3-[[3-(2,3-dichlorophenoxy)phenyl] [(3-pentafluoroethyl)cyclohexylmethyl]amino]-1,1,1-trifluoro-2-propanol;
   3-[[3-(2,3-dichlorophenoxy)phenyl] [(3-trifluoromethoxy)cyclohexylmethyl]amino]-1,1,1-trifluoro-2-propanol;
   3-[[3-(2,3-dichlorophenoxy)phenyl][[3-(1,1,2,2-tetrafluoroethoxy)cyclohexylmethyl]amino]-1,1,1-trifluoro-2-propanol;
   3-[[3-(4-fluorophenoxy)phenyl](cyclohexylmethyl)amino]-1,1,1-trifluoro-2-propanol;
   3-[[3-(4-fluorophenoxy)phenyl](cyclopentylmethyl)amino-1,1,1-trifluoro-2-propanol;
   3-[[3-(4-fluorophenoxy)phenyl](cyclopropylmethyl)amino]-1,1,1-trifluoro-2-propanol;
   3-[[3-(4-fluorophenoxy)phenyl][(3-trifluoromethyl)cyclohexylmethyl]amino]-1,1,1-trifluoro-2-propanol;
   3-[[3-(4-fluorophenoxy)phenyl][(3-pentafluoroethyl)cyclohexylmethyl]amino]-1,1,1-trifluoro-2-propanol;
   3-[[3-(4-fluorophenoxy)phenyl][(3-trifluoromethoxy)cyclohexylmethyl]amino]-1,1,1-trifluoro-2-propanol,
   3-[[3-(4-fluorophenoxy)phenyl][3-(1,1,2,2-tetrafluoroethoxy)cyclohexylmethyl]amino]-1,1,1-trifluoro-2-propanol;
   3-[[3-(3-trifluaromethoxybenzyloxy)phenyl](cyclohexylmethyl)amino]-1,1,1-trifluoro-2-propanol;
   3-[[3-(3-trifluaromethoxybenzyloxy)phenyl](cyclopentylmethyl)arnino]-1,1,1-trifluoro-2-propanol;
   3-[[3-(3-trifluoromethoxybenzyloxy)phenyl](cyclopropylmethyl)amino]-1,1,1-trifluoro-2-propanol;
   3-[[3-(3-trifluoromethoxybenzyloxy)phenyl][(3-trifluoromethyl)cyclohexylmethyl]amino]-1,1,1-trifluoro-2-propanol;
   3-[[3-(3-trifluoromethoxybenzyloxy)phenyl][(3-pentafluoroethyl)cyclohexylmethyl]amino]-1,1,1-trifluoro-2-propanol;
   3-[[3-(3-trifluoromethoxybenzyloxy)phenyl][(3-trifluoromethoxy)cyclohexylmethyl]amino]-1,1,1-trifluoro-2-propanol;
   3-[[3-(3-trifluoromethoxybensyloxy)phenyl][[3-(1,1,2,2-tetrafluoroethoxy)cyclohexylmethyl]amino]-1,1,1-trifluoro-2-propanol;
   3-[[3-(3-trifluoromethylbenzyloxy)phenyl](cyclohexylmethyl)amino]-1,1,1-trifluoro-2-pxopanal;
   3-[[3-(3-trifluoromethylbenzyloxy)phenyl](cyclopentylmethyl)amino]-1,1,1-trifluoro-2-propanol;
   3-[[3-(3-trifluoromethylbenzyloxy)phenyl](cyclopropylmethyl)amino]-1,1,1-trifluoro-2-propanol;
   3-[[3-(3-trifluoromethylbenzyloxy)phenyl][(3-trifluoromethyl)cyclohexylmethyl]amino]-1,1,1-trifluoro-2-propanol;
   3-[[3-(3-trifluoromethylbenzyloxy)phenyl][(3-pentafluoroethyl)cyclohexylmethyl]amino]-1,1,-trifluoro-2-propanol;
   3-[[3-(3-trifluoromethylbenzyloxy)phenyl][[(3-trifluoromethoxy)cyclohexylmethyl]amino]-1,1,1-trifluoro-2-propanol;
   3-[[3-(3-trifluoromethylbenzyloxy)phenyl][3-(1,1,2,2-tetrafluoroethoxy)cyclohexylmethyl]amino]-1,1,1-trifluoro-2-propanol;
   3-[[[(3-trifluoromethyl)phenyl]methyl](cyclohexyl)amino]-1,1,1-trifluoro-2-propanol;
   3-[[[(3-pentafluoroethyl)phenyl]methyl](cyclohexyl)amino]-1,1,1-trifluoro-2-propanol;
   3-[[[(3-trifluoromethoxy)phenyl]methyl](cyclohexyl)amino]-1,1,1-trifluoro-2-propanol;
   3-[[[3-(1,1,2,2-tetrafluoroethoxy)phenyl]methyl](cyclohexyl)amino]-1,1,1-trifluoro-2-propanol;
   3-[[[(3-trifluoromethyl)phenyl]methyl](4-methylcyclohexyl)amino]-1,1,1-trifluoro-2-propanol;
   3-[[[(3-pentafluoroethyl)phenyl]methyl][(4-methylcyclohexyl)amino]-1,1,1-trifluoro-2-propanol;
   3-[[[(3-trifluoromethoxy)phenyl]methyl][[4-methylcyclohexyl)amino]-1,1,1-trifluoro-2-propanol;
   3-[[[3-(1,1,2,2-tetrafluoroethoxy)phenyl]methyl](4-methylcyclohexyl)amino]-1,1,1-trifluoro-2-propanol;
   3-[[[(3-trifluoromethyl)phenyl]methyl] (3-trifluoromethylcyclohexyl)amino]-1,1,1-trifluoro-2-propanol;
   3-[[[(3-pentafluoroethyl)phenyl]methyl](3-trifluoromethylcyclohexyl)amino]-1,1,1-trifluoro-2-propanol;
   3-[[[(3-trifluoromethoxy)phenyl]methyl](3-trifluoromethylcyclohexyl)amino]-1,1,1-trifluoro-2-propanol;
   3-[[[3-(1,1,2,2-tetrafluoroethoxy)phenyl]methyl](3-trifluoromethylcyclohexyl)amino]-1,1,1-trifluoro-2-propanol;
   3-[[[(3-trifluoromethyl)phenyl]methyl][3-(4-chloro-3-ethylphenoxy)cyclohexyl]amino]-1,1,1-trifluoro-2-propanol;
   3-[[[(3-pentafluoroethyl)phenyl]methyl][3-(4-chloro-3-ethylphenoxy)cyclahexyl]amino]-1,1,1-trifluoro-2-propanol;
   3-[[[(3-trifluoromethoxy)phenyl]methyl][3-(4-chloro-3-ethylphenoxy)cyclohexyl]amino]-1,1,1-trifluoro-2-propanol;
   3-[[[3-(1,1,2,2-tetrafluoroethoxy)phenyl]methyl][3-(4-chloro-3-ethylphenoxy)cyclohexyl]amino]-1,1,1-trifluoro-2-propanol;
   3-[[[(3-trifluoromethyl)phenyl]methyl](3-phenoxycyclohexyl)amino]-1,1,1-trifluoro-2-propanol;
   3-[[[(3-pentafluoroethyl)phenyl]methyl](3-phenoxycyclohexyl)amino]-1,1,1-trifluoro-2-propanol;
   3-[[[(3-trifluoromethoxy)phenyl]methyl](3-phenoxycyclohexyl)amino]-1,1,1-trifluoro-2-propanol;
   3-[[[3-(1,1,2,2-tetrafluoroethoxy)phenyl]methyl](3-phenoxycyclohexyl)amino]-1,1,1-trifluoro-2-propanol;
   3-[[[(3-trifluoromethyl)phenyl]methyl](3-isopropoxycyclohexyl)amino]-1,1,1-trifluoro-2-propanol;
   3-[[[(3-pentafluoroethyl)phenyl]methyl](3-isopropoxycyclohexyl)amino]-1,1,1-trifluoro-2-propanol;
   3-[[[(3-trifluoromethoxy)phenyl]methyl](3-isopropoxycyclohexyl)amino]-1,1,1-trifluoro-2-propanol;
   3-[[[3-(1,1,2,2-tetrafluoroethoxy)phenyl]methyl](3-isopropoxycyclohexyl)amino]-1,1,1-trifluoro-2-propanol;
   3-[[[(3-trifluoromethyl)phenyl]methyl](3-cyclopentyloxycyclohexyl)amino]-1,1,1-trifluoro-2-propanol; 3-[[[(3-pentafluoroethyl)phenyl]mtethyl](3-cyclopentyloxycyclohexyl)amino]-1,1,1-trifluoro-2-propanol; 3-[[[(3-trifluoromethoxy)phenyl]methyl](3-cyclopentyloxycyclohexyl)amino]-1,1,1-trifluoro-2-propanol;
   3-[[[3-(1,1,2,2-tetrafluoroethoxy)phenyl]methyl](3-cyclopentyloxycyclohexyl)amino]-1,1,1-trifluoro-2-propanol;
   3-[[[(2-trifluoromethyl)pyrid-6-yl]methyl](3-isopropoxycyclohexyl)amino]-1,1,1-trifluoro-2-propanol;
   3-[[[(2-trifluoromethyl)pyrid-6-yl]methyl](3-cyclopentyloxycyclohexyl)amino]-1,1,1-trifluoro-2-propanol;
   3-[[[(2-trifluoromethyl)pyrid-6-yl]methyl](3-phenoxycyclohexyl)amino]-1,1,1-trifluoro-2-propanol;
   3-[[[(2-trifluoromethyl)pyrid-6-yl]methyl](3-trifluoromethylcyclohexyl)amino]-1,1,1-trifluoro-2-propanol;
   3-[[[(2-trifluoromethyl)pyrid-6-yl]methyl][3-(4-chloro-3-ethylphenoxy)cyclohexyl]amino]-1,1,1-trifluoro-2-propanol;
   3-[[[(2-trifluoromethyl)pyrid-6-yl]methyl][3-(1,2,2-tetrafluoroethoxy)cyclohexyl]amino]-1,1,1-trifluoro-2-propanol;
   3-[[[(2-trifluoromethyl)pyrid-6-yl]methyl](3-pentafluoroethylcyclohexyl)amino]-1,1,1-trifluoro-2-propanol;
   3-[[[(2-trifluoromethyl)pyrid-6-yl]methyl](3-trifluoromethoxycyclohexyl)amino]-1,1,1-trifluoro-2-propanol;
   3-[[[(3-trifluoromethyl)phenyl]methyl][3-(4-chloro-3-ethylphenoxy)propyl]amino]-1,1,1-trifluoro-2-propanol;
   3-[[[(3-pentafluoroethyl)phenyl]methyl][3-(4-chloro-3-ethylphenoxy)propyl]amino]-1,1,1-trifluoro-2-propanol;
   3-[[[(3-trifluoromethoxy)phenyl]methyl][3-(4-chloro-3-ethylphenoxy)propyl]amino]-1,1,1-trifluoro-2-propanol;
   3-[[[3-(1,1,2,2-tetrafluoroethoxy)phenyl]methyl][3-(4-chloro-3-ethylphenoxy)propyl]amino]-1,1,1-trifluoro-2-propanol;
   3-[[[(3-trifluoromethyl)phenyl]methyl][3-(4-chloro-3-ethylphenoxy)-2,2-di-fluoropropyl]amino]1,1,1-trifluoro-2-propanol;
   3-[[[(3-pentafluoroethyl)phenyl]methyl][3-(4-chloro-3-ethylphenoxy)-2,2-difluoropropyl]amino]-1,1,1-trifluoro-2-propanol;
   3-[[[(3-trifluoromethoxy)phenyl]methyl][3-(4-chloro-3-ethylphenoxy)-2,2-difluoropropyl]amino]-1,1,1-trifluoro-2-propanol;
   3-[[[(3-(1,1,2,2-tetrafluoroethoxy)phenyl]methyl][3-(4-chloro-3-ethylphenoxy)-2,2-difluoropropyl]amino]-1,1,1-trifluoro-2-propanol;
   3-[[[(3-trifluoromethyl)phenyl]methyl][3-(isopropoxy)propyljamino]-1,1,1-trifluoro-2-propanol;
   3-[[[(3-pentafluoroethyl)phenyl]methyl][3-(isopropoxy)propyl]amino]-1,1,1-trifluoro-2-propanol;
   3-[[[(3-trifluoromethoxy)phenyl]methyl][3-(isopropoxy)propyl]amino]-1,1,1-trifluoro-2-propanol;
   3-[[[3-(1,1,2,2-tetrafluoroethoxy)phenyl]methyl][3-(isopropoxy)propyljamino]-1,1,1-trifluoro-2-propanol; and
   3-[[[3-(1,1,2,2-tetrafluoroethoxy)phenyl]methyl][3-(phenoxy)propyl]amino]-1,1,1-trifluoro-2-propanol, or a pharmaceutically acceptable salt thereof.
[83] The method of [1], [3] or [6], wherein the compound having a CETP inhibitory activity is cycloalkano-pyridine of the formula (XVIII) 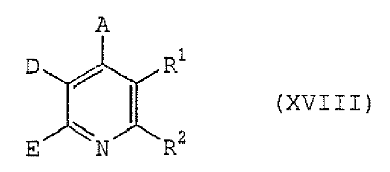 wherein A is an aryl having 6 to 10 carbon atoms, which can be optionally substituted up to 5 times in an identical or different manner by halogen, nitro, hydroxyl, trifluoromethyl or trifluoromethoxy, or by linear or branched chain alkyl, acyl, hydroxyalkyl or alkoxy, each having up to 7 carbon atoms, or by a group of the formula - NR³R⁴, wherein R³ and R⁴ are identical or different and each is hydrogen, phenyl or linear or branched chain alkyl having up to 6 carbon atoms, D is aryl having 6 to 10 carbon atoms, which is optionally substituted by phenyl, nitro, halogen, trifluoromethyl or trifluoromethoxy, or is a group represented by the formula R⁵-L-, 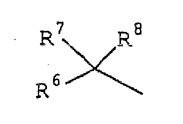 or R⁹-T-V-X-
   wherein R⁵, R⁶ and R⁹ independently of one another denote cycloalkyl having 3 to 6 carbon atoms, or aryl having 6 to 10 carbon atoms, or a 5- to 7-membered, optionally benzo-fused, saturated or unsaturated, mono-, bi- or tricyclic heterocycle having up to 4 heteroatoms from the series S, N and/or O, where the cycles are substituted, if appropriate, in the case of a nitrogen-containing rings also via an N functional group, up to 5 times in an identical or different manner by halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl, each having up to 6 carbon atoms, by aryl-or trifluoromethyl-substituted aryl, each having 6 to 10 carbon atoms, or by an optionally benzo-fused, aromatic 5-to 7-membered heterocycle having up to 3 heteroatoms from the series S, N and/or O, and/or are substituted by a group of the formula -OR¹⁰, -SR¹¹ -SO₂R¹² or -NR¹³R¹⁴, wherein R¹⁰, R¹¹ and R¹² independently of one another denote aryl having 6 to 10 carbon atoms, which for its part is substituted up to 2 times in an identical or different manner by phenyl, halogen or by straight-chain or branched alkyl having up to 6 carbon atoms, R¹³ and R¹⁴ are identical or different and have the meaning of R³ and R⁴ indicated above, or R⁵ and/or R⁶ denote a group of the formula 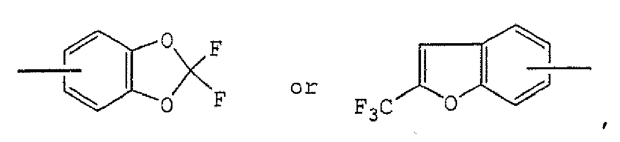 R⁷ denotes a hydrogen or halogen, R⁸ denotes hydrogen, halogen, azido, trifluoromethyl, hydroxyl, trifluoromethoxy, straight-chain or branched alkoxy or alkyl each having up to 6 carbon atoms or a group of the formula -NR¹⁵R¹⁶, wherein R¹⁵ and R¹⁶ are identical or different and have the meaning of R³ and R⁴ indicated above, or R⁷ and R⁸ together form a group of the formula =O or =NR¹⁷wherein R¹⁷ is a hydrogen or linear or branched chain alkyl, alkoxy or acyl, each having up to 6 carbon atoms, L is a linear or branched chain alkylene or alkenylene chain each having up to 8 carbon atoms, each of which is optionally substituted up to 2 times by hydroxyl, T and X are identical or different and denote a straight-chain or branched alkylene chain having up to 8 carbon atoms, or T or X denotes a bond, V represents an oxygen or sulfur atom or an -NR¹⁸ group, wherein R¹⁸ is a hydrogen or linear or branched chain alkyl having up to 6 carbon atoms or phenyl, E denotes cycloalkyl having 3 to 8 carbon atoms, or linear or branched chain alkyl having up to 8 carbon atoms, which is optionally substituted by cycloalkyl having 3 to 8 carbon atoms or hydroxyl, or represents phenyl which is optionally substituted by halogen or trifluoromethyl, R¹ and R² together form linear or branched alkylene chain having up to 7 carbon atoms, which must be substituted by a carbonyl group and/or a group of the formula, 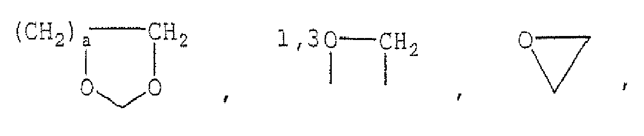 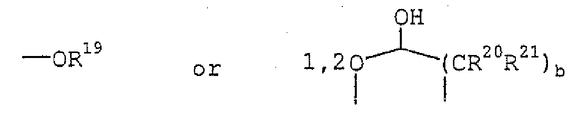 wherein a and b are identical or different and denote a number 1, 2 or 3, R¹⁹ denotes hydrogen, cycloalkyl having 3 to 7 carbon atoms, straight-chain or branched silylalkyl having up to 8 carbon atoms or straight-chain or branched alkyl having up to 8 carbon atoms, which is optionally substituted by hydroxyl, straight-chain or branched alkoxy having up to 6 carbon atoms or by phenyl, which for its part can be substituted by halogen, nitro, trifluoromethyl, trifluoromethoxy or by phenyl- or tetrazole-substituted phenyl, and alkyl is optionally substituted by a group of the formula -OR²², wherein R²² is linear or branched chain acyl or benzyl having up to 4 carbon atoms, or R¹⁹ is linear or branched chain acyl having up to 20 carbon atoms, or benzoyl which is optionally substituted by halogen, trifluoromethyl, nitro or trifluoromethoxy, or denotes straight-chain or branched fluoroacyl having up to 8 carbon atoms and up to 9 fluorine atoms, R²⁰ and R²¹ are identical or different, and denote hydrogen, phenyl or straight-chain or branched alkyl having up to 6 carbon atoms, or R²⁰ and R²¹ together form a 3 to 6-membered carbocycle, and, if appropriate also geminally, the carbocycles formed are optionally substituted up to 6 times in an identical or different manner by trifluoromethyl, hydroxyl, halogen, carboxyl, nitro, azido, cyano, cycloalkyl or cycloalkyloxy each having 3 to 7 carbon atoms, by straight-chain or branched alkoxycarbonyl, alkoxy or alkylthio each having up to 6 carbon atoms or by straight-chain or branched alkyl having up to 6 carbon atoms, which for its part is substituted up to 2 times in an identical or different manner by hydroxyl, benzyloxy, trifluoromethyl, benzoyl, straight-chain or branched alkoxy, oxyacyl or carboxyl each having up to 4 carbon atoms and/or phenyl, which for its part can be substituted by halogen, trifluoromethyl or trifluoromethoxy, and/or the carbocycles formed, also geminally, are optionally substituted up to 5 times in an identical or different manner by phenyl, benzoyl, thiophenyl or sulphonylbenzyl, which for their part are optionally substituted by halogen, trifluoromethyl, trifluoromethoxy or nitro, and/or are optionally substituted by a group of the formula 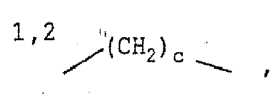 -SO₂-C₆H₅, -(CO)_{d}-NR²³R²⁴ or =O
   wherein c denotes a number 1, 2, 3 or 4, d denotes a number 0 or 1, R²³ and R²⁴ are identical or different and denote hydrogen, cycloalkyl having 3 to 6 carbon atoms, straight-chain or branched alkyl having up to 6 carbon atoms, benzyl or phenyl, which is optionally substituted up to 2 times in an identical or different manner by halogen, trifluoromethyl, cyano, phenyl or nitro, and/or the carbocycles formed are optionally substituted by a spiro-linked group of the formula 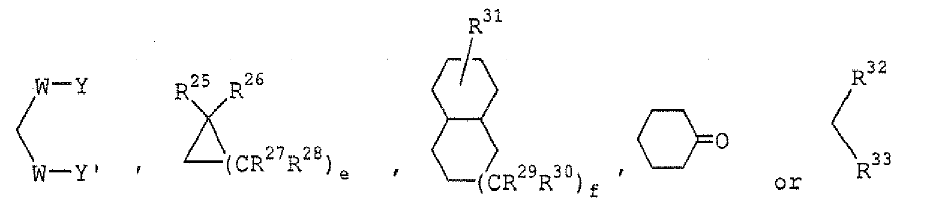 wherein W denotes an oxygen or a sulphur atom, Y and Y' together form a 2- to 6-membered straight-chain or branched alkylene chain, e denotes a number 1, 2, 3, 4, 5, 6 or 7, f denotes a number 1 or 2, R²⁵, R²⁶, R²⁷, R²⁸, R²⁹, R³⁰ and R³¹ are identical or different and denote hydrogen, trifluoromethyl, phenyl, halogen or straight-chain or branched alkyl or alkoxy each having up to 6 carbon atoms, or R²⁵ and R²⁶ or R²⁷ and R²⁸ in each case together form a straight-chain or branched alkyl chain having up to 6 carbon atoms, or R²⁵ and R²⁶ or R¹⁷ and R²⁸ in each case together form a group of the formula 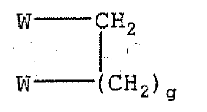 wherein W has the meaning indicated above, g denotes a number 1, 2, 3, 4, 5, 6 or 7, R³² and R³³ together form a 3-to 7-membered heterocycle which contains an oxygen or sulphur atom or a group of the formula SO, SO² or -NR³⁴, wherein R³⁴ denotes hydrogen, phenyl, benzyl or straight-chain or branched alkyl having up to 4 carbon atoms, a salt thereof or N-oxide thereof, with the exception of 5(6H)-quinolone, 3-benzoyl-7,8-dihydro-2,7,7-trimethyl-4-phenyl.
[84] The inhibitor of [2], [4] or [7], wherein the compound having a CETP inhibitory activity is cycloalkano-pyridine of the formula (XVIII) of [83], or a salt thereof or N-oxide thereof.
[85] The inhibitor of [5] or [8], which is cycloalkano-pyridine of the formula (XVIII) of [83], or a salt thereof or N-oxide thereof.
[86] The method of [1], [3] or [6], wherein the compound having a CETP inhibitory activity is substituted quinoline of the formula (XIX) 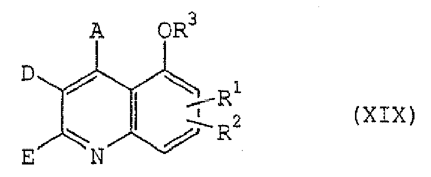 wherein A is aryl having 6 to 10 carbon atoms, which is optionally substituted up to 5 times in an identical or different manner by halogen, hydroxyl, trifluoromethyl, trifluoromethoxy, nitro, or straight chain or branched alkyl, acyl, hydroxyalkyl, or alkoxy, each having up to 7 carbon atoms, or a group of the formula -NR⁴R⁵, wherein R⁴ and R⁵ are identical or different and denote hydrogen, phenyl or straight chain or branched alkyl having up to 6 carbon atoms, D is aryl having 6 to 10 carbon atoms, which is optionally substituted by phenyl, nitro, halogen, trifluoromethyl or trifluoromethoxy, or denote a group of the formula R⁶-L- , 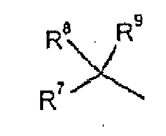 or R¹⁰-T-V-X- ,
   wherein R⁶, R⁷ and R¹⁰ independently of one another denote cycloalkyl having 3 to 6 carbon atoms, or aryl having 6 to 10 carbon atoms, or a 5- to 7-membered, optionally benzo-fused, saturated or unsaturated, mono-, bi- or tricyclic heterocycle having up to 4 heteroatoms from the group consisting of S, N and/or O, where the cycles are optionally substituted in the case of the nitrogen-containing rings also via the N functional group, up to 5 times in an identical or different manner by halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl, each having up to 6 carbon atoms, by aryl having 6 to 10 carbon atoms, or by an optionally benzo-fused, aromatic 5- to 7-membered heterocycle having up to 3 heteroatoms from the group consisting of S, N and/or O, and/or are substituted by a group of the formula -OR¹¹, -SR¹², -SO₂R¹³ or -NR¹⁴R¹⁵, wherein R¹¹, R¹² and R¹³ independently of one another denote aryl having 6 to 10 carbon atoms, which for its part is substituted up to 2 times in an identical or different manner by phenyl, halogen or by straight-chain or branched alkyl having up to 6 carbon atoms, R¹⁴ and R¹⁵ are identical or different and have the meaning of R⁴ and R⁵ indicated above, or R⁶ and/or R⁷ denote a group of the formula 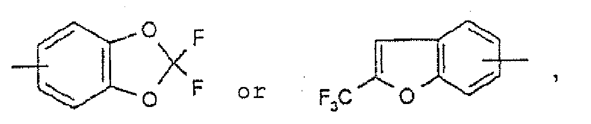 R⁸ denotes a hydrogen or halogen, R⁹ denotes hydrogen, halogen, azido, trifluoromethyl, hydroxyl, trifluoromethoxy, straight-chain or branched alkoxy or alkyl each having up to 6 carbon atoms or a group of the formula -NR¹⁶R¹⁷, wherein R¹⁶ and R¹⁷ are identical or different and have the meaning of R⁴ and R⁵ indicated above, or R⁸ and R⁹ together form a group of the formula =O or =NR¹⁸, wherein R¹⁸ is hydrogen or linear or branched alkyl, alkoxy or acyl, each having up to 6 carbon atoms, L is a linear or branched alkyl or alkenyl each having up to 8 carbon atoms, each of which is optionally substituted up to 2 times by hydroxyl, T and X are identical or different and denote straight-chain or branched alkyl having up to 8 carbon atoms, or T or X denotes a bond, V represents oxygen or sulfur atom or -NR¹⁹ group, wherein R¹⁹ is hydrogen or linear or branched alkyl having up to 6 carbon atoms or phenyl, E denotes cycloalkyl having 3 to 8 carbon atoms, or linear or branched alkyl having up to 8 carbon atoms, which is optionally substituted by cycloalkyl having 3 to 8 carbon atoms or hydroxyl, or represents phenyl which is optionally substituted by halogen or trifluoromethyl, R¹ and R² are identical or different and denote cycloalkyl having 3 to 8 carbon atoms, hydrogen, nitro, halogen, trifluoromethyl, trifluoromethoxy, carboxyl, hydroxyl, cyano, or straight chain or branched acyl, alkoxycarbonyl or alkoxy each having up to 6 carbon atoms, or a group of the formula - NR²⁰R²¹, wherein R²⁰ and R²¹ are identical or different and denote hydrogen, phenyl, or straight chain or branched alkyl having up to 6 carbon atoms and/or R¹ and/or R² denote straight chain or branched alkyl having up to 6 carbon atoms, which are optionally substituted by halogen, trifluoromethoxy, hydroxyl or straight chain or branched alkoxy having up to 4 carbon atoms, and/or aryl having 6 to 10 carbon atoms, which are optionally substituted up to 5 times in an identical or different manner by halogen, cyano, hydroxyl, trifluoromethyl, trifluoromethoxy, nitro, or straight chain or branched alkyl, acyl, hydroxyalkyl or alkoxy each having up to 7 carbon atoms, or a group of the formula -NR²²R²³, wherein R²² and R²³ are identical or different and denote hydrogen, phenyl, or straight chain or branched alkyl having up to 6 carbon atoms, and/or R¹ and R² together form straight chain or branched alkyl chain or alkenyl chain each having up to 6 carbon atoms, which is optionally substituted by halogen, trifluoromethyl, hydroxyl, or straight chain or branched alkoxy having up to 5 carbon atoms, R³ denotes hydrogen, straight chain or branched acyl or benzoyl having up to 20 carbon atoms, which is optionally substituted by halogen, trifluoromethyl, nitro or trifluoromethoxy, or straight chain or branched fluoroacyl having up to 8 carbon atoms and up to 7 fluorine atoms, cycloalkyl having 3 to 7 carbon atoms, or straight chain or branched alkyl having up to 8 carbon atoms, which is optionally substituted by hydroxyl, straight chain or branched alkoxy having up to 6 carbon atoms, or phenyl, which for its part can be substituted by halogen, nitro, trifluoromethyl, trifluoromethoxy or phenyl or tetrazol-substituted phenyl, and/or alkyl is optionally substituted by a group of the formula -OR²⁴, wherein R²⁴ denotes straight chain or branched acyl or benzyl having up to 4 carbon atoms, or a salt thereof.
[87] The inhibitor of [2], [4] or [7], wherein the compound having a CETP inhibitory activity is substituted quinoline of the formula (XIX) of [86] or a salt thereof.
[88] The inhibitor of [5] or [8], which is substituted quinoline of the formula (XIX) of [86] or a salt thereof.
[89] The method of [1], [3] or [6], wherein the compound having a CETP inhibitory activity is hetero tetrahydroquinoline of the formula (XX) 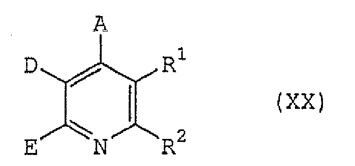 wherein A represents cycloalkyl having 3 to 8 carbon atoms or, represents a 5- to 7-membered saturated, partially unsaturated or unsaturated, optionally benzo-fused heterocycle having up to 3 heteroatoms from the group consisting of S, N and O, which, in the case of a saturated heterocycle with a nitrogen functional group, is optionally also attached via this functional group, and where the abovementioned ring systems are optionally substituted up to 5 times by identical or different substituents from the group consisting of halogen, nitro, hydroxyl, trifluoromethyl, trifluoromethoxy and straight-chain or branched alkyl, acyl, hydroxyalkyl or alkoxy having in each case up to 7 carbon atoms, or by a group of the formula -NR³R⁴ wherein R³ and R⁴ are identical or different and represent hydrogen, phenyl or straight-chain or branched alkyl having up to 6 carbon atoms, or A represents a group of the formula 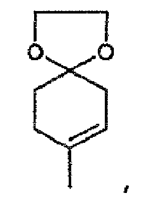 D represents aryl having 6 to 10 carbon atoms which is optionally substituted by phenyl, nitro, halogen, trifluoromethyl or trifluoromethoxy, or represents a group of the formula R⁵-L- , 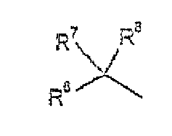 or R⁹-T-V-X- ,
   wherein R⁵, R⁶ and R⁹ independently of one another represent cycloalkyl having 3 to 6 carbon atoms, or represent aryl having 6 to 10 carbon atoms or represent a 5- to 7-membered optionally benzo-fused saturated or unsaturated mono-, bi-or tricyclic hetreocycle having up to 4 heteroatoms from the group consisting of S, N and O, where the cycles are optionally substituted, in the case of a nitrogen-containing rings also via an N functional group, up to 5 times by identical or different substituents from the group consisting of halogen, trifluoromethyl, nitro, hydroxyl, cyano carboxyl, trifluoromethoxy, and straight-chain or branched acyl, alkyl, alkylthio, alkylalkoxy, alkoxy or alkoxycarbonyl having in each case up to 6 carbon atoms, by aryl or trifluoromethyl-substituted aryl having in each case 6 to 10 carbon atoms, or by an optionally benzo-fused aromatic 5- to 7-membered heterocycle having up to 3 heteroatoms from the group consisting of S, N and O and/or by a group of the formula -OR¹⁰, -SR¹¹, -SO₂R¹² or -NR¹³R¹⁴, wherein R¹⁰, R¹¹ and R¹² independently of one another represent aryl having 6 to 10 carbon atoms which for its part is substituted up to 2 times by identical or different substituents from the group consisting of phenyl, halogen and straight-chain or branched alkyl having up to 6 carbon atoms, R¹³ and R¹⁴ are identical or different and have the meaning of R³ and R⁴ given above, or R⁵ and/or R⁶ represent(s) a group of the formula 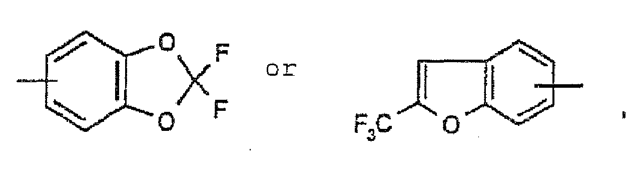 R⁷ is a hydrogen or halogen, R⁸ represents hydrogen, halogen, azido, trifluoromethyl, hydroxyl, trifluoromethoxy, straight-chain or branched alkoxy or alkyl having in each case up to 6 carbon atoms or a group of the formula -NR¹⁵R¹⁶, wherein R¹⁵ and R¹⁶ are identical or different and have the meaning of R³ and R⁴ given above, or R⁷ and R⁸ together form a group of the formula =O or =NR¹⁷ wherein R¹⁷ represents hydrogen or straight-chain or branched alkyl, alkoxy or acyl having in each case up to 6 carbon atoms, L represents a straight-chain or branched alkylene or alkenylene chain having in each case up to 8 carbon atoms, which is optionally substituted up to 2 times by hydroxyl, T and X are identical or different and represent a straight-chain or branched alkylene chain having up to 8 carbon atoms, or T or X represents a bond, V represents an oxygen or sulphur atom or represents an -NR¹⁸ group wherein R¹⁸ represents hydrogen or straight-chain or branched alkyl having up to 6 carbon atoms or phenyl, E represents cycloalkyl having 3 to 8 carbon atoms, or represents straight-chain or branched alkyl having up to 8 carbon atoms which is optionally substituted by cycloalkyl having 3 to 8 carbon atoms or hydroxy, or represents phenyl which is optionally substituted by halogen or trifluoromethyl, R¹ and R² together form a straight-chain or branched alkylene chain having up to 7 carbon atoms, which has to be substituted by a carbonyl group and/or by a group of the formula 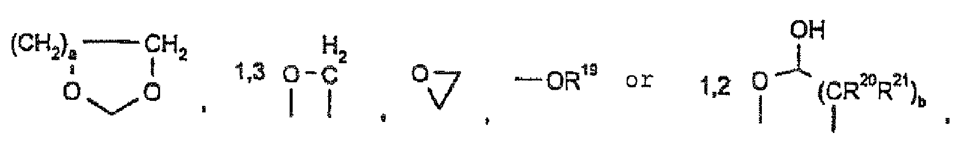 wherein a and b are identical or different and represent a number 1, 2 or 3, R¹⁹ represents hydrogen, cycloalkyl having 3 to 7 carbon atoms, straight-chain or branched silylalkyl having up to 8 carbon atoms, or straight-chain or branched alkyl having up to 8 carbon atoms, which is optionally substituted by hydroxyl, straight-chain or branched alkoxy having up to 6 carbon atoms or by phenyl which for its part may be substituted by halogen, nitro, trifluoromethyl, trifluoromethoxy or by phenyl or tetrazole-substituted phenyl, and alkyl is optionally substituted by a group of the formula -OR²², in which R²² represents straight-chain or branched acyl having up to 4 carbon atoms or benzyl, or R¹⁹ represents straight-chain or branched acyl having up to 20 carbon atoms or benzoyl, which is optionally substituted by halogen, trifluoromethyl, nitro or trifluoromethoxy, or represents straight-chain or branched fluoroacyl having up to 8 carbon atoms and up to 9 fluorine atoms, R²⁰ and R²¹ are identical or different, represent hydrogen, phenyl or straight-chain or branched alkyl having up to 6 carbon atoms, or R²⁰ and R²¹ together form 3 to 6-membered carbocycle and, if appropriate also geminally, the carbocycles formed are optionally substituted up to 6 times by identical or different substituents from the group consisting of trifluoromethyl, hydroxyl, nitrile, halogen, carboxyl, nitro, azido, cyano, cycloalkyl or cycloalkyloxy having in each case 3 to 7 carbon atoms, straight-chain or branched alkoxycarbonyl, alkoxy or alkylthio having in each case up to 6 carbon atoms and straight-chain or branched alkyl having up to 6 carbon atoms, which for its part is substituted up to 2 times by identical or different substituents from the group consisting of hydroxyl, benzyloxy, trifluoromethyl, benzoyl, straight-chain or branched alkoxy, oxyacyl or carboxyl having in each case up to 4 carbon atoms and phenyl, which for its part may be substituted by halogen, trifluoromethyl or trifluoromethoxy, and/or the carbocycles formed are optionally substituted, also geminally, up to 5 times by identical or different substituents from the group consisting of phenyl, benzoyl, thiophenyl and sulphonylbenzyl, which for their part are optionally substituted by halogen, trifluoromethyl, trifluoromethoxy or nitro, and/or are optionally substituted by a group of the formula 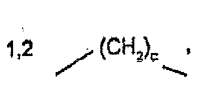 -SO₂-C₆H₅, -(CO)_{d}NR²³R²⁴ or =O,
   wherein c represents a number 1, 2, 3 or 4, d represents a number 0 or 1, R²³ and R²⁴ are identical or different and represent hydrogen, cycloalkyl having 3 to 6 carbon atoms, straight-chain or branched alkyl having up to 6 carbon atoms, benzyl or phenyl which is optionally substituted up to 2 times by identical or different substituents from the group consisting of halogen, trifluoromethyl, cyano, phenyl and nitro, and/or the carbocycles formed are optionally substituted by a spiro-linked group of the formula 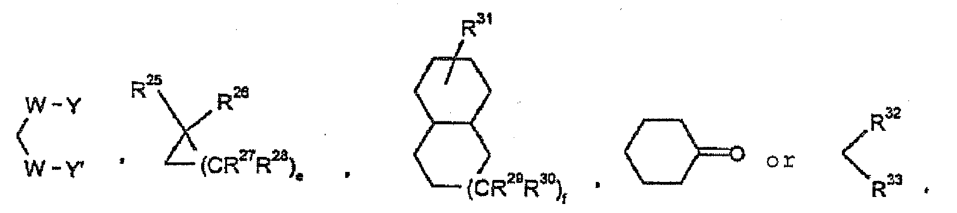 wherein W represents either an oxygen or a sulphur atom, Y and Y' together form a 2- to 6-membered straight-chain or branched alkylene chain, e represents a number 1, 2, 3, 4, 5, 6 or 7, f represents a number 1 or 2, R²⁵, R²⁶, R²⁷, R²⁸, R²⁹, R³⁰ and R³¹ are identical or different and represent hydrogen, trifluoromethyl, phenyl, halogen or straight-chain or branched alkyl or alkoxy having in each case up to 6 carbon atoms, or R²⁵ and R²⁶ or R²⁷ and R²⁸ in each case together form straight-chain or branched alkyl chain having up to 6 carbon atoms, or R²⁵ and R²⁶ or R²⁷ and R²⁸ in each case together form a group of the formula 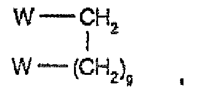 wherein W is as defined above, g represents a number 1, 2, 3, 4, 5, 6 or 7, R³² and R³³ together form 3- to 7-membered heterocycle which contains an oxygen or sulphur atom or a group of the formula SO, SO₂ or -NR³⁴, in which R³⁴ represents hydrogen, phenyl, benzyl or straight-chain or branched alkyl having up to 4 carbon atoms, a salt thereof or N-oxide thereof.
[90] The inhibitor of [2], [4] or [7], wherein the compound having a CETP inhibitory activity is hetero-tetrahydroquinoline of the formula (XX) of [89] or a salt thereof or N-oxide thereof.
[91] The inhibitor of [5] or [8], which is hetero-tetrahydroquinoline of the formula (XX) of [89] or a salt thereof, or N-oxide thereof.
[92] The method of [1], [3] or [6], wherein the compound having a CETP inhibitory activity is tetrahydroquinoline of the formula (XXI) 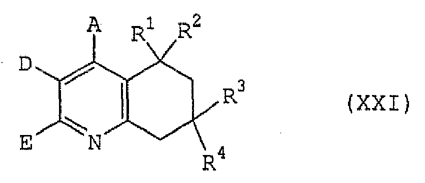 in which
   A represents phenyl which is optionally substituted up to 2 times by identical or different substituents from the group consisting of halogen, trifluoromethyl and straight-chain or branched alkyl or alkoxy having in each case up to 3 carbon atoms,
   D represents a group of the formula 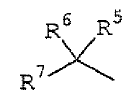 or R⁸-CH₂-O-CH₂-
      in which
   R⁵ and R⁶ together form a carbonyl group (=O), or
   R⁵ represents hydrogen and
   R⁶ represents halogen or hydroxyl, or
   R⁵ and R⁶ represent hydrogen,
   R⁷ and R⁸ are identical or different and represent phenyl, naphthyl, benzothiazolyl, quinolyl, pyrimidyl or pyridyl which are optionally substituted up to 4 times by identical or different substituents from the group consisting of halogen, trifluoromethyl, nitro, cyano, trifluoromethoxy, or by a group of the formula -SO₂-CH₃ or -NR⁹R¹⁰, in which R⁹ and R¹⁰ are identical or different and represent hydrogen or straight-chain or branched alkyl having up to 3 carbon atoms,
   E represents cycloalkyl having 3 to 6 carbon atoms, or represents straight-chain or branched alkyl having up to 8 carbon atoms,
   R¹ represents hydroxyl, and
   R² represents hydrogen or represents methyl,
   R³ and R⁴ are identical or different and represent straight-chain or branched alkyl having up to 3 carbon atoms, or
   R³ and R⁴ together form a spiro-linked alkyl chain having 2 to 4 carbon atoms,
   or a salt thereof or N-oxide thereof.
[93] The inhibitor of [2], [4] or [7], wherein the compound having a CETP inhibitory activity is tetrahydroquinoline of the formula (XXI) of [92], or a salt thereof or N-oxide thereof.
[94] The inhibitor of [5] or [8], which is tetrahydroquinoline of the formula (XXI) of [92], or a salt thereof or N-oxide thereof.
[95] The method of [1], [3] or [6], wherein the compound having a CETP inhibitory activity is a compound of the formula (XXII) 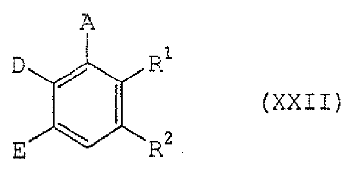 wherein A is cycloalkyl having 3 to 8 carbon atoms, aryl having 6 to 10 carbon atoms, or 5- to 7-membered saturated, partially unsaturated or unsaturated optionally benzo-fused heterocycle having up to 4 hetero atoms from the group consisting of S, N and o, wherein the ring systems of the aryl and said heterocycle are optionally substituted up to 5 times by identical or different substituents from the group consisting of cyano, halogen, nitro, carboxyl, hydroxyl, trifluoromethyl and trifluoromethoxy, or straight chain or branched alkyl, acyl, hydroxyalkyl, alkylthio, alkoxycarbonyl, oxyalkoxycarbonyl or alkoxy, each having up to 7 carbon atoms, or a group of the formula -NR³R⁴, wherein R³ and R⁴ are identical or different and denote hydrogen, phenyl or straight chain or branched alkyl having up to 6 carbon atoms, D denotes a group of the formula R⁵-L- , 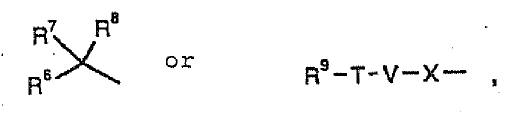 wherein R⁵, R⁶ and R⁹ independently of one another denote aryl having 6 to 10 carbon atoms, or 5- to 7-membered, optionally benzo-fused, saturated or unsaturated, mono-, bi- or tricyclic heterocycle having up to 4 heteroatoms from the group consisting of S, N and O, where the cycles are optionally substituted, in the case of nitrogen-containing rings also via an N functional group, up to 5 times, by identical or different substituents from the group consisting of halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl, each having up to 6 carbon atoms, by aryl-or trifluoromethyl-substituted aryl, each having 6 to 10 carbon atoms, or by an optionally benzo-fused, aromatic 5-to 7-membered heterocycle having up to 3 heteroatoms from the group consisting of S, N and O, and/or by a group of the formula -OR¹⁰, -SR¹¹, -SO₂R¹² or -NR¹³R¹⁴, wherein R¹⁰, R¹¹ and R¹² independently of one another denote aryl having 6 to 10 carbon atoms, which for its part is substituted up to 2 times by identical or different substituents from the group consisting of phenyl and halogen, or by straight-chain or branched alkyl having up to 6 carbon atoms, R¹³ and R¹⁴ are identical or different and have the meaning of R³ and R⁴ indicated above, or R⁵ and/or R⁶ denote a group of the formula 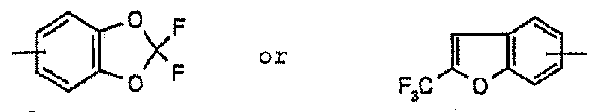 R⁷ denotes a hydrogen, halogen or methyl, R⁸ denotes hydrogen, halogen, azido, trifluoromethyl, hydroxyl, trifluoromethoxy, straight-chain or branched alkoxy or alkyl each having up to 6 carbon atoms or a group of the formula -NR¹⁵R¹⁶, wherein R¹⁵ and R¹⁶ are identical or different and have the meaning of R³ and R⁴ indicated above, or R⁷ and R⁸ together form a group of the formula =O or =NR¹⁷, wherein R¹⁷ is hydrogen or linear or branched alkyl, alkoxy or acyl, each having up to 6 carbon atoms, L is a linear or branched alkylene or alkenylene chain each having up to 8 carbon atoms, each of which is optionally substituted up to 2 times by hydroxyl, T and X are identical or different and denote straight-chain or branched alkylene chain having up to 8 carbon atoms, or T or X denotes a bond, V represents an oxygen or sulfur atom or -NR¹⁸- group, wherein R¹⁸ is a hydrogen or linear or branched alkyl having up to 6 carbon atoms or phenyl, E denotes cycloalkyl having 3 to 8 carbon atoms, or linear or branched alkyl having up to 8 carbon atoms, which is optionally substituted by cycloalkyl having 3 to 8 carbon atoms or hydroxyl, or represents phenyl which is optionally substituted by halogen or trifluoromethyl, R¹ denotes straight chain or branched alkyl having up to 6 carbon atoms, which is substituted by hydroxyl or a group of the formula 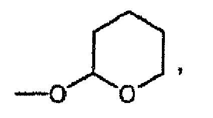 R² denotes hydrogen, or straight chain or branched alkyl or alkenyl each having up to 8 carbon atoms, which is optinonally substituted by hydroxy, halogen, phenyl, cycloalkyl having 3 to 6 carbon atoms, or a group of the formula -O-R¹⁹ 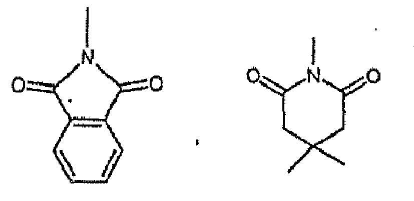 or 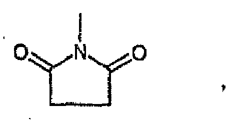 wherein R¹⁹ denotes a group of the formula -Si(CH₃)₂C(CH₃)₃, or straight chain or branched alkyl having up to 6 carbon atoms, or 5- to 7-membered saturated, partially unsaturated or unsaturated heterocycle having up to 3 hetero atoms from the group consisting of S, N and O, or phenyl or benzyl, wherein all ring systems for R¹⁹ are optionally substituted up to 2 times by identical or different substituents from the group consisting of trifluoromethyl, fluorine, nitro, hydroxyl, straight chain or branched alkoxy or alkoxycarbonyl having in each case up to 4 carbon atoms, or by straight chain or branched alkyl having up to 4 carbon atoms optionally substituted by hydroxyl, or a salt thereof.
[96] The inhibitor of [2], [4] or [7], wherein the compound having a CETP inhibitory activity is a compound of the formula (XXII) of [95], or a salt thereof.
[97] The inhibitor of [5] or [8], which is a compound of the formula (XXII) of [95], or a salt thereof.
[98] The method of [1], [3] or [6], wherein the compound having a CETP inhibitory activity is a compound represented by the formula (XXIII) 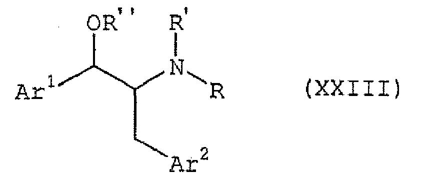 wherein Ar¹ is an aromatic ring group optionally having substituents, Ar² is an aromatic ring group having substituent, OR" is an optionally protected hydroxyl group, R is an acyl group, R' is a hydrogen atom or a hydrocarbon group optionally having substituents, or a salt thereof with the exception of tert-butyl benzyl-[2(S)-hydroxy-2-thiazol-2-yl-1(S)-(4-trifluoromethyl-benzyl)-ethyl]-carbamate.
[99] The inhibitor of [2], [4] or [7], wherein the compound having a CETP inhibitory activity is a compound of the formula (XXIII) of [98], or a salt thereof.
[100] The inhibitor of [5] or [8], which is a compound of the formula (XXIII) of [98], or a salt thereof.
[101] The method of [98], wherein the compound of the formula (XXIII) is a compound selected from the group consisting of N-[(1RS,2SR)-2-(4-fluorophenyl)-2-hydroxy-1-[4-(trifluoromethyl)benzyl]ethyl]-6,7-dihydro-5H-benzo[a]cycloheptene-1-carboxamide, 4-fluoro-N-((1R,2S)-2-(4-fluorophenyl)-2-hydroxy-1-((4-(trifluoromethyl)phenyl)methyl)ethyl)-1-naphthalenecarboxamide, N-[(1R,2S)-2-(4-fluorophenyl)-2-hydroxy-1-[3-(1,1,2,2-tetrafluoroethoxy)benzyl]ethyl]-6,7-dihydro-5H-benzo[a]cycloheptane-1-carboxamide, N-[(1RS,2SR)-2-(4-fluorophenyl)-2-hydroxy-1-[3-(1,1,2,2-tetrafluoroethoxy)benzyl]ethyl]-5,6-dihydronaphthalene-1-carboxamide, N-[(1RS,2SR)-2-(4-fluorophenyl)-2-hydroxy-1-[3-(1,1,2,2-tetrafluoroethoxy)benzyl]ethyl]-6,7,8,9-tetrahydro-5H-benzo[a]cycloheptene-1-carboxamide, 4-fluoro-N-[(1R,2S)-2-(4-fluorophenyl)-2-hydroxy-1-[3-(1,1,2,2-tetrafluoroethoxy)benzyl]ethyl]naphthalene-1-carboxamide, N-[(1RS,2SR)-2-(4-fluorophenyl)-2-hydroxy-1-[3-(1,1,2,2-tetrafluoroethoxy)benzyl]ethyl]-5,6,7,8-tetrahydrobenzo[a]cyclooctene-1-carboxamide, N-[(1RS,2SR)-2-(4-fluorophenyl)-2-hydroxy-1-(4-isopropylbenzyl)ethyl]-6,7-dihydro-5H-benzo[a]cycloheptane-1-carboxamide, N-((1RS,2SR)-2-(3-fluorophenyl)-2-hydroxy-1-((4-(trifluoromethyl)phenyl)methyl)ethyl)-6,7-dihydro-5H-benzo[a]cycloheptane-1-carboxamide, N-(1RS,2SR)-2-hydroxy-2-(4-phenoxyphenyl)-1-((4-(trifluoromethyl)phenyl)methyl)ethyl)-6,7-dihydro-5H-benzo[a]cycloheptane-1-carboxamide, N-[(1RS,2SR)-2-(4-chlorophenyl)-2-hydroxy-1-[3-(1,1,2,2-tetrafluoroethoxy)benzyl]ethyl]-6,7-dihydro-5H-benzo[a]cycloheptane-1-carboxamide, N-(1RS,2SR)-2-hydroxy-2-(4-(phenyloxy)phenyl)-1-((3-((1,1,2,2-tetrafluoroethyl)oxy)phenyl)methyl)ethyl)-6,7-dihydro-5H-benzo[a]cycloheptene-1-carboxamide, N-((1RS,2SR)-2-(4-((4-chloro-3-ethylphenyl)oxy)phenyl)-2-hydroxy-1-((3-((1,1,2,2-tetrafluoroethyl)oxy)phenyl)methyl)ethyl)-6,7-dihydro-5H-benzo[a]cycloheptene-1-carboxamide, N-((1RS,2SR)-2-(2-fluoropyridin-4-yl)-2-hydroxy-1-((3-(1,1,2,2-tetrafluoroethoxy)phenyl)methyl)ethyl)-6,7-dihydro-5H-benzo[a]cycloheptene-1-carboxamide, N-((1RS,2RS)-2-(6-fluoropyridin-2-yl)-2-hydroxy-1-((3-(1,1,2,2-tetrafluoroethoxy)phenyl)methyl)ethyl)-6,7-dihydro-5H-benzo[a]cycloheptane-1-carboxamide, N-[(1RS,2SR)-1-(4-tert-butylbenzyl)-2-(3-chlorophenyl)-2-hydroxyethyl]-5-chloro-1-naphthamide and 4-fluoro-N-{(1RS,2SR)-2-(4-fluarcphenyl)-2-hydroxy-1-[(2,2,3,3-tetrafluoro-2,3-dihydro-1,4-benzodioxyn-6-yl)methyl]ethyl}-1-naphthamide, or a salt thereof.
[102] The inhibitor of [99] or [100], wherein the compound of the formula (XXIII) is a compound selected from the group consisting of N-[(1RS,2SR)-2-(4-fluorophenyl)-2-hydroxy-1-[4-(trifluoromethyl)benzyl]ethyl]-6,7-dihydro-5H-benzo[a]cycloheptene-1-carboxamide, 4-fluoro-N-((1R,2S)-2-(4-fluorophenyl)-2-hydroxy-1-((4-(trifluoromethyl)phenyl)methyl)ethyl)-1-naphthalenecarboxamide, N-[(1R,2S)-2-(4-fluorophenyl)-2-hydroxy-1-[3-(1,1,2,2-tetrafluoroethoxy)benzyl]ethyl]-6,7-dihydro-5H-benzo[a]cycloheptane-1-carboxamide, N-[(1RS,2SR)-2-(4-fluorophenyl)-2-hydroxy-1-[3-(1,1,2,2-tetrafluoroethoxy)benzyl]ethyl]-5,6-dihydronaphthalene-1-carboxamide, N-[(1RS,2SR)-2-(4-fluorophenyl)-2-hydroxy-1-[3-(1,1,2,2-tetrafluoroethoxy)benzyl]ethyl]-6,7,8,9-tetrahydro-5H-benzo[a]cycloheptene-1-carboxamide, 4-fluoro-N-[(1R,2S)-2-(4-fluorophenyl)-2-hydroxy-1-[3-(1,1,2,2-tetrafluoroethoxy)benzyl]ethyl]naphthalene-1-carboxamide, N-[(1RS,2SR)-2-(4-fluorophenyl)-2-hydroxy-1-[3-(1,1,2,2-tetrafluoroethoxy)benzyl]ethyl]-5,6,7,8-tetrahydrobenzo[a]cyclooctene-1-carboxamide, N-[(1RS,2SR)-2-(4-fluorophenyl)-2-hydroxy-1-(4-isopropylbenzyl)ethyl]-6,7-dihydro-5H-benzo[a]cycloheptane-1-carboxamide, N-((1RS,2SR)-2-(3-fluorophenyl)-2-hydroxy-1-((4-(trifluoromethyl)phenyl)metbyl)ethyl)-6,7-dihydro-5H-benzo[a]cycloheptane-1-carboxamide, N-((1RS,2SR)-2-hydroxy-2-(4-phenoxyphenyl)-1-((4-(trifluoromethyl)phenyl)methyl)ethyl)-6,7-dihydro-5H-benzo[a]cycloheptane-1-carboxamide, N- [(1RS,2SR)-2-(4-chlorophenyl)-2-hydroxy-1-[3-(1,1,2,2-tetrafluoroethoxy)benzyl]ethyl]-6,7-dihydro-5H-benzo[a]cycloheptane-1-carboxamide, N-((1RS,2SR)-2-hydroxy-2-(4-(phenyloxy)phenyl)-1-((3-((1,1,2,2-tetrafluoroethyl)oxy)phenyl)methyl)ethyl)-6,7-dihydro-5H-benzo[a]cycloheptene-1-carboxamide, N-((1RS,2SR)-2-(4-((4-chloro-3-ethylphenyl)oxy)phenyl)-2-hydroxy-1-((3-((1,1,2,2-tetrafluoroethyl)oxy)phenyl)methyl)ethyl)-6,7-dihydro-5H-benzo[a]cycloheptene-1-carboxamide, N-((1RS,2SR)-2-(2-fluoropyridin-4-yl)-2-hydroxy-1-((3-(1,1,2,2-tetrafluoroethoxy)phenyl)methyl)ethyl)-6,7-dihydro-5H-benzo[a]cycloheptene-1-carboxamide, N-((1RS,2RS)-2-(6-fluoropyridin-2-yl)-2-hydroxy-1-((3-(1,1,2,2-tetrafluoroethoxy)phenyl)methyl)ethyl)-6,7-dihydro-5H-benzo[a]cycloheptane-1-carboxamide, N-[(1RS,2SR)-1-(4-tert-butylbenzyl)-2-(3-chlorophenyl)-2-hydroxyethyl]-5-chloro-1-naphthamide and 4-fluoro-N-{(1RS,2SR)-2-(4-fluorophenyl)-2-hydroxy-1-[(2,2,3,3-tetrafluoro-2,3-dihydro-1,4-benzodioxyn-6-yl)methyl]ethyl}-1-naphthamide, or a salt thereof.
[103] The method of [1], [3] or [6], wherein the compound having a CEPT inhibitory activity is a compound selected from the group consisting of Code Names: JTT-705, CP-529414, SC-795, SC-744, SC-554, SC-71952, SC-56960, SC-57201, PD-140195, WK-5344A, WK-5344B, CETi-1(CETP vaccine), BM99-1 and BM99-2, or a salt thereof .
[104] The inhibitor of [2], [4] or [7], wherein the compound having a CEPT inhibitory activity is a compound selected from the group consisting of Code Names: JTT-705, CP-529414, SC-795, SC-744, SC-554, SC-71952, SC-56960, SC-57201, PD-140195, WK-5344A, WK-5344B, CETi-1(CETP vaccine), BM99-1 and BM99-2, or a salt thereof.
[105] The inhibitor of [5] or [8], which is a compound selected from the group consisting of Code Names: JTT-705, CP-529414, SC-795, SC-744, SC-554, SC-71952, SC-56960, SC-57201, PD-140195, WK-5344A, WK-5344B, CETi-1(CETP vaccine), BM99-1 and BM99-2, or a salt thereof.
[106] A method for the prophylaxis or treatment of a disease selected from the group consisting of hyperlipidemia, arteriosclerosis and hyper-remnant-emia, which comprises administering an effective amount of an inhibitor of [2], [4], [5], [7] or [8].
[107] An agent for the prophylaxis or treatment of a disease selected from the group consisting of hyperlipidemia, arteriosclerosis and hyper-remnant-emia, which comprises an inhibitor of [2], [4], [5], [7] or [8] as an active ingredient.

### Detailed Description of the Invention

The present invention provides a prophylactic drug or a therapeutic drug of hyperlipidemia, arteriosclerosis or hyper-remnant lipoproteinemia in which a remnant lipoprotein is involved (e.g., hereditary hyper-remnant lipoproteinemia, such as familiar dysbetalipidemia, familial lipase deficiency, familial combined hyperlipidemia and the like; or secondary hyper-remnant lipoproteinemia that expresses subsequent to diabetic hypertriglyceridemia, hypothyroidism, nephritic syndrome and the like) by selectively inhibiting CETP.

### Best Mode for Carrying Out the Invention

Remnant lipoproteins refer to lipoproteins produced during catabolism of triglyceride-rich lipoproteins (chylomicron, very low density lipoprotein (VLDL)). Remnant lipoproteins are lipoprotein particles which are commonly characterized by being rich in triglyceride, cholesterol and apolipoprotein E (apoE), and having apoB (chylomicron remnant has apoB-48 and a small amount of apoB-100, VLDL remnant has apoB-100). The metabolism of chylomicron and VLDL starts from hydrolysis of triglyceride by LPL and completes through remnant lipoproteins. The former is called a chylomicron remnant and the latter is called a VLDL remnant,

CETP is an abbreviation of cholesteryl ester transfer protein. Due to CETP action, triglyceride in triglyceride-rich lipoproteins is transferred to a high density lipoprotein (HDL) and cholesteryl ester in HDL is transferred to a triglyceride-rich lipoproteins.

A compound having a CEPT inhibitory action of the present invention (hereinafter to be also referred to as CEPT inhibitor) may be any as long as it has a CEPT inhibitory action, and is exemplified by compounds disclosed in WO2003/026672, WO2002/088085, WO2002/088069, WO2002/083128 WO2002/064549, WO2002/059077, WO2002/013797, WO2002/011710, WO2000/053792, WO2000/038726, WO2000/033724, WO2000/038721, WO2000/018724, WO2000/018723, WO2000/018721, WO99/41237, WO99/15504, WO99/14215, WO99/14204, WO99/14174, WO98/39299, WO98/34920, WO98/04528, WO96/15141, WO95/06626, WW93/11782, WO99/1548, WO03/028727, WO2004/039453, WO99/41237, WO03/026672, WO95/06626, US patent No. 6,727,277, US patent application publication 2003/040545, US patent application publication 2003/032644, US patent application publication 2003/027826, US patent application publication 2002/177708, US patent application publication 2002/165231, US patent application publication 2002/120011, US patent application publication 2001/018446, US patent 6521607, US patent No. 6482862, US patent No. 6476075, US patent No. 6462092, US patent No. 6458849, US patent No. 6458803, US patent No. 6455519, US patent No. 6451823, US patent No. 6207671, US patent No. 6063788, US patent No. 5932587, US patent No. 5925645, US patent No. 5519001, JP-A-2002-326987, JP patent No. 9059155, AU2002027728, JP-T-516732/2001 (WO99/14174), JP-T-510478/2001 (WO98/34920), JP-A-10-167967, JP-A-9-255574, JP-A-9-59155, JP-A-293764/2002, JP-A-221376/2003 and the like.

Specifically, the compound disclosed in WO00/17164 (JP-T-2002-526475) can be mentioned:
a compound of the formula (I) 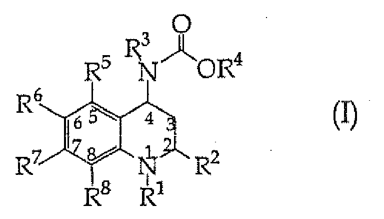 wherein
   - R¹: is a hydrogen, Y, W-X or W-Y;
   - W: is carbonyl, thiocarbonyl, sulfinyl or sulfonyl;
   - X: is -O-Y, -S-Y, -N(H)-Y or -N-(Y)₂; wherein Y in each case is independently Z or a fully saturated, partially unsaturated or fully unsaturated 1- to 10-membered straight chain or branched carbon chain, wherein the carbons other than a connecting carbons may be replaced with 1 or 2 heteroatoms independently selected from oxygen, sulfur and nitrogen, and the carbon may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon chain may be mono-substituted by Z;
   Z is a partially saturated, fully saturated or fully unsaturated 3- to 8-membered ring optionally having 1 to 4 heteroatoms independently selected from oxygen, sulfur and nitrogen, or a bicyclic ring consisting of two fused partially saturated, fully saturated or fully unsaturated 3- to 6-membered rings, taken independently, which may have 1 to 4 heteroatoms independently selected from oxygen, sulfur and nitrogen;
   the Z substituent may be independently mono-, di-or tri-substituted by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, wherein the (C₁-C₆)alkyl substituent may be independently mono-, di- or tri-substituted by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino and the (C₁-C₆)alkyl substituent may be substituted by 1 to 9 fluorines;
   - R²: is a partially saturated, fully saturated or fully unsaturated 1- to 6-membered straight chain or branched carbon chain, wherein the carbons other than a connecting carbons may be replaced with 1 or 2 heteroatoms independently selected from oxygen, sulfur and nitrogen, the carbon atom may be independently mono-, di- or tri-substituted by halo, the carbon may be mono-substituted by oxo, the carbon may be mono-substituted by hydroxy, the sulfur may be mono or di-substituted by oxo, the nitrogen may be mono or di-substituted by oxo; or R² is a partially saturated, fully saturated or fully unsaturated 3- to 7-membered ring optionally having 1 or 2 heteroatoms independently selected from oxygen, sulfur and nitrogen, wherein the R² ring may be bonded via (C₁-C₄)alkyl; wherein the R² ring may be independently mono-, di- or tri-substituted by halo, (C₂-C₆)alkenyl, (C₁-C₆)alkyl, hydroxy, (C₁-C₆)alkoxy, (C₁-C₄)alkylthio, amino, nitro, cyano,oxo, carboxy, (C₁-C₆)alkyloxycarbonyl, mono-N- or di-N,N-(C₁-C₆)alkylamino, and the (C₁-C₆)alkyl substituent may be independently mono-, di- or tri-substituted by halo, hydroxy, (C₁-C₆)alkoxy, (C₁-C₄)alkylthio, oxo or (C₁-C₆)alkyloxycarbonyl; provided that R² is not methyl;
   - R³: is a hydrogen or Q; wherein Q is a fully saturated, partially unsaturated or fully unsaturated 1- to 6-membered straight chain or branched carbon chain, wherein the carbons other than a connecting carbons may be replaced with one heteroatom selected from oxygen, sulfur and nitrogen, the carbon may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon chain may be mono-substituted by V; V is a partially saturated, fully saturated or fully unsaturated 3- to 8-membered ring optionally having 1 to 4 heteroatoms independently selected from oxygen, sulfur and nitrogen or a bicyclic ring consisting of two fused partially saturated, fully saturated or fully unsaturated 3- to 6-membered rings, taken independently, which may have 1 to 4 heteroatoms independently selected from oxygen, sulfur and nitrogen; wherein the V substituent may be independently mono-, di- or tri-substituted by halo, (C₁- C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl, mono-N- or di-N,N-(C₁-C₆)alkylamino, wherein the (C₁-C₆)alkyl or (C₂-C₆)alkenyl substituent may be independently mono-, di- or tri-substituted by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent or (C₂-C₆)alkenyl substituent may be substituted by 1 to 9 fluorines;
   - R⁴: is Q¹ or V¹;
   wherein Q¹ is a fully saturated, partially unsaturated or fully unsaturated 1- to 6-membered straight chain or branched carbon chain, wherein the carbons other than a connecting carbons may be replaced with one heteroatom selected from oxygen, sulfur and nitrogen, the carbon may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon chain may be mono-substituted by V¹;
   V¹ is a partially saturated, fully saturated or fully unsaturated 3- to 6-membered ring optionally having 1 or 2 heteroatoms independently selected from oxygen, sulfur and nitrogen; V¹ substituent may be independently mono-, di-, tri- or tetra-substituted independently by halo, (C₁-C₆)alkyl, (C₁-C₆)alkoxy, amino, nitro, cyano, (C₁-C₆)alkyloxycarbonyl, mono-N- or di-N,N-(C₁-C₆)alkylamino wherein the (C₁-C₆)alkyl substituent is optionally mono-substituted independently by oxo or the (C₁-C₆)alkyl substituent is optionally substituted by 1 to 9 fluorines; wherein any R³ should contain V, or R⁴ should contain V¹; and
   - R⁵, R⁶, R⁷ and R⁸: are each independently hydrogen, a bond, nitro or halo, wherein the bond is substituted by T or a partially saturated, fully saturated or fully unsaturated straight chain or branched carbon chain having 1 to 12 carbon atoms, wherein the carbons may be replaced with 1 or 2 heteroatoms independently selected from oxygen, sulfur and nitrogen, wherein the carbon atom may be independently mono-, di- or tri-substituted by halo, the carbon may be mono-substituted by hydroxy, the carbon may be mono-substituted by oxo, the sulfur may be mono or di-substituted by oxo, the nitrogen may be mono or di-substituted by oxo, and the carbon may be mono-substituted by T; T is a partially saturated, fully saturated or fully unsaturated 3- to 8-membered ring optionally having 1 to 4 heteroatoms independently selected from oxygen, sulfur and nitrogen, or a bicyclic ring consisting of two fused partially saturated, fully saturated or fully unsaturated 3- to 6-membered rings, taken independently, which may have 1 to 4 heteroatoms independently selected from oxygen, sulfur and nitrogen; wherein the T substituent is optionally mono-, di-or tri-substituted by halo, (C₁-C₆)alkyl, (C₂-C₆)alkenyl, hydroxy, (C₁-C₆)alkoxy, (C₁- C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent is optionally substituted by 1 to 9 fluorines; and
   - R⁵ and R⁶, or R⁶ and R⁷, and/or R⁷ and R⁸: may be linked to form a partially saturated or fully unsaturated 4- to 8-membered ring optionally having 1 to 3, at least 1, heteroatom independently selected from nitrogen, sulfur and oxygen;
   wherein the ring (one or plural) formed by R⁵ and R⁶, or R⁶ and R⁷, and/or R⁷ and R⁸ may be independently mono-, di- or tri-substituted by halo, (C₁-C₆)alkyl, (C₁-C₄)alkylsulfonyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(G₁-C₆)alkylamino, the (C₁-C₆)alkyl substituent may be independently mono-, di- or tri-substituted by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent may be substituted by 1 to 9 fluorines;
provided that when R² is carboxyl or (C₁-C₄)alkylcarboxyl, R¹ is not a hydrogen, a prodrug thereof or a pharmaceutically acceptable salt of the compound or the prodrug.

The expression of "prodrug" of the formula (I) refers to a compound which is a drug precursor that releases, following administration, a drug according to a specific chemical or physiological process *in vivo* (e.g., prodrug is converted to a desired drug form by setting to a physiological pH or by an enzyme action). An exemplary prodrug releases a corresponding free acid upon cleavage, and examples of such hydrolytic ester forming residue of the compound of the formula (I) includes, but not limited to, those wherein its free hydrogen of carboxyl moiety is substituted by (C₁-C₄)alkyl, (C₂-C₇)alkanoyloxymethyl, 1-(alkanoyloxy)ethyl having 4 to 9 carbon atoms, 1-methyl-1-(alkanoyloxy)-ethyl having 5 to 10 carbon atoms, alkoxycarbonyloxymethyl having 3 to 6 carbon atoms, 1-(alkoxycarbonyloxy)ethyl having 4 to 7 carbon atoms, 1-methyl-1-(alkoxycarbonyloxy)ethyl having 5 to 8 carbon atoms, N-(alkoxycarbonyl)aminomethyl having 3 to 9 carbon atoms, 1-(N-(alkoxycarbonyl)amino)ethyl having 4 to 10 carbon atoms, 3-phthalidyl, 4-crotonolactonyl, γ-butyrolacton-4-yl, di-N,N-(C₁-C₂)alkylamino(C₂-C₃)alkyl (e.g., b-dimethylaminoethyl), carbamoyl-(C₁-C₂)alkyl, N,N-di(C₁-C₂)alkylcarbamoyl-(C₁-C₂)alkyl, piperidino-, pyrrolidino- and morpholino(C₂-C₃)alkyl.

In the following paragraphs, exemplary rings (one kind or plural kinds) are described for the explanation of general rings included herein. An exemplary 5- or 6-membered ring in the formula (I), which may have 1 or 2 heteroatoms independently selected from oxygen, nitrogen and sulfur, includes phenyl, furyl, thienyl, pyrrolyl, oxazolyl, thiazolyl, imidazolyl, pyrazolyl, isoxazolyl, isothiazolyl, pyridinyl, pyridiazinyl, pyrimidinyl and pyrazinyl.

An exemplary partially saturated, fully saturated or fully unsaturated 5- to 8-membered ring in the formula (I), which may have 1 to 4 heteroatoms independently selected from oxygen, sulfur and nitrogen includes cyclopentyl, cyclohexyl, cycloheptyl, cyclooctyl and phenyl. A further exemplary 5-membered ring in the formula (I) includes 2H-pyrrolyl, 3H-pyrrolyl, 2-pyrrolinyl, 3-pyrrolinyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathiolyl, 1,2,3-oxadiazolyl, 1,2,4-oxadiazolyl, 1,2,5-oxadiazolyl, 1,3,4-oxadiazolyl, 1,2,3-triazolyl, 1,2,4-triazolyl, 1,3,4-thiadiazolyl, 1,2,3,4-oxatriazolyl, 1,2,3,5-oxatriazolyl, 3H-1,2,3-dioxazolyl, 1,2,4-dioxazolyl, 1,3,2-dioxazolyl, 1,3,4-dioxazolyl, 5H-1,2,5-oxathiazolyl and 1,3-oxathiolyl.

A further exemplary 6-membered ring in the formula (I) includes 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxadinyl, p-isoxadinyl, 1,2,5-oxathiazinyl, 1,2,6-oxathiazinyl, 1,4,2-oxadiazinyl and 1,3,5,2-oxadiazinyl.

A further exemplary 7-membered ring in the formula (I) includes azepinyl, oxepinyl and thiepinyl.

A further exemplary 8-membered ring in the formula (I) includes cyclooctyl, cyclooctenyl and cyclooctadienyl.

An exemplary bicyclic ring consisting of two fused partially saturated, fully saturated or fully unsaturated 5- or 6-membered rings, taken independently, optionally having 1 to 4 heteroatoms independently selected from nitrogen, sulfur and oxygen includes indolizinyl, indolyl, isoindolyl, 3H-indolyl, 1H-isoindolyl, indolinyl, cyclopenta(b)pyridinyl, pyrano(3,4-b)pyrrolyl, benzofuryl, isobenzofuryl, benzo(b)thienyl, benzo(c)thienyl, 1H-indazolyl, indoxazinyl, benzoxazolyl, benzimidazolyl, benzothiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, indenyl, isoindenyl, naphthyl, tetralinyl, decalinyl, 2H-1-benzopyranyl, pyrido(3,4-b)-pyridinyl, pyrido(3,2-b)-pyridinyl, pyrido(4,3-b)-pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl and 4H-1,4-benzoxazinyl.

What alkylene in the formula (I) means is a saturated hydrocarbon (straight chain or branched chain) wherein a hydrogen atom has been removed from each terminal carbon. Examples of such group include (assuming that the designated length encompasses specific examples) methylene, ethylene, propylene, butylene, pentylene, hexylene and heptylene.

What the halo in the formula (I) means is chloro, bromo, iodo or fluoro.

What the alkyl in the formula (I) means is a saturated straight chain hydrocarbon or a saturated branched chain hydrocarbon. Examples of such alkyl group (assuming that the designated length encompasses specific examples) include methyl, ethyl, propyl, isopropyl, butyl, sec-butyl, tert-butyl, pentyl, isopentyl, neopentyl, tert-pentyl, 1-methylbutyl, 2-methylbutyl, 3-methylbutyl, hexyl, isohexyl, heptyl and octyl.

What the alkoxy in the formula (I) means is a saturated straight chain alkyl or a saturated branched chain alkyl, each being bonded via oxy. Examples of such alkoxy group (assuming that the designated length encompasses specific examples) include methoxy, ethoxy, propoxy, isopropoxy, butoxy, isobutoxy, tert-butoxy, pentoxy, isopentoxy, neopentoxy, tert-pentoxy, hexoxy, isohexoxy, heptoxy and octoxy.

The terms mono-N- and di-N,N-(C₁-Cₓ)alkyl ... used for the formula (I) refer to (C₁-Cₓ)alkyl moiety taken independently, when it is di-N,N-(C₁-Cₓ)alkyl ... (x means an integer).

It should be appreciated that, when, in the formula (I), a monocyclic or heterocyclic moiety can be bonded to a designated substrate via a different ring atom, without indicating a specific binding site, or can be linked by a different method, any point with such possibility is intended, whether via a carbon atom, or via, for example, a trivalent nitrogen atom. For example, the term "pyridyl" means 2-, 3- or 4-pyridyl, and "thienyl" means 2-, or 3-thienyl.

A reference to a term "the carbon" in the phrases, "the carbon may be independently mono-, di- or tri-substituted by halo, the carbon may be mono-substituted by hydroxy, the carbon may be mono-substituted by oxo" in the formula (I) means each carbon in a carbon chain including a connecting carbon.

A reference to "nitrogen may be ... di-substituted by oxo" in the formula (I) means a terminal nitrogen constituting a nitro functionality.

The expression "a pharmaceutically acceptable salt" in the formula (I) means a non-toxic anion salt including anion, which is exemplified by, though not limited to, chloride, bromide, iodide, sulfate, hydrogen sulfate, phosphate, acetate, maleate, fumarate, oxalate, lactate, tartrate, citrate, gluconate, methanesulfonate and 4-toluene-sulfonate. This expression also means, but not limited to, non-toxic cation salts such as sodium, potassium, calcium, magnesium, ammonia or protonated benzathine (N,N'-dibenzylethylenediamine), choline, ethanolamine, diethanolamine, ethylenediamine, meglamine (N-methyl-glucamine), benethamine (N-benzylphenethylamine), piperazine and tromethamine (2-amino-2-hydroxymethyl-1,3-propanediol).

Specifically, the compound disclosed in WO00/17166 (JP-T-2002-526477) can be mentioned:
a compound of the formula (II) : 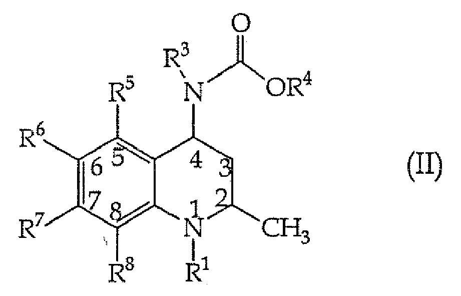
wherein R¹ is hydrogen, Y, W-X, W-Y;
W is carbonyl, thiocarbonyl, sulfinyl or sulfonyl;
X is -O-Y, -S-Y, -N(H)-Y or -N-(Y)₂;
Y in each case is independently, Z or a fully saturated, partially unsaturated or fully unsaturated 1- to 10-membered straight or branched carbon chain wherein the carbons other than the connecting carbon, may be replaced with 1 or 2 heteroatoms selected independently from oxygen, sulfur and nitrogen and aforementioned carbon is optionally mono-, di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon chain is optionally mono-substituted by Z ;
Z is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein the aforementioned Z substituent is optionally mono-, di- or tri-substituted independently by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the aforementioned (C₁-C₆)alkyl substituent is optionally mono-, di- or tri-substituted independently by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the aforementioned (C₁-C₆)alkyl is optionally substituted by 1 to 9 fluorines;
R³ is hydrogen or Q;
wherein Q is a fully saturated, partially unsaturated or fully unsaturated 1- to 6-membered straight or branched carbon chain wherein the carbons other than the connecting carbon, may be replaced with one heteroatom selected from oxygen, sulfur and nitrogen, the aforementioned carbon is optionally mono-, di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon chain is optionally mono-substituted by V;
wherein V is a partially saturated, fully saturated or fully unsaturated 3- to 12-membered ring optionally having 1 to 4 heteroatoms selected independently from oxygen, sulfur and nitrogen or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
the aforementioned V substituent is optionally mono-, di-, tri- or tetra-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl, mono-N- or di-N,N-(C₁-C₆)alkylamino wherein the aforementioned (C₁-C₆)alkyl or (C₂-C₆)alkenyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino or the aforementioned (C₁-C₆)alkyl or (C₂-C₆)alkenyl substituent is optionally substituted by 1 to 9 fluorines;
R⁴ is Q¹ or V¹;
wherein Q¹ is a fully saturated, partially unsaturated or fully unsaturated 1- to 6-membered straight or branched carbon chain wherein the carbons other than the connecting carbon, may be replaced with one heteroatom selected from oxygen, sulfur and nitrogen and the aforementioned carbon is optionally mono-, di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon chain is optionally mono-substituted by V¹;
wherein V¹ is a partially saturated, fully saturated or fully unsaturated 3- to 6-membered ring optionally having 1 or 2 heteroatoms selected from oxygen, sulfur and nitrogen; wherein the aforementioned V¹ substituent is optionally mono-, di-, tri- or tetra-substituted independently by halo, (C₁-C₆)alkyl, (C₁-C₆)alkoxy, amino, nitro, cyano, (C₁-C₆)alkyloxycarbonyl, mono-N- or di-N,N-(C₁-C₆)alkylamino, the aforementioned (C₁-C₆)alkyl substituent is optionally mono-substituted by oxo, the aforementioned (C₁-C₆)alkyl substituent is optionally substituted by 1 to 9 fluorines; wherein either R³ should contain V or R⁴ should contain V¹; and R⁵, R⁶, R⁷ and R⁸ are each independently hydrogen, a bond, nitro or halo wherein the aforementioned bond is substituted by T or a partially saturated, fully saturated or fully unsaturated straight or branched carbon chain having 1 to 12 carbon atoms wherein the carbons may be replaced with 1 or 2 heteroatoms selected independently from oxygen, sulfur and nitrogen wherein the aforementioned carbon atom is optionally mono- di- or tri-substituted independently by halo, the aforementioned carbon is optionally mono-substituted by hydroxy, the aforementioned carbon is optionally mono-substituted by oxo, the aforementioned sulfur is optionally mono- or di-substituted by oxo, the aforementioned nitrogen is optionally mono- or di-substituted by oxo, and the aforementioned carbon is optionally mono-substituted by T;
wherein T is a partially saturated, fully saturated or fully unsaturated 3- to 12-membered ring optionally having 1 to 4 heteroatoms selected independently from oxygen, sulfur and nitrogen or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein the aforementioned T substituent is optionally mono-, di- or tri-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the aforementioned (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the aforementioned (C₁-C₆)alkyl substituent is optionally substituted by 1 to 9 fluorines; provided that at least one of substituents R⁵, R⁶, R⁷ and R⁸ is not hydrogen and is not linked to the quinoline moiety through oxy, a prodrug thereof or a pharmaceutically acceptable salt of the compound or the prodrug.

The expression of "prodrug" of the formula (II) refers to a compound of a drug precursor that releases a drug in vivo according to some specific chemical or physiological process after administration (e.g., prodrug is converted to a desired drug form by achieving a physiological pH or by an enzyme action). A typical prodrug cleaves and releases a corresponding free acid. An ester forming residue of a hydrolyzable compound of the formula (II) has a carboxyl moiety, and is exemplified by, but not limited to, those wherein its free hydrogen has been substituted by (C₁-C₄)alkyl, (C₂-C₇)alkanoyloxymethyl, 1-(alkanoyloxy)ethyl having 4 to 9 carbon atoms, 1-methyl-1-(alkanoyloxy)ethyl having 5 to 10 carbon atoms, alkoxycarbonyloxymethyl having 3 to 6 carbon atoms, 1-(alkoxycarbonyloxy)ethyl having 4 to 7 carbon atoms, 1-methyl-1-(alkoxycarbonyloxy)ethyl having 5 to 8 carbon atoms, N-(alkoxycarbonyl)aminomethyl having 3 to 9 carbon atoms, 1-(N-(alkoxycarbonyl)amino)ethyl having 4 to 10 carbon atoms, 3-phthalidyl, 4-crotonolactonyl, γ-butyrolacton-4-yl, di-N,N-(C₁-C₂)alkylamino(C₂-C₃)alkyl (e.g., b-dimethylaminoethyl), carbamoyl-(C₁-C₂)alkyl, N,N-di(C₁-C₂)alkylcarbamoyl-(C₁-C₂)alkyl, piperidino-, pyrrolidino- or morpholino(C₂-C₃)alkyl.

Examples of the 5- or 6-membered aromatic ring in the formula (II), which has 1 or 2 heteroatoms independently selected from oxygen, nitrogen and sulfur as desired, include phenyl, furyl, thienyl, pyrrolyl, oxazolyl, thiazolyl, imidazolyl, pyrazolyl, isoxazolyl, isothiazolyl, pyridinyl, pyridiazinyl, pyrimidinyl, pyrazinyl and the like.

Examples of the partially saturated, fully saturated or fully unsaturated 5- to 8-membered ring in the formula (II), which has 1 to 4 heteroatoms independently selected from oxygen, sulfur and nitrogen as desired, include cyclopentyl, cyclohexyl, cycloheptyl, cyclooctyl, phenyl and the like. Further examples of the 5-membered ring include 2H-pyrrolyl, 3H-pyrrolyl, 2-pyrrolinyl, 3-pyrrolinyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathiolyl, 1,2,3-oxadiazolyl, 1,2,4-oxadiazolyl, 1,2,5-oxadiazolyl, 1,3,4-oxadiazolyl, 1,2,3-triazolyl, 1,2,4-triazolyl, 1,3,4-thiadiazolyl, 1,2,3,4-oxatriazolyl, 1,2,3,5-oxatriazolyl, 3H-1,2,3-dioxazolyl, 1,2,4-dioxazolyl, 1,3,2-dioxazolyl, 1,3,4-dioxazolyl, 5H-1,2,5-oxathiazolyl, 1,3-oxathiolyl and the like.

A further exemplary 6-membered ring includes 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxadinyl, p-isoxadinyl, 1,2,5-oxathiazinyl, 1,2,6-oxathiazinyl, 1,4,2-oxadiazinyl, 1,3,5,2-oxadiazinyl and the like.

Further examples of the 7-membered ring include azepinyl, oxepinyl, thiepinyl and the like.

Further examples of the 8-membered ring include cyclooctyl, cyclooctenyl, cyclooctadienyl and the like.

In the case of the formula (II), examples of a bicyclic ring consisting of two fused partially saturated, fully saturated or fully unsaturated 5- or 6-membered rings (independently selected) optionally having 1 to 4 heteroatoms independently selected from nitrogen, sulfur and oxygen include indolizinyl, indolyl, isoindolyl, 3H-indolyl, 1H-isoindolyl, indolinyl, cyclopenta(b)pyridinyl, pyrano(3,4-b)pyrrolyl, benzofuryl, isobenzofuryl, benzo(b)thienyl, benzo(c)thienyl, 1H-indazolyl, indoxazinyl, benzoxazolyl, benzimidazolyl, benzothiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, indenyl, isoindenyl, naphthyl, tetralinyl, decalinyl, 2H-1-benzopyranyl, pyrido(3,4-b)-pyridinyl, pyrido(3,2-b)-pyridinyl, pyrido(4,3-b)-pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl, 4H-1,4-benzoxazinyl and the like.

The alkylene in the formula (II) means a saturated hydrocarbon (straight chain or branched) wherein a hydrogen atom has been removed from each terminal carbon. Examples of such group include (assuming that the designated length encompasses specific examples) methylene, ethylene, propylene, butylene, pentylene, hexylene and heptylene.

The halo in the formula (II) means chloro, bromo, iodo or fluoro.

The alkyl in the formula (II) means a saturated straight chain hydrocarbon or a saturated branched chain hydrocarbon. Examples of such alkyl group (assuming that the designated length encompasses specific examples) include methyl, ethyl, propyl, isopropyl, butyl, sec-butyl, tert-butyl, pentyl, isopentyl, neopentyl, tert-pentyl, 1-methylbutyl, 2-methylbutyl, 3-methylbutyl, hexyl, isohexyl, heptyl and octyl.

The alkoxy in the formula (II) means a saturated straight chain alkyl or a saturated branched chain alkyl, each being bonded via oxy. Examples of such alkoxy group (assuming that the designated length encompasses specific examples) include methoxy, ethoxy, propoxy, isopropoxy, butoxy, isobutoxy, tert-butoxy, pentoxy, isopentoxy, neopentoxy, tert-pentoxy, hexoxy, isohexoxy, heptoxy and octoxy.

The terms mono-N- and di-N,N-(C₁-Cₓ)alkyl ... used for the formula (II) refer to (C₁-Cₓ)alkyl moiety taken independently, when it is di-N,N-(C₁-Cₓ)alkyl ... (x means an integer).

The "aforementioned carbon" in the "aforementioned carbon may be independently mono-, di- or tri-substituted by halo, the aforementioned carbon may be mono-substituted by hydroxy, the aforementioned carbon may be mono-substituted by oxo" in the formula (II) refers to each carbon in the carbon chain containing a connecting carbon.

The "nitrogen is ... di-substituted by oxo" in the formula (II) refers to the terminal nitrogen constituting the nitro functionality.

It should be appreciated that, when a carbon or heterocyclic moiety can be bonded or attached to a designated substrate via a different ring atom, without indicating a specific binding site, any point with such possibility is intended, whether via a carbon atom, or, for example, via a trivalent nitrogen atom. For example, the term "pyridyl" means 2-, 3- or 4-pyridyl, "thienyl" means 2-, or 3-thienyl, and the like.

The expression, "a pharmaceutically acceptable salt" in the formula (II) means, but not limited to, non-toxic anion salts including anion such as chloride, bromide, iodide, sulfate, hydrogen sulfate, phosphate, acetate, maleate, fumarate, oxalate, lactate, tartrate, citrate, gluconate, methanesulfonate and 4-toluene-sulfonate. This expression also means, but not limited to, non-toxic cation salts such as sodium, potassium, calcium, magnesium, ammonium or protonated benzathine (N,N'-dibenzylethylenediamine), choline, ethanolamine, diethanolamine, ethylenediamine, meglamine (N-methyl-glucamine), benethamine (N-benzylphenethylamine), piperazine and tromethamine (2-amino-2-hydroxymethyl-1,3-propanediol).

Specifically, the compound disclosed in WO00/17165 (JP-T-2002-526476) can be mentioned:
a compound of the formula (III) 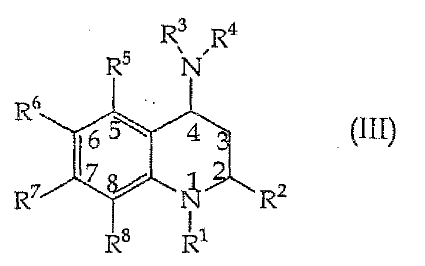
wherein R¹ is Y, W-X or W-Y;
wherein W is a carbonyl, thiocarbonyl, sulfinyl or sulfonyl;
X is -O-Y, -S-Y, -N(H)-Y or -N-(Y)₂;
Y in each case is independently Z or fully saturated, partially unsaturated or fully unsaturated 1- to 10-membered straight chain or branched carbon chain wherein the carbons other than the connecting carbon, may be replaced with 1 or 2 heteroatoms selected independently from oxygen, sulfur and nitrogen and the carbon is mono-, di- or tri-substituted independently by halo, the carbon is optionally mono-substituted by hydroxy, the carbon is optionally mono-substituted by oxo, the sulfur is optionally mono or di-substituted by oxo, the nitrogen is optionally mono or di-substituted by oxo, and the carbon chain is optionally mono-substituted by Z;
wherein Z is a partially saturated, fully saturated or fully unsaturated 3- to 8-membered ring optionally having 1 to 4 heteroatoms independently selected from oxygen, sulfur and nitrogen, or a bicyclic ring consisting of two fused partially saturated, fully saturated or fully unsaturated 3- to 6-membered rings, taken independently, optionally having 1 to 4 heteroatoms from nitrogen, sulfur and oxygen; wherein the Z substituent is optionally mono-, di- or tri-substituted independently by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl substituent is optionally substituted by 1 to 9 fluorines;
R² is a partially saturated, fully saturated or fully unsaturated 1- to 6-membered straight chain or branched carbon chain wherein the carbons other than the connecting carbon, may be replaced with 1 or 2 heteroatoms selected independently from oxygen, sulfur and nitrogen wherein the carbon atom is optionally mono-, di- or tri-substituted independently by halo, the carbon is optionally mono-substituted by oxo, the carbon is optionally mono-substituted by hydroxy, the sulfur is optionally mono or di-substituted by oxo, the nitrogen is optionally mono or di-substituted by oxo; or the R² is a partially saturated, fully saturated or fully unsaturated 3- to 7-membered ring optionally having 1 or 2 heteroatoms selected independently from oxygen, sulfur and nitrogen, wherein the R² ring is optionally bonded via (C₁-C₄)alkyl;
wherein the R² ring is optionally mono-, di- or tri-substituted by halo, (C₂-C₆)alkenyl, (C₁-C₆)alkyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by halo, hydroxy, (C₁-C₆)alkoxy, (C₁-C₄)alkylthio, oxo or (C₁-C₆)alkyloxycarbonyl;
R³ is hydrogen or Q;
wherein Q is a fully saturated, partially unsaturated or fully unsaturated 1- to 6-membered straight chain or branched carbon chain wherein the carbons, other than connecting carbon, may be replaced with one heteroatom selected from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is mono or di-substituted by oxo, the nitrogen is optionally mono or di-substituted by oxo, and the carbon chain is optionally mono-substituted by V;
wherein V is a partially saturated, fully saturated or fully unsaturated 3- to 8-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein the V substituent is optionally mono, di, tri- or tetra-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl, mono-N- or di-N,N-(C₁-C₆)alkylamino wherein the (C₁-C₆)alkyl substituent or (C₂-C₆)alkenyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl substituent or (C₂-C₆)alkenyl substituent is optionally substituted by 1 to 9 fluorines;
R⁴ is cyano, formyl, W¹Q¹, W¹V¹, (C₁-C₄)alkylene V¹ or V²; wherein W¹ is carbonyl, thiocarbonyl, SO or SO₂; Q¹ is a fully saturated, partially unsaturated or fully unsaturated 1- to 6-membered straight chain or branched carbon chain wherein the carbons may be replaced with one heteroatom selected from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is optionally mono or di-substituted by oxo, the nitrogen is optionally mono or di-substituted by oxo, and the carbon chain is optionally mono-substituted by V¹;
wherein V¹ is a partially saturated, fully saturated or fully unsaturated 3- to 6-membered ring optionally having 1 or 2 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein the V¹ substituent is optionally mono, di, tri- or tetra-substituted independently by halo, (C₁-C₆)alkyl, (C₁-C₆)alkoxy, hydroxy, oxo, amino, nitro, cyano, (C₁-C₆)alkyloxycarbonyl, mono-N- or di-N,N-(C₁-C₆)alkylamino wherein the (C₁-C₆)alkyl substituent is optionally mono-substituted by oxo, the (C₁-C₆)alkyl substituent is also optionally substituted by 1 to 9 fluorines;
wherein V² is a partially saturated, fully saturated or fully unsaturated 5- to 7-membered ring containing 1 to 4 heteroatoms independently from oxygen, sulfur and nitrogen; wherein the V² substituent is optionally mono-, di- or tri-substituted independently by halo, (C₁-C₂)alkyl, (C₁-C₂)alkoxy, hydroxy or oxo, wherein the (C₁-C₂)alkyl optionally has 1 to 5 fluorines; and
wherein R⁴ does not include oxycarbonyl bonded directly to the C⁴ nitrogen;
wherein either R³ should contain V or R⁴ should contain V¹; R⁵, R⁶, R⁷ and R⁸ are independently hydrogen, a bond, nitro or halo wherein the bond is substituted by T or a partially saturated, fully saturated or fully unsaturated straight chain or branched carbon chain having 1 to 12 carbon atoms wherein the carbon is replaced with 1 or 2 heteroatoms selected independently from oxygen, sulfur and nitrogen, wherein the carbon atom is optionally mono-, di- or tri-substituted independently by halo, the carbon is optionally mono-substituted by hydroxy, the carbon is optionally mono-substituted by oxo, the sulfur is optionally mono or di-substituted by oxo, the nitrogen is optionally mono or di-substituted by oxo, and the carbon chain is optionally mono-substituted by T;
wherein T is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein the T substituent is optionally mono-, di- or tri-substituted by halo, (C₁-C₆)alkyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent also optionally has 1 to 9 fluorines;
wherein R⁵ and R⁶, or R⁶ and R⁷, and/or R⁷ and R⁸ may also be linked to form at least one ring that is a partially saturated or fully unsaturated 4- to 8-membered ring optionally having 1 to 3 heteroatoms independently selected from nitrogen, sulfur and oxygen;
wherein the ring formed by R⁵ and R⁶, or R⁶ and R⁷, and/or R⁷ and R⁸ are optionally mono-, di- or tri-substituted by halo, (C₁-C₆)alkyl, (C₁-C₄)alkylsulfonyl, (C₂-C₆)alkenyl, hydroxy, (C₁-C₆)alkoxy; (C₁-C₄)alkylthio, amino, nitro, cyano, oxo, carboxy, (C₁-C₆)alkyloxycarbonyl mono-N- or di-N,N-(C₁-C₆)alkylamino wherein the (C₁-C₆)alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent also optionally has 1 to 9 fluorines, a prodrug thereof or a pharmaceutically acceptable salt of the compound or the prodrug.

The expression of "prodrug" of the formula (III) refers to a compound of a drug precursor that releases a drug *in vivo* due to some specific chemical or physiological process after administration (e.g., prodrug is converted to a desired drug form by achieving a physiological pH or by an enzyme action). Examples of the prodrug that releases a corresponding free acid upon cleavage and the residue forming hydrolytic ester of the compound of the formula (III) include, but not limited to, those having a carboxyl moiety, wherein its free hydrogen has been substituted by the following groups: (C₁-C₄)alkyl, (C₂-C₇)alkanoyloxymethyl, 1-(alkanoyloxy)ethyl having 4 to 9 carbon atoms, 1-methyl-1-(alkanoyloxy)ethy having 5 to 10 carbon atoms, alkoxycarbonyloxymethyl having 3 to 6 carbon atoms, 1-(alkoxycarbonyloxy)ethyl having 4 to 7 carbon atoms, 1-methyl-1-(alkoxycarbonyloxy)ethyl having 5 to 8 carbon atoms, N-(alkoxycarbonyl)aminomethyl having 3 to 9 carbon atoms, 1-(N-(alkoxycarbonyl)amino)ethyl having 4 to 10 carbon atoms, 3-phthalidyl, 4-crotonolactonyl, γ-butyrolacton-4-yl, di-N,N-(C₁-C₂)alkylamino(C₂-C₃)alkyl (e.g., b-dimethylaminoethyl), carbamoyl-(C₁-C₂)alkyl , N, N-di(C₁-C₂)alkylcarbamoyl-(C₁-C₂)alkyl, piperidino-, pyrrolidino- or morpholino (C₂-C₃)alkyl.

Examples of the 5- or 6-membered aromatic ring in the formula (III), which has 1 or 2 heteroatoms as desired, which are independently selected from oxygen, nitrogen and sulfur, include phenyl, furyl, thienyl, pyrrolyl, oxazolyl, thiazolyl, imidazolyl, pyrazolyl, isoxazolyl, isothiazolyl, pyridinyl, pyridiazinyl, pyrimidinyl and pyrazinyl.

Examples of the partially saturated, fully saturated or fully unsaturated 5- to 8-membered ring in the formula (III), which has 1 to 4 heteroatoms as desired, which are independently selected from oxygen, sulfur and nitrogen, include cyclopentyl, cyclohexyl, cycloheptyl, cyclooctyl, phenyl and the like. Further examples of the 5-membered ring include the following: 2H-pyrrolyl, 3H-pyrrolyl, 2-pyrrolinyl, 3-pyrrolznyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathialyl, 1,2,3-oxadiazolyl, 1,2,4-oxadiazolyl, 1,2,5-oxadiazolyl, 1,3,4-oxadiazolyl, 1,2,3-triazolyl, 1,2,4-triazolyl, 1,3,4-thiadiazolyl, 1,2,3,4-oxatriazolyl, 1,2,3,5-oxatriazolyl, 3H-1,2,3-dioxazolyl, 1,2,4-dioxazolyl, 1,3,2-dioxazolyl, 1,3,4-dioxazolyl, 5H-1,2,5-oxathiazolyl and 1,3-oxathialyl.

Further examples of the 6-membered ring include the following: 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxadinyl, p₋isoxadinyl, 1,2,5-oxathiazinyl, 1,2,6-oxathiazinyl, 1,4,2-oxadiazinyl and 1,3,5,2-oxadiazinyl.

Further examples of the 7-membered ring include azepinyl, oxepinyl and thiepinyl.

Further examples of the 8-membered ring include cyclooctyl, cyclooctenyl and cyclooctadienyl.

In the case of the formula (III), examples of a bicyclic ring consisting of two fused partially saturated, fully saturated or fully unsaturated 5- or 6-membered rings, taken independently, having 1 to 4 heteroatoms as desired, which are independently selected from nitrogen, sulfur and oxygen, include the following: indolizinyl, indolyl, isoindolyl, 3H-indolyl, 1H-isoindolyl, indolinyl, cyclopenta(b)pyridinyl, pyrano(3,4-b)pyrrolyl, benzofuryl, isobenzofuryl, benzo(b)thienyl, benzo(c)thienyl, 1H-indazolyl, indoxazinyl, benzoxazolyl, benzimidazolyl, benzothiazolyl, purinyl, 4H-quinolizanyl, quinolinyl, isoquinolinyl, cinnolinyl, phthalazinyl, quinazolinyl, quinoxalinyl, 1,8-naphthyridinyl, pteridinyl, indenyl, isoindenyl, naphthyl, tetralinyl, decalinyl, 2H-1-benzopyranyl, pyrido(3,4-b)-pyridinyl, pyrido(3,2-b)-pyridinyl, pyrido(4,3-b)-pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl and 4H-1,4-benzoxazinyl.

The alkylene in the formula (III) means a saturated hydrocarbon (straight chain or branched chain) wherein a hydrogen atom has been removed from each terminal carbon. Examples of such group include (assuming that the designated length encompasses specific examples) methylene, ethylene, propylene, butylene, pentylene, hexylene and heptylene.

The halo in the formula (III) means chloro, bromo, iodo or fluoro.

The alkyl in the formula (III) means a saturated straight chain hydrocarbon or a saturated branched chain hydrocarbon. Examples of such alkyl group (assuming that the designated length encompasses specific examples) include methyl, ethyl, propyl, isopropyl, butyl, sec-butyl, t-butyl, pentyl, isopentyl, neopentyl, t-pentyl, 1-methylbutyl, 2-methylbutyl, 3-methylbutyl, hexyl, isohexyl, heptyl and octyl.

The alkoxy in the formula (III) means a saturated straight chain alkyl or a saturated branched chain alkyl bonded via oxy. Examples of such alkoxy group (assuming that the designated length encompasses specific examples) include methoxy, ethoxy, propoxy, isopropoxy, butoxy, isobutoxy, t-butoxy, pentoxy, isopentoxy, neopentoxy, t-pentoxy, hexoxy, isohexoxy, heptoxy and octoxy.

The terms mono-N- and di-N,N-(C₁-Cₓ)alkyl ... used for the formula (III) refer to (C₁-Cₓ)alkyl moiety taken independently, when it is di-N,N-(C₁-Cₓ)alkyl ... (x means an integer).

The "aforementioned carbon" in the "aforementioned carbon is mono-, di- or tri-substituted as desired by halo, the aforementioned carbon is mono-substituted as desired by hydroxy, the aforementioned carbon is mono-substituted as desired by oxo" in the formula (II) refers to each carbon containing a connecting carbon in the carbon chain.

The "nitrogen is ... di-substituted by oxo" in the formula (III) refers to the terminal nitrogen constituting the nitro functional group.

It should be appreciated that, in the formula (III), when a carbon ring or heterocyclic moiety is bonded to a designated substrate via various ring atoms, or attached thereto in a different form, without indicating a specific binding site, any point with such possibility is intended, whether via a carbon atom, or, for example, via a trivalent nitrogen atom. For example, the term "pyridyl" means 2-, 3- or 4-pyridyl, "thienyl" means 2-, or 3-thienyl, and the like, and hereinafter the same.

The expression "a pharmaceutically acceptable salt" in the formula (III) means, but not limited to, a non-toxic anion salt including anion such as chloride, bromide, iodide, sulfate, hydrogen sulfate, phosphate, acetate, maleate, fumarate, oxalate, lactate, tartrate, citrate, gluconate, methanesulfonate and 4-toluene-sulfonate. This expression also means, but not limited to, non-toxic cation salts such as sodium, potassium, calcium, magnesium, ammonium or protonated benzathine (N,N'-dibenzylethylenediamine), choline, ethanolamine, diethanolamine, ethylenediamine, meglamine (N-methyl-glucamine), benethamine (N-benzylphenethylamine), piperazine and tromethamine (2-amino-2-hydroxymethyl-1,3-propanediol).

Specifically, the compound disclosed in JP-A-2000-95764 can be mentioned:
a compound of the formula (IV): 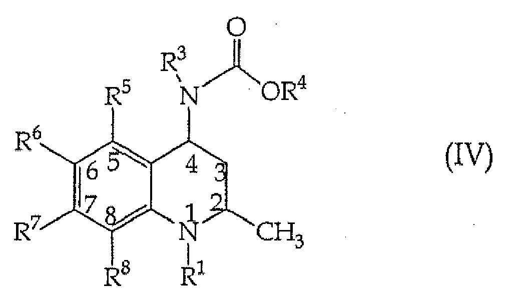
wherein R¹ is hydrogen, Y, W-X, W-Y;
wherein W is a carbonyl, thiocarbonyl, sulfinyl or sulfonyl;
X is -O-Y, -S-Y, -N (H) -Y or -N-(Y)₂;
Y in each case is independently Z or a fully saturated, partially unsaturated or fully unsaturated 1- to 10-membered straight chain or branched carbon chain wherein the carbons other than the connecting carbon, may be replaced with 1 or 2 heteroatoms selected independently from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is optionally mono- or di-substituted by oxo, the nitrogen is optionally mono- or di-substituted by oxo, and the carbon chain is optionally mono-substituted by Z; wherein Z is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein Z substituent is optionally mono-, di- or tri-substituted independently by halo, (C₂-C₆)alkenyl, (C₁-C₆)alkyl, hydroxy, (C₁-C₆)alkoxy, (C₁-C₄)alklylthio, amino, nitro, cyano, oxo, carboy, (C₁-C₆)alkyloxycarbonyl, mono-N- or di-N,N-(C₁-C₆)alkylamino wherein (C₁-C₆)alkyl substituent is optionally mono-, di- or tri-substituted independently by halo, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, the (C₁-C₆)alkyl is optionally substituted by 1 to 9 fluorines;
R³ is hydrogen or Q;
wherein Q is a fully saturated, partially unsaturated or fully unsaturated 1- to 6-membered straight chain or branched carbon chain wherein the carbons other than the connecting carbon, may be replaced with one heteroatom selected from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is optionally mono- or di-substituted by oxo, the nitrogen is optionally mono- or di-substituted by oxo, the carbon chain is optionally mono-substituted by V; wherein V is a partially saturated, fully saturated or fully unsaturated 3- to 12-membered ring optionally having 1 to 4 heteroatoms selected independently from oxygen, sulfur and nitrogen, or a bicyclic ring consisting of two fused partially saturated, fully saturated or fully unsaturated 3- to 6-membered rings, taken independently, optionally having 1 to 4 heteroatoms selected independently from nitrogen, sulfur and oxygen;
wherein the V substituent is optionally mono-, di-, tri- or tetra-substituted independently by halo, (C₁-C₆)alkyl, (C₂-C₆)alkenyl, hydroxy, (C₁-C₆)alkoxy, (C₁-C₄)alkylthio, amino, nitro, cyano, oxo, carboxamoyl, mono-N- or di-N,N-(C₁-C₆)alkylcarboxamoyl, carboxy, (C₁-C₆)alkyloxycarbonyl , mono-N- or di-N,N-(C₁-C₆)alkylamino wherein the (C₁-C₆)alkyl or (C₂-C₆)alkenyl substituent is optionally mono-, di- or tri-substituted independently by hydroxy, (C₁-C₆)alkoxy, (C₁-C₄)alkylthio, amino, nitro, cyano, oxo, carboxy, (C¹-C₆)alkyloxycarbonyl, mono-N- or di-N,N-(C₁-C₆)alkylamino, the (C₁-C₆)alkyl or (C₂-C₆)alkenyl is optionally substituted by 1 to 9 fluorines;
R⁴ is Q¹ or V¹; wherein Q¹ is a fully saturated, partially unsaturated or fully unsaturated 1- to 6-membered straight chain or branched carbon chain wherein the carbons other than the connecting carbon, may be replaced with one heteroatom selected independently from oxygen, sulfur and nitrogen and the carbon is optionally mono-, di- or tri-substituted independently by halo, the carbon is optionally mono-substituted by hydroxy, the carbon is optionally mono-substituted by oxo, the sulfur is mono- or di-substituted by oxo, the nitrogen is optionally mono- or di-substituted by oxo, the carbon chain is optionally mono-substituted by V¹; wherein V¹ is a partially saturated, fully saturated or fully unsaturated 3- to 6-membered ring optionally having 1 or 2 heteroatoms selected independently from oxygen, sulfur and nitrogen; the V¹ substituent is optionally mono-, di-, tri- or tetra-substituted independently by halo, (C₁-C₆)alkyl, (C₁-C₆)alkoxy, amino, nitro, cyano, (Ci-C₆)alkyloxycarbonyl, mono-N- or di-N,N-(C₁-C₆)alkylamino wherein the (C₁-C₆)alkyl substituent is optionally mono-substituted by oxo, the (C₁-C₆)alkyl substituent optionally has 1 to 9 fluorines; either R³ should contain V or R⁴ should contain V¹; and
R⁵ and R⁶, or R⁶ and R⁷, and/or R⁷ and R⁸ are linked to form at least one partially saturated or fully unsaturated 4- to 8-membered ring optionally having 1 to 3 heteroatoms independently selected from nitrogen, sulfur and oxygen; the rings (one or more) formed by R⁵ and R⁶, or R⁶ and R⁷, and/or R⁷ and R⁸ are optionally mono-, di- or tri-substituted independently by halo, (C₁-C₆)alkyl, (C₁-C₆)alkylsulfonyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino wherein the (C₁-C₆) alkyl substituent is optionally mono-, di- or tri-substituted independently by hydroxy, (C₁-C₆)alkoxy, (C₁-C₄)alkylthio, amino, nitro, cyano, oxo, carboxy, (C₁-C₆)alkyloxycarbonyl, mono-N- or di-N,N-(C₁-C₆)alkylamino, and the (C₁-C₆)alkyl substituent optionally has 1, to 9 fluorines; provided that when the R⁵, R⁶, R⁷ and/or R⁸ do not form at least one ring, they are each independently hydrogen, halo, (C₁-C₆)alkoxy or (C₁-C₆)alkyl, wherein the (C₁-C₆)alkyl optionally has 1 to 9 fluorines, a prodrug thereof or a pharmaceutically acceptable salt of the compound or the prodrug.

The expression of "prodrug" of the formula (IV) refers to a compound of a drug precursor that releases a drug in vivo according to some specific chemical or physiological process after administration (e.g., prodrug becomes an object drug form by achieving a physiological pH or by an enzyme action). Examples of the prodrug that releases a corresponding free acid upon cleavage and the residue forming hydrolytic ester of the compound of the formula (IV) include, but not limited to, those having a carboxy moiety, wherein its free hydrogen has been substituted by the following groups: (C₁-C₄)alkyl, (C₂-C₇)alkanoyloxymethyl, 1-(alkanoyloxy)ethyl having 4 to 9 carbon atoms, 1-methyl-1-(alkanoyloxy)ethyl having 5 to 10 carbon atoms, alkoxycarbonyloxymethyl having 3 to 6 carbon atoms, 1-(alkoxycarbonyloxy)ethyl having 4 to 7 carbon atoms, 1-methyl-1-(alkoxycarbonyloxy)ethyl having 5 to 8 carbon atoms, N-(alkoxycarbonyl)aminomethyl having 3 to 9 carbon atoms, 1-(N-(alkoxycarbonyl)amino)ethyl having 4 to 10 carbon atoms, 3-phthalidyl, 4-crotonolactonyl, γ-butyrolacton-4-yl, di-N,N-(C₁-C₂)alkylamino(C₂-C₃)alkyl (e.g., b-dimethylaminoethyl), carbamoyl-(C₁-C₂)alkyl, N,N-di(C₁-C₂)alkylcarbamoyl-(C₁-C₂)alkyl, piperidino-, pyrrolidino- or morpholino(C₂-C₃)alkyl.

Examples of the 5- or 6-membered aromatic ring in the formula (IV), which may have 1 or 2 heteroatoms independently selected from oxygen, nitrogen and sulfur include phenyl, furyl, thienyl, pyrrolyl, oxazolyl, thiazolyl, imidazolyl, pyrazolyl, isoxazolyl, isothiazolyl, pyridinyl, pyridiazinyl, pyrimidinyl and pyrazinyl.

Examples of the partially saturated, fully saturated or fully unsaturated 5- to 8-membered ring in the formula (IV), which may have 1 to 4 heteroatoms independently selected from oxygen, sulfur and nitrogen, include cyclohexyl, cycloheptyl, cyclooctyl and phenyl. Examples of other 5-membered ring include the following: 2H-pyrrolyl, 3H-pyrrolyl, 2-pyrrolinyl, 3-pyrrolinyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathiolyl, 1,2,3-oxadiazolyl, 1,2,4-oxadiazolyl, 1,2,5-oxadiazolyl, 1,3,4-oxadiazolyl, 1,2,3-thiazolyl, 1,2,4-thiazolyl, 1,3,4-thiazolyl, 1,2,3,4-oxatriazolyl, 1,2,3,5-oxatriazolyl, 3H-1,2,3-dioxazolyl, 1,2,4-dioxazolyl, 1,3,2-dioxazolyl, 1,3,4-dioxazolyl, 5H-1,2,5-oxathiazolyl and 1,3-oxathiolyl.

Examples of other 6-membered ring in the formula (IV) include the following: 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxadinyl, p-isoxadinyl, 1,2,5-oxathiazinyl, 1,2,6-oxathiazinyl, 1,4,2-oxadiazinyl and 1,3,5,2-oxadiazinyl.

Further examples of the 7-membered ring include azetidinyl, oxepinyl and thiepinyl. Further examples of the 8-membered ring include cyclooctyl, cyclooctenyl and cyclooctadienyl.

Examples of the two fused partially saturated, fully saturated or fully unsaturated 5- or 6-membered rings, which independently optionally have 1 to 4 heteroatoms independently selected from nitrogen, sulfur and oxygen, include the following: indolizinyl, indolyl, isoindolyl, 3H-indolyl, 1H-isoindolyl, indolinyl, cyclopenta(b)pyridinyl, pyrano(3,4-b)pyrrolyl, benzofuryl, isobenzofuryl, benzo(b)thienyl, benzo(c)thienyl, 1H-indazolyl, indoxazinyl, benzoxazolyl, benzimidazolyl, benzothiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, indenyl, isoindenyl, naphthyl, tetralinyl, decalinyl, 2H-1-benzopyranyl, pyrido(3,4-b)-pyridinyl, pyrido(3,2-b)-pyridinyl, pyrido(4,3-b)-pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl and 4H-1,4-benzoxazinyl.

The alkylene in the formula (IV) means a saturated hydrocarbon (straight chain or branched chain) wherein a hydrogen atom has been removed from each terminal carbon. Examples of such group include (assuming that the designated length encompasses specific examples) methylene, ethylene, propylene, butylene, pentylene, hexylene and heptylene.

The halo in the formula (IV) means chloro, bromo, iodo or fluoro. The alkyl in the formula (IV) means a saturated straight chain hydrocarbon or a saturated branched chain hydrocarbon. Examples of such alkyl group (assuming that the designated length encompasses specific examples) include methyl, ethyl, propyl, isopropyl, butyl, sec-butyl, t-butyl, pentyl, isopentyl, neopentyl, t-pentyl, 1-methylbutyl, 2-methylbutyl, 3-methylbutyl, hexyl, isohexyl, heptyl and octyl.

The alkoxy in the formula (IV) means a saturated straight chain alkyl or a saturated branched chain alkyl bonded via oxy. Examples of such alkoxy group (assuming that the designated length encompasses specific examples) include methoxy, ethoxy, propoxy, isopropoxy, butoxy, isobutoxy, t-butoxy, pentoxy, isopentoxy, neopentoxy, t-pentoxy, hexoxy, isohexoxy, heptoxy and octoxy.

The terms mono-N- and di-N,N-(C₁-Cₓ)alkyl ... used for the formula (IV) refer to (C₁-Cₓ) alkyl moiety taken independently, when it is di-N,N-(C₁-Cₓ)alkyl ... (x means an integer).

It should be appreciated that, in the formula (IV), when a carbon ring or heterocyclic moiety is bonded to a designated substrate via various ring atoms, or attached thereto in a different form, without indicating a specific binding site, any point with such possibility is intended, whether via a carbon, or via, for example, a trivalent nitrogen atom. For example, the term "pyridyl" means 2-, 3- or 4-pyridyl, "thienyl" means 2- or 3-thienyl, and hereinafter the same.

The "aforementioned carbon" in the "aforementioned carbon may be independently mono-, di- or tri-substituted by halo, the aforementioned carbon may be mono-substituted by hydroxy, the aforementioned carbon may be mono-substituted by oxo" in the formula (IV) refers to each carbon containing a connecting carbon in the carbon chain.

The "nitrogen may be ... di-substituted by oxo" in the formula (IV) refers to the terminal nitrogen constituting the nitro functional group. The expression "a pharmaceutically acceptable salt" in the formula (IV) means, but not limited to, non-toxic anion salts including anion such as chloride, bromide, iodide, sulfate, hydrogen sulfate, phosphate, acetate, maleate, fumarate, oxalate, lactate, tartrate, citrate, gluconate, methanesulfonate and 4-toluene-sulfonate. This expression also means, but not limited to, non-toxic cation salts such as sodium, potassium, calcium, magnesium, ammonia or protonated benzathine (N,N'-dibenzylethylenediamine), choline, ethanolamine, diethanolamine, ethylenediamine, meglamine (N-methyl-glucamine), benethamine (N-benzylphenethylamine), piperazine and tromethamine (2-amino-2-hydroxymethyl-1,3-propanediol .

Specifically, the compound disclosed in JP-A-2000-191645 can be mentioned:
a compound of the formula (V) 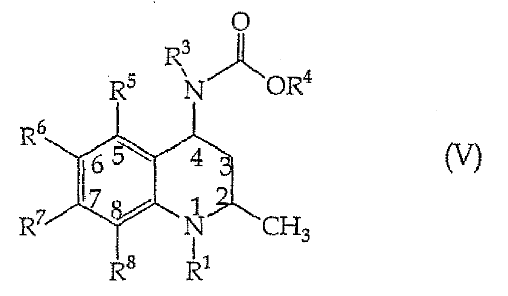 wherein
   - R¹: is a hydrogen, Y, W-X or W-Y;
   wherein W is carbonyl, thiocarbonyl, sulfinyl or sulfonyl;
   X is -O-Y, -S-Y, -N (H) -Y or -N-(Y)₂;
   Y for each bond is independently Z or a fully saturated, partially unsaturated or fully unsaturated 1- to 10-membered straight chain or branched carbon chain, wherein the carbons other than a connecting carbons may be replaced with 1 or 2 heteroatoms selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted by Z as desired; wherein Z is a partially saturated, fully saturated or fully unsaturated 3- to 8-membered ring having 1 to 4 heteroatoms independently selected from oxygen, sulfur and nitrogen as desired, or a bicyclic ring consisting of two fused partially saturated, fully saturated or fully unsaturated 3-to 6-membered rings, taken independently, to have 1 to 4 heteroatoms independently selected from nitrogen, sulfur and oxygen as desired; the aforementioned Z substituent is independently mono-substituted, di-substituted or tri-substituted as desired by halogen, (C₂-C₆) alkenyl, (C₁-C₆) alkyl, hydroxy, (C₁-C₆) alkoxy, (C₁-C₄)alkylthio, amino, nitro, cyano, oxo, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent is independently mono-substituted, di-substituted or tri-substituted as desired by halogen, hydroxy, (C₁-C₆)alkoxy, (C₁-C₄) alkylthio, amino, nitro, cyano, oxo, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent is further substituted by 1 to 9 fluorines as desired;
   - R³: is a hydrogen or Q; wherein Q is a fully saturated, partially unsaturated or fully unsaturated 1- to 6-membered straight chain or branched carbon chain wherein the carbons other than a connecting carbons may be replaced with one heteroatom selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted by V as desired; wherein V is a partially saturated, fully saturated or fully unsaturated 3- to 8-membered ring having 1 to 4 heteroatoms independently selected from oxygen, sulfur and nitrogen as desired, or a bicyclic ring consisting of two fused partially saturated, fully saturated or fully unsaturated 3- to 6-membered rings, taken independently, to have 1 to 4 heteroatoms independently selected from nitrogen, sulfur and oxygen as desired; the aforementioned V substituent is independently mono-substituted, di- substituted, tri-substituted or tetra-substituted as desired by halogen, (C₁-C₆)alkyl, (C₂-C₆)alkenyl, hydroxy, (C₁-C₆)alkoxy, (C₁-C₄)alkylthio, amino, nitro, cyano, oxo, carbamoyl, mono-N-(C₁-C₆)alkylcarbamoyl, di-N,N-(C₁-C₆)alkylcarbamoyl, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent or (C₂-C₆)alkenyl substituent is independently mono-substituted, di-substituted or tri-substituted as desired by hydroxy, (C₁-C₆)alkoxy, (C₁-C₄)alkylthio, amino, nitro, cyano, oxo, carboxyl, (C₁-C₆)alkyloxycarbonyl, mono-N-(C₁-C₆)alkylamino or di-N,N-(C₁-C₆)alkylamino, the aforementioned (C₁-C₆)alkyl substituent or (C₂-C₆)alkenyl substituent is further substituted by 1 to 9 fluorines as desired;
   - R⁴: is Q¹ or V¹; wherein Q¹ is a fully saturated, partially unsaturated or fully unsaturated 1- to 6-membered straight chain or branched carbon chain, wherein the carbons other than a connecting carbon may be replaced with one heteroatom selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted by V¹ as desired; wherein V¹ is a partially saturated, fully saturated or fully unsaturated 3- to 6-membered ring having, as desired, 1 or 2 heteroatoms independently selected from oxygen, sulfur and nitrogen; the aforementioned V¹ substituent is independently mono-substituted, di-substituted, tri-substituted or tetra-substituted as desired by halogen, (C₁-C₆)alkyl, (C₁-C₆)alkoxy, amino, nitro, cyano, (C₁-C₆)alkyloxycarbonyl, mono-N-(C₁-C₆) alkylamino or di-N,N-(C₁-C₆)alkylamino, the aforementioned (C₁-C₆)alkyl substituent is mono-substituted as desired by oxo, and the aforementioned (C₁-C₆)alkyl substituent is further substituted by 1 to 9 fluorines as desired; R³ should contain V or R⁴ should contain V¹; and
   - R⁵, R⁶, R⁷, and R⁸: are each independently hydrogen, hydroxy or oxy, wherein the aforementioned oxy is substituted by T or a partially saturated, fully saturated or fully unsaturated 1- to 12-membered straight chain or branched carbon chain, the carbons other than a connecting carbons may be replaced with 1 or 2 heteroatoms independently selected from oxygen, sulfur and nitrogen as desired, the aforementioned carbon is independently mono-substituted, di-substituted or tri-substituted by halogen as desired, the aforementioned carbon is mono-substituted by hydroxy as desired, the aforementioned carbon is mono-substituted by oxo as desired, the aforementioned sulfur is mono-substituted or di-substituted by oxo as desired, the aforementioned nitrogen is mono-substituted or di-substituted by oxo as desired, and the aforementioned carbon chain is mono-substituted as desired by T; wherein T is a partially saturated, fully saturated or fully unsaturated 3- to 8-membered ring having 1 to 4 heteroatoms independently selected from oxygen, sulfur and nitrogen as desired, or a bicyclic ring consisting of two fused partially saturated, fully saturated or fully unsaturated 3- to 6-membered rings, taken independently, to have 1 to 4 heteroatoms independently selected from nitrogen, sulfur and oxygen as desired; the aforementioned T substituent is independently mono-substituted, di-substituted or tri-substituted as desired by halogen, (C₁-C₆)alkyl, (C₂-C₆)alkenyl, hydroxy, (C₁-C₆)alkoxy, (C₁-C₄)alkylthio, amino, nitro, cyano, oxo, carboxy, (C₁-C₆)alkyloxycarbonyl, mono-N-(C₁-C₆)alkylamino or di-N,N-(C₁-C₆)alkylamino, the aforementioned (C₁-C₆)alkyl substituent is independently mono-substituted, di-substituted or tri-substituted as desired by hydroxy, (C₁-C₆)alkoxy, (C₁-C₄)alkylthio, amino, nitro, cyano, oxo, carboxy, (C₁-C₆)alkyloxycarbonyl, mono-N-(C₁-C₆)alkylamino or di-N,N-(C₁-C₆)alkylamino, and the aforementioned (C₁-C₆)alkyl substituent is further substituted as desired by 1 to 9 fluorines,
a prodrug thereof, a pharmaceutically acceptable salt of the compound or a pharmaceutically acceptable salt of the prodrug.

The "prodrug" of the formula (V) refers to a compound which is a drug precursor that releases a drug in vivo via some specific chemical or physiological process after administration (e.g., prodrug is converted to a desired drug form by achieving a physiological pH or by an enzyme action). A typical prodrug produces a corresponding free acid upon cleavage and the residue forming such hydrolytic ester of the compound of the formula (V) includes, but not limited to, a residue having a carboxyl moiety, in which a free hydrogen is substituted by the following groups: (C₁-C₄) alkyl, (C₂-C₇) alkanoyloxymethyl, 1-(alkanoyloxy)ethyl having 4 to 9 carbon atoms, 1-methyl-1-(alkanoyloxy)ethyl having 5 to 10 carbon atoms, alkoxycarbonyloxymethyl having 3 to 6 carbon atoms, 1-(alkoxycarbonyloxy)ethyl having 4 to 7 carbon atoms, 1-methyl-1-(alkoxycarbonyloxy)ethyl having 5 to 8 carbon atoms, N-(alkoxycarbonyl)aminomethyl having 3 to 9 carbon atoms, 1-(N-(alkoxycarbonyl)amino)ethyl having 4 to 10 carbon atoms, 3-phthalidyl, 4-crotonolactonyl, γ-butyrolacton-4-yl, di-N,N-(C₁-C₂)alkylamino(C₂-C₃)alkyl (e.g., B-dimethylaminoethyl), carbamoyl-(C₁-C₂) alkyl, N,N-di(C₁-C₂)alkylcarbamoyl-(C₁-C₂)alkyl, piperidino (C₂-C₃ alkyl), pyrrolidino(C₂-C₃)alkyl or morpholino(C₂-C₃)alkyl.

The following paragraphs describe representative rings relative to the general ring names contained in the formula (V).

Examples of the representative 5- or 6-membered aromatic ring in the formula (V), which has 1 or 2 heteroatoms as desired, which are independently selected from oxygen, nitrogen and sulfur, include phenyl, furyl, thienyl, pyrrolyl, oxazolyl, thiazolyl, imidazolyl, pyrazolyl, isoxazolyl, isothiazolyl, pyridinyl, pyridazinyl, pyrimidinyl, pyrazinyl and the like.

Representative examples of the partially saturated, fully saturated or fully unsaturated 5- to 8-membered ring in the formula (V), which has 1 to 4 heteroatoms, which are independently selected from oxygen, sulfur and nitrogen, include cyclopentyl, cyclohexyl, cycloheptyl, cyclooctyl, phenyl and the like. Further examples of the representative 5-membered ring in the formula (V) include the following: 2H-pyrrolyl, 3H-pyrrolyl, 2-pyrrolinyl, 3-pyrrolinyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathiolyl, 1,2,3-oxadiazolyl, 1,2,4-oxadiazolyl, 1,2,5-oxadiazolyl, 1,3,4-oxadiazolyl, 1,2,3-triazolyl, 1,2,4-triazolyl, 1,3,4-thiadiazolyl, 1,2,3,4-oxatriazolyl, 1,2,3,5-oxatriazolyl, 3H-1,2,3-dioxazalyl, 1,2,4-dioxazolyl, 1,3,2-dioxazolyl, 1,3,4-dioxazolyl, 5H-1,2,5-oxathiazolyl, 1,3-oxathiolyl and the like.

Further examples of representative 6-membered ring in the formula (V) include the following: 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxadinyl, p-isoxadinyl, 1,2,5-oxathiazinyl, 1,2,6-oxathiazinyl, 1,4,2-oxadiazinyl, 1,3,5,2-oxadiazinyl and the likes.

Further examples of the representative 7-membered ring in the formula (V) include azepinyl, oxepinyl, thiepinyl and the like. Further examples of the representative 8-membered ring in the formula (V) include cyclooctyl, cyclooctenyl, cyclooctadienyl and the like.

Examples of the representative bicyclic ring in the formula (V), consisting of two fused partially saturated, fully saturated or fully unsaturated 5- or 6-membered rings, taken independently, optionally having 1 to 4 heteroatoms as desired, which are independently selected from nitrogen, sulfur and oxygen, include the following: indolizinyl, indolyl, isoindolyl, 3H-indolyl, 1H-isoindolyl, indolinyl, cyclopenta(b)pyridinyl, pyrano(3,4-b)pyrrolyl, benzofuryl, isobenzofuryl, benzo (b) thienyl, benzo(c)thienyl, 1H-indazolyl, indoxazinyl, benzoxazolyl, benzimidazolyl, benzothiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, indenyl, isoindenyl, naphthyl, tetralinyl, decalinyl, 2H-1-benzopyranyl, pyrido(3,4-b)-pyridinyl, pyrido(3,2-b)-pyridinyl, pyrido(4,3-b)-pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl, 4H-1,4-benzoxazinyl and the like.

The alkylene in the formula (V) means a saturated hydrocarbon (straight chain or branched chain) wherein hydrogen atom has been removed from each terminal carbon. Examples of such group include (assuming that the designated length encompasses specific examples) methylene, ethylene, propylene, butylene, pentylene, hexylene and heptylene.

The halo in the formula (V) means chloro, bromo, iodo or fluoro. The alkyl in the formula (V) means a saturated straight chain hydrocarbon or a saturated branched chain hydrocarbon. Examples of such alkyl group (assuming that the designated length encompasses specific examples) include methyl, ethyl, propyl, isopropyl, butyl, sec-butyl, tert-butyl, pentyl, isopentyl, neopentyl, tert-pentyl, 1-methylbutyl, 2-methylbutyl, 3-methylbutyl, hexyl, isohexyl, heptyl and octyl.

The alkoxy in the formula (V) means a saturated straight chain alkyl or a saturated branched chain alkyl bonded via oxy. Examples of such alkoxy group (assuming that the designated length encompasses specific examples) include methoxy, ethoxy, propoxy, isopropoxy, butoxy, isobutoxy, tert-butoxy, pentoxy, isopentoxy, neopentoxy, tert-pentoxy, hexoxy, isohexoxy, heptoxy and octoxy.

The terms mono-N- and di-N,N-(C₁-Cₓ)alkyl ... used for the formula (V) refer to (C₁-Cₓ)alkyl moiety taken independently, when it is di-N,N-(C₁-Cₓ)alkyl ... (x means an integer).

It should be appreciated that, in the formula (V), when a carbon ring moiety or heterocyclic moiety is bonded or attached to a designated substrate via a different ring atom, without indicating a specific binding site, any point with such possibility is intended, whether via a carbon atom, or via, for example, a trivalent nitrogen atom. For example, the term "pyridyl" means 2-, 3- or 4-pyridyl, "thienyl" means 2-, or 3-thienyl, and the like.

The "aforementioned carbon" in the "aforementioned carbon is independently mono-substituted, di-substituted or tri-substituted as desired by halogen, the aforementioned carbon is mono-substituted as desired by hydroxy, the aforementioned carbon is mono-substituted as desired by oxo" in the formula (V) refers to each carbon in the carbon chain containing binding carbons.

The "nitrogen di-substituted by oxo" in the formula (V) refers to the terminal nitrogen constituting the nitro functionality. The expression, "a pharmaceutically acceptable salt" in the formula (V) means, but not limited to, non-toxic anion salts including anion, such as chloride ion, bromide ion, iodide ion, sulfate ion, hydrogen sulfate ion, phosphate ion, acetate ion, maleate ion, fumarate ion, oxalate ion, lactate ion, tartrate ion, citrate ion, gluconate ion, methanesulfonate ion and 4-toluene-sulfonate ion. This expression also means, but not limited to, non-toxic cation salts including cation, such as sodium ion, potassium ion, calcium ion, magnesium ion, ammonium or protonated benzathine. (N,N'-dibenzylethylenediamine), choline, ethanolamine, diethanolamine, ethylenediamine, meglamine (N-methyl-glucamine), benethamine (N-benzylphenethylamine), piperazine and tromethamine (2-amino-2-hydroxymethyl-1,3-propanediol) .

Specifically, the compound disclosed in WO98/35937 and JP-A-H11-49743 can be mentioned:
a compound represented by the formula (VII) 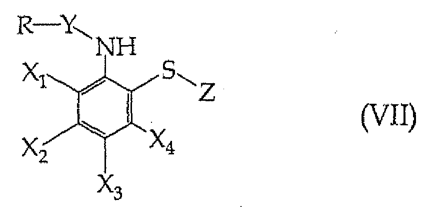 wherein
   - R: is a straight chain or branched C₁₋₁₀ alkyl group, a straight chain or branched C₂₋₁₀ alkenyl group, a halogenated C₁₋₄ lower alkyl group, an optionally substituted C₃₋₁₀ cycloalkyl group, an optionally substituted C₅₋₈ cycloalkenyl group, an optionally substituted C₃₋₁₀ cycloalkyl C₁₋₁₀ alkyl group, an optionally substituted aryl group, an optionally substituted aralkyl group or an optionally substituted 5- or 6-membered heterocyclic group having 1 to 3 from nitrogen atom, oxygen atom and sulfur atom;
   - X₁, X₂, X₃ and X₄: are the same or different and each is a hydrogen atom, a halogen atom, a C₁₋₄ lower alkyl group, a halogenated C₁₋₄ lower alkyl group, a C₁₋₄ lower alkoxy group, a cyano group, a nitro group, an acyl group or an aryl group;
   - Y: is -CO- or -SO₂-; and
   - Z: is a hydrogen atom or a mercapto protecting group,
a prodrug compound thereof, a pharmaceutically acceptable salt thereof, or a hydrate thereof or a solvate thereof.

The "straight chain or branched C₁₋₁₀ alkyl group" in the formula (VII) means a straight chain or branched alkyl group having 1 to 10 carbon atoms, which is specifically methyl group, ethyl group, propyl group, isopropyl group, butyl group, isobutyl group, sec-butyl group, tert-butyl group, pentyl group, isopentyl group, neopentyl group, tert-pentyl group, 1-ethylbutyl group, 2-ethylbutyl group, 1-propylbutyl group, 1,1-dimethylbutyl group, 1-isobutyl-3-methylbutyl group, 1-ethylpentyl group, 1-propylpentyl group, 1-isobutylpentyl group, 2-ethylpentyl group, 2-isopropylpentyl group, 2-tert-butylpentyl group, 3-ethylpentyl group, 3-isopropylpentyl group, 4-methylpentyl group, 1,4-dimethylpentyl group, 2,4-dimethylpentyl group, 1-ethyl-4-methylpentyl group, hexyl group, 1-ethylhexyl group, 1-propylhexyl group, 2-ethylhexyl group, 2-isopropylhexyl group, 2-tert-butylhexyl group, 3-ethylhexyl group, 3-isopropylhexyl group, 3-tert-butylhexyl group, 4-ethylhexyl group, 5-methylhexyl group, heptyl group, 1-ethylheptyl group, 1-isopropylheptyl group, 2-ethylheptyl group, 2-isopropylheptyl group, 3-propylheptyl group, 4-propylheptyl group, 5-ethylheptyl group, 6-methylheptyl group, octyl group, 1-ethyloctyl group, 2-ethyloctyl group, nonyl group, 1-methylnonyl group, 2-methylnonyl group, decyl group and the like, with preference given to a straight chain or branched alkyl group having 1 to 8 carbon atoms.

The "C₁₋₄ lower alkyl group" in the formula (VII) means a straight chain or branched alkyl group having 1 to 4 carbon atoms, which is specifically methyl group, ethyl group, propyl group, isopropyl group, butyl group, isobutyl group, sec-butyl group, tert-butyl group and the like.

The "straight chain or branched C₂₋₁₀ alkenyl group" in the formula (VII) means a straight chain or branched alkenyl group having 2 to 10 carbon atoms and at least one double bond, which is specifically allyl group, vinyl group, isopropenyl group, 1-propenyl group, 1-methyl-2-propenyl group, 2-methyl-2-propenyl group, 1-methyl-1-butenyl group, crotyl group, 1-methyl-3-butenyl group, 3-methyl-2-butenyl group, 1,3-dimethyl-2-butenyl group, 1-pentenyl group, 1-methyl-2-pentenyl group, 1-ethyl-3-pentenyl group, 4-pentenyl group, 1,3-pentadienyl group, 2,4-pentadienyl group, 1-hexenyl group, 1-methyl-2-hexenyl group, 3-hexenyl group, 4-hexenyl group, 1-butyl-5-hexenyl group, 1,3-hexadienyl group, 2,4-hexadienyl group, 1-heptenyl group, 2-heptenyl group, 3-heptenyl group, 4-heptenyl group, 5-heptenyl group, 6-heptenyl group, 1,3-heptadienyl group, 2,4-heptadienyl group, 1-octenyl group, 2-octenyl group, 3-octenyl group, 4-octenyl group, 5-octenyl group, 6-octenyl group, 7-octenyl group, 1-nonenyl group, 2-nonenyl group, 3-nonenyl group, 4-nonenyl group, 5-nonenyl group, 6-nonenyl group, 7-nonenyl group, 8-nonenyl group, 9-decenyl group and the like, with preference given to a straight chain or branched alkenyl group having 2 to 8 carbon atoms.

The "halogen atom" in the formula (VII) means fluorine atom, chlorine atom, bromine atom and the like.

The "halogenated C₁₋₄ alkyl group" in the formula (VII) means the aforementioned C₁₋₄ lower alkyl group substituted by 1 to 3 the same or different halogen atoms, which is specifically fluoromethyl group, chloromethyl group, bromomethyl group, difluoromethyl group, dichloromethyl group, trifluoromethyl group, trichloromethyl group, chloroethyl group, difluoroethyl group, trifluoroethyl group, pentachloroethyl group, bromopropyl group, dichloropropyl group, trifluorobutyl group and the like, with preference given to trifluoromethyl group and chloroethyl group.

The "C₁₋₄ lower alkoxy group" in the formula (VII) means an alkoxy group having the aforementioned C₁₋₄ lower alkyl group, which is specifically methoxy group, ethoxy group, propoxy group, isopropoxy group, butoxy group, isobutoxy group, sec-butoxy group, tert-butoxy group and the like.

The "C₁₋₄ lower alkylthio group" in the formula (VII) means an alkylthio group having the aforementioned C₁₋₄ lower alkyl group, which is specifically methylthio group, ethylthio group, propylthio group, isopropylthio group, butylthio group, isobutylthio group, sec-butylthio group, tert-butylthio group and the like.

The "C₃₋₁₀ cycloalkyl group" in the formula (VII) means a monocyclic or polycyclic cycloalkyl group having 3 to 10 carbon atoms, which is specifically cyclopropyl group, cyclobutyl group, cyclopentyl group, cyclohexyl group, cycloheptyl group, cyclooctyl group, octahydroindenyl group, decahydronaphthyl group, bicyclo[2.2.1]heptyl group, adamantyl group and the like, with preference given to cyclopentyl group, cyclohexyl group and cycloheptyl group, each of which having 5 to 7 carbon atoms.

The "C₅₋₈ cycloalkenyl group" in the formula (VII) means a cycloalkenyl group having 5 to 8 carbon atoms and one or more double bonds on the ring, which is specifically cyclopentenyl group, cyclohexenyl group, cycloheptenyl group, cyclooctenyl group, cyclopentadienyl group, cyclohexadienyl group, cycloheptadienyl group, cyclooctadienyl group and the like, with preference given to cyclopentenyl group, cyclohexenyl group and cycloheptenyl group, each of which having 5 to 7 carbon atoms.

The "C₃₋₁₀ cycloalkyl C₁₋₁₀ alkyl group" in the formula (VII) means the aforementioned straight chain or branched C₁₋₁₀ alkyl group substituted by the aforementioned C₃₋₁₀ cycloalkyl group, which is specifically cyclopropylmethyl group, cyclopentylmethyl group, cyclohexylmethyl group, cyclohexylcyclopentylmethyl group, dicyclohexylmethyl group, 1-cyclopentylethyl group, 1-cyclohexylethyl group, 2-cyclopropylethyl group, 2-cyclopentylethyl group, 2-cyclohexylethyl group, 2-cycloheptylethyl group, 1-cyclohexyl-1-methylethyl group, 1-cyclohexylpropyl group, 2-cyclopentylpropyl group, 3-cyclobutylpropyl group, 3-cyclopentylpropyl group, 3-cyclohexylpropyl group, 3-cycloheptylpropyl group, 1-cyclopropyl-1-methylpropyl group, 1-cyclohexyl-2-methylpropyl group, 1-cyclopentylbutyl group, 1-cyclohexylbutyl group, 3-cyclohexylbutyl group, 4-cyclopropylbutyl group, 4-cyclobutylbutyl group, 4-cyclopentylbutyl group, 1-cyclohexyl-1-methylbutyl group, 1-cyclopentyl-2-ethylbutyl group, 1-cyclohexyl-3-methylbutyl group, 1-cyclopentylpentyl group, 1-cyclohexylpentyl group, 1-cyclohexylmethylpentyl group, 2-cyclohexylpentyl group, 2-cyclohexylmethylpentyl group, 3-cyclopentylpentyl group, 1-cyclohexyl-4-methylpentyl group, 5-cyclopentylpentyl group, 1-cyclopentylhexyl group, 1-cyclohexylhexyl group, 1-cyclopentylmethylhexyl group, 2-cyclopentylhexyl group, 2-cyclopropylethylhexyl group, 3-cyclopentylhexyl group, 1-cyclohexylheptyl group, 1-cyclopentyl-1-methylheptyl group, 1-cyclohexyl-1,6-dimethylheptyl group, 1-cycloheptyloctyl group, 2-cyclopentyloctyl group, 3-cyclohexyloctyl group, 2-cyclopentylmethyloctyl group, 1-cyclopentylnonyl group, 1-cyclohexylnonyl group, 3-cyclopropylnonyl group, 1-cyclopentyldecyl group, 1-cyclohexylundecyl group, 1-cyclopentyltridecyl group, 2-cyclohexyltridecyl group and the like.

The "aryl group" in the formula (VII) means phenyl group, naphthyl group, anthryl group, phenanthryl group, biphenyl group and the like, with preference given to phenyl group, naphthyl group and biphenyl group.

The "aralkyl group" in the formula (VII) means the aforementioned C₁₋₄ lower alkyl group substituted by one or more the aforementioned aryl groups, which is specifically benzyl group, benzhydryl group, trityl group, phenethyl group, 3-phenylpropyl group, 2-phenylpropyl group, 4-phenylbutyl group, naphthylmethyl group, 2-naphthylethyl group, 4-biphenylmethyl group, 3-(4-biphenyl)propyl group and the like.

The "arylalkenyl group" in the formula (VII) means alkenyl group having 2 to 4 carbon atoms substituted by the aforementioned aryl group, which is specifically 2-phenylvinyl group, 3-phenyl-2-propenyl group, 3-phenyl-2-methyl-2-propenyl group, 4-phenyl-3-butenyl group, 2-(1-naphthyl)vinyl group, 2-(2-naphthyl)vinyl group, 2-(4-biphenyl)vinyl group and the like.

The "arylthio group" in the formula (VII) means arylthio group having the aforementioned aryl group, which is specifically phenylthio group, naphthylthio group and the like.

The "heterocyclic group" in the formula (VII) means a 5- or 6-membered aromatic or nonaromatic heterocyclic group having one or more, specifically 1 to 4, preferably 1 to 3, heteroatoms selected from nitrogen atom, oxygen atom and sulfur atom, which is specifically aromatic heterocyclic groups such as thiatriazolyl group, tetrazolyl group, dithiazolyl group, oxadiazolyl group, thiadiazolyl group, triazolyl group, oxazolyl group, pyrazolyl group, pyrrolyl group, furyl group, thienyl group, tetrazinyl group, triazinyl group, pyrazinyl group, pyridazinyl group, pyrimidinyl group, pyridyl group and the like and nonaromatic heterocyclic groups such as dioxolanyl group, pyrrolidinyl group, tetrahydrofuryl group, tetrahydrothienyl group, dithiadiazinyl group, thiadiazinyl group, morpholino group, morpholinyl group, oxazinyl group, thiazinyl group, piperazinyl group, piperidyl group, piperidino group, pyranyl group, thiopyranyl group and the like. Preferred are an aromatic heterocyclic (heteroaryl, group such as furyl group, thienyl group, pyrrolyl group, pyridyl group and the like and a nonaromatic heterocyclic group having at least one nitrogen atom, such as pyrrolidinyl group, tetrahydrofuryl group, piperazinyl group, piperidyl group, piperidino group and the like.

The "heteroarylalkyl group" in the formula (VII) means the aforementioned C₁₋₄ lower alkyl substituted by the above-mentioned 5- or 6-membered aromatic heterocyclic (heteroaryl) group, which is specifically 2-thienylmethyl group, 2-furylmethyl group, 2-pyridylmethyl group, 3-pyridylmethyl group, 2-thienyl-2-ethyl group, 3-furyl-1-ethyl group, 2-pyridyl-3-propyl group and the like.

The "acyl group" in the formula (VII) specifically means formyl group, acetyl group, propionyl group, butyryl group, isobutyryl group, valeryl group, isovaleryl group, pivaloyl group, hexanoyl group, acryloyl group, propioloyl group, metacryloyl group, crotonoyl group, benzoyl group, naphthoyl group, toluoyl group, hydroatropoyl group, atropoyl group, cinnamoyl group, furoyl group, thenoyl group, nicotinoyl group, isonicotinoyl group, glycoloyl group, lactoyl group, glyceroyl group, tropoyl group, benziloyl group, salicyloyl group, anisoyl group, vanilloyl group, veratroyl group, piperonyloyl group, protocatechuoyl group, galloyl group, cyclopentanecarbonyl group, cyclohexanecarbonyl group, cycloheptanecarbonyl group, 1-methylcyclohexanecarbonyl group, 1-isopentylcyclopentanecarbonyl group, 1-isopentylcyclohexanecarbonyl group, tert-butoxycarbonyl group, methoxycarbonyl group, ethoxycarbonyl group, 2-(1-isopentylcyclohexanecarbonylamino)phenylthiocarbonyl group and the like. Preferred are acetyl group, tert-butoxycarbonyl group, benzoyl group, 1-methylcyclohexanecarbonyl group, 1-isopentylcyclopentanecarbonyl group, 1-isopentylcyclohexanecarbonyl group and 2-(1-isopentylcyclohexanecarbonylamino)phenylthiocarbonyl group.

In the formula (VII), the "optionally substituted" of the "optionally substituted C₃₋₁₀ cycloalkyl group", "optionally substituted C₅₋₈ cycloalkenyl group" and "optionally substituted C_{3**-**10} cycloalkyl C₁₋₁₀ alkyl group" for R and the like means being optionally substituted by 1 to 4 substituents. The substituent may be the same or different and the position of substituent is optional and free of any particular limitation. Specifically, the aforementioned straight chain or branched C₁₋₁₀ alkyl group; the aforementioned straight chain or branched C₂₋₁₀ alkenyl group; the aforementioned C₃₋₁₀ cycloalkyl group; the aforementioned C₅₋₈ cycloalkenyl group; the aforementioned C₃₋₁₀ cycloalkyl C₁₋₁₀ alkyl group; the aforementioned aryl group; amino group; C₁₋₄ lower alkylamino group such as methylamino group, ethylamino group and the like; acylamino group such as acetylamino group, propionylamino group, benzylamino group and the like; oxo group; the aforementioned aralkyl group; the aforementioned arylalkenyl group and the like can be mentioned. The above substituents are recommended mainly as the substituent for R.

In the formula (VII), the "optionally substituted" of the "optionally substituted aryl group", "optionally substituted 5- or 6-membered heterocyclic group having 1 to 3 from nitrogen atom, oxygen atom and sulfur atom" and "optionally substituted aralkyl group" for R and the like means being optionally substituted by 1 to 4, preferably 1 to 3, substituents. The substituent may be the same or different and the position of substituent is optional and free of any particular limitation. Specifically, the aforementioned straight chain or branched C₁₋₁₀ alkyl group, preferably straight chain or branched C₁₋₆ alkyl group; the aforementioned straight chain or branched C₂₋₁₀ alkenyl group, preferably straight chain or branched C₂₋₆ alkenyl group; the aforementioned halogen atom; nitro group; the aforementioned C₁₋₄ lower alkyl group or amino group optionally substituted by the aforementioned acyl group; hydroxyl group; the aforementioned C₁₋₄ lower alkoxy group; the aforementioned C₁₋₄ lower alkylthio group; the aforementioned halogenated C₁₋₄ lower alkyl group; the aforementioned acyl group; oxo group and the like can be mentioned. Of these, the aforementioned straight chain or branched C₁₋₆ alkyl, group, the aforementioned halogen atom, and nitro group are particularly preferable.

In the formula (VII), the "mercapto-protecting group" for Z means a conventionally used mercapto-protecting group, and is free of any particular limitation as long as it is an organic residue liberated in living organisms. It may also form a disulfide form, which is a dimer. Specifically, those such as the following C₁₋₄ lower alkoxymethyl group; C₁₋₄ lower alkylthiomethyl group; aralkyloxymethyl group; aralkylthiomethyl group; C₃₋₁₀ cycloalkyloxymethyl group; C₅₋₈ cycloalkenyloxymethyl group; C₃₋₁₀ cycloalkyl C₁₋₁₀ alkoxymethyl group; aryloxymethyl group; arylthiomethyl group; acyl group; acyloxy group; aminocarbonyloxymethyl group; thiocarbonyl group; and thio group can be mentioned. More specifically, C₁₋₄ lower alkoxymethyl group having the aforementioned C₁₋₄ lower alkoxy group; C₁₋₄ lower alkylthiomethyl group having the aforementioned C₁₋₄ lower alkylthio group; aralkyloxymethyl group having the aforementioned aralkyl group; aralkylthiomethyl group having the aforementioned aralkyl group; C₃₋₁₀ cycloalkyloxymethyl group having the aforementioned C₃₋₁₀ cycloalkyl group; C₅₋₈ cycloalkenyloxymethyl group having the aforementioned C₅₋₈ cycloalkenyl group; C₃₋₁₀ cycloalkyl, C₁₋₁₀ alkoxymethyl group having the aforementioned C₃₋₁₀ cycloalkyl C₁₋₁₀ alkyl group; aryloxymethyl group having the aforementioned aryl group; arylthiomethyl group having the aforementioned arylthio group; acyl group having the aforementioned optionally substituted straight chain or branched C₁₋₁₀ alkyl group, the aforementioned halogenated C₁₋₄ lower alkyl group, the aforementioned C₁₋₄ lower alkoxy group, the aforementioned C₁₋₄ lower alkylthio group, the aforementioned optionally substituted amino group, the aforementioned optionally substituted ureido group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted arylthio group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl, group; acyloxy group having the aforementioned optionally substituted straight chain or branched C₁₋₁₀ alkyl group, the aforementioned halogenated C₁₋₄ lower alkyl group, the aforementioned C₁₋₄ lower alkoxy group, the aforementioned C₁₋₄ lower alkylthio group, the aforementioned optionally substituted amino group, the aforementioned optionally substituted ureido group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted arylthio group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl, group; aminocarbonyloxymethyl group optionally substituted by the aforementioned optionally substituted straight chain or branched C₁₋₁₀ alkyl group, the aforementioned halogenated C₁₋₄ alkyl group, the aforementioned C₁₋₄ lower alkoxy group, the aforementioned C₁₋₄ lower alkylthio group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl, group; thiocarbonyl group having the aforementioned optionally substituted straight chain or branched C₁₋₁₀ alkyl group, the aforementioned halogenated C₁₋₄ lower alkyl group, the aforementioned C₁₋₄ lower alkoxy group, the aforementioned C₁₋₄ lower alkylthio, group, the aforementioned optionally substituted amino group, the aforementioned optionally substituted ureido group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted arylthio group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl group; and thio group having the aforementioned optionally substituted C₁₋₄ lower alkyl group or aryl group can be mentioned.

To be more concrete, as the "straight chain or branched C₁₋₁₀ alkyl group" for R of the formula (VII), methyl group, ethyl group, isopropyl group, butyl group, isobutyl group, tert-butyl group, heptyl group, 1-propylbutyl group and 1-isobutyl-3-methylbutyl group are preferable.

As the "straight chain or branched C₂₋₁₀ alkenyl group" for R of the formula (VII), allyl group, vinyl group, isopropenyl group, 1-methyl-2-propenyl group, 2-methyl-2-propenyl group, 1-methyl-1-butenyl group, crotyl group, 1,3-dimethyl-2-butenyl group, 1-pentenyl group and 1-methyl-2-pentenyl group are preferable.

The "halogenated C₁₋₄ lower alkyl group" for R of the formula (VII) means C₁₋₄ lower alkyl group (particularly preferably methyl group) substituted by the aforementioned halogen atom (particularly preferably fluorine atom, chlorine atom), with preference given to trifluoromethyl group.

The "optionally substituted C₃₋₁₀ cycloalkyl group" for R of the formula (VII) is C₃₋₁₀ cycloalkyl group (particularly preferably cyclopropyl group, cyclobutyl group, cyclopentyl group, cyclohexyl group, cycloheptyl group, octahydroindenyl group, decahydronaphthyl group, adamantyl group, bicyclo[2.2.1]heptyl group) optionally substituted by 1 to 4 substituents selected from the aforementioned straight chain or branched C₁₋₁₀ alkyl group (particularly preferably C₁₋₈ alkyl group such as methyl group, ethyl group, propyl group, isopropyl group, butyl group, isobutyl group, tert-butyl group, pentyl group, isopentyl group, 2,2-dimethylpropyl group, 4-methylpentyl group, 2-ethylbutyl group etc.), the aforementioned straight chain or branched C₂₋₁₀ alkenyl group (particularly preferably C₂₋₈ alkenyl group such as 1-methylvinyl group, 2-methylvinyl group, 3-methyl-3-propenyl group etc.), the aforementioned C₃₋₁₀ cycloalkyl group (particularly preferably C₃₋₇ cycloalkyl group such as cyclopropyl group, cyclopentyl group, cyclohexyl group etc.), the aforementioned C₅₋₈ cycloalkenyl group (particularly preferably C₅₋₆ cycloalkenyl group such as cyclopentenyl group, cyclohexenyl group etc.), the aforementioned C₃₋₁₀ cycloalkyl C₁₋₁₀ alkyl group (particularly preferably C₃₋₇ cycloalkyl C₁₋₄ alkyl group such as cyclopropylmethyl group, 2-cyclopropylethyl group, 2-cyclopentylethyl group, cyclohexylmethyl group, 2-cyclohexylethyl group etc.), the aforementioned aryl group (particularly preferably phenyl group), oxo group, the aforementioned aralkyl group (particularly preferably phenyl C₁₋₄ lower alkyl group such as benzyl group, phenethyl group etc.) and the aforementioned arylalkenyl group (particularly preferably 2-phenylvinyl group). Preferred are 2,2,3,3-tetramethylcyclopropyl group, 1-isopentylcyclobutyl group, 1-isopropylcyclopentyl group, 1-isobutylcyclopentyl group, 1-isopentylcyclopentyl group, 1-cyclohexylmethylcyclopentyl group, cyclohexyl group, 1-methylcyclohexyl group, 1-ethylcyclohexyl group, 1-propylcyclohexyl group, 1-isopropylcyclohexyl group, 1-butylcyclohexyl group, 1-isobutylcyclohexyl group, 1-pentylcyclohexyl group, 1-isopentylcyclohexyl group, 1-(2,2-dimethylpropyl)cyclohexyl group, 1-(4-methylpentyl)cyclohexyl group, 1-(2-ethylbutyl)cyclohexyl group, 4-tert-butyl-1-isopentylcyclohexyl group, 1-cyclopropylcyclohexyl group, 1-bicyclohexyl group, 1-phenylcyclohexyl group, 1-cyclopropylmethylcyclohexyl group, 1-cyclohexylmethylcyclohexyl group, 1-(2-cyclopropylethyl)cyclohexyl group, 1-(2-cyclopentylethyl)cyclohexyl group, 1-(2-cyclohexylethyl)cyclohexyl group, 4-methylcyclohexyl group, 4-propylcyclohexyl group, 4-isopropylcyclohexyl group, 4-tert-butylcyclohexyl group, 4-pentylcyclohexyl group, 4-bicyclohexyl group, 1-isopentylcycloheptyl group, 3a-octahydroindenyl group, 4a-decahydronaphthyl group, 1-adamantyl group and 7,7-dimethyl-1-(2-oxo)bicyclo[2.2.1]heptyl group. While the position of substitution is not particularly limited, it is particularly preferably the 1-position. While the substituent may be any of the above-mentioned substituents, it is particularly preferably straight chain or branched C₁₋₁₀ alkyl group.

The substituent of the "optionally substituted C₅₋₈ cycloalkenyl group" for R of the formula (VII) is completely the same as in the case of the aforementioned "optionally substituted C₃₋₁₀ cycloalkyl group". Specific examples thereof include cycloalkenyl group (particularly cyclopentenyl group, cyclohexenyl group) optionally substituted by 1 to 4 substituents selected from the aforementioned straight chain or branched C₁₋₁₀ alkyl group (particularly preferably C₁₋₈ alkyl group such as methyl group, ethyl group, propyl group, isopropyl group, butyl group, isobutyl group, pentyl group, isopentyl group, 2,2-dimethylpropyl group, 4-methylpentyl group etc.), the aforementioned straight chain or branched C₂₋₁₀ alkenyl group (particularly preferably C₂₋₈ alkenyl group such as 1-methylvinyl group, 2-methylvinyl group, 3-methyl-3-propenyl group etc.), the aforementioned C₃₋₁₀ cycloalkyl group (particularly preferably C₃₋₇ cycloalkyl group such as cyclopropyl group, cyclopentyl group, cyclohexyl group etc.), the aforementioned C₅₋₈ cycloalkenyl group (particularly preferably C₅₋₆ cycloalkenyl group such as cyclopentenyl group, cyclohexenyl group etc.), the aforementioned C₃₋₁₀ cycloalkyl C₁₋₁₀ alkyl group (particularly preferably C₃₋₇ cycloalkyl C₁₋₄ lower alkyl, group such as cyclopropylmethyl group, 2-cyclopropylethyl group, 2-cyclopentylethyl group, cyclohexylmethyl group, 2-cyclohexylethyl group etc.), the aforementioned aryl group (particularly preferably phenyl group), oxo group, the aforementioned aralkyl group (particularly preferably phenyl C₁₋₄ lower alkyl group such as benzyl group, phenethyl group etc.) and the aforementioned arylalkenyl group (particularly preferably 2-phenylvinyl group) . Preferred are 1-isopropyl-2-cyclopentenyl group, 1-isopropyl-3-cyclopentenyl group, 1-isobutyl-2-cyclopentenyl group, 1-isobutyl-3-cyclopentenyl group, 1-isopentyl-2-cyclopentenyl group, 1-isopentyl-3-cyclopentenyl group, 1-cyclohexylmethyl-2-cyclopentenyl group, 1-cyclohexylmethyl-3-cyclopentenyl group, 1-cyclohexenyl group, 2-cyclohexenyl group, 3-cyclohexenyl group, 1-methyl-2-cyclohexenyl group, 1-methyl-3-cyclohexenyl group, 1-ethyl-2-cyclohexenyl group, 1-ethyl-3-cyclohexenyl group, 1-propyl-2-cyclohexenyl group, 1-propyl-3-cyclohexenyl group, 1-isopropyl-2-cyclohexenyl group, 1-isopropyl-3-cyclohexenyl group, 1-butyl-2-cyclohexenyl group, 1-butyl-3-cyclohexenyl group, 1-isobutyl-2-cyclohexenyl group, 1-isobutyl-3-cyclohexenyl group, 1-pentyl-2-cyclohexenyl group, 1-pentyl-3-cyclohexenyl group, 1-isopentyl-2-cyclohexenyl group, 1-isopentyl-3-cyclohexenyl group, 1-(2,2-dimethylpropyl)-2-cyclohexenyl group, 1-(2,2-dimethylpropyl)-3-cyclohexenyl group, 1-(4-methylpentyl)-2-cyclohexenyl group, 1-(4-methylpentyl)-3-cyclohexenyl group, 1-cyclopropyl-2-cyclohexenyl group, 1-cyclopropyl-3-cyclohexenyl group, 1-cyclohexyl-2-cyclohexenyl group, 1-cyclohexyl-3-cyclohexenyl group, 1-phenyl-2-cyclohexenyl group, 1-phenyl-3-cyclohexenyl group, 1-cyclopropylmethyl-2-cyclohexenyl group, 1-cyclopropylmethyl-3-cyclohexenyl group, 1-cyclohexylmethyl-2-cyclohexenyl group, 1-cyclohexylmethyl-3-cyclohexenyl group, 1-(2-cyclopropylethyl)-2-cyclohexenyl group, 1-(2-cyclopropylethyl)-3-cyclohexenyl group, 1-(2-cyclopentylethyl)-2-cyclohexenyl group, 1-(2-cyclopentylethyl)-3-cyclohexenyl group, 1-(2-cyclohexylethyl)-2-cyclohexenyl group and 1-(2-cyclohexylethyl)-3-cyclohexenyl group. While the position of substitution is not particularly limited, it is particularly preferably the 1-position. While the substituent may be any of the above-mentioned substituents, it is particularly preferably straight chain or branched C₁₋₁₀ alkyl group or C₃₋₁₀ cycloalkyl C₁₋₄ alkyl group.

The "optionally substituted C₃₋₁₀ cycloalkyl C₁₋₁₀ alkyl group" for R of the formula (VII) means C₃₋₁₀ cycloalkyl C₁₋₁₀ alkyl group (particularly preferably cyclohexylmethyl group, 1-cyclohexylethyl group, 1-cyclohexyl-1-methylethyl group, 1-cyclohexyl-2-methylpropyl group, 1-cyclohexyl-3-methylbutyl group, 1-cyclohexylhexyl group, 1-cyclohexyl-4-methylpentyl group, 1-cyclohexylheptyl group) optionally substituted by 1 to 4 substituents selected from the aforementioned C₃₋₁₀ cycloalkyl group (particularly preferably C₃₋₇ cycloalkyl group such as cyclopentyl group, cyclohexyl group etc.), the aforementioned C₅₋₈ cycloalkenyl group (particularly preferably C₅₋₇ cycloalkenyl group such as cyclopentenyl group and cyclohexenyl group) and the aforementioned aryl group (particularly preferably phenyl group), wherein C₁₋₁₀ alkyl group is straight chain or branched. The position of substitution is not particularly limited, and the straight chain or branched C₁₋₁₀ alkyl group moiety may have a substituent. Preferred are cyclohexylmethyl group, 1-cyclohexylethyl group, cyclohexylcyclopentylmethyl group, dicyclohexylmethyl group, 1-cyclohexyl-1-methylethyl group, 1-cyclohexyl-2-methylpropyl group, l-cyclohexyl-3-methylbutyl group, 1-cyclohexyl-4-methylpentyl group, 1-cyclohexylhexyl group and 1-cyclohexylheptyl group.

The "optionally substituted aryl group" for R of the formula (VII) means aryl group (particularly preferably phenyl group) optionally substituted by 1 to 4 substituents selected from the aforementioned straight chain or branched C₁₋₆ alkyl group (particularly preferably tert-butyl group), the aforementioned halogen atom (particularly preferably fluorine atom, chlorine atom) and nitro group. Preferred are phenyl group, 2-chlorophenyl group, 4-nitrophenyl group and 3,5-di-tert-butylphenyl group.

The "optionally substituted aralkyl group" for R of the formula (VII) means aralkyl group (particularly preferably benzyl group, benzhydryl group, trityl group) optionally substituted by the substituent selected from the aforementioned halogen atom (particularly preferably fluorine atom, chlorine atom), nitro group and hydroxyl group, wherein the C₁₋₄ lower alkyl group is straight chain or branched. The position of substitution is not particularly limited and the straight chain or branched C₁₋₄ lower alkyl group moiety may have said substituent. Preferred are benzyl group and trityl group.

The "optionally substituted 5- or 6-membered heterocyclic group having 1 to 3 from nitrogen atom, oxygen atom and sulfur atom" for R of the formula (VII) means the aforementioned heterocyclic group optionally substituted by 1 to 4 substituents selected from the aforementioned straight chain or branched C₁₋₆ alkyl group (particularly preferably tert-butyl group), the aforementioned halogen atom (particularly preferably fluorine atom, chlorine atom) and nitro group, with preference given to aromatic heterocyclic group. Particularly preferred are furyl group, thienyl group and pyridyl group.

The "halogen atom" for X₁, X₂, X₃, X₄ in the formula (VII) means halogen atom such as fluorine atom, chlorine atom, bromine atom and the like, with preference given to fluorine atom and chlorine atom.

The "C₁₋₄ lower alkyl group" for X₁, X₂, X₃, X₄ in the formula (VII) is preferably methyl group.

The "halogenated C₁₋₄ lower alkyl group" for X₁, X₂, X₃, X₄ in the formula (VII) is C₁₋₄ lower alkyl group (particularly preferably methyl group) substituted by the aforementioned halogen atom (particularly preferably fluorine atom, chlorine atom), with preference given to trifluoromethyl group.

The "C₁₋₄ lower alkoxy group" for X₁, X₂, X₃, X₄ in the formula (VII) is preferably methoxy group.

In the formula (VII), the "acyl group" for X₁, X₂, X₃, X₄ is preferably benzoyl group.

In the formula (VII), the "aryl group" for X₁, X₂, X₃, X₄ is preferably phenyl group.

The "prodrug compound" of the formula (VII) is a derivative of the compound of the formula (VII), which has a chemically or metabolically degradable group and shows pharmaceutical activity by hydrolysis or solvent decomposition, or by degradation under physiological conditions.

The "pharmaceutically acceptable salt" of the formula (VII) may be any as long as it forms a nontoxic salt with a compound represented by the above-mentioned formula (VII). For example, inorganic acid salts such as hydrochloride, hydrobromide, hydroiodide, sulfate, nitrate, phosphate, carbonate, hydrogen carbonate, perchlorate etc.; organic acid salts such as formate, acetate, trifluoroacetate, propionate, oxalate, glycolate, succinate, lactate, maleate, hydroxymaleate, methylmaleate, fumarate, adipate, tartrate, malate, citrate, benzoate, cinnamate, ascorbate, salicylate, 2-acetoxyhenzoate, nicotinate, isonicotinate etc.; sulfonates such as methanesulfonate, ethanesulfonate, isethionate, benzenesulfonate, p-toluenesulfonate, naphthalenesulfonate etc.; acidic amino acid salts such as aspartate, glutamate etc.; alkali metal salts such as sodium salt, potassium salt etc.; alkaline earth metal salts such as magnesium salt, calcium salt etc.; ammonium salt; organic base salts such as trimethylamine salt, triethylamine salt, pyridine salt, picoline salt, dicyclohexylamine salt, N,N'-dibenzylethylenediamine salt etc.; and amino acid salts such .as lysin salt, arginine salt etc. can be mentioned. In some cases, it may be a hydrate or a solvate with alcohol and the like.

Specifically, the compound disclosed in JP-A-H11-222428 can be mentioned:
a compound represented by the formula (VIII) 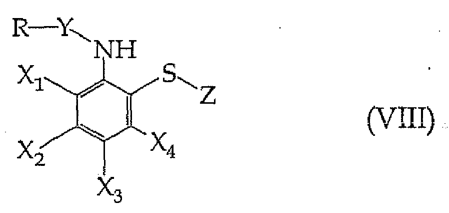 wherein
   - R: is a straight chain or branched C₁₋₁₀ alkyl groups, a straight chain or branched C₂₋₁₀ alkenyl group, a halogenated C₁₋₄ lower alkyl group, a C₃₋₁₀ cycloalkyl group, a C₅₋₈ cycloalkenyl group, a C₃₋₁₀ cycloalkyl C₁₋₁₀ alkyl group (wherein these cycloalkyl group, cycloalkenyl group and cycloalkylalkyl group are each optionally substituted by a group selected from straight chain or branched C₁₋₁₀ alkyl group, straight chain or branched C₂₋₁₀ alkenyl group, C₃₋₁₀ cycloalkyl, group, C₅₋₈ cycloalkenyl group, C₃₋₁₀ cycloalkyl C₁₋₁₀ alkyl group, aryl group, amino group, C₁₋₄ lower alkylamino group, acylamino group, oxo group, aralkyl group and arylalkenyl group), an aryl group, an aralkyl group or a 5- or 6-membered heterocyclic group having 1 to 3 from nitrogen atom, oxygen atom and sulfur atom (wherein these aryl group, aralkyl group and heterocyclic group are each optionally substituted by a group selected from straight chain or branched C₁₋₁₀ alkyl group, straight chain or branched C₂₋₁₀ alkenyl group, halogen atom, nitro group, amino group optionally substituted by C₁₋₄ lower alkyl group or acyl group, hydroxyl group, C₁₋₄ lower alkoxy group, C₁₋₄ lower alkylthio group, halogenated C₁₋₄ lower alkyl group, acyl group and oxo group);
   - X₁, X₂, X₃ and X₄: may be the same or different and each is a hydrogen atom, a halogen atom, a C₁₋₄ lower alkyl group, a halogenated C₁₋₄ lower alkyl group, a C₁₋₄ lower alkoxy group, a cyano group, a nitro group, an acyl group or an aryl group;
   - Y: is -CO- or -SO₂-;
   - Z: is a hydrogen atom or a mercapto-protecting group selected from a group that forms a disulfide form, which is a dimer, C₁₋₄ lower alkoxymethyl group, C₁₋₄ lower alkylthiomethyl group, aralkyloxymethyl group, aralkylthiomethyl group, C₃₋₁₀ cycloalkyloxymethyl group, C₅-₈ cycloalkenyloxymethyl group, C₃-₁₀ cycloalkyl C₁₋₁₀ alkoxymethyl group, aryloxymethyl group, arylthiomethyl group, acyl group, acyloxy group, aminocarbonyloxymethyl group, thiocarbonyl group and thio group,
a prodrug compound thereof, a pharmaceutically acceptable salt thereof, or a hydrate thereof or a solvate thereof.

The "straight chain or branched C₁₋₁₀ alkyl group" in the formula (VIII) means a straight chain or branched alkyl group having 1 to 10 carbon atoms, which is specifically methyl group, ethyl group, propyl group, isopropyl group, butyl group, isobutyl group, sec-butyl group, tert-butyl group, pentyl group, isopentyl group, neopentyl group, tert-pentyl group, 1-ethylbutyl group, 2-ethylbutyl group, 1-propylbutyl group, 1,1-dimethylbutyl group, 1-isobutyl-3-methylbutyl group, 1-ethylpentyl group, 1-propylpentyl group, 1-isobutylpentyl group, 2-ethylpentyl group, 2-isopropylpentyl group, 2-tert-butylpentyl group, 3-ethylpentyl group, 3-isopropylpentyl group, 4-methylpentyl group" 1,4-dimethylpentyl group, 2,4-dimethylpentyl group, 1-ethyl-4-methylpentyl group, hexyl group, 1-ethylhexyl group, 1-propylhexyl group, 2-ethylhexyl group, 2-isopropylhexyl group, 2-tert-butylhexyl group, 3-ethylhexyl group, 3-isopropylhexyl group, 3-tert-butylhexyl group, 4-ethylhexyl group, 5-methylhexyl group, heptyl group, 1-ethylheptyl group, 1-isopropylheptyl group, 2-ethylheptyl group, 2-isopropylheptyl group, 3-propylheptyl group, 4-propylheptyl group, 5-ethylheptyl group, 6-methylheptyl group, octyl group, I-ethyloctyl group, 2-ethyloctyl group, nonyl group, 1-methyLnony.l group, 2-methylnonyl group, decyl group and the like, with preference given to a straight chain or branched alkyl group having 1 to 8 carbon atoms.

The "C₁₋₄ lower alkyl group" in the formula (VIII) means a straight or branched alkyl group having 1 to 4 carbon atoms, which is specifically methyl group, ethyl group, propyl group, isopropyl group, butyl group, isobutyl group, sec-butyl group, tert-butyl group and the like.

The "straight chain or branched C₂₋₁₀ alkenyl group" in the formula (VIII) means a straight chain or branched alkenyl group having 2 to 10 carbon atoms and at least one double bond, which is specifically allyl group, vinyl group, isopropenyl group, 1-propenyl group, 1-methyl-2-propenyl group, 2-methyl-2-propenyl group, 1-methyl-l-butenyl group, crotyl group, 1-methyl-3-butenyl group, 3-methyl-2-butenyl group, 1,3-dimethyl-2-butenyl group, 1-pentenyl group, 1-methyl-2-pentenyl group, 1-ethyl-3-pentenyl group, 4-pentenyl group, 1,3-pentadienyl group, 2,4-pentadienyl group, 1-hexenyl group, 1-methyl-2-hexenyl group, 3-hexenyl group, 4-hexenyl group, 1-butyl-5-hexenyl group, 1,3-hexadienyl group, 2,4-hexadienyl group, 1-heptenyl group, 2-heptenyl group, 3-heptenyl group, 4-heptenyl group, 5-heptenyl group, 6-heptenyl group, 1,3-heptadienyl group, 2,4-heptadienyl group, 1-octenyl group, 2-octenyl group, 3-octenyl group, 4-octenyl group, 5-octenyl group, 6-octenyl group, 7-octenyl group, 1-nonenyl group, 2-nonenyl group, 3-nonenyl group, 4-nonenyl group, 5-nonenyl group, 6-nonenyl group, 7-nonenyl group, 8-nonenyl group, 9-decenyl group and the like, with preference given to a straight chain or branched alkenyl group having 2 to 8 carbon atoms.

The "halogen atom" in the formula (VIII) means fluorine atom, chlorine atom, bromine atom and the like.

The "halogenated C₁₋₄ alkyl group" in the formula (VIII) means the aforementioned C₁₋₄ lower alkyl group substituted by 1 to 3 the same or different halogen atoms, which is specifically fluoromethyl group, chloromethyl group, bromomethyl group, difluoromethyl group, dichloromethyl group, trifluoromethyl group, trichloromethyl group, chloroethyl group, difluoroethyl group, trifluoroethyl group, pentachloroethyl group, bromopropyl group, dichloropropyl group, trifluorobutyl group and the like, with preference given to trifluoromethyl group and chloroethyl group.

The "C₁₋₄ lower alkoxy group" in the formula (VIII) means an alkoxy group having the aforementioned C₁₋₄ lower alkyl group, which is specifically methoxy group, ethoxy group, propoxy group, isopropoxy group, butoxy group, isobutoxy group, sec-butoxy group, tert-butoxy group and the like.

The "C₁₋₄ lower alkylthio, group" in the formula (VIII) means an alkylthio group having the aforementioned C₁₋₄ lower alkyl group, which is specifically methylthio group, ethylthio group, propylthio group, isopropylthio group, butylthio group, isobutylthio group, sec-butylthio group, tert-butylthio group and the like.

The "C₃₋₁₀ cycloalkyl group" in the formula (VIII) means a monocyclic or polycyclic cycloalkyl group having 3 to 10 carbon atoms, which is specifically cyclopropyl group, cyclobutyl group, cyclopentyl group, cyclohexyl group, cycloheptyl group, cyclooctyl group, octahydroindenyl group, decahydronaphthyl group, bicyclo[2.2.1]heptyl group, adamantyl group and the like, with preference given to cyclopentyl group, cyclohexyl group and cycloheptyl group, each having 5 to 7 carbon atoms.

The "C₅₋₈ cycloalkenyl group" in the formula (VIII) means a cycloalkenyl group having 5 to 8 carbon atoms and one or more double bonds on the ring, which is specifically cyclopentenyl group, cyclohexenyl group, cycloheptenyl group, cyclooctenyl group, cyclopentadienyl group, cyclohexadienyl group, cycloheptadienyl group, cyclooctadienyl group and the like, with preference given to cyclopentenyl group, cyclohexenyl group and cycloheptenyl group, each having 5 to 7 carbon atoms.

The "C₃₋₁₀ cycloalkyl C₁₋₁₀ alkyl group" in the formula (VIII) means the aforementioned straight chain or branched C₁₋₁₀ alkyl group substituted by the aforementioned C₃₋₁₀ cycloalkyl group, which is specifically cyclopropylmethyl group, cyclopentylmethyl group, cyclohexylmethyl group, cyclohexylcyclopentylmethyl group, dicyclohexylmethyl group, 1-cyclopentylethyl group, 1-cyclohexylethyl group, 2-cyclopropylethyl group, 2-cyclopentylethyl group, 2-cyclohexylethyl group, 2-cycloheptylethyl group, 1-cyclohexyl-1-methylethyl group, 1-cyclohexylpropyl group, 2-cyclopentylpropyl group, 3-cyclobutylpropyl group, 3-cyclopentylpropyl group, 3-cyclohexylpropyl group, 3-cycloheptylpropyl group, 1-cyclopropyl-1-methylpropyl group, 1-cyclohexyl-2-methylpropyl group, 1-cyclopentylbutyl group, 1-cyclohexylbutyl group, 3-cyclohexylbutyl group, 4-cyclopropylbutyl group, 4-cyclobutylbutyl group, 4-cyclopentylbutyl group, 1-cyclohexyl-1-methylbutyl group, 1-cyclopentyl-2-ethylbutyl group, 1-cyclohexyl-3-methylbutyl group, 1-cyclopentylpentyl group, 1-cyclohexylpentyl group, 1-cyclohexylmethylpentyl group, 2-cyclohexylpentyl group, 2-cyclohexylmethylpentyl group, 3-cyclopentylpentyl group, 1-cyclohexyl-4-methylpentyl group, 5-cyclopentylpentyl group, 1-cyclopentylhexyl group, 1-cyclohexylhexyl group, 1-cyclopentylmethylhexyl group, 2-cyclopentylhexyl group, 2-cyclopropylethylhexyl group, 3-cyclopentylhexyl group, 1-cyclohexylheptyl group, 1-cyclopentyl-1-methylheptyl group, 1-cyclohexyl-1,6-dimethylheptyl group, 1-cycloheptyloctyl group, 2-cyclopentyloctyl group, 3-cyclohexyloctyl group, 2-cyclopentylmethyloctyl group, 1-cyclopentylnonyl group, 1-cyclohexylnonyl group, 3-cyclopropylnonyl group, 1-cyclopentyldecyl group, 1-cyclohexylundecyl group, 1-cyclopentyltridecyl group, 2-cyclohexyltridecyl group and the like.

The "aryl group" in the formula (VIII) means phenyl group, naphthyl group, anthryl group, phenanthryl group, biphenyl group and the like, with preference given to phenyl group, naphthyl group and biphenyl group.

The "aralkyl group" in the formula (VIII) means the aforementioned C₁-₄ lower alkyl group substituted by one or more aryl groups mentioned above, which is specifically benzyl group, benzhydryl group, trityl group, phenethyl group, 3-phenylpropyl group, 2-phenylpropyl group, 4-phenylbutyl group, naphthylmethyl group, 2-naphthylethyl group, 4-biphenylmethyl group, 3-(4-biphenyl)propyl group and the like.

The "arylalkenyl group" in the formula (VIII) means alkenyl group having 2 to 4 carbon atoms substituted by aryl group mentioned above, which is specifically 2-phenylvinyl group, 3-phenyl-2-propenyl group, 3-phenyl-2-methyl-2-propenyl group, 4-phenyl-3-butenyl group, 2-(1-naphthyl)vinyl group, 2-(2-naphthyl)vinyl group, 2-(4-biphenyl)vinyl group and the like.

The "arylthio group" in the formula (VIII) means arylthio group having the aforementioned aryl group, which is specifically phenylthio group, naphthylthio group and the like.

The "heterocyclic group" in the formula (VIII) means a 5- or 6-membered aromatic or nonaromatic heterocyclic group having one or more, specifically 1 to 4, preferably 1 to 3, heteroatoms selected from nitrogen atom, oxygen atom and sulfur atom, which is specifically aromatic heterocyclic groups such as thiatriazolyl group, tetrazolyl group, dithiazolyl group, oxadiazolyl group, thiadiazolyl group, triazolyl group, oxazolyl group, pyrazolyl group, pyrrolyl group, furyl group, thienyl group, tetrazinyl group, triazinyl group, pyrazinyl group, pyridazinyl group, pyrimidinyl group, pyridyl group and the like and nonaromatic heterocyclic groups such as dioxolanyl group, pyrrolidinyl group, tetrahydrofuryl group, tetrahydrothienyl group, dithiadiazinyl group, thiadiazinyl group, morpholino group, morpholinyl group, oxazinyl group, thiazinyl group, piperazinyl group, piperidyl group, piperidino group, pyranyl group, thiopyranyl group and the like. Preferred are an aromatic heterocyclic (heteroaryl) group such as furyl, group, thienyl group, pyrrolyl group, pyridyl group and the like and a nonaromatic heterocyclic group having at least one nitrogen atom, such as pyrrolidinyl group, tetrahydrofuryl group, piperazinyl group, piperidyl group, piperidino group and the like.

The "acyl group" in the formula (VIII) specifically means formyl group, acetyl group, propionyl group, butyryl group, isobutyryl group, valeryl group, isovaleryl group, pivaloyl group, hexanoyl group, acryloyl group, propioloyl group, metacryloyl group, crotonoyl group, benzoyl group, naphthoyl group, toluoyl group, hydroatropoyl group, atropoyl group, cinnamoyl group, furoyl group, thenoyl group, nicotinoyl group, isonicotinoyl group, glycoloyl group, lactoyl group, glycerol group, tropoyl group, benziloyl group, salicyloyl group, anisoyl group, vanilloyl group, veratroyl group, piperonyloyl group, protocatechuoyl group, galloyl group, cyclopentanecarbonyl group, cyclohexanecarbonyl group, cycloheptanecarbonyl group, 1-methylcyclohexanecarbonyl group, 1-isopentylcyclopentanecarbonyl group, 1-isopentylcyclohexanecarbonyl group, tert-butoxycarbonyl group, methoxycarbonyl group, ethoxycarbonyl group, 2-(1-isopentylcyclohexanecarbonylamino)phenylthiocarbonyl group and the like. Preferred are acetyl group, tert-butoxycarbonyl group, benzoyl group, 1-methylcyclohexanecarbonyl group, 1-isopentylcyclopentanecarbonyl group, 1-isopentylcyclohexanecarbonyl group and 2-(1-isopentylcyclohexanecarbonylamino)phenylthiocarbonyl group.

In the formula (VIII), the "optionally substituted of the "optionally substituted C₃₋₁₀ cycloalkyl group", "optionally substituted C₅₋₈ cycloalkenyl group" and "optionally substituted C₃₋₁₀ cycloalkyl C₁₋₁₀ allyl group" for R and the like means being optionally substituted by 1 to 4 substituents. The substituent may be the same or different and the position of substituent is optional and free of any particular limitation. Specifically, the aforementioned straight chain or branched C₁₋₁₀ alkyl group; the aforementioned straight chain or branched C₂₋₁₀ alkenyl group; the aforementioned C₃₋₁₀ cycloalkyl group; the aforementioned C₅₋₈ cycloalkenyl group; the aforementioned C₃₋₁₀ cycloalkyl C₁₋₁₀ alkyl group; the aforementioned aryl group; amino group; C₁₋₄ lower alkylamino group such as methylamino group, ethylamino group and the like; acylamino group such as acetylamino group, propionylamino group, benzylamino group and the like; oxo group; the aforementioned aralkyl group; the aforementioned arylalkenyl group and the like can be mentioned. The above substituents are recommended mainly as the substituent for R.

In the formula (VIII), the "optionally substituted" of the "optionally substituted aryl group", "optionally substituted 5- or 6-membered heterocyclic group having 1 to 3 from nitrogen atom, oxygen atom and sulfur atom", "optionally substituted aralkyl group", "optionally substituted arylalkenyl group", "optionally substituted arylthio group" and "optionally substituted 5- or 6-membered heteroarylalkyl group" for R and the like means being optionally substituted by 1 to 4, preferably 1 to 3, substituents. The substituent may be the same or different and the position of substituent is optional and free of any particular limitation. Specifically, the aforementioned straight chain or branched C₁₋₁₀ alkyl group, preferably straight chain or branched C₁₋₆ alkyl group; the aforementioned straight chain or branched C₂₋₁₀ alkenyl group, preferably straight chain or branched C₂₋₆ alkenyl group; the aforementioned halogen atom; nitro group; the aforementioned C₁₋₄ lower alkyl group or amino group optionally substituted by the aforementioned acyl group; hydroxyl group; the aforementioned C₁₋₄ lower alkoxy group; the aforementioned C₁₋₄ lower alkylthio group; the aforementioned halogenated C₁₋₄ lower alkyl group; the aforementioned acyl group; oxo, group and the like can be mentioned. Of these, the aforementioned straight chain or branched C₁₋₆ alkyl group; the aforementioned halogen atom; and nitro group are particularly preferable.

In the formula (VIII), the "optionally substituted" of the "optionally substituted amino group" means being optionally substituted by one or more, preferably 1 or 2, substituents. The substituents may be the same or different and the position of substitution is optional and is not particularly limited. Specific examples thereof include the aforementioned aryl group optionally substituted by the aforementioned C₁₋₄ lower alkyl group, and the like; hydroxyl group; the aforementioned acyl group; and the aforementioned C₁₋₄ lower alkoxy group.

In the formula (VIII), the "mercapto-protecting group" for Z means a conventionally used mercapto-protecting group, and is free of any particular limitation as long as it is an organic residue liberated in living organisms. It may also form a disulfide form, which is a dimer. Specifically, those such as the following C₁₋₄ lower alkoxymethyl group; C₁₋₄ lower alkylthiomethyl group; aralkyloxymethyl group; aralkylthiomethyl group; C₃₋₁₀ cycloalkyloxymethyl group; C₅₋₈ cycloalkenyloxymethyl group; C₃₋₁₀ cycloalkyl C₁₋₁₀ alkoxymethyl group; aryloxymethyl group; arylthiomethyl group; acyl group; acyloxy group; aminocarbonyloxymethyl group; thiocarbonyl group; and thio group can be mentioned. More specifically, C₁₋₄ lower alkoxymethyl group having the aforementioned C₁₋₄ lower alkoxy group; C₁₋₄ lower alkylthiomethyl group having the aforementioned C₁₋₄ lower alkylthio group; aralkyloxymethyl group having the aforementioned aralkyl, group; aralkylthiomethyl group having the aforementioned aralkyl group; C₃₋₁₀ cycloalkyloxymethyl group having the aforementioned C₃₋₁₀ cycloalkyl group; C₅₋₈ cycloalkenyloxymethyl group having the aforementioned C₅₋₈ cycloalkenyl group; C₃₋₁₀ cycloalkyl C₁₋₁₀ alkoxymethyl group having the aforementioned C₃₋₁₀ cycloalkyl C₁₋₁₀ alkyl group; aryloxymethyl group having the aforementioned aryl group; arylthiomethyl group having the aforementioned arylthio group; acyl group having the aforementioned optionally substituted straight chain or branched C₁₋₁₀ alkyl group, the aforementioned halogenated C₁₋₄ lower alkyl group, the aforementioned C₁₋₄ lower alkoxy group, the aforementioned C₁₋₄ lower alkylthio group, the aforementioned optionally substituted amino group, the aforementioned optionally substituted ureido group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl, group, the aforementioned optionally substituted arylthio group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl group; acyloxy group having the aforementioned optionally substituted straight chain or branched C₁₋₁₀ alkyl group, the aforementioned halogenated C₁₋₄ lower alkyl group, the aforementioned C₁₋₄ lower alkoxy group, the aforementioned C₁₋₄ lower alkylthio group, the aforementioned optionally substituted amino group, the aforementioned optionally substituted ureido group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted arylthio group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl group; aminocarbonyloxymethyl group optionally substituted by the aforementioned optionally substituted straight chain or branched C₁₋₁₀ alkyl group, the aforementioned halogenated C₁₋₄ alkyl group, the aforementioned C₁₋₄ lower alkoxy group, the aforementioned C₁₋₄ lower alkylthio group, the aforementioned optionally substituted C₃₋₁₀ cycloalkyl group, the aforementioned optionally substituted C₃₋₁₀ cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl, group; thiocarbonyl group having the aforementioned optionally substituted straight chain or branched C₁₋₁₀ alkyl group, the aforementioned halogenated C₁₋₄ lower alkyl group, the aforementioned C₁₋₄ lower alkoxy group, the aforementioned C₁₋₄ lower alkylthio group, the aforementioned optionally substituted amino group, the aforementioned optionally substituted ureido group, the aforementioned optionally substituted C₃₋₁₀ cycloalkyl group, the aforementioned optionally substituted C₃₋₁₀cycloalkyl C₁₋₁₀ alkyl group, the aforementioned optionally substituted aryl group, the aforementioned optionally substituted aralkyl group, the aforementioned optionally substituted arylalkenyl group, the aforementioned optionally substituted arylthio group, the aforementioned optionally substituted 5- or 6-membered heterocyclic group having 1 to 3 from nitrogen atom, oxygen atom and sulfur atom or the aforementioned optionally substituted 5- or 6-membered heteroarylalkyl group; and thio group having the aforementioned optionally substituted C₁₋₄ lower alkyl group or aryl group can be mentioned.

To be more concrete, as the "straight chain or branched C₁₋₁₀ alkyl group" for R of the formula (VIII), methyl group, ethyl group, isopropyl group, butyl group, isobutyl group, tert-butyl group, heptyl group, 1-propylbutyl group and 1-isobutyl-3-methylbutyl group are preferable.

As the "straight chain or branched C₂₋₁₀ alkenyl group" for R of the formula (VIII), allyl group, vinyl group, isopropenyl group, 1-methyl-2-propenyl group, 2-methyl-2-propenyl group, 1-methyl-1-buteny.l group, crotyl group, 1;3-dimethyl-2-butenyl group, 1-pentenyl group and 1-methyl-2-pentenyl group are preferable.

The "halogenated C₁₋₄ lower alkyl group" for R of the formula (VIII) means C₁₋₄ lower alkyl group (particularly preferably methyl group) substituted by the aforementioned halogen atom (particularly preferably fluorine atom, chlorine atom), with preference given to trifluoromethyl group.

The "optionally substituted C₃₋₁₀ cycloalkyl group" for R of the formula (VIII) is C₃₋₁₀ cycloalkyl group (particularly preferably cyclopropyl group, cyclobutyl group, cyclopentyl group, cyclohexyl group, cycloheptyl group, octahydroindenyl group, decahydronaphthyl group, adamantyl group, bicyclo[2.2.1]heptyl group) optionally substituted by 1 to 4 substituents selected from the aforementioned straight chain or branched C₁₋₁₀ alkyl group (particularly preferably C₁₋₈ alkyl group such as methyl group, ethyl group, propyl group, isopropyl group, butyl group, isobutyl group, tert-butyl group, pentyl group, isopentyl group, 2,2-dimethylpropyl group, 4-methylpentyl group, 2-ethylbutyl group etc.), the aforementioned straight chain or branched C₂₋₁₀ alkenyl group (particularly preferably C₂₋₈ alkenyl group such as 1-methylvinyl group, 2-methylvinyl group, 3-methyl-3-propenyl group etc.), the aforementioned C₃₋₁₀ cycloalkyl group (particularly preferably C₃₋₇ cycloalkyl group such as cyclopropyl group, cyclopentyl group, cyclohexyl group etc.), the aforementioned C₅₋₈ cycloalkenyl group (particularly preferably C₅₋₆ cycloalkenyl group such as cyclopentenyl group, cyclohexenyl group etc.), the aforementioned C₃₋₁₀ cycloalkyl C₁₋₁₀ alkyl group (particularly preferably C₃₋₇ cycloalkyl C₁₋₄ alkyl group such as cyclopropylmethyl group, 2-cyclopropylethyl group, 2-cyclopentylethyl group, cyclohexylmethyl group, 2-cyclohexylethyl group etc.), the aforementioned aryl group (particularly preferably phenyl group), oxo group, the aforementioned aralkyl group (particularly preferably phenyl C₁₋₄ lower alkyl group such as benzyl group, phenethyl group etc.) and the aforementioned arylalkenyl group (particularly preferably 2-phenylvinyl group). Preferred are 2,2,3,3-tetramethylcyclopropyl group, 1-isopentylcyclobutyl group, l-isopropylcyclopentyl group, 1-isobutylcyclopentyl group, 1-isopentylcyclopentyl group, 1-cyclohexylmethylcyclopentyl group, cyclohexyl group, 1-methylcyclohexyl group, 1-ethylcyclohexyl group, 1-propylcyclohexyl group, 1-isopropylcyclohexyl group, 1-butylcyclohexyl group, 1-isobutylcyclohexyl group, 1-pentylcyclohexyl group, 1-isopentylcyclohexyl group, 1-(2,2-dimethylpropyl)cyclohexyl group, 1-(4-methylpentyl)cyclohexyl group, 1-(2-ethylbutyl)cyclohexyl group, 4-tert-butyl-1-isopentylcyclohexyl group, 1-cyclopropylcyclohexyl group, 1-bicyclohexyl group, 1-phenylcyclohexyl group, 1-cyclopropylmethylcyclohexyl group, 1-cyclohexylmethylcyclohexyl group, 1-(2-cyclopropylethyl)cyclohexyl group, 1-(2-cyclopentylethyl)cyclohexyl group, 1-(2-cyclohexylethyl)cyclohexyl group, 4-methylcyclohexyl group, 4-propylcyclohexyl group, 4-isopropylcyclohexyl group, 4-tert-butylcyclohexyl group, 4-pentylcyclohexyl group, 4-bicyclohexyl group, 1-isopentylcycloheptyl group, 3a-octahydroindenyl group, 4a-decahydronaphthyl group, 1-adamantyl group and 7,7-dimethyl-1-(2-oxo)bicyclo[2.2.1]heptyl group. While the position of substitution is not particularly limited, it is particularly preferably the 1-position. While the substituent may be any of the above-mentioned substituents, it is particularly preferably straight chain or branched C₁₋₁₀ alkyl group.

The substituent of the "optionally substituted C₅₋₈ cycloalkenyl groups" for R of the formula (VIII) is completely the same as in the case of the aforementioned "optionally substituted C₃₋₁₀ cycloalkyl group". Specific examples thereof include cycloalkenyl group (particularly cyclopentenyl group, cyclohexenyl group) optionally substituted by 1 to 4 substituents selected from the aforementioned straight chain or branched C₁₋₁₀ alkyl group (particularly preferably C₁₋₈ alkyl group such as methyl group, ethyl group, propyl group, isopropyl group, butyl group, isobutyl group, pentyl group, isopentyl group, 2,2-dimethylpropyl group, 4-methylpentyl group etc.), the aforementioned straight chain or branched C₂₋₁₀ alkenyl group (particularly preferably C₂₋₈ alkenyl group such as 1-methylvinyl group, 2-methylvinyl group, 3-methyl-3-propenyl group etc.), the aforementioned C₃₋₁₀ cycloalkyl group (particularly preferably C₃₋₇ cycloalkyl group such as cyclopropyl group, cyclopentyl group, cyclohexyl group etc.), the aforementioned C₅₋₈ cycloalkenyl group (particularly preferably C₅₋₆ cycloalkenyl group such as cyclopentenyl group, cyclohexenyl group etc.), the aforementioned C₃₋₁₀ cycloalkyl C₁₋₁₀ alkyl group (particularly preferably C₃₋₇ cycloalkyl C₁₋₄ lower alkyl group such as cyclopropylmethyl group, 2-cyclopropylethyl group, 2-cyclopentylethyl group, cyclohexylmethyl group, 2-cyclohexylethyl group etc.), the aforementioned aryl group (particularly preferably phenyl group), oxo group, the aforementioned aralkyl group (particularly preferably phenyl C₁₋₄ lower alkyl group such as benzyl group, phenethyl group etc.) and the aforementioned arylalkenyl group (particularly preferably 2-phenylvinyl group). Preferred are 1-isopropyl-2-cyclopentenyl group, 1-isopropyl-3-cyclopentenyl group, l-isobutyl-2-cyclopentenyl group, l-isobutyl-3-cyclopentenyl group, 1-isopentyl-2-cyclopentenyl group, l-isopentyl-3-cyclopentenyl group, 1-cyclohexylmethyl-2-cyclopentenyl group, 1-cyclohexylmethyl-3-cyclopentenyl group, 1-cyclohexenyl group, 2-cyclohexenyl group, 3-cyclohexenyl group, 1.-methyl-2-cyclohexenyl group, 1-methyl-3-cyclohexenyl group, l-ethyl-2-cyclohexenyl group, l-ethyl-3-cyclohexenyl group, 1-propyl-2-cyclohexenyl group, 1-propyl-3-cyclohexenyl group, 1-isopropyl-2-cyclohexenyl group, 1-isopropyl-3-cyclohexenyl group, 1-butyl,-2-cyclohexenyl group, 1-butyl-3-cyclohexenyl group, 1-isobutyl-2-cyclohexenyl group, 1-isobutyl-3-cyclohexenyl group, 1-pentyl-2-cyclohexenyl group, 1-pentyl-3-cyclohexenyl group, 1-isopentyl-2-cyclohexenyl group, 1-isopentyl-3-cyclohexenyl group, 1-(2,2-dimethylpropyl)-2-cyclohexenyl group, 1-(2,2-dimethylpropyl)-3-cyclohexenyl group, 1-(4-methylpentyl)-2-cyclohexenyl group, 1-(4-methylpentyl)-3-cyclohexenyl group, 1-cyclopropyl-2-cyclohexenyl group, 1-cyclopropyl-3-cyclohexenyl group, 1-cyclohexyl-2-cyclohexenyl group, 1-cyclohexyl-3-cyclohexenyl group, 1-phenyl-2-cyclohexenyl group, 1-phenyl-3-cyclohexenyl.group, 1-cyclopropylmethyl-2-cyclohexenyl group, 1-cyclopropylmethyl-3-cyclohexenyl group, 1-cyclohexylmethyl-2-cyclohexenyl group, 1-cyclohexylmethyl-3-cyclohexenyl group, 1-(2-cyclopropylethyl)-2-cyclohexenyl group, 1-(2-cyclopropylethyl)-3-cyclohexenyl group, 1-(2-cyclopentylethyl)-2-cyclohexenyl group, 1-(2-cyclopentylethyl)-3-cyclohexenyl group, 1-(2-cyclohexylethyl)-2-cyclohexenyl group and 1-(2-cyclohexylethyl)-3-cyclohexenyl group. While the position of substitution is not particularly limited, it is particularly preferably the 1-position. While the substituent may be any of the above-mentioned substituents, it is particularly preferably straight chain or branched C₁₋₁₀ alkyl group or C₃₋₁₀ cycloalkyl C₁₋₄ alkyl group.

The "optionally substituted C₃₋₁₀ cycloalkyl C₁₋₁₀ alkyl groups for R of the formula (VIII) means C₃₋₁₀ cycloalkyl C₁₋₁₀ alkyl group (particularly preferably cyclohexylmethyl group, 1-cyclohexylethyl group, 1-cyclohexyl-l-methylethyl group, 1-cyclahexyl-2-methylpropyl group, 1-cyclohexyl-3-methylbutyl group, 1-cyclohexylhexyl group, l-cyclohexyl-4-methylpentyl group, 1-cyclohexylheptyl group) optionally substituted by 1 to 4 substituents selected from the aforementioned C₃₋₁₀ cycloalkyl group (particularly preferably C₃₋₇ cycloalkyl group such as cyclopentyl group, cyclohexyl group etc.), the aforementioned C₅₋₈ cycloalkenyl group (particularly preferably C₅₋₇ cycloalkenyl group such as cyclopentenyl group and cyclohexenyl group) and the aforementioned aryl group (particularly preferably phenyl group), wherein C₁₋₁₀ alkyl group is straight chain or branched. The position of substitution is not particularly limited, and the straight chain or branched C₁₋₁₀ alkyl group moiety may have a substituent. Preferred are cyclohexylmethyl group, 1-cyclohexylethyl group, cyclohexylcyclopentylmethyl group, dicyclohexylmethyl group, 1-cyclohexyl-1-methylethyl group, 1-cyclohexyl-2-methylpropyl group, 1-cyclohexyl-3-methylbutyl group, 1-cyclohexyl-4-methylpentyl group, 1-cyclohexylhexyl group and 1-cyclohexylheptyl group.

The "optionally substituted aryl group" for R of the formula (VIII) means aryl group (particularly preferably phenyl group) optionally substituted by 1 to 4 substituents selected from the aforementioned straight chain or branched C₁₋₆ alkyl group (particularly preferably tert-butyl group), the aforementioned halogen atom (particularly preferably fluorine atom, chlorine atom) and nitro group. Preferred are phenyl group, 2-chlorophenyl group, 4-nitrophenyl group and 3,5-di-tert-butylphenyl group.

The "optionally substituted aralkyl group" for R of the formula (VIII) means aralkyl group (particularly preferably benzyl group, benzhydryl group, trityl group) optionally substituted by the substituent selected from the aforementioned halogen atom (particularly preferably fluorine atom, chlorine atom), nitro group and hydroxyl group, wherein the C₁₋₄ lower alkyl group is straight chain or branched. The position of substitution is not particularly limited and the straight chain or branched C₁₋₄ lower alkyl group moiety may have said substituent. Preferred are benzyl group and trityl group.

The "optionally substituted 5- or 6-membered heterocyclic group having 1 to 3 from nitrogen atom, oxygen atom and sulfur atom" for R of the formula (VIII) means the aforementioned heterocyclic group optionally substituted by 1 to 4 substituents selected from the aforementioned straight chain or branched C₁₋₆ alkyl group (particularly preferably tert-butyl group), the aforementioned halogen atom (particularly preferably fluorine atom, chlorine atom) and nitro group, with preference given to aromatic heterocyclic group. Particularly preferred are furyl group, thienyl group and pyridyl group.

The "halogen atom for X₁, X₂, X₃, X₄ in the formula (VIII) means halogen atom such as-fluorine atom, chlorine atom, bromine atom and the like, with preference given to fluorine atom and chlorine atom.

The "C₁₋₄ lower alkyl group" for X₁, X₂, X₃, X₄ in the formula (VIII) is preferably methyl group.

The "halogenated C₁₋₄ lower alkyl group" for X₁, X₂, X₃, X₄ in the formula (VIII) is C₁₋₄ lower alkyl group (particularly preferably methyl group) substituted by the aforementioned halogen atom (particularly preferably fluorine atom, chlorine atom), with preference given to trifluoromethyl group.

The "group" of the "C₁₋₄ lower alkoxy group" for X₁, X₂, X₃, X₄ in the formula (VIII) is preferably methoxy group.

In the formula (VIII), the "acyl group" for X₁, X₂, X₃, X₄ is preferably benzoyl group.

In the formula (VIII), the "aryl group" for X₁, X₂, X₃, X₄ is preferably phenyl group.

The "prodrug compound" of the formula (VIII) is a derivative of the compound of the formula (VIII), which has a chemically or metabolically degradable group and shows pharmaceutical activity by hydrolysis or solvent decomposition, or by degradation under physiological conditions.

The "pharmaceutically acceptable salt" of the formula (VIII) may be any as long as it forms a nontoxic salt with a compound represented by the above-mentioned formula (VII). For example, inorganic acid salts such as hydrochloride, hydrobromide, hydroiodide, sulfate, nitrate, phosphate, carbonate, hydrogen carbonate, perchlorate etc.; organic acid salts such as formate, acetate, trifluoroacetate, propionate, oxalate, glycolate, succinate, lactate, maleate, hydroxymaleate, methylmaleate, fumarate, adipate, tartrate, malate, citrate, benzoate, cinnamate, ascorbate, salicylate, 2-acetoxybenzoate, nicotinate, isonicotinate etc.; sulfonates such as methanesulfonate, ethanesulfonate, isethionate, benzenesulfonate, p-toluenesulfonate, naphthalenesulfonate etc.; acidic amino acid salts such as aspartate, glutamate etc.; alkali metal salts such as sodium salt, potassium salt etc.; alkaline earth metal salts such as magnesium salt, calcium salt etc.; ammonium salt; organic base salts such as trimethylamine salt, triethylamine salt, pyridine salt, picoline salt, dicyclohexylamine salt, N,N'-dibenzylethylenediamine salt etc.; and amino acid salts such as lysin salt, arginine salt etc. can be mentioned. In some cases, it may be a hydrate or a solvate with alcohol and the like.

In addition, the compounds disclosed in WO2004/020393 and Patent Application No. 2003-308156 can be mentioned.

A dibenzylamine compound represented by the formula (IX) 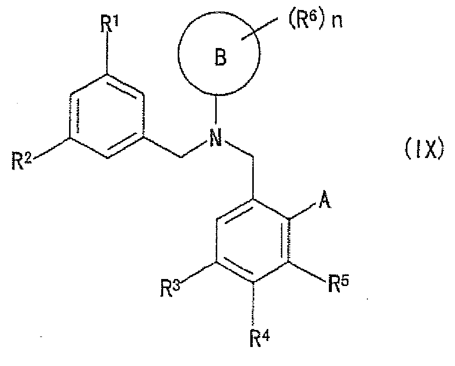 wherein
- R¹ and R²: are the same or different and each is a halogen atom, a nitro group, a cyano group or a C₁₋₆ alkyl group optionally substituted by halogen atom;
- R³, R⁴ and R⁵: are the same or different and each is a hydrogen atom, a halogen atom, a C₁₋₆ alkyl group optionally substituted by halogen atom, a C₁₋₆ alkylthio group optionally substituted by halogen atom or a C₁₋₆ alkoxy group optionally substituted by halogen atom, or R³ and R⁴ or R⁴ and R⁵ may form, together with a carbon atom they are bonded to, a homocyclic ring optionally having substituent(s) or a heterocyclic ring optionally having substituent(s);
- A: is -N(R⁷)(R⁸) (wherein R⁷ and R⁸ are the same or different and each is a hydrogen atom, a C₁₋₆ alkyl group (wherein C₁₋₆ alkyl group is optionally substituted by phenyl group or -(CH₂)ₘ-COOR⁹ (wherein R⁹ is a hydrogen atom or a C₁₋₆ alkyl group and m is 0 or an integer of 1 to 5)) or a C₄₋₁₀ cycloalkylalkyl group (wherein C₄₋₁₀ cycloalkylalkyl group is optionally substituted by 1 to 3 substituents from halogen atom, nitro group, amino group, hydroxyl group, cyano group, acyl group, C₁₋₆ alkoxy group, C₁₋₆ alkyl group (wherein C₁₋₆ alkyl group is optionally substituted by hydroxyl group, C₁₋₆ alkoxy group or phosphono group), -(CH₂)_{q}-CON(R²⁰)(R²¹) (wherein R²⁰ and R²¹ are the same or different and each is hydrogen atom or C₁₋₆ alkyl group and q is 0 or an integer of 1 to 5) or -(CH₂)ᵣ-COOR¹⁰ (wherein R¹⁰ is hydrogen atom or C₁₋₆ alkyl group and r is 0 or an integer of 1 to 5)), -C(R¹¹) (R¹²)(R¹³) (wherein R¹¹, R¹² and R¹³ are the same or different and each is a hydrogen atom, a C₁₋₆ alkyl group (wherein C₁₋₆ alkyl group is optionally substituted by phenyl group or -COOR⁹ (wherein R⁹ is as defined above)) or a C₄₋₁₀ cycloalkylalkyl group (wherein C₄₋₁₀ cycloalkylalkyl, group is optionally substituted by 1 to 3 substituents from halogen atom, nitro group, amino group, hydroxyl group, cyano group, acyl group, C₁₋₆ alkoxy group, C₁₋₆ alkyl group (wherein C₁₋₆ alkyl group is optionally substituted by hydroxyl group, C₁₋₆ alkoxy group or phosphono group), -(CH₂)_{q}-CON(R²⁰)(R²¹) (wherein R²⁰, R²¹ and q are as defined above) or -(CH₂)ᵣ-COOR¹⁰ (wherein R¹⁰ and r are as defined above))) or -O-C(R¹¹)(R¹²)(R¹³) (wherein R¹¹, R¹² and R¹³ are as defined above);
- ring B: is an aryl group or a heterocyclic residue;
- R⁶: is a hydrogen atom, a halogen atom, a nitro group, an amino group, a hydroxyl group, a cyano group, an acyl group, a C₁₋₆ alkoxy group, a C₂₋₆ alkenyl group or a C₁₋₆ alkyl group (wherein C₁₋₆ alkyl group is optionally substituted by hydroxyl group or -COOR¹⁴ (wherein R¹⁴ is hydrogen atom or C₁₋₆ alkyl, group)); and
- n: is an integer of 1 to 3
or a prodrug thereof or a pharmaceutically acceptable salt thereof.

The "halogen atom" in the formula (IX) is a chlorine atom, a bromine atom, a fluorine atom and the like. The halogen atom for R¹, R², R³, R⁴, R⁵ or R⁶ is preferably a chlorine atom or a fluorine atom and preferable halogen atom as a substituent of C₄₋₁₀ cycloalkylalkyl group for R⁷, R⁸, R¹¹, R¹² or R¹³ is a chlorine atom or a fluorine atom.

The "C₂₋₆ alkenyl group" in the formula (IX) is a straight chain or branched alkenyl group having 2 to 6 carbon atoms. Examples thereof include ethenyl group (vinyl graup), 1-propenyl group, 2-propenyl group (allyl group), isopropenyl group, 1-butenyl group, 2-butenyl group, 3-butenyl group, 1-methyl-1-propenyl group, 1-methyl-2-propenyl group, 2-methyl-2-propenyl group, 1-ethylvinyl group, 1-pentenyl group, 2-pentenyl group, 3-pentenyl group, 4-pentenyl group, 1,2-dimethyl-1-propenyl group, 1,2-dimethyl-2-propenyl group, 1-ethyl-1-propenyl group, 1-ethyl-2-propenyl group, 1-methyl-1-butenyl group, 1-methyl-2-butenyl group, 2-methyl-1-butenyl group, 1-isopropylvinyl group, 2,4-pentadienyl group, 1-hexenyl group, 2-hexenyl group, 3-hexenyl group, 4-hexenyl group, 5-hexenyl group, 2,4-hexadienyl group and 1-methyl-l-pentenyl group and the like, with preference given to alkenyl group having 2 to 4 carbon atoms, which may be a straight chain or branched. Particularly preferred are ethenyl group, isopropenyl group and 2-methyl-2-propenyl group.

The "C₁₋₆ alkyl group" in the formula (IX) is a straight chain or branched alkyl, group having 1 to 6 carbon atoms. Examples thereof include methyl group, ethyl group, propyl group, isopropyl group, butyl group, isobutyl group, sec-butyl group, tert-butyl group, pentyl group, isopentyl group, neopentyl group, tert-pentyl group, hexyl group and the like, with preference given to straight chain and branched allyl group having 1 to 4 carbon atoms. Particularly preferred are methyl group, ethyl group and isopropyl group. For R²⁰ or R²¹, it is preferably methyl group. For R⁶, it is preferably methyl group or ethyl group, for R⁷ R⁸, R¹¹, R¹² or R¹³, it is preferably ethyl group, propyl, group or butyl group, and for R⁹ or R¹⁰, it is preferably methyl group or ethyl group. Preferable C₁₋₆ alkyl group as a substituent of C₄₋₁₀ cycloalkylalkyl group for R⁷, R⁸, R¹¹, R¹² or R¹³ is methyl group or ethyl group. For R¹⁴, it is preferably methyl group or ethyl group.

The "C₁₋₆ alkyl group optionally substituted by halogen atom" in the formula (IX) is the aforementioned C₁₋₆ alkyl group optionally substituted by the aforementioned halogen atom. Examples thereof include methyl group, ethyl group, propyl group, isopropyl group, butyl group, isobutyl group, sec-butyl group, tert-butyl group, pentyl group, isopentyl group, neopentyl group, tert-pentyl group, hexyl group, trifluoromethyl group, 1- or 2-chloroethyl group, 1-or 2-bromoethyl group, 1- or 2-fluoroethyl group, 1-, 2- or 3-chloropropyl group, 1-, 2- or 3-bromopropyl group, 1-, 2-or 3-fluoropropyl group, 1-, 2-, 3- or 4-chlorobutyl group, 1-, 2-, 3- or 4-bromobutyl group, 1-, 2-, 3- or 4-fluorobutyl group and the like, with preference given to methyl group, ethyl group and trifluoromethyl group. For R¹, R², R³, R⁴ or R⁵, it is preferably methyl group, ethyl group or trifluoromethyl group.

The "C₁₋₆ alkoxy group" in the formula (IX) is a straight chain or branched alkoxy group having 1 to 6 carbon atoms. Examples thereof include methoxy group, ethoxy group, propoxy group, isopropoxy group, butoxy group, tert-butoxy group, pentyloxy group, tert-pentyloxy group and hexyloxy group, with preference given to methoxy group, ethoxy group, isopropoxy group, butoxy group and tert-butoxy group, all having 1 to 4 carbon atoms. Particularly preferred are methoxy group and ethoxy group. For R⁶, it is preferably methoxy group. Preferable C₁₋₆ alkoxy group as a substituent of C₄₋₁₀ cycloalkylalkyl, group for R⁷, R⁸, R¹¹, R¹² or R¹³ is methoxy group or methoxy group.

The "C₁₋₆ alkoxy group optionally substituted by halogen atom" in the formula (IX) is the aforementioned C₁₋₆ alkoxy group optionally substituted by the aforementioned halogen atom. Examples thereof include methoxy group, ethoxy group, propoxy group, isopropoxy group, butoxy group, tert-butoxy group, pentyloxy group, tert-pentyloxy group, hexyloxy group, trifluoromethoxy group, 1- or 2-chloroethoxy group, 1- or 2-bromoethoxy group, 1- or 2-fluoroethoxy group, 1-, 2- or 3-chloropropoxy group, 1-, 2-or 3-bromopropoxy group, 1-, 2- or 3-fluoropropoxy group, 1-, 2-, 3- or 4-chlorobutoxy group, 1-, 2-, 3- or 4-bromobutoxy group, 1-, 2-, 3- or 4-fluorobutoxy group and the like, with preference given to methoxy group, ethoxy group and trifluoromethoxy group. For R³, R⁴ or R⁵, it is preferably methoxy group, ethoxy group or trifluoromethoxy group.

The "C₁₋₆ alkylthio group optionally substituted by halogen atom" in the formula (IX) is C₁₋₆ alkylthio group optionally substituted by the aforementioned halogen atom. Examples thereof include methylthio group, ethylthio group, propylthio group, isopropylthio group, butylthio group, tert-butylthio group, pentylthio group, tert-pentylthio group, hexylthio group, trifluoromethylthio group, 1- or 2-chloroethylthio group, 1- or 2-bromoethylthio group, 1- or 2-fluoroethylthio group, 1-, 2- or 3-chloropropylthio group, 1-, 2- or 3-bromopropylthio group, 1-, 2- or 3-fluoropropylthio group, 1-, 2-, 3- or 4-chlorobutylthio group, 1-, 2-, 3- or 4-bromobutylthio group, 1-, 2-, 3- or 4-fluorobutylthio group and the like, with preference given to methylthio group, ethylthio group and trifluoromethylthio group. For R³, R⁴ or R⁵, it is preferably methylthio group, ethylthio group or trifluoromethylthio group.

The "C₄₋₁₀ cycloalkylalkyl group" in the formula (IX) is C₁₋₃ alkyl group substituted by C₃₋₇ cycloalkyl group. As used herein, the "C₃₋₇ cycloalkyl group" means a cycloalkyl group having 3 to 7 carbon atoms. Specific examples thereof include cyclopropyl group, cyclobutyl group, cyclopentyl group, cyclohexyl group and cycloheptyl group, with preference given to cycloalkyl group having 3 to 6 carbon atoms. Specific examples thereof include cyclopropyl group, cyclobutyl group, cyclopentyl group and cyclohexyl group. The "C₁₋₃ alkyl group" is a straight chain or branched alkyl group having 1 to 3 carbon atoms. Examples thereof include methyl group, ethyl group, propyl group and isopropyl group, with preference given to methyl group, ethyl group and propyl group.

Specific examples of the "C₄₋₁₀ cycloalkylalkyl group" in the formula (IX) include cyclopropylmethyl group, cyclobutylmethyl group, cyclopentylmethyl group, cyclohexylmethyl group, cycloheptylmethyl group, cyclopentylethyl group (1- or 2-(cyclopentyl)ethyl group), cyclohexylethyl group (1- or 2-(cyclohexyl)ethyl group), cyclopentylpropyl group (1-, 2- or 3-(cyclopentyl)propyl group) and cyclohexylpropyl group (1-, 2- or 3-(cyclohexyl)propyl group). Preferred is cycloalkylalkyl group having 3 to 7 carbon atoms. Specific examples thereof include cyclopropylmethyl group, cyclobutylmethyl group, cyclopentylmethyl group and cyclohexylmethyl group. Preferable C₄₋₁₀ cycloalkylalkyl group for R⁷, R⁸, R¹¹, R¹² or R¹³ is cyclopentylmethyl group, cyclohexylmethyl group or 2-(cyclopentyl)ethyl group.

The "acyl group" in the formula (IX) is an alkylcarbonyl group such as acetyl group, propionyl group, butyryl group, pivaloyl group and the like; or an arylcarbonyl group such as benzoyl group, naphthoyl and the like, with preference given to acetyl group. For R⁶, it is preferably acetyl group. Preferable acyl group as a substituent of C₄₋₁₀ cycloalkylalkyl group for R⁷, R⁸, R¹¹, R¹² or R¹³ is acetyl group.

The "aryl group" in the formula (IX) is phenyl group, naphthyl group, biphenyl group and the like, with preference given to phenyl group.

The "heterocyclic residue" in the formula (IX) is a 5- to 8-membered aromatic heterocyclic group having 1 to 4 heteroatoms selected from oxygen atom, sulfur atom and nitrogen atom, and the like other than carbon atom, and bicyclic or tricyclic condensed heterocyclic group fused thereto and the like. Examples thereof include pyrrolyl group (1-, 2- or 3-pyrrolyl group), furyl group (2- or 3-furyl group), thienyl group (2- or 3-thienyl group), imidazolyl group (1-, 2-, 4- or 5-imidazolyl group), oxazolyl group (2-, 4- or 5-oxazolyl group), thiazolyl group (2-, 4- or 5-thiazolyl group), pyrazolyl group (1-, 3-, 4- or 5-pyrazolyl group), isoxazolyl group (3-, 4- or 5-isoxazolyl group), isothiazolyl group (3-, 4- or 5-isothiazolyl group), oxadiazolyl group (1,2,4-oxadiazol-3 or 5-yl group, 1,3,4-oxadiazol-2-yl group, 1,2,5-oxadiazol-3-yl group), thiadiazolyl group (1,2,4-thiadiazol-3 or 5-yl group, 1,3,4-thiadiazol-2-yl group, 1,2,5-thiadiazol-3-yl group), triazolyl group (1,2,4-triazol-1, 3, 4 or 5-yl group, 1,2,3-triazol-1, 2 or 4-yl group), indolyl group (1-, 2-, 3-, 4-, 5-, 6- or 7-indolyl group), benzofuryl group (2-, 3-, 4-, 5-, 6- or 7-benzofuryl group), benzothienyl group (2-, 3-, 4-, 5-, 6- or 7-benzothienyl group), benzimidazolyl group (1-, 2-, 4-, 5-, 6- or 7-benzimidazolyl group), benzoxazolyl group (2-, 4-, 5-, 6-or 7-benzoxazolyl group), benzothiazolyl group (2-, 4-, 5-, 6- or 7-benzothiazolyl group), pyridyl group (2-, 3- or 4-pyridyl group), pyridine-1-oxide group (2-, 3- or 4-pyridine-1-oxide group), pyrimidinyl group (2-, 4- or 5-pyrimidinyl group), tetrazolyl group (IH-tetrazol-1 or 5-yl group, 2H-tetrazol-2 or 5-yl group), quinolyl group (2-, 3-, 4-, 5-, 6-, 7- or 8-quinolyl group), isoquinolyl group (1-, 3-, 4-, 5-, 6-, 7- or 8-isoquinolyl group) and the like.

As the homocyclic ring" in the formula (IX), optionally condensed 3- to 7-membered carbocyclic ring and the like can be used. Examples thereof include C₆₋₁₀ arene (C₆₋₁₀ aryl) (e.g., benzene (phenyl), naphthalene (naphthyl) etc.), C₃₋₇ cycloalkane (C₃₋₇ cycloalkyl) (e.g., cyclopropane (cyclopropyl), cyclobutane (cyclobutyl), cyclopentane (cyclopentyl), cyclohexane (cyclohexyl), cycloheptane (cycloheptyl) etc.), C₃₋₇ cycloalkene (C₃₋₇ cycloalkenyl) (e.g., cyclopropene (cyclopropenyl), cyclobutene (cyclobutenyl), cyclopentene (cyclopentenyl), cyclohexene (cyclohexenyl), cycloheptane (cycloheptenyl) etc.) and the like. The parenthesis attached to each homocyclic ring shows a homocyclic group corresponding to the homocyclic ring.

As the substituent that the above-mentioned homocyclic ring may have, for example, (1) C₁₋₆ alkyl group optionally substituted by halogen (of which C₁₋₆ alkyl group substituted by halogen is preferable.), (2) C₃₋₁₀ cycloalkyl group, (3) C₂₋₁₀ alkenyl group, (4) C₂₋₁₀ alkynyl group, (5) C₆₋₁₀ aryl group, (6) C₇₋₂₀ aralkyl group, (7) nitro group, (8) hydroxy group, (9) mercapto group, (10) oxo group, (11) thioxo group, (12) cyano group, (13) carbamoyl group, (14) carboxyl group, (15) C₁₋₆ alkoxycarbonyl group (e.g., ethoxycarbonyl group, ethoxycarbonyl group etc.), (16) sulfo, (17) halogen atom, (18) C₁₋₆ alkoxy group, (19) C₆₋₁₀ aryloxy group (e.g., phenoxy group etc.), (20) C₁₋₆ aryloxy group (e.g. , acetoxy, propionyloxy), (21) C₁₋₅ alkylthio group (e.g., methylthio group, ethylthio group, n-propylthio group, isopropylthio group, n-butylthio group, tert-butylthio group etc.), (22) C₆₋₁₀ arylthio, group (e.g., phenylthio group etc.), (23) C₁₋₆ alkylsulfinyl group (e.g., methylsulfinyl group, ethylsulfinyl group etc.), (24) C₆₋₁₀ arylsulfinyl group (e.g., phenylsulfinyl group etc.), (25) C₁₋₆ alkylsulfonyl group (e.g., methylsulfonyl group, ethylsulfonyl group etc.), (26) C₆₋₁₀ arylsulfonyl group (e.g., phenylsulfonyl group etc.), (27) amino group, (28) C₁₋₆ acylamino group (e.g., acetylamino group, propionylamino group etc.), (29) mono- or di-C₁₋₄ alkylamino group (e.g., methylamino group, ethylamino group, n-propylamino group, isopropylamino group, n-butylamino group, dimethylamino group, diethylamino group etc.), (30) C₃₋₈ cycloalkylamino group (e.g., cyclopropylamino group, cyclobutylamino group, cyclopentylamino group, cyclohexylamino group etc.), (31) C₆₋₁₀ arylamino group (e.g., anilino etc.), (32) C₁₋₆ alkanoyl group (e.g., formyl group, acetyl group, hexanoyl group etc.), (33) C₆₋₁₀ arylcarbonyl group (e.g., benzoyl group etc.), (34) 5- or 6-membered heterocyclic group (e.g., 2- or 3-thienyl group, 2- or 3-furyl group, 3-, 4- or 5-pyrazolyl group, 2-, 4- or 5-thiazolyl group, 3-, 4- or 5-isothiazolyl group, 2-, 4-or 5-oxazolyl group, 3-, 4- or 5-isoxazolyl group, 2-, 4-or 5-imidazolyl group, 1,2,3- or 1,2,4-triazolyl (1,2,4-triazol-1,3,4 or 5-yl group, 1,2,3-triazol-1, 2 or 4-yl group), 1H or 2H-tetrazolyl (1Fi-tetrazol-1 or 5-yl group, 2H-tetrazol-2 or 5-yl group), 2-, 3- or 4-pyridyl group, 2-, 4- or 5-pyrimidyl group, 3- or 4-pyridazinyl group, quinolyl group (2-, 3-, 4-, 5-, 6-, 7- or 8-quinolyl group), isoquinolyl group (1-, 3-, 4-, 5-, 6-, 7- or 8-isoquinolyl group), indolyl group (1-, 2-, 3-, 4-, 5-, 6- or 7-indolyl group) etc.) having 1 to 4 heteroatoms selected from oxygen, sulfur, nitrogen and the like other than carbon atom, and the like can be mentioned. The number of substitutions is 1 to 6, preferably 1 to 3, more preferably 1 or 2.

Preferable examples of the "homocyclic ring optionally having substituents" in the formula (IX), which may be formed by R³ and R⁴ or R⁴ and R⁵ together with a carbon atom bonded thereto is C₃₋₇ cycloalkane and benzene, more preferably cyclopentane and cyclohexane.

As the "heterocyclic ring" in the formula (IX), a 5-to 8-membered heterocyclic ring having 1 to 4 heteroatoms selected from oxygen atom, sulfur atom and nitrogen atom and the like other than carbon atom, and bicyclic or tricyclic condensed heterocyclic ring fused thereto and the like can be mentioned. Specific examples of the heterocyclic ring include (1) 5-membered heterocyclic ring having 1 to 4 heteroatoms selected from oxygen atom, sulfur atom, nitrogen atom and the like other than carbon atom, such as thiophene (thienyl group), furan (furyl group), pyrrole (pyrrolyl group), pyrroline (pyrrolinyl group), pyrrolidine (pyrrolidinyl group), 1,3-dioxole (1,3-dioxolyl group), oxazole (oxazolyl group), thiazole (thiazolyl group), pyrazole (pyrazolyl group), imidazole (imidazolyl group), imidazoline (imidazolinyl group), isoxazole (isoxazolyl group), isothiazole (isothiazolyl group), furazan (furazanyl group), 1,2,3-thiadiazole (1,2,3-thiadiazolyl group), 1,2,5-thiadiazole (1,2,5-thiadiazolyl group), 1,2,3-triazole (1,2,3-triazolyl group), 1,2,3-triazolidine (triazolidinyl group) and the like; (2) 6-membered heterocyclic ring having 1 to 4 heteroatoms selected from oxygen atom, sulfur atom, nitrogen atom and the like other than carbon atom, such as pyridine (pyridyl group), pyrimidine (pyrimidinyl, group), thiomorpholine (thiomorpholinyl group), morpholine (morpholinyl group), 1,2,3-triazine, 1,2,4-triazine (triazinyl group), piperidine (piperidinyl group), pyran (pyranyl group), thiopyran (thiopyranyl group), 1,4-oxazine (1,4-oxazinyl group), 1,4-dioxane (1,4-dioxanyl group), 1,4-thiazine (1,4-thiazinyl group), 1,3-thiazine 1,3-thiazinyl group), piperazine (piperazinyl group), oxotriazine (oxotriazinyl group), pyridazine (pyridazinyl group), pyrazine (pyrazinyl group) and the like can be mentioned. As the bicyclic or tricyclic condensed heterocyclic ring, a bicyclic or tricyclic condensed heterocyclic ring having 1 to 4 heteroatoms selected from oxygen atom, sulfur atom, nitrogen atom and the like other than carbon atom can be mentioned. Examples thereof include benzofuran (benzofuryl group), benzothiazole (benzothiazolyl group), benzoxazole (benzoxazolyl group), tetrazolo[1,5-b]pyridazine (tetrazolo[1,5-b]pyridazinyl group), triazolo[4,5-b]pyridazine (triazaolo[4,5-b]pyridazinyl group), benzimidazole (benzimidazolyl group), quinoline (quinolyl group), isoquinoline (isoquinolyl group), cinnoline (cinnolinyl group), phthalazine (phthalazinyl group), quinazoline (quinazolinyl group), quinoxaline (quinoxalinyl group), indolizine (indolizinyl group), indole (indolyl group), quinolizidine (quinolizinyl group), 1,8-naphthyridine (1,8-naphthyridinyl group), pteridine (pteridinyl group), dibenzofuran (dibenzofuranyl group), carbazole (carbazolyl group), acridine (acrydinyl group), phenanthridine (phenanthridinyl group), chromane (chromanyl group), benzoxazine (benzoxazinyl group), phenazine (phenazinyl group), phenothiazine (phenothiazinyl group), phenoxazine (phenoxazinyl group) and the like. The parenthesis attached to each heterocyclic ring shows a heterocyclic group corresponding to the heterocyclic ring.

As the substituent that the above-mentioned heterocyclic ring may have, for example, (1) C₁₋₆ alkyl group, (2) C₂₋₆ alkenyl group, (3) C₂₋₆ alkynyl group, (4) C₃₋₆ cycloalkyl group, (5) cycloalkenyl group, (6) C₇₋₁₁ aralkyl group, (7) C₆₋₁₄ aryl group, (8) C₁₋₆ alkoxy group, (9) C₆₋₁₄ aryloxy group (e.g., phenoxy group etc.), (10) C₁₋₆ alkanoyl group (e.g., formyl group, acetyl group, propionyl group, n-butyryl group, iso-butyryl group etc.), (11) C₆₋₁₄ arylcarbonyl group (e.g., benzoyl group etc.), (12) C₁₋₆ alkanoyloxy group (e.g., formyloxy group, acetyloxy group, propionyloxy group, n-butyryloxy group, iso-butyryloxy group etc.), (13) C₆₋₁₄ arylcarbonyloxy group (e.g., benzoyloxy group etc.), (14) carboxyl group, (15) C₁₋₆ alkoxycarbonyl group (e.g., methoxycarbonyl group, ethoxycarbonyl group, n-propoxycarbonyl group, isopropoxycarbonyl group, n-butoxycarbonyl group, isobutoxycarbonyl group, tert-butoxycarbonyl group etc.), (16) carbamoyl group, (17) N-mono-C₁₋₄ alkylcarbamoyl group (e.g., N-methylcarbamoyl group, N-ethylcarbamoyl group, N-propylcarbamoyl group, N-isopropylcarbamoyl group, N-butylcarbamoyl group etc.), (18) N,N-di-C₁₋₄ alkylcarbamoyl group (e.g., N,N-dimethylcarbamoyl group, N,N-diethylcarbamoyl group, N,N-dipropylcarbamoyl group, N,N-dibutylcarbamoyl group etc.), (19) cyclic aminocarbonyl group (e.g., 1-aziridinylcarbonyl group, 1-azetidinylcarbonyl group, 1-pyrrolidinylcarbonyl group, 1-piperidinylcarbonyl group, N-methylpiperazinylcarbonyl group, morpholinocarbonyl group etc.), (20) halogen atom, (21) C₁₋₆ alkyl group optionally substituted by halogen atom (e.g., chloromethyl group, dichloromethyl group, trifluoromethyl group, trifluoroethyl group etc.), (22) oxo group, (23) amidino group, (24) imino group, (25) amino group, (26) mono- or di-C₁₋₄ alkylamino group (e.g., methylamino group, ethylamino group, propylamino group, isopropylamino group, butylamino group, dimethylamino group, diethylamino group, dipropylamino group, diisopropylamino group, dibutylamino group etc.), (27) 3- to 6-membered cyclic amino group (e.g., aziridinyl group, azetidinyl group, pyrrolidinyl group, pyrrolinyl group, pyrrolyl group, imidazolyl group, pyrazolyl group, imidazolidinyl group, piperidino group, morpholino group, dihydropyridyl group, pyridyl group, N-methylpiperazinyl group, N-ethylpiperazinyl group etc.) which may have 1 to 3 heteroatoms selected from oxygen atom, sulfur atom, nitrogen atom and the like other than carbon atom and one nitrogen atom, (28) C₁₋₆ alkanoylamino group (e.g., formamide group, acetamide group, trifluoroacetamide group, propionyl amide group, butyryl amide group, isobutyryl amide group etc.), (29) benzamide group, (30) carbamoylamino group, (31) N-C₁₋₄ alkylcarbamoylamino group (e.g., N-methylcarbamoylamino group, N-ethylcarbamoylamino group, N-propylcarbamoylamino group, N-isopropylcarbamoylamino group, N-butylcarbamoylamino group etc.), (32) N,N-di-C₁₋₄ alkylcarbamoylamino group (e.g., N,N-dimethylcarbamoylamino group, N,N-diethylcarbamoylamino group, N,N-dipropycarbamoylamino group, N,N-dibutylcarbamoylamino group etc.), (33) C₁₋₃ alkylenedioxy group (e.g., methylenedioxy group, ethylenedioxy group etc.), (34) -B(OH)₂, (35) hydroxy group, (36) epoxy group (-O-), (37) nitro group, (38) cyano group, (39) mercapto group, (40) sulfo group, (41) sulfino group, (42) phosphono group, (43) sulfamoyl group, (44) C₁₋₆ alkylsulfamoyl group (e.g., N-methylsulfamoyl group, N-ethylsulfamoyl group, N-propylsulfamoyl group, N-isopropylsulfamoyl group, N-butylsulfamoyl group etc.), (45) di-C₁₋₆ alkylsulfamoyl group (e.g., N,N-dimethylsulfamoyl group, N,N-diethylsulfamoyl group, N,N-dipropylsulfamoyl group, N,N-dibutylsulfamoyl group etc.), (46) C₁₋₆ alkylthio group (e.g., methylthio group, ethylthio group, propylthio group, isopropylthio group, n-butylthio group, sec-butylthio group, tert-butylthio group etc.), (47) phenylthio group, (48) C₁₋₆ alkylsulfinyl group (e.g., methylsulfinyl group, ethylsulfinyl group, propylsulfinyl group, butylsulfinyl group etc.), (49) phenylsulfinyl group, (50) C₁₋₆ alkylsulfonyl group (e.g., methylsulfonyl group, ethylsulfonyl group, propylsulfonyl group, butylsulfonyl group etc.), (51) phenylsulfonyl group and the like can be mentioned. The number of substitutions is 1 to 6, preferably 1 to 3, more preferably 1 to 2.

Preferable examples of the "heterocyclic ring optionally having substituents" in the formula (IX), which may be formed by R³ and R⁴ or R⁴ and R⁵ together with a carbon atom bonded thereto, include thiophene, furan, pyrrole, pyrroline, oxazole, thiazole, pyrazole, imidazole, imidazoline, isoxazole, isothiazole, furazan, 1,2,3-thiadiazole, 1,2,5-thiadiazole, 1,2,3-triazole, 1,2,3-triazine, 1,2,4-triazine, 1,2,3-triazolidine, 2,2-difluoro-1,3-dioxole and 2,2,3,3-tetrafluoro1,4-dioxane.

In the above-mentioned embodiment of the formula (IX), it is preferable that the substituent in the "homocyclic ring optionally having substituents" and the substituent in the "heterocyclic ring optionally having substituents" are selected from the group consisting of (1) C₁₋₆ alkyl group optionally substituted by halogen atom, (2) nitro group, (3) hydroxy group, (4) mercapto group, (5) cyano group, (6) carbamoyl group, (7) carboxyl group, (8) C₁₋₆ alkoxycarbonyl group, (9) sulfo group, (10) halogen atom, (11) C₁₋₆ alkoxy group, (12) C₁₋₆ alkylthio group, (13) C₁₋₆ alkylsulfinyl group, (14) C₁₋₆ alkylsulfonyl group, (15) amino group, (16) mono- or di-C₁₋₄ alkylamino group, (17) C₁₋₆ alkanoyl group and (18) C₁₋₆ alkanoyloxy group .

The "pharmaceutically acceptable salt" of the formula (IX) may be any as long as it forms a nontoxic salt with a compound represented by the above-mentioned formula (IX). Examples thereof include, but not limited to, inorganic acid salts such as hydrochloride, hydrobromide, hydroiodide, sulfate, nitrate, phosphate, carbonate, hydrogen carbonate, perchlorate etc.; organic acid salts such as formate, acetate, trifluoroacetate, propionate, oxalate, glycolate, succinate, lactate, maleate, hydroxymaleate, methylmaleate, fumarate, adipate, tartrate, malate, citrate, benzoate, cinnamate, ascorbate, salicylate, 2-acetoxybenzoate, nicotinate, isonicotinate etc.; sulfonates such as methanesulfonate, ethanesulfonate, isethionate, benzenesulfonate, p-toluenesulfonate, naphthalenesulfonate etc.; acidic amino acid salts such as aspartate, glutamate etc.; alkali metal salts such as sodium salt, potassium salt etc.; alkaline earth metal salts such as magnesium salt, calcium salt etc.; ammonium salt; organic base salts such as trimethylamine salt, triethylamine salt, pyridine salt, picoline salt, dicyclohexylamine salt, N,N'-dibenzylethylenediamine salt etc.; and amino acid salts such as lysin salt, arginine salt etc. In some cases, it may be a water-containing product, a hydrate or a solvate with alcohol and the like.

In the compound represented by the above-mentioned formula (IX), various isomers can be present. Examples thereof include E form and Z form as geometric isomers, and, when an asymmetric carbon atom exists, enantiomer and diastereomer are present as stereoisomers based thereon, and tautomer can be present. Accordingly, the compound of the formula (IX) encompasses all these isomers and a mixture thereof.

Here, the compound of the formula (IX) also encompasses prodrug compounds and metabolites.

The "prodrug compound" of the formula (IX) is a derivative of the compound of the formula (IX), which has a chemically or metabolically degradable group and restores to the original compound to show an intrinsic efficacy after administration to a living organism. It includes a complex and a salt not based on a covalent bond. As a prodrug compound of the compound represented by the formula (IX) of the present invention, a compound represented by the formula (IX) wherein a carboxyl group has been modified by ethyl group, pivaloyloxymethyl group, 1-(acetyloxy)ethyl group, 1-(ethoxycarbonyloxy)ethyl group, 1-(cyclohexyloxycarbonyloxy)ethyl group, carboxylmethyl group, (5-methyl-2-oxo-1,3-dioxol-4-yl)methyl group, phenyl group, o-tolyl group and the like; a compound represented by the formula (IX), wherein a hydroxyl group has been modified by acetyl group, propionyl group, isobutyryl group, pivaloyl group, benzoyl group, 4-methylbenzoyl group, dimethylcarbamoyl group or sulfo group; a compound represented by the formula (IX), wherein an amino group has been modified by hexylcarbamoyl group, 3-methylthio-1-(acetylamino)propylcarbonyl group, 1-sulfo-1-(3-ethoxy-4-hydroxyphenyl)methyl group, (5-methyl-2-oxo-1,3-dioxol-4-yl)methyl group and the like, and the like can be mentioned.

Specifically, the following compound disclosed in JP-A-2003-221376 can be mentioned:
a compound represented by the formula (X) 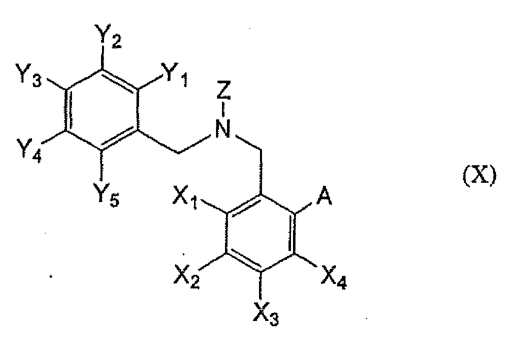
wherein X₁-X₄ and Y₁-Y₅ are the same or different and each is a hydrogen atom or any substituent, Z is the formula (XI); -Z₁-Z₂ wherein Z₁ is -CO-, -CS- or -SO₂- Z₂ is a hydrogen atom, an optionally substituted hydrocarbon group having 1 to 20 carbon atoms, an optionally substituted amino group, an optionally substituted alkoxy group or an optionally substituted alkylthio group, A is a disubstituted amino group, an optionally substituted alkoxy group, an optionally substituted alkylthio group or an optionally substituted hydrocarbon group having 1 to 20 carbon atoms, or X₂ and X₃, X₃ and X₄ or X₄ and A may be taken together to form a homocyclic ring group optionally having substituents or a heterocyclic group optionally having substituents, or a salt thereof.

Each term used for the formulas (X) and (XI) is as described in the above-mentioned publication.

Specifically, the following compound disclosed in WO00/18724 (JP-T-2002-525351) can be mentioned:
a compound having the formula (XII): 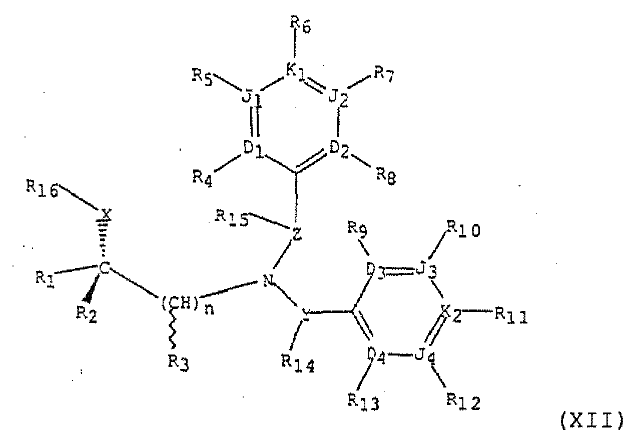 or a pharmaceutically acceptable salt thereof,
   wherein
n is an integer selected from 1 through 4:
X is oxy;
R₁ is selected from the group consisting of haloalkyl, haloalkenyl, haloalkoxymethyl, and haloalkenyloxymethyl with the proviso that R₁ has a higher Cahn-Ingold-Prelog stereochemical system ranking than both R₂ and (CHR₃)ₙ-N(A)Q
wherein A is the formula (XIII) and Q is the formula (XIV); 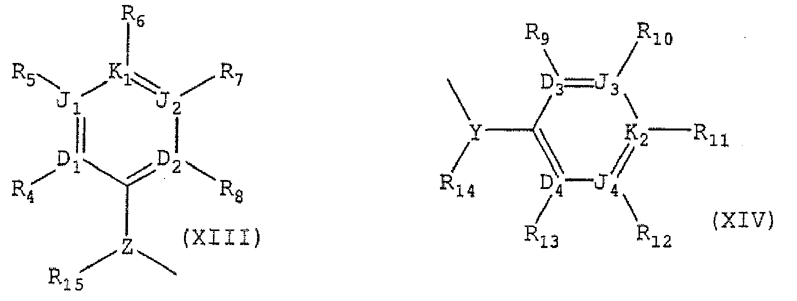
R₁₆ is selected from the group consisting of hydrido; alkyl; acyl; aroyl; heteroaroyl; trialkylsilyl; and a spacer selected from the group consisting of a covalent single bond and a linear spacer moiety having a chain length of 1 to 4 atoms linked to the point of bonding of any aromatic substituent selected from the group consisting of R₄, R₈, R₉ and R₁₃ to form a heterocyclyl, ring having from 5 through 10 continuous members:
D₁, D₂, J₁, J₂ and K₁ are independently selected from the group consisting of C, N, O, S and a covalent bond with the proviso that no more than one of D₁, D₂, J₁, J₂ and K₁ is a covalent bond; no more than one of D₁, D₂, J₁, J₂ and K₁ is O; no more than one of D₁, D₂, J₁, J₂ and K₁ is S; one of D₁, D₂, J₁, J₂ and K₁ must be a covalent bond when two of D₁, D₂, J₁, J₂ and K₁ are O and S; and no more than four of D₁ , D₂, J₁, J₂ and K₁ are N;
D₃, D₄, J₃, J₄ and K₂ are independently selected from the group consisting of C, N, O, S and a covalent bond with the proviso that no more than one of D₃, D₄, J₃, J₄ and K₂ is a covalent bond; no more than one of D₃, D₄, J₃, J₄ and K₂ is O; no more than one of D₃, D₄, J₃, J₄ and K₂ is S; no more than two of D₃, D₄, J₃, J₄ and K₂ are O and S; one of D₃, D₄, J₃, J₄ and K₂ must be a covalent bond when two of D₃, D₄, J₃, J₄ and K₂ are O and S; and no more than four of D₃, D₄, J₃, J₄ and K₂ are N;
R₂ is selected from the group consisting of hydrido, aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl with the proviso that R₂ has a lower Cahn-Ingold-Prelog system ranking than both R₁ and (CHR₃)ₙ-N(A)Q;
R₃ is selected from the group consisting of hydride, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl with the provisos that (CHR₃)ₙ-N(A)Q has a lower Cahn Ingold-Prelog stereochemical system ranking than R₁ and a higher Cahn-Tngold-Prelog stereochemical system ranking than R₂;
Y is selected from a group consisting of a covalent single bond, (C(R₁₄)₂)_{q} wherein q is an integer selected from 1 and 2 and (CH(R14))_{g}-W-(CH(R₁₄)ₚ wherein g and p are integers independently selected from 0 and 1;
R₁₄ is selected from the group consisting of hydrido, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl; Z is selected from the group consisting of a covalent single bond, (C(R₁₅)₂)_{q} wherein q is an integer selected from 1 and 2, and (CH(R₁₅))ⱼ-W-(CH(R₁₅))ₖ wherein j and k are integers independently selected from 0 and 1: W is selected from the group consisting of O, C(O), C(S), C(O)N(R₁₄), C(S)N(R₁₄), (R₁₄)NC(O), (R₁₄)NC(S), S, S(O), S (O)₂, S(O)₂N(R₁₄), (R₁₄)NC(O)₂, and N(R₁₄) with the proviso that R₁₄ is other than cyano;
R₁₅ is selected from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl; R₄, R₅, R₆, R₇, R₈, R₉, R₁₀, R₁₁, R₁₂, and R₁₃ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl-amidosulfonyl, dialkyl-amidosulfonyl, monoarylamidosulfonyl, arylsulfonamido, diarylamidosulfonyl, monoalkyl-monoaryl-amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, heteroaralkyl, arylalkenyl, heteroarylalkenyl, carboxyalkyl, carboalkoxy, alkoxycarboxamido, alkylaminodocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that R₄, R₅, R₆, R₇, R₈, R₉, R₁₀, R₁₁, R₁₂, and R₁₃ are each independently selected to maintain the tetravalent nature of carbon, the trivalent nature of nitrogen, the divalent nature of sulfur, and the divalent nature of oxygen;
R₄ and R₅, R₅ and R₆, R₆ and R₇, R₇ and R₈, R₉ and R₁₀, R₁₀ and R₁₂, R₁₁ and R₁₂, and R₁₂ and R₁₃ are independently selected to form spacer pairs wherein a spacer pair is taken together to form a linear moiety having from 3 through 6 continuous atoms connecting the points of bonding of said spacer pair members to form a ring selected from the group consisting of a cycloalkenyl ring having 5 through 8 continuous members, a partially saturated heterocyclyl ring having 5 through 8 continuous members, a heteroaryl ring having 5 through 6 continuous members, and an aryl with the provisos that no more than one of the group consisting of spacer pairs R₄ and R₅, R₅ and R₆, R₆ and R₇, and R₇ and R₈, is used at the same time and that no more than one of the group consisting of spacer pairs R₉ and R₁₀, R₁₀ and R₁₁, R₁₁ and R₁₂, and R₁₂ and R₁₃ is used at the same time;
R₄ and R₉, R₄ and R₁₃, R₈ and R₉ , and R₈ and R₁₃ are independently selected to form a spacer pair wherein said spacer pair is taken together to form a linear moiety wherein said linear moiety forms a ring selected from the group consisting of a partially saturated heterocyclyl ring having from 5 through 8 continuous members and a heteroaryl ring having from 5 through 6 continuous members with the proviso that no more than one of the group consisting of spacer pairs R₄ and R₉, R₄ and R₁₃, R₈ and R₉, and R₈ and R₁₃ is used at the same time.

Each term used for the formulas (XII), (XIII) and (XIV) is as described in the above-mentioned publication.

Specifically, the following compound disclosed in WO00/18721 (JP-T-2002-525348) can be mentioned:
a compound represented by the formula (XV): 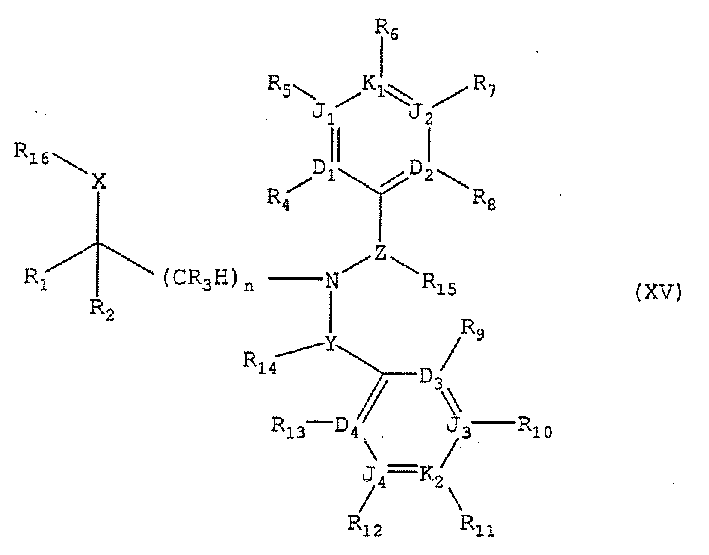 or a pharmaceutically acceptable salt thereof, wherein;
n is an integer selected from 0 through 5;
R₁ is selected from the group consisting of haloalkyl, haloalkenyl, haloalkoxyalkyl and haloalkenyloxyalkyl;
X is selected from the group consisting of O, H, F, S, S(O), NH, N(OH), N(alkyl), and N(alkoxy);
R₁₆ is selected from the group consisting of hydrido, alkyl, alkenyl, alkynyl, aryl, aralkyl, aryloxyalkyl, alkoxyalkyl, alkenyloxyalkyl, alkylthioalkyl, arylthioalkyl, aralkoxyalkyl, heteroaralkoxyalkyl, alkylsulfinylalkyl, alkylsulfonylalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxyalkyl, haloalkenyloxyalkyl, halocycloalkoxyalkyl, halocycloalkenyloxyalkyl, perhaloaryl, perhaloaralkyl, perhaloaryloxyalkyl, heteroaryl, heteroarylalkyl, monocarboalkoxyalkyl, monocarboalkoxy, dicarboalkoxyalkyl, monocarboxamido, monocyanoalkyl, dicyanoalkyl, carboalkoxycyanoalkyl, acyl, aroyl, heteroaroyl, heteroaryloxyalkyl, dialkoxyphosphonoalkyl, trialklylsilyl, and a spacer selected from the group consisting of a covalent single bond and a linear spacer moiety having from 1 through 4 continuous atoms linked to the point of bonding of an aromatic substituent selected from the group consisting of R₄, R₈, R₉, and R₁₃ to form a heterocyclyl ring having from 5 through 10 continuous members with the provisos that said spacer moiety is other than a covalent single bond when R₂ is alkyl and there is no R₁₆ wherein X is H or F;
D₁, D₂, J₁, J₂ and K₁ are independently selected from the group consisting of C, N, O, S and a covalent bond with the provisos that no more than one of D₁, D₂, J₁, J₂ and K₁ is a covalent bond, no more than one of D₁, D₂, J₁, J₂ and K₁ is O, no more than one of D₁, D₂, J₁, J₂ and K₁ is S, one of D₁, D₂, J₁, J₂ and K₁ must be a covalent bond when two of D₁, D₂, J₁, J₂ and K₁ are O and S, and no more than four of D₁ D₂, J₁, J₂ and K₁ are N;
D₃, D₄, J₃, J₄ and K₂ are independently selected from the group consisting of C, N, O, S and a covalent bond with the provisos that no more than one of D₃, D₄, J₃, J₄ and K₂ is a covalent bond, no more than one of D₃, D₄, J₃, J₄ and K₂ is O, no more than one of D₃, D₄, J₃, J₄ and K₂ is S, one of D₃, D₄, J₃, J₄ and K₂ must be a covalent bond when two of D₃, D₄, J₃, J₄ and K₂ are O and S, and no more than four of D₃, D₄, J₃, J₄ and K₂ are N;
R₂ is independently selected from the group consisting of hydrido, hydroxy, hydroxyalkyl, amino, aminoalkyl, alkylamino, dialkylamino, alkyl, alkenyl, alkynyl, aryl, aralkyl, aralkoxyalkyl, aryloxyalkyl, alkoxyalkyl, heteroaryloxyalkyl, alkenyloxyalkyl, alkylthioalkyl, aralkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, alkylsulfinylalkyl, alkylsulfonylalkyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl and diaralkoxyphosphonoalkyl;
R₂ and R₃ are taken together to form a linear spacer moiety selected from the group consisting of a covalent single bond and a moiety having from 1 through 6 continuous atoms to form a ring selected from the group consisting of a cycloalkyl having from 3 through 8 continuous members, a cycloalkenyl having from 5 through 8 continuous members, and a heterocyclyl having from 4 through 8 continuous members;
R₃ is selected from the group consisting of hydrido, hydroxy, halo, cyano, aryloxy, hydroxyalkyl, amino, alkylamino, dialkylamino, acyl, sulfhydryl, acylamido, alkoxy, alkylthio, arylthio, alkyl, alkenyl, alkynyl, aryl, aralkyl, aryloxyalkyl, alkoxyalkyl, heteroarylthio, aralkylthio, aralkoxyalkyl, alkylsulfinylalkyl, alkylsulfonylalkyl, aroyl, heteroaroyl, aralkylthioalkyl, heteroaralkylthioalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl and diaralkoxyphosphonoalkyl;
Y is selected from a group consisting of a covalent single bond, (C(R₁₄)₂)_{q} wherein q is an integer selected from 1 and 2, and (CH(R₁₄))_{g}-W- (CH(R₁₄))ₚ wherein g and p are integers independently selected from 0 and 1;
R₁₄ is independently selected from the group consisting of hydrido, hydroxy, halo, cyano, aryloxy, amino, alkylamino, dialkylamino, hydroxyalkyl, acyl, aroyl, heteroaroyl, heteroaryloxyalkyl, sulfhydryl, acylamido, alkoxy, alkylthio, arylthio, alkyl, alkenyl, alkynyl, aryl, aralkyl, aryloxyalkyl, aralkoxyalkylalkoxy, alkylsulfinylalkyl, alkylsulfonylalkyl, aralkylthioalkyl, heteroaralkoxythioalkyl, alkoxyalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl, diaralkoxyphosphonoalkyl, a spacer selected from a moiety having a chain length of 3 to 6 atoms connected to the point of bonding selected from the group consisting of R₉ and R₁₃ to form a ring selected from the group consisting of a cycloalkenyl ring having from 5 through 8 continuous members and a heterocyclyl ring having from 5 through 8 continuous members, and a spacer selected from a moiety having a chain length of 2 to 5 atoms connected to the point of bonding selected from the group consisting of R₄ and R₈ to form a heterocyclyl having from 5 through 8 continuous members with the proviso that, when Y is a covalent bond, R₁₄ substituent is not attached to Y;
R₁₄ and R₁₄, when bonded to the different atoms, are taken together to form a group selected from the group consisting of a covalent bond, alkylene, haloalkylene, and a spacer selected from a group consisting of a moiety having a chain length of 2 to 5 atoms connected to form a ring selected from the group of a saturated cycloalkyl having from 5 through 8 continuous members, a cycloalkenyl having from 5 through 8 continuous members, and a heterocyclyl having from 5 through 8 continuous members;
R₁₄ and R₁₄, when bonded to the same atom, are taken together to form a group selected from the group consisting of oxo, thiono, alkylene, haloalkylene, and a spacer selected from the group consisting of a moiety having a chain length of 3 to 7 atoms connected to form a ring selected from the group consisting of a cycloalkyl having from 4 through 8 continuous members, a cycloalkenyl having from 4 through 8 continuous members, and a heterocyclyl having from 4 through 8 continuous members;
W is selected from the group consisting of O, C(O), C(S), C(O)N(R₁₄), C(S)N(R₁₄), (R₁₄)NC(O), (R₁₄)NC(S), S, S(O), S(O)₂, S(O)₂N(R₁₄), (R₁₄)NS(O)₂, and N(R₁₄) with the proviso that R₁₄ is selected from other than halo and cyano;
Z is independently selected from a group consisting of a covalent single bond, (C(R₁₅)_{q}) wherein q is an integer selected from 1 and 2, and (CH(R₁₅))ⱼ-W-(CH(R₁₅))ₖ wherein j and k are integers independently selected from 0 and 1 with the proviso that, when Z is a covalent single bond, R₁₅ substituent is not attached to Z;
R₁₅ is independently selected, when Z is (C(R₁₅)₂)_{q} wherein q is an integer selected from 1 and 2, from the group consisting of hydrido, hydroxy, halo, cyano, aryloxy, amino, alkylamino, dialkylamino, hydroxyalkyl, acyl, aroyl, heteroaroyl, heteroaryloxyalkyl, sulfhydryl, acylamido, alkoxy, alkylthio, arylthio, alkyl, alkenyl, alkynyl, aryl, aralkyl, aryloxyalkyl, aralkoxyalkyl, alkylsulfinylalkyl, alkylsulfonylalkyl, aralkylthioalkyl, heteroaralkylthioalkyl, alkoxyalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenlyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl, diaralkoxyphosphonoalkyl, a spacer selected from a moiety having a chain length of 3 to 6 atoms connected to the point of bonding selected from the group consisting of R₄ and R₈ to form a ring selected from the group consisting of a cycloalkenyl ring having from 5 through 8 continuous members and a heterocyclyl ring having from 5 through 8 continuous members, and a spacer selected from a moiety having a chain length of 2 to 5 atoms connected to the point of bonding selected from the group consisting of R₉ and R₁₃, to form a heterocyclyl having from 5 through 8 continuous members;
R₁₅ and R₁₅, when bonded to the different atoms, are taken together to form a group selected from the group consisting of a covalent bond, alkylene, haloalkylene, and a spacer selected from a group consisting of a moiety having a chain length of 2 to 5 atoms connected to form a ring selected from the group consisting of a saturated cycloalkyl having from 5 through 8 continuous members, a cycloalkenyl having from 5 through 8 continuous members, and a heterocyclyl having from 5 through 8 continuous members;
R₁₅ and R₁₅, when bonded to the same atom, are taken together to form a group selected from the group consisting of oxo, thiono, alkylene, haloalkylene, and a spacer selected from the group consisting of a moiety having a chain length of 3 to 7 atoms connected to form a ring selected from the group consisting of a cycloalkyl having from 4 through 8 continuous members, a cycloalkenyl having from 4 through 8 continuous members, and a heterocyclyl having from 4 through 8 continuous members;
R₁₅ is independently selected, when Z is (CH(R₁₅))ⱼ-W-(CH(R₁₅))ₖ wherein j and k are integers independently selected from 0 and 1, from the group consisting of hydrido, halo, cyano, aryloxy, carboxyl, acyl, aroyl, heteroaroyl, hydroxyalkyl, heteroaryloxyalkyl, acylamido, alkoxy, alkylthio, arylthio, alkyl, alkenyl, alkynyl, aryl, aralkyl, aryloxyalkyl, alkoxyalkyl, heteroaryloxyalkyl, aralkoxyalkyl, heteroaralkoxyalkyl, alkylsulfonylalkyl, alkylsulfinylalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxyalkyl, carboalkoxy, carboxamide, carboxamidoalkyl, carboaralkoxy, dialkoxyphosphonoalkyl, diaralkoxyphosphonoalkyl, a spacer selected from a linear moiety having a chain length of 3 to 6 atoms connected to the point of bonding selected from the group consisting of R₄ and R₈ to form a ring selected from the group consisting of a cycloalkenyl ring having from 5 through 8 continuous members and a heterocyclyl ring having from 5 through 8 continuous members, and a spacer selected from a linear moiety having a chain length of 2 to 5 atoms connected to the point of bonding selected from the group consisting of R₉ and R₁₃ to form a heterocyclyl ring having from 5 through 8 continuous members;
R₄, R₅, R₆, R₇, R₈, R₉, R₁₀, R₁₁, R₁₂, and R₁₃ are independently selected from the group consisting of perhaloaryloxy, alkanoylalkyl, alkanoylalkoxy, alkanoyloxy, N-aryl-N-alkylamino, heterocyclylalkoxy, heterocyclylthio, hydroxyalkoxy, carboxamidoalkoxy, alkoxycarbonylalkoxy, alkoxycarbonylalkenyloxy, aralkanoylalkoxy, aralkenoyl, N-alkylcarboxamido, N-haloalkylcarboxamido, N-cycloalkylcarboxamido, N-arylcarboxamidoalkoxy, cycloalkylcarbonyl, cyanoalkoxy, heterocyclylcarbonyl, hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkylamidosulfonyl, dialkylamidosulfonyl, monoarylamidosulfonyl, arylsulfonamido, diarylamidosulfonyl, monoalkylmonoarylamidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloaralkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that there are one to five non-hydrido ring substituents R₄, R₅, R₆, R₇, and R₈ present, that there are one to five non-hydrido ring substituents R₉, R₁₀, R₁₁, R₁₂, and R₁₃ present, and R₄, R₅, R₆, R₇, R₈, R₉, R₁₀, R₁₁, R₁₂, and R₁₃ are each independently selected to maintain the tetravalent nature of carbon, the trivalent nature of nitrogen, the divalent nature of sulfur, and the divalent nature of oxygen;
R₄ and R₅, R₅ and R₆, R₆ and R₇, R₇ and R₈, R₉ and R₁₀, R₁₀ and R₁₁, R₁₁ and R₁₂, and R₁₂ and R₁₃ are independently selected to form spacer pairs wherein a spacer pair is taken together to form a linear moiety having from 3 through 6 continuous atoms connecting the points of bonding of said spacer pair members to form a ring selected from the group consisting of a cycloalkenyl ring having 5 through 8 continuous members, a partially saturated heterocyclyl ring having 5 through 8 continuous members, a heteroaryl ring having 5 through 6 continuous members, and an aryl with the provisos that no more than one of the group consisting of spacer pairs R₄ and R₅, R₅ and R₆, R₆ and R₇, and R₇ and R₈, is used at the same time and that no more than one of the group consisting of spacer pairs R₉ and R₁₀, R₁₀ and R₁₁, R₁₁ and R₁₂, and R₁₂ and R₁₃ is used at the same time;
R₄ and R₉, R₄ and R₁₃, R₈ and R₉, and R₈ and R₁₃ are independently selected to form a spacer pair wherein said spacer pair is taken together to form a linear moiety wherein said linear moiety forms a ring selected from the group consisting of a partially saturated heterocyclyl ring having from 5 through 8 continuous members and a heteroaryl ring having from 5 through 6 continuous members with the proviso that no more than one of the group consisting of spacer pairs R₄ and R₉, R₄ and R₁₃, R₈ and R₉, and R₈ and R₁₃ is used at the same time.

Each term used for the formula (XV) is as described in the above-mentioned publication.

Specifically, the following compound disclosed in WO00/18723 (JP-T-2002-525350) can be mentioned:
a compound having the formula (XVI): 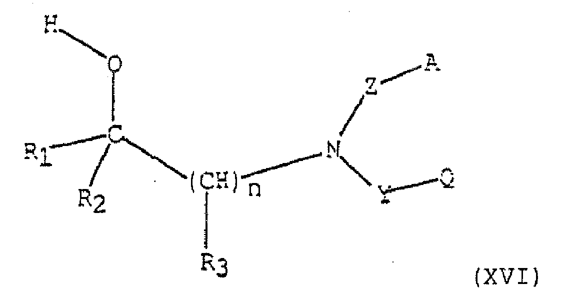 or a pharmaceutically acceptable salt thereof,
   wherein
n is an integer selected from 1 through 2;
A and Q are independently selected from the group consisting of -CH₂(CR₃₇R₃₈)ᵥ- (CR₃₃R₃₄)ᵤ-T-(CR₃₅R₃₆)_{w}-H, 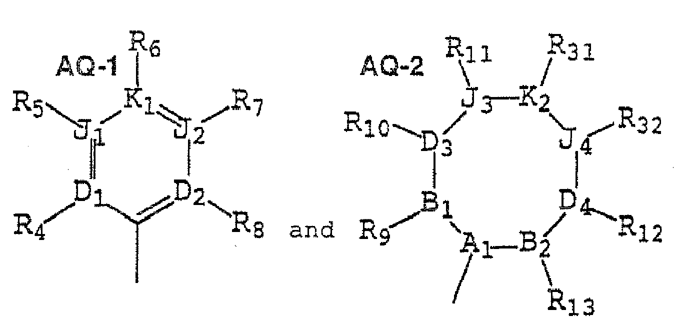 with the provisos that one of A and Q must be AQ-1 and that one of A and Q must be selected from the group consisting of AQ-2 and -CH₂(CR₃₇R₃₈)ᵥ-(CR₃₃R₃₄)ᵤ-T-(CR₃₅R₃₆)_{w}-H;
T is selected from the group consisting of a single covalent bond, O, S, S(O), S(O)₂, C(R₃₃)=C(R₃₅), and C≡C;
v is an integer selected from 0 through 1 with the proviso that v is 1 when any one of R₃₃, R₃₄, R₃₅, and R₃₆ is aryl or heteroaryl;
u and w are integers independently selected from 0 through 6;
A₁ is C(R₃₀);
D₁, D₂, J₁, J₂ and K₁ are independently selected from the group consisting of C, N, O, S and a covalent bond with the provisos that no more than one of D₁, D₂, J₁, J₂ and K₁ is a covalent bond, no more than one of D₁, D₂, J₁, J₂ and K₁ is O, no more than one of D₁, D₂, J₁, J₂ and K₁ is S, one of D₁, D₂, J₁, J₂ and K₁ must be a covalent bond when two of D₁, D₂, J₁, J₂ and K₁ are O and S, and no more than four of D₁, D₂, J₁, J₂ and K₁ are N;
B₁, B₂, D₃, D₄, J₃, J₄ and K₂ are independently selected from the group consisting of C, C(R₃₀), N, 0, S and a covalent bond with the provisos that no more than 5 of B₁, B₂, D₃, D₄, J₃, J₄ and K₂ are a covalent bond, no more than two of B₁, B₂, D₃, D₄, J₃, J₄ and K₂ are O, no more than two of B₁, 8₂, D₃, D₄, J₃, J₄ and K₂ are S, no more than two of B₁, B₂, D₃, D₄, J₃, J₄ and K₂ are simultaneously O and S, and no more than two of B₁, B₂, D₃, D₄, J₃, J₄ and K₂ are N; B₁ and D₃, D₃ and J₃, J₃ and K₂, K₂ and J₄, J₄ and D₄, and D₄ and B₂ are independently selected to form an in-ring spacer pair wherein said spacer pair is selected from the group consisting of C(R₃₃)=C(R₃₅) and N=N with the provisos that AQ-2 must be a ring of at least five continuous members, that no more than two of the group of said spacer pairs are simultaneously C(R₃₃)=C(R₃₅), and that no more than one of the group of said spacer pairs are N=N unless the other spacer pairs are other than C(R₃₃)=C(R₃₅), O, N, and S; R₁ is selected from the group consisting of haloalkyl and haloalkoxymethyl;
R₂ is selected from the group consisting of hydrido, aryl, alkyl, alkenyl, haloalkyl, haloalkoxy, haloalkoxyalkyl, perhaloaryl, perhaloaralkyl, perhaloaryloxyalkyl and heteroaryl;
R₃ is selected from the group consisting of hydrido, aryl, alkyl, alkenyl, haloalkyl and haloalkoxyalkyl; Y is selected from a group consisting of a covalent single bond, (CH₂)_{q} wherein q is an integer selected from 1 through 2, and (CH₂)ⱼ-O-(CH₂)ₖ wherein j and k are integers independently selected from 0 through 1;
Z is selected from the group consisting of a covalent single bond, (CH₂)_{q} wherein q is an integer selected from 1 through 2, and (CH₂)ⱼ-O-(CH₂)ₖ wherein j and k are integers independently selected from 0 through 1;
R₄, R₈, R₉ and R₁₃ are independently selected from the group consisting of hydrido, halo, haloalkyl and alkyl; R₃₀ is selected from the group consisting of hydrido, alkoxy, alkoxyalkyl, halo, haloalkyl, alkylamino, alkylthio, alkylthioalkyl, alkyl, alkenyl, haloalkoxy, and haloalkoxyalkyl with the proviso that R₃₀ is selected to maintain the tetravalent nature of carbon, the trivalent nature of nitrogen, the divalent nature of sulfur, and the divalent nature of oxygen;
R₃₀, when bonded to A₁, is taken together to form an intra-ring linear spacer connecting the A₁-carbon at the point of attachment of R₃₀ to the point of bonding of a group selected from the group consisting of R₁₀, R₁₁, R₁₂, R₃₁, and R₃₂ wherein said intra-ring linear spacer is selected from the group consisting of a covalent single bond and a spacer moiety having from 1 through 6 continuous atoms to form a ring selected from the group consisting of a cycloalkyl having from 3 through 10 continuous members, a cycloalkenyl having from 5 through 10 continuous members, and a heterocyclyl having from 5 through 10 continuous members; R₃₀, when bonded to A₁, is taken together to form an intra-ring branched spacer connecting the A₁-carbon at the point of attachment of R₃₀ to the points of bonding of each member of any one of substituent pairs selected from the group consisting of substituent pairs R₁₀ and R₁₁, R₁₀ and R₃₁, R₁₀ and R₃₂, R₁₀ and R₁₂, R₁₁ and R₃₁, R₁₁ and R₃₂, R₁₁ and R₁₂, R₃₁ and R₃₂, R₃₁ and R₁₂, and R₃₂ and R₁₂ and wherein said intra-ring branched spacer is selected to form two rings selected from the group consisting of cycloalkyl having from 3 through 10 continuous members, cycloalkenyl having from 5 through 10 continuous members, and heterocyclyl having from 5 through 10 continuous members;
R₄, R₅, R₆, R₇, R₈, R₉, R₁₀, R₁₁, R₁₂, R₁₃, R₃₁, R₃₂, R₃₃, R₃₄, R₃₅, and R₃₆ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl-monoaryl-amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the provisos that R₄, R₅, R₆, R₇, R₈, R₉, R₁₀, R₁₁, R₁₂, R₁₃, R₃₁, R₃₂, R₃₃, R₃₄, R₃₅, and R₃₆ are each independently selected to maintain the tetravalent nature of carbon, the trivalent nature of nitrogen, the divalent nature of sulfur, and the divalent nature of oxygen, that no more than three of the R₃₃ and R₃₄ substituents are simultaneously selected from other than the group consisting of of hydrido and halo, and that no more than three of the R₃₅ and R₃₆ substituents are simultaneously selected from other than the group consisting of of hydrido, and halo:
R₉, R₁₀, R₁₁, R₁₂, R₁₃, R₃₁, and R₃₂ are independently selected to be oxo with the provisos that B₁, B₂, D₃, D₄, J₃, J₄ and K₂ are independently selected from the group consisting of C and S, no more than two of R₉, R₁₀, R₁₁, R₁₂, R₁₃, R₃₁, and R₃₂ are simultaneously oxo, and that R₉, R₁₀, R₁₁, R₁₂, R₁₃, R₃₁, and R₃₂ are each independently selected to maintain the tetravalent nature of carbon, the trivalent nature of nitrogen, the divalent nature of sulfur, and the divalent nature of oxygen;
R₄ and R₅, R₅ and R₆, R₆ and R₇, R₇ and R₈, R₉ and R₁₀, R₁₀ and R₁₁, R₁₁ and R₃₁, R₃₁ and R₃₂, R₃₂ and R₁₂, and R₁₂ and R₁₃ are independently selected to form spacer pairs wherein a spacer pair is taken together to form a linear moiety having from 3 through 6 continuous atoms connecting the points of bonding of said spacer pair members to form a ring selected from the group consisting of a cycloalkenyl ring having 5 through 8 continuous members, a partially saturated heterocyclyl ring having 5 through 8 continuous members, a heteroaryl ring having 5 through 6 continuous members, and an aryl with the provisos that no more than one of the group consisting of spacer pairs R₄ and R₅, R₅ and R₆, R₆ and R₇, and R₇ and R₈, are used at the same time and that no more than one of the group consisting of spacer pairs R₉ and R₁₀, R₁₀ and R₁₁, R₁₁ and R₃₁, R₃₁ and R₃₂, R₃₂ and R₁₂, and R₁₂ and R₁₃ are used at the same time;
R₉ and R₁₁, R₉ and R₁₂, R₉ and R₁₃, R₉ and R₃₁, R₉ and R₃₂, R₁₀ and R₁₂, R₁₀ and R₁₃, R₁₀ and R₃₁, R₁₀ and R₃₂, R₁₁ and R₁₂, R₁₁ and R₁₃, R₁₁ and R₃₂, R₁₂ and R₃₁, R₁₃ and R₃₁, and R₁₃ and R₃₂ are independently selected to form a spacer pair wherein said spacer pair is taken together to form a linear spacer moiety selected from the group consisting of a covalent single bond and a moiety having from 1 through 3 continuous atoms to form a ring selected from the group consisting of a cycloalkyl having from 3 through 8 continuous members, a cycloalkenyl having from 5 through 8 continuous members, a saturated heterocyclyl having from 5 through 8 continuous members and a partially saturated heterocyclyl having from 5 through 8 continuous members with the provisos that no more than one of said group of spacer pairs is used at the same time;
R₃₇ and R₃₈ are independently selected from the group consisting of hydrido, alkoxy, alkoxyalkyl, hydroxy, amino, thio, halo, haloalkyl, alkylamino, alkylthio, alkylthioalkyl, cyano, alkyl, alkenyl, haloalkoxy, and haloalkoxyalkyl.

Each term used for the formula (XVI) is as described in the above-mentioned publication.

Specifically, the compound designated with Code Name PD-140195 [Compound (XVII)], which is disclosed in WO99/14204, and the like can be mentioned.

Specifically, the compound disclosed in JP-A-10-67746 can be mentioned:
Cycloalkano-pyridine of the formula (XVIII) 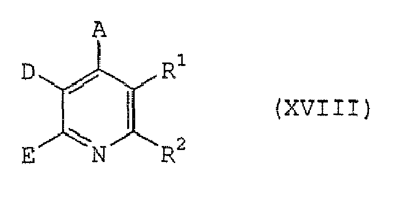
wherein A is an aryl having 6 to 10 carbon atoms, which can be optionally substituted up to 5 times in an identical or different manner by halogen, nitro, hydroxyl, trifluoromethyl or trifluoromethoxy, or by linear or branched chain alkyl, acyl, hydroxyalkyl or alkoxy, each having up to 7 carbon atoms, or by a group of the formula - NR³R⁴, wherein R³ and R⁴ are identical or different and each is hydrogen, phenyl or linear or branched chain alkyl having up to 6 carbon atoms, D is aryl having 6 to 10 carbon atoms, which is optionally substituted by phenyl, nitro, halogen, trifluoromethyl or trifluoromethoxy, or is a group represented by the formula R⁵-L-, 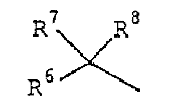 or R⁹-T-V-X
wherein R⁵, R⁶ and R⁹ independently of one another denote cycloalkyl having 3 to 6 carbon atoms, or aryl having 6 to 10 carbon atoms, or a 5- to 7-membered, optionally benzo-fused, saturated or unsaturated, mono-, bi- or tricyclic heterocycle having up to 4 heteroatoms from the series S, N and/or O, where the cycles are substituted, if appropriate, in the case of a nitrogen-containing rings also via an N functional group, up to 5 times in an identical or different manner by halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl, each having up to 6 carbon atoms, by aryl-or trifluoromethyl-substituted aryl, each having 6 to 10 carbon atoms, or by an optionally benzo-fused, aromatic 5-to 7-membered heterocycle having up to 3 heteroatoms from the series S, N and/or 0, and/or are substituted by a group of the formula -OR¹⁰, -SR¹¹, -SO₂R¹² or -NR¹³R¹⁴, wherein R¹⁰, R¹¹ and R¹² independently of one another denote aryl having 6 to 10 carbon atoms, which for its part is substituted up to 2 times in an identical or different manner by phenyl, halogen or by straight-chain or branched alkyl having up to 6 carbon atoms, R¹³ and R¹⁴ are identical or different and have the meaning of R³ and R⁴ indicated above, or R⁵ and/or R⁶ denote a group of the formula 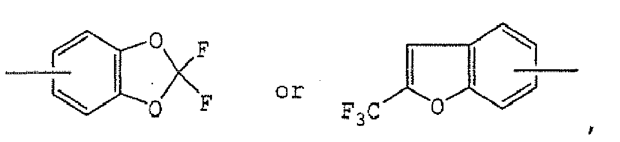
R⁷ denotes a hydrogen or halogen, R⁸ denotes hydrogen, halogen, azido, trifluoromethyl, hydroxyl, trifluoromethoxy, straight-chain or branched alkoxy or alkyl each having up to 6 carbon atoms or a group of the formula -NR¹⁵R¹⁶, wherein R¹⁵ and R¹⁶ are identical or different and have the meaning of R³ and R⁴ indicated above, or R⁷ and R⁸ together form a group of the formula =O or =NR¹⁷, wherein R¹⁷ is a hydrogen or linear or branched chain alkyl, alkoxy or acyl, each having up to 6 carbon atoms, L is a linear or branched chain alkylene or alkenylene chain each having up to 8 carbon atoms, each of which is optionally substituted up to 2 times by hydroxyl, T and X are identical or different and denote a straight-chain or branched alkylene chain having up to 8 carbon atoms, or T or X denotes a bond, V represents an oxygen or sulfur atom or an -NR¹⁸ group, wherein R¹⁸ is a hydrogen or linear or branched chain alkyl having up to 6 carbon atoms or phenyl, E denotes cycloalkyl having 3 to 8 carbon atoms, or linear or branched chain alkyl having up to 8 carbon atoms, which is optionally substituted by cycloalkyl having 3 to 8 carbon atoms or hydroxyl, or represents phenyl which is optionally substituted by halogen or trifluoromethyl, R¹ and R² together form linear or branched alkylene chain having up to 7 carbon atoms, which must be substituted by a carbonyl group and/or a group of the formula 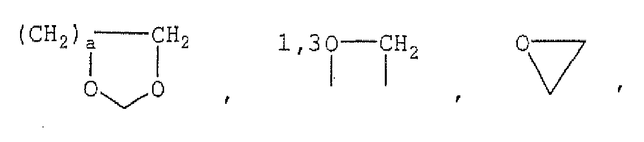 -OR¹⁹ or 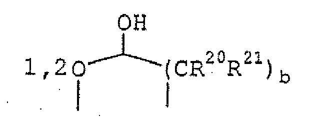
wherein a and b are identical or different and denote a number 1, 2 or 3, R¹⁹ denotes hydrogen, cycloalkyl having 3 to 7 carbon atoms, straight-chain or branched silylalkyl having up to 8 carbon atoms or straight-chain or branched alkyl having up to 8 carbon atoms, which is optionally substituted by hydroxyl, straight-chain or branched alkoxy having up to 6 carbon atoms or by phenyl, which for its part can be substituted by halogen, nitro, trifluoromethyl, trifluoromethoxy or by phenyl- or tetrazole-substituted phenyl, and alkyl is optionally substituted by a group of the formula -OR²², wherein R²² is linear or branched chain acyl or benzyl having up to 4 carbon atoms, or R¹⁹ is linear or branched chain acyl having up to 20 carbon atoms, or benzoyl which is optionally substituted by halogen, trifluoromethyl, nitro or trifluoromethoxy, or denotes straight-chain or branched fluoroacyl having up to 8 carbon atoms and up to 9 fluorine atoms, R²⁰ and R²¹ are identical or different, and denote hydrogen, phenyl or straight-chain or branched alkyl having up to 6 carbon atoms, or R²⁰ and R²¹ together form a 3 to 6-membered carbocycle, and, if appropriate also geminally, the carbocycles formed are optionally substituted up to 6 times in an identical or different manner by trifluoromethyl, hydroxyl, halogen, carboxyl, nitro, azido, cyano, cycloalkyl or cycloalkyloxy each having 3 to 7 carbon atoms, by straight-chain or branched alkoxycarbonyl, alkoxy or alkylthio each having up to 6 carbon atoms or by straight-chain or branched alkyl having up to 6 carbon atoms, which for its part is substituted up to 2 times in an identical or different manner by hydroxyl, benzyloxy, trifluoromethyl, benzoyl, straight-chain or branched alkoxy, oxyacyl or carboxyl each having up to 4 carbon atoms and/or phenyl, which for its part can be substituted by halogen, trifluoromethyl or trifluoromethoxy, and/or the carbocycles formed, also geminally, are optionally substituted up to 5 times in an identical or different manner by phenyl, benzoyl, thiophenyl or sulphonylbenzyl, which for their part are optionally substituted by halogen, trifluoromethyl, trifluoromethoxy or nitro, and/or are optionally substituted by a group of the formula 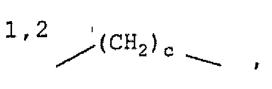 -SO₂-C₆H₅ , -(CO)_{d}-NR²³R²⁴ or =O.
wherein c denotes a number 1, 2, 3 or 4, d denotes a number 0 or 1, R²³ and R²⁴ are identical or different and denote hydrogen, cycloalkyl having 3 to 6 carbon atoms, straight-chain or branched alkyl having up to 6 carbon atoms, benzyl or phenyl, which is optionally substituted up to 2 times in an identical or different manner by halogen, trifluoromethyl, cyano, phenyl or nitro, and/or the carbocycles formed are optionally substituted by a spiro-linked group of the formula 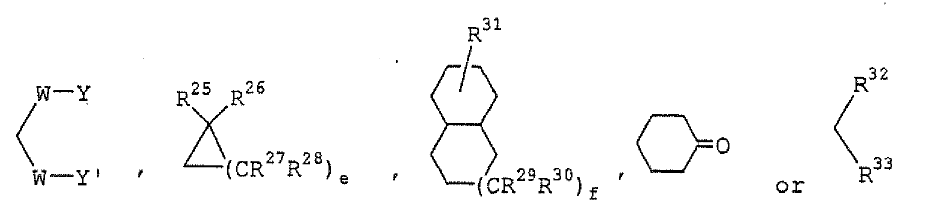
wherein W denotes an oxygen or a sulphur atom, Y and Y' together form a 2- to 6-membered straight-chain or branched alkylene chain, e denotes a number 1, 2, 3, 4, 5, 6 or 7, f denotes a number 1 or 2, R²⁵, R²⁶, R²⁷, R²⁸, R²⁹, R³⁰ and R³¹ are identical or different and denote hydrogen,
   trifluoromethyl, phenyl, halogen or straight-chain or branched alkyl or alkoxy each having up to 6 carbon atoms, or R²⁵ and R²⁶ or R²⁷ and R²⁸ in each case together form a straight-chain or branched alkyl chain having up to 6 carbon atoms, or R²⁵ and R²⁶ or R²⁷ and R²⁸ in each case together form a group of the formula 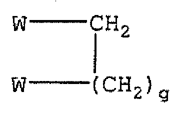
wherein W has the meaning indicated above, g denotes a number 1, 2, 3, 4, 5, 6 or 7, R³² and R³³ together form a 3-to 7-membered heterocycle which contains an oxygen or sulphur atom or a group of the formula SO, SO₂ or -NR³⁴, wherein R³⁴ denotes hydrogen, phenyl, benzyl or straight-chain or branched alkyl having up to 4 carbon atoms, a salt thereof or N-oxide thereof, with the exception of 5(6H)-quinolone, 3-benzoyl-7,8-dihydro-2,7,7-trimethyl-4-phenyl.

Each term used for the formula (XVIII) is as described in the above-mentioned publication.

Specifically, the following compound disclosed in WO98/39299 (JP-T-2001-513790) can be mentioned:
Substituted quinoline of the formula (XIX) 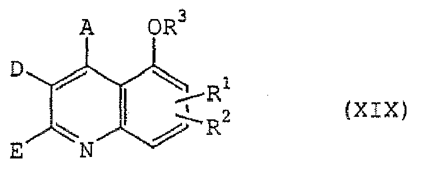
wherein A is aryl having 6 to 10 carbon atoms, which is optionally substituted up to 5 times in an identical or different manner by halogen, hydroxyl, trifluoromethyl, trifluoromethoxy, nitro, or straight chain or branched alkyl, acyl, hydroxyalkyl or alkoxy, each having up to 7 carbon atoms, or a group of the formula -NR⁴R⁵, wherein R⁴ and R⁵ are identical or different and denote hydrogen, phenyl or straight chain or branched alkyl having up to 6 carbon atoms, D is aryl having 6 to 10 carbon atoms, which is optionally substituted by phenyl, nitro, halogen, trifluoromethyl or trifluoromethoxy, or denote a group of the formula R⁶-L- , 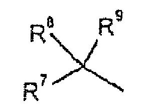 or R¹⁰-T-V-X-,
wherein R⁶, R⁷ and R¹⁰ independently of one another denote cycloalkyl having 3 to 6 carbon atoms, or aryl having 6 to 10 carbon atoms, or a 5- to 7-membered, optionally benzo-fused, saturated or unsaturated, mono-, bi- or tricyclic heterocycle having up to 4 heteroatoms from the group consisting of S, N and/or O, where the cycles are optionally substituted in the case of the nitrogen-containing rings also via the N functional group, up to 5 times in an identical or different manner by halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl, each having up to 6 carbon atoms, by aryl having 6 to 10 carbon atoms, or by an optionally benzo-fused, aromatic 5- to 7-membered heterocycle having up to 3 heteroatoms from the group consisting of S, N and/or O, and/or are substituted by a group of the formula -OR¹¹, -SR¹², -SO₂R¹³ or -NR¹⁴R¹⁵, wherein R¹¹, R¹² and R¹³ independently of one another denote aryl having 6 to 10 carbon atoms, which for its part is substituted up to 2 times in an identical or different manner by phenyl, halogen or by straight-chain or branched alkyl having up to 6 carbon atoms, R¹⁴ and R¹⁵ are identical or different and have the meaning of R⁴ and R⁵ indicated above, or R⁶ and/or R⁷ denote a group of the formula 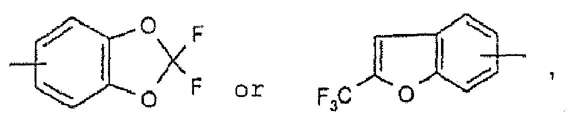
R⁸ denotes a hydrogen or halogen, R⁹ denotes hydrogen, halogen, azido, trifluoromethyl, hydroxyl, trifluoromethoxy, straight-chain or branched alkoxy or alkyl each having up to 6 carbon atoms or a group of the formula -NR¹⁶R¹⁷, wherein R¹⁶ and R¹⁷ are identical or different and have the meaning of R⁴ and R⁵ indicated above, or R⁸ and R⁹ together form a group of the formula =O or =NR¹⁸, wherein R¹⁸ is hydrogen or linear or branched alkyl, alkoxy or acyl, each having up to 6 carbon atoms, L is a linear or branched alkyl or alkenyl each having up to 8 carbon atoms, each of which is optionally substituted up to 2 times by hydroxyl, T and X are identical or different and denote straight-chain or branched alkyl having up to 8 carbon atoms, or T or X denotes a bond, V represents oxygen or sulfur atom or -NR¹⁹ group, wherein R¹⁹ is hydrogen or linear or branched alkyl having up to 6 carbon atoms or phenyl, E denotes cycloalkyl having 3 to 8 carbon atoms, or linear or branched alkyl having up to 8 carbon atoms, which is optionally substituted by cycloalkyl having 3 to 8 carbon atoms or hydroxyl, or represents phenyl which is optionally substituted by halogen or trifluoromethyl, R¹ and R² are identical or different and denote cycloalkyl having 3 to 8 carbon atoms, hydrogen, nitro, halogen, trifluoromethyl, trifluoromethoxy, carboxyl, hydroxyl, cyano, or straight chain or branched acyl, alkoxycarbonyl or alkoxy each having up to 6 carbon atoms, or a group of the formula - NR²⁰R²¹, wherein R²⁰ and R²¹ are identical or different and denote hydrogen, phenyl, or straight chain or branched alkyl having up to 6 carbon atoms and/or R¹ and/or R² denote straight chain or branched alkyl having up to 6 carbon atoms, which are optionally substituted by halogen, trifluoromethoxy, hydroxyl or straight chain or branched alkoxy having up to 4 carbon atoms, and/or aryl having 6 to 10 carbon atoms, which are optionally substituted up to 5 times in an identical or different manner by halogen, cyano, hydroxyl, trifluoromethyl, trifluoromethoxy, nitro, or straight chain or branched alkyl, acyl, hydroxyalkyl or alkoxy each having up to 7 carbon atoms, or a group of the formula -NR²²R²³, wherein R²² and R²³ are identical or different and denote hydrogen, phenyl, or straight chain or branched alkyl having up to 6 carbon atoms, and/or R¹ and R² together form straight chain or branched alkyl chain or alkenyl chain each having up to 6 carbon atoms, which is optionally substituted by halogen, trifluoromethyl, hydroxyl, or straight chain or branched alkoxy having up to 5 carbon atoms, R³ denotes hydrogen, straight chain or branched acyl or benzoyl having up to 20 carbon atoms, which is optionally substituted by halogen, trifluoromethyl, nitro or trifluoromethoxy, or straight chain or branched fluoroacyl having up to 8 carbon atoms and up to 7 fluorine atoms, cycloalkyl having 3 to 7 carbon atoms, or straight chain or branched alkyl having up to 8 carbon atoms, which is optionally substituted by hydroxyl, straight chain or branched alkoxy having up to 6 carbon atoms, or phenyl, which for its part can be substituted by halogen, nitro, trifluoromethyl, trifluoromethoxy or phenyl or tetrazol-substituted phenyl, and/or alkyl is optionally substituted by a group of the formula -OR²⁴, wherein R²⁴ denotes straight chain or branched acyl or benzyl having up to 4 carbon atoms, or a salt thereof.

Each term used for the formula (XIX) is as described in the above-mentioned publication.

Specifically, the following compound disclosed in WO99/14215 (JP-T-2001-516757) can be mentioned:
Hetero-tetrahydroquinoline of the formula (XX) 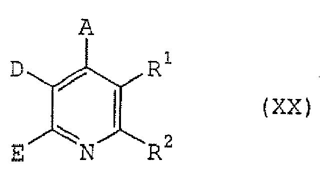
wherein A represents cycloalkyl having 3 to 8 carbon atoms or, represents a 5- to 7-membered saturated, partially unsaturated or unsaturated, optionally benzo-fused heterocycle having up to 3 heteroatoms from the group consisting of S, N and O, which, in the case of a saturated heterocycle with a nitrogen functional group, is optionally also attached via this functional group, and where the abovementioned ring systems are optionally substituted up to 5 times by identical or different substituents from the group consisting of halogen, nitro, hydroxyl, trifluoromethyl, trifluoromethoxy and straight-chain or branched alkyl, acyl, hydroxyalkyl or alkoxy having in each case up to 7 carbon atoms, or by a group of the formula -NR³R⁴ wherein R³ and R⁴ are identical or different and represent hydrogen, phenyl or straight-chain or branched alkyl having up to 6 carbon atoms, or A represents a group of the formula 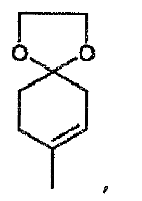
D represents aryl having 6 to 10 carbon atoms which is optionally substituted by phenyl, nitro, halogen, trifluoromethyl or trifluoromethoxy, or represents a group of the formula R⁵-L-, 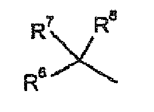 or R⁹-T-V-X- ,
wherein R⁵, R⁶ and R⁹ independently of one another represent cycloalkyl having 3 to 6 carbon atoms, or represent aryl having 6 to 10 carbon atoms, or represent a 5- to 7-membered optionally benzo-fused saturated or unsaturated mono-, bi- or tricyclic hetreocycle having up to 4 heteroatoms from the group consisting of S, N and O, where the cycles are optionally substituted, in the case of nitrogen-containing rings also via an N functional group, up to 5 times by identical or different substituents from the group consisting of halogen, trifluoromethyl, nitro, hydroxyl, cyano, carboxyl, trifluoromethoxy, and straight-chain or branched acyl, alkyl, alkylthio, alkylalkoxy, alkoxy or alkoxycarbonyl having in each case up to 6 carbon atoms, by aryl or trifluoromethyl-substituted aryl having in each case 6 to 10 carbon atoms, or by an optionally benzo-fused aromatic 5- to 7-membered heterocycle having up to 3 heteroatoms from the group consisting of S, N and O and/or by a group of the formula -OR¹⁰, -SR¹¹, -SO₂R¹² or - NR¹³R¹⁴, wherein R¹⁰, R¹¹ and R¹² independently of one another represent aryl having 6 to 10 carbon atoms which for its part is substituted up to 2 times by identical or different substituents from the group consisting of phenyl, halogen and straight-chain or branched alkyl having up to 6 carbon atoms, R¹³ and R¹⁴ are identical or different and have the meaning of R³ and R⁴ given above, or R⁵ and/or R⁶ represent(s) a group of the formula 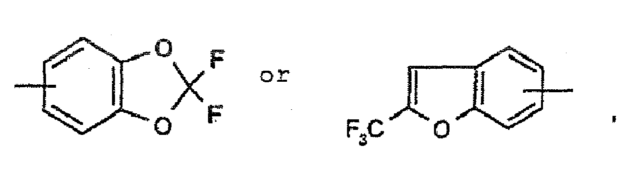
R⁷ is a hydrogen or halogen, R⁸ represents hydrogen, halogen, azido, trifluoromethyl, hydroxyl, trifluoromethoxy, straight-chain or branched alkoxy or alkyl having in each case up to 6 carbon atoms or a group of the formula -NR¹⁵R¹⁶, wherein R¹⁵ and R¹⁶ are identical or different and have the meaning of R³ and R⁴ given above, or R⁷ and R⁸ together form a group of the formula =O or =NR¹⁷ wherein R¹⁷ represents hydrogen or straight-chain or branched alkyl, alkoxy or acyl having in each case up to 6 carbon atoms, L represents a straight-chain or branched alkylene or alkenylene chain having in each case up to 8 carbon atoms, which is optionally substituted up to 2 times by hydroxyl, T and X are identical or different and represent a straight-chain or branched alkylene chain having up to 8 carbon atoms, or T or X represents a bond, V represents an oxygen or sulphur atom or represents an -NR¹⁸ group wherein R¹⁸ represents hydrogen or straight-chain or branched alkyl having up to 6 carbon atoms or phenyl, E represents cycloalkyl having 3 to 8 carbon atoms, or represents straight-chain or branched alkyl having up to 8 carbon atoms which is optionally substituted by cycloalkyl having 3 to 8 carbon atoms or hydroxyl, or represents phenyl which is optionally substituted by halogen or trifluoromethyl, R¹ and R² together form a straight-chain or branched alkylene chain having up to 7 carbon atoms, which has to be substituted by a carbonyl group and/or by a group of the formula 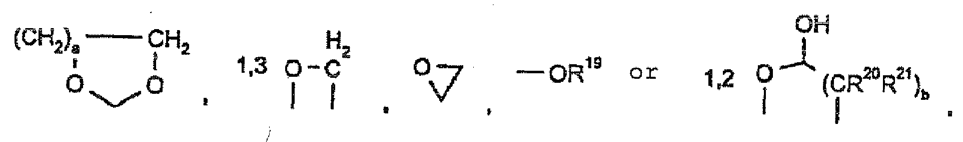
wherein a and b are identical or different and represent a number 1, 2 or 3, R¹⁹ represents hydrogen, cycloalkyl having 3 to 7 carbon atoms, straight-chain or branched silylalkyl having up to 8 carbon atoms, or straight-chain or branched alkyl having up to 8 carbon atoms, which is optionally substituted by hydroxyl, straight-chain or branched alkoxy having up to 6 carbon atoms or by phenyl which for its part may be substituted by halogen, nitro, trifluoromethyl, trifluoromethoxy or by phenyl or tetrazole-substituted phenyl, and alkyl is optionally substituted by a group of the formula -OR²², in which R²² represents straight-chain or branched acyl having up to 4 carbon atoms or benzyl, or R¹⁹ represents straight-chain or branched acyl having up to 20 carbon atoms or benzoyl, which is optionally substituted by halogen, trifluoromethyl, nitro or trifluoromethoxy, or represents straight-chain or branched fluoroacyl having up to 8 carbon atoms and up to 9 fluorine atoms, R²⁰ and R²¹ are identical or different, represent hydrogen, phenyl or straight-chain or branched alkyl having up to 6 carbon atoms, or R²⁰ and R²¹ together form 3 to 6-membered carbocycle and, if appropriate also geminally, the carbocycles formed are optionally substituted up to 6 times by identical or different substituents from the group consisting of trifluoromethyl, hydroxyl, nitrile, halogen, carboxyl, nitro, azido, cyano, cycloalkyl or cycloalkyloxy having in each case 3 to 7 carbon atoms, straight-chain or branched alkoxycarbonyl, alkoxy or alkylthio having in each case up to 6 carbon atoms and straight-chain or branched alkyl having up to 6 carbon atoms, which for its part is substituted up to 2 times by identical or different substituents from the group consisting of hydroxyl, benzyloxy, trifluoromethyl, benzoyl, straight-chain or branched alkoxy, oxyacyl or carboxyl having in each case up to 4 carbon atoms and phenyl, which for its part may be substituted by halogen, trifluoromethyl or trifluoromethoxy, and/or the carbocycles formed are optionally substituted, also geminally, up to 5 times by identical or different substituents from the group consisting of phenyl, benzoyl, thiophenyl and sulphonylbenzyl, which for their part are optionally substituted by halogen, trifluoromethyl, trifluoromethoxy or nitro, and/or are optionally substituted by a group of the formula 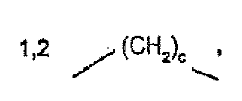
-SO₂-C₆H₅, -(CO)_{d}NR²³R²⁴ or =O, wherein c represents a number 1, 2, 3 or 4, d represents a number 0 or 1, R²³ and R²⁴ are identical or different and represent hydrogen, cycloalkyl having 3 to 6 carbon atoms, straight-chain or branched alkyl having up to 6 carbon atoms, benzyl or phenyl which is optionally substituted up to 2 times by identical or different substituents from the group consisting of halogen, trifluoromethyl, cyano, phenyl and nitro, and/or the carbocycles formed are optionally substituted by a spire-linked group of the formula 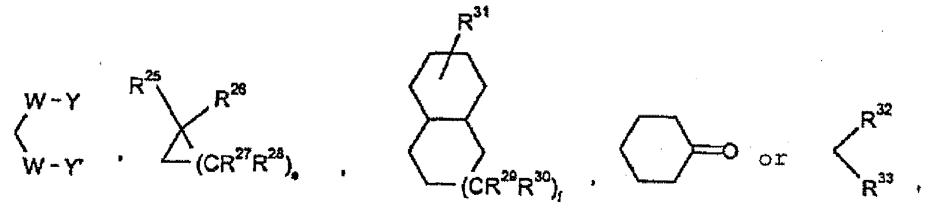
wherein W represents either an oxygen or a sulphur atom, Y and Y' together form a 2- to 6-membered straight-chain or branched alkylene chain, e represents a number 1, 2, 3, 4, 5, 6 or 7, f represents a number 1 or 2, R²⁵, R²⁶, R²⁷, R²⁸, R²⁹, R³⁰ and R³¹ are identical or different and represent hydrogen, trifluoromethyl, phenyl, halogen or straight-chain or branched alkyl or alkoxy having in each case up to 6 carbon atoms, or R²⁵ and R²⁶ or R²⁷ and R²⁸ in each case together form straight-chain or branched alkyl chain having up to 6 carbon atoms, or R²⁵ and R²⁶ or R²⁷ and R²⁸ in each case together form a group of the formula 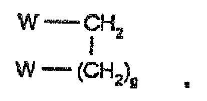
wherein W is as defined above, g represents a number 1, 2, 3, 4, 5, 6 or 7, R³² and R³³ together form 3- to 7-membered heterocycle which contains an oxygen or sulphur atom or a group of the formula SO, SO₂ or -NR³⁴, in which R³⁴ represents hydrogen, phenyl, benzyl or straight-chain or branched alkyl having up to 4 carbon atoms, a salt thereof or N-oxide thereof.

Each term used for the formula (XX) is as described in the above-mentioned publication.

Specifically, the following compound disclosed in WO99/155504 (JP-T-2001-517655) can be mentioned:
Tetrahydro-quinoline of the formula (XXI) 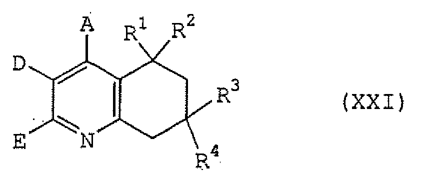 in which
A represents phenyl which is optionally substituted up to 2 times by identical or different substituents from the group consisting of halogen, trifluoromethyl and straight-chain or branched alkyl or alkoxy having in each case up to 3 carbon atoms,
D represents a group of the formula 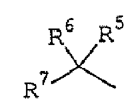 or R⁸-CH₂-O-CH₂-
   in which
R⁵ and R⁶ together form a carbonyl group (=O), or
R⁵ represents hydrogen and
R⁶ represents halogen or hydroxyl, or
R⁵ and R⁶ represent hydrogen,
R⁷ and R⁸ are identical or different and represent phenyl, naphthyl, benzothiazolyl, quinolyl, pyrimidyl or pyridyl which are optionally substituted up to 4 times by identical or different substituents from the group consisting of halogen, trifluoromethyl, nitro, cyano, trifluoromethoxy, or by a group of the formula -SO₂-CH₃ or -NR⁹R¹⁰, in which R⁹ and R¹⁰ are identical or different and represent hydrogen or straight-chain or branched alkyl having up to 3 carbon atoms,
E represents cycloalkyl having 3 to 6 carbon atoms, or represents straight-chain or branched alkyl having up to 8 carbon atoms,
R¹ represents hydroxyl, and
R² represents hydrogen or represents methyl,
R³ and R⁴ are identical or different and represent straight-chain or branched alkyl having up to 3 carbon atoms, or
R³ and R⁴ together form a spire-linked alkyl chain having 2 to 4 carbon atoms,
or a salt thereof or N-oxide thereof.

Each term used for the formula (XXI) is as described in the above-mentioned publication.

Specifically, the following compound disclosed in WO99/15487 (JP-T-2001-517646) can be mentioned:
a compound of the formula (XXII) 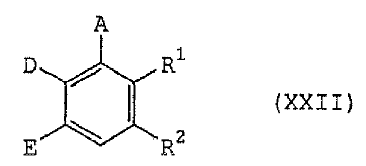
wherein A is cycloalkyl having 3 to 8 carbon atoms, aryl having 6 to 10 carbon atoms, or 5- to 7-membered saturated, partially unsaturated or unsaturated optionally benzo-fused heterocycle having up to 4 hetero atoms from the group consisting of S, N and O, wherein the ring systems of the aryl and said heterocycle are optionally substituted up to 5 times by identical or different substituents from the group consisting of cyano, halogen, nitro, carboxyl, hydroxyl, trifluoromethyl and trifluoromethoxy, or straight chain or branched alkyl, acyl, hydroxyalkyl, alkylthio, alkoxycarbonyl, oxyalkoxycarbonyl or alkoxy, each having up to 7 carbon atoms, or a group of the formula -NR³R⁴, wherein R³ and R⁴ are identical or different and denote hydrogen, phenyl or straight chain or branched alkyl having up to 6 carbon atoms, D denotes a group of the formula R⁵-L- , 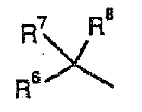 or R⁹-T-V-X- ,
wherein R⁵, R⁶ and R⁹ independently of one another denote aryl having 6 to 10 carbon atoms, or 5- to 7-membered, optionally benzo-fused, saturated or unsaturated, mono-, bi- or tricyclic heterocycle having up to 4 heteroatoms from the group consisting of S, N and O, where the cycles are optionally substituted, in the case of nitrogen-containing rings also via an N functional group, up to 5 times, by identical or different substituents from the group consisting of halogen, trifluoromethyl, nitro, hydroxyl, cyano, carboxyl, trifluoromethoxy, straight-chain or branched acyl, alkyl, alkylthio, alkylalkoxy, alkoxy or alkoxycarbonyl, each having up to 6 carbon atoms, by aryl-or trifluoromethyl-substituted aryl, each having 6 to 10 carbon atoms, or by an optionally benzo-fused, aromatic 5-to 7-membered heterocycle having up to 3 heteroatoms from the group consisting of S, N and O, and/or by a group of the formula -OR¹⁰, -SR¹¹, -SO₂R¹² or -NR¹³R¹⁴, wherein R¹⁰, R¹¹ and R¹² independently of one another denote aryl having 6 to 10 carbon atoms, which for its part is substituted up to 2 times by identical or different substituents from the group consisting of phenyl and halogen, or by straight-chain or branched alkyl having up to 6 carbon atoms, R¹³ and R¹⁴ are identical or different and have the meaning of R³ and R⁴ indicated above, or R⁵ and/or R⁶ denote a group of the formula 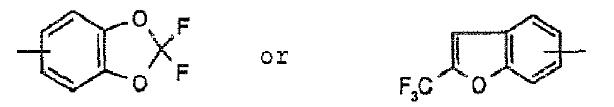
R⁷ denotes a hydrogen, halogen or methyl, R⁸ denotes hydrogen, halogen, azido, trifluoromethyl, hydroxyl, trifluoromethoxy, straight-chain or branched alkoxy or alkyl each having up to 6 carbon atoms or a group of the formula -NR¹⁵R¹⁶, wherein R¹⁵ and R¹⁶ are identical or different and have the meaning of R³ and R⁴ indicated above, or R⁷ and R⁸ together form a group of the formula =O or =NR¹⁷, wherein R¹⁷ is hydrogen or linear or branched alkyl, alkoxy or acyl, each having up to 6 carbon atoms, L is a linear or branched alkylene or alkenylene chain each having up to 8 carbon atoms, each of which is optionally substituted up to 2 times by hydroxyl, T and X are identical or different and denote straight-chain or branched alkylene chain having up to 8 carbon atoms, or T or X denotes a bond, V represents an oxygen or sulfur atom or -NR¹⁸- group, wherein R¹⁸ is a hydrogen or linear or branched alkyl having up to 6 carbon atoms or phenyl, E denotes cycloalkyl having 3 to 8 carbon atoms, or linear or branched alkyl having up to 8 carbon atoms, which is optionally substituted by cycloalkyl having 3 to 8 carbon atoms or hydroxyl, or represents phenyl which is optionally substituted by halogen or trifluoromethyl, R¹ denotes straight chain or branched alkyl having up to 6 carbon atoms, which is substituted by hydroxyl or a group of the formula 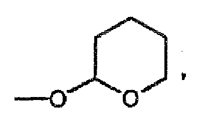
R² denotes hydrogen, or straight chain or branched alkyl or alkenyl each having up to 8 carbon atoms, which is optinonally substituted by hydroxyl, halogen, phenyl, cycloalkyl having 3 to 6 carbon atoms, or a group of the formula -O-R¹⁹, 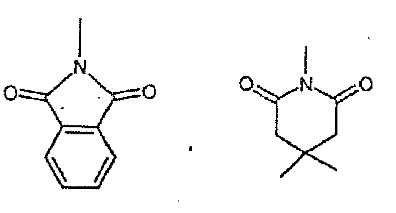 or 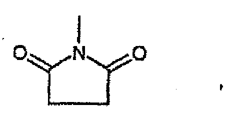
wherein R¹⁹ denotes a group of the formula -Si(CH₃)₂C(CH₃)₃, or straight chain or branched alkyl having up to 6 carbon atoms, or 5- to 7-membered saturated, partially unsaturated or unsaturated heterocycle having up to 3 hetero atoms from the group consisting of S, N and O, or phenyl or benzyl, wherein all ring systems for R¹⁹ are optionally substituted up to 2 times by identical or different substituents from the group consisting of trifluoromethyl, fluorine, nitro, hydroxyl, straight chain or branched alkoxy or alkoxycarbonyl having in each case up to 4 carbon atoms, or by straight chain or branched alkyl having up to 4 carbon atoms optionally substituted by hydroxyl, or a salt thereof.

Each term used for the formula (XXII) is as described in the above-mentioned publication.

Specifically, the compound disclosed in WO00/53792 (Code Names WK-5344A and WK-5344B) can be mentioned.

Specifically, the following compound disclosed in JP-A-2002-293764 can be mentioned:
a compound represented by the formula (XXIII) 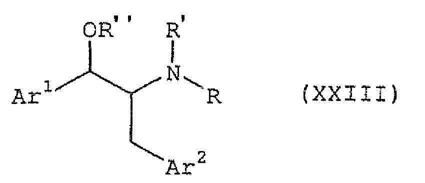
wherein Ar¹ is an aromatic ring group optionally having substituents, Ar² is an aromatic ring group having substituent, OR" is an optionally protected hydroxyl group, R is an acyl group, R' is a hydrogen atom or a hydrocarbon group optionally having substituents, or a salt thereof with the exception of tert-butyl benzyl-[2(S)-hydroxy-2-thiazol-2-yl-1(S)-(4-trifluoromethyl-benzyl)-ethyl]-carbamate.

Each term used for the formula (XXIII) is as described in the above-mentioned publication.

Specifically, CETi-1 (CETP vaccine) disclosed in WO99/20302 (JP-T-2001-520204) can be mentioned.

Furthermore, as a compound having a CETP inhibitory activity in the present invention, a compound selected from the group consisting of Code Names: JTT-705, CP-529414, SC-795, SC-744, SC-554, SC-71952, SC-56960, SC-57201, PD-140195, WK-5344A, WK-5344B, CETi-1 (CETP vaccine), BM99-1 and BM99-2, and the like can be mentioned.

Of these, as a CETP inhibitor, S-{2-([[1-(2-ethylbutyl)cyclohexyl]carbonyl]amino)phenyl} 2-methylpropanethioate, as well as ethyl (2R,4S)-4-[[3,5-bis(trifluoromethyl)benzyl](methoxycarbonyl)amino]-2-ethyl-6-(trifluoromethyl)-3,4-dihydroquinoline-1(2H)-carboxylate, an anhydride thereof, an ethanolate thereof, crystals thereof (JP-T-2003-515592), methyl (3-cyano-5-trifluoromethylbenzyl)-[6-(N-cyclopentylmethyl-N-ethylamino)indan-5-ylmethyl]-carbamate (JP-A-221376/2003) and trans-(4-{[N-(2-{[N'-[3,5-bis(trifluoromethyl)benzyl]-N'-(2-methyl-2H-tetrazo1-5-yl)amino]methyl)-5-methyl-4-trifluoromethylphenyl)-N-ethylamino]methyl}cyclohexyl)-acetic acid methanesulfonate (WO2004/020393) are particularly preferable.

The above-mentioned compounds having a CETP inhibitory activity can be used for
(1) a remnant lipoprotein production inhibitor comprising a compound having a CETP inhibitory activity as an active ingredient,
(2) an inhibitor of transfer of cholesteryl ester in HDL to chylomicron and/or VLDL, which comprises a compound having a CETP inhibitory activity as an active ingredient,
(3) a CETP inhibitor having an inhibitory activity on the transfer of cholesteryl ester in HDL to chylomicron and/or VLDL,
(4) the inhibitor of the above-mentioned (1), wherein the compound having a CETP inhibitory activity inhibits transfer of cholesteryl ester in HDL to chylomicron and/or VLDL due to a CETP inhibitory activity thereof and
(5) a CETP inhibitor that inhibits transfer of cholesteryl ester in HDL to chylomicron and/or VLDL and has a remnant lipoprotein production inhibitory activity.

Moreover, the present invention relates to a commercial package comprising at least one of the inhibitors of the above-mentioned (1) to (5) and a written matter relating to the use thereof.

Each inhibitor (remnant lipoprotein production inhibitor, inhibitor of transfer of cholesteryl ester in HDL to chylomicron and/or VLDL, CETP inhibitor) in the present invention can be generally admixed with pharmacologically acceptable carrier, excipient, diluent, extender, disintegrant, stabilizer, preservative, buffer, emulsifier, aromatic, coloring agent, sweetening agent, thickener, corrigent, dissolution aids, and other additives concretely exemplified by water, vegetable oil, alcohol such as ethanol and benzyl alcohol, polyethylene glycol, glycerol triacetate gelatin, carbohydrates such as lactose, starch and the like, magnesium stearate, talc, lanolin, vaseline and the like, which are known per se, and systemically or topically, orally or parenterally administered to an administration subject (mammals such as human, bovine, horse, dog, cat, rat, mouse, hamster etc.) in the form of tablet, pill, powder, granule, suppository, injection, eye drop, liquid, capsule, troche, aerozol, elixir, suspension, emulsion, syrup and the like. While the dose varies depending on various factors such as the kind of inhibitor, age and body weight of the administration subject, disease, symptoms, treatment effect and the like, and determined appropriately upon consideration of these factors and administered once or several times a day. In addition, the inhibitor of the present invention can be used concurrently with other one or more pharmaceutical agents by a method generally employed for pharmaceutical agents.

The compound having the CEPT inhibitory activity encompasses "pharmaceutically acceptable salt".

The "pharmaceutically acceptable salt" may be any as long as it forms a nontoxic salt with the compound. Examples thereof include, but not limited to, inorganic acid salts such as hydrochloride, hydrobromide, hydroiodide, sulfate, nitrate, phosphate, carbonate, hydrogen carbonate, perchlorate etc.; organic acid salts such as formate, acetate, trifluoroacetate, propionate, oxalate, glycolate, succinate, lactate, maleate, hydroxymaleate, methylmaleate, fumarate, adipate, tartrate, malate, citrate, benzoate, cinnamate, ascorbate, salicylate, 2-acetoxybenzoate, nicotinate, isonicotinate etc.; sulfonates such as methanesulfonate, ethanesulfonate, isethionate, benzenesulfonate, p-toluenesulfonate, naphthalenesulfonate etc.; acidic amino acid salts such as aspartate, glutamate etc.; alkali metal salts such as sodium salt, potassium salt etc.; alkaline earth metal salts such as magnesium salt, calcium salt etc.; ammonium salt; organic base salts such as trimethylamine salt, triethylamine salt, pyridine salt, picoline salt, dicyclohexylamine salt, N,N'-dibenzylethylenediamine salt etc.; and amino acid salts such as lysin salt, arginine salt etc. In some cases, it may be a water-containing product, a hydrate or a solvate with alcohol and the like.

In the compound, various isomers can be present. Examples thereof include E form and Z form as geometric isomers, and, when an asymmetric carbon atom exists, enantiomer and diastereomer are present as stereoisomers based thereon, and tautomer can be present. Accordingly, the compound encompasses all these isomers and a mixture thereof.

Here, the compound having the CEPT inhibitory activity also encompasses prodrug compounds and metabolites.

The "prodrug compound" of the compound having the CEPT inhibitory activity is a derivative of the compound, which has a chemically or metabolically degradable group and restores to the original compound to show an intrinsic efficacy after administration to a living organism. It includes a complex and a salt not based on a covalent bond.

As the prodrug compound, a compound represented by the above formula wherein a carboxyl group has been modified by ethyl group, pivaloyloxymethyl group, 1-(acetyloxy)ethyl group, 1-(ethoxycarbonyloxy)ethyl group, 1-(cyclohexyloxycarbonyloxy)ethyl group, carboxylmethyl group, (5-methyl-2-oxo-1,3-dioxol-4-yl)methyl group, phenyl group, o-tolyl group and the like; a compound represented by the above formula, wherein a hydroxyl group has been modified by acetyl group, propionyl group, isobutyryl group, pivaloyl group, benzoyl group, 4-methylbenzoyl group, dimethylcarbamoyl group or sulfo group; a compound represented by the above formula, wherein an amino group has been modified by hexylcarbamoyl group, 3-methylthio-1-(acetylamino)propylcarbonyl group, 1-sulfo-1-(3-ethoxy-4-hydroxyphenyl)methyl group, (5-methyl-2-oxo-1,3-dioxol-4-yl)methyl group and the like, and the like can be mentioned.

The present invention is useful for the prophylaxis or treatment of diseases such as hyperlipidemia, arteriosclerosis and hyper-remnant lipoproteinemia (e.g., hereditary hyper-remnant lipoproteinemia such as familial dysbetalipidemia, familial lipase deficiency, familial combined hyperlipidemia etc.; and secondary hyper-remnant lipoproteinemia that expresses subsequent to diabetic hypertriglyceridemia, hypothyroidism, nephritic syndrome etc.) and the like.

The present invention is more specifically explained in the following by referring to Examples. The present invention is not limited at all by the Examples. Compound A used in the following experiments refers to S-{2-([[1-(2-ethylbutyl)cyclohexyl]carbonyl]amino)phenyl} 2-methylpropanethioate; Compound B refers trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl)cyclohexyl)acetic acid methanesulfonate, Compound C refers methyl (3-cyano-5-trifluoromethylbenzyl)-[6-(N-cyclopentylmethyl-N-ethylamino)indan-5-ylmethyl]-carbamate [i.e., methyl N-(3-cyano-5-trifluoromethylbenzyl)-[6-(N'-cyclopentylmethyl-N'-ethylamino)indan-5-ylmethyl]carbamate], Compound D refers ethyl (2R,4S)-4-[[3,5-bis(trifluoromethyl)benzyl](methoxycarbonyl)amino]-2-ethyl-6-(trifluoromethyl)-3,4-dihydroquinoline-1(2H)-carboxylic acid ester [i.e., ethyl (2R,4S)-4-[N-[3,5-bis(trifluoromethyl)benzyl]-N-(methoxycarbonyl)amino]-2-ethyl-6-(trifluoromethyl)-3,4-dihydroquinoline-1(2H)-carboxylic acid ester].

### Examples

### Preparation of Donor Lipoprotein

Potassium bromide (KBr) was added to plasma (40 mL) of healthy subject and the mixture was adjusted to have a specific density d=1.125 g/mL. The mixture was subjected to density gradient centrifugation (227,000×g, 4°C, 17 hr) and a fraction showing a specific density of d>1.125 g/mL (HDL₃ fraction) was harvested. The obtained fraction was dialyzed against PBS solution [10 mmol/L Na₂HPO₄; 10 mmol/L NaH₂PO₄; 0.15 mol/L NaCl; 1 mmol/L EDTA (pH 7.4)]. Then, tritium-labeled cholesterol (37 MBq) was dissolved in 95% ethanol, gradually added to the above-mentioned HDL₃ fraction with stirring and incubated at 37°C for 18 hr. [By this operation, tritium-labeled cholesterol was esterified by the action of lecithin cholesterol acyl transferase (LCAT) present on the HDL₃ surface and taken up into HDL₃ as tritium-labeled cholesteryl ester ([³H]CE)]. After incubation, KBr was added, the mixture was adjusted to have a specific density d=1.21 g/mL, subjected to density gradient centrifugation (227,000×g, 4°C, 17 hr) and a fraction of specific density d>1.21 g/mL was harvested. The obtained fraction was dialyzed against the aforementioned PBS solution to give HDL₃ incorporating [³H]CE ([³H]CE-HDL₃, specific density: 1.125<d<1.21, specific activity: 22,000 - 33,000 dpm/µL), which was used as a donor lipoprotein.

### Experimental Example 1: in vitro effect on remnant lipoprotein production in plasma

Compound A, Compound B, Compound C and Compound D were previously dissolved in dimethyl sulfoxide (DMSO). Plasma (200 µL) of healthy subject, and a solution (2 µL) of each compound in DMSO or DMSO (2 µL) were added into a microtube. On the other hand, a monoclonal antibody against human CETP was diluted with physiological saline and added into a microtube together with plasma (190 µL) of healthy subject and a diluted solution (10 µL) of the monoclonal antibody or physiological saline (10 µL). These were incubated at 37°C or 4°C for 6 hr (Compound A) or 4 hr (Compound B, Compound C, Compound D, the monoclonal antibody) . After ice-cooling, the cholesterol amount in the remnant fraction was measured using "RLP-cholesterol "JIMRO II" " (manufactured by Japan Immunoresearch Laboratories Co., Ltd.). The difference in the measured values from incubation of solvent alone at 4°C and 37°C, namely, an increase in the remnant cholesterol was taken as a 100% production amount of remnant lipoprotein, and the percent of decrease in the measured values of incubation at 37°C with the addition of each compound or the antibody was taken as percent inhibition of remnant lipoprotein production. The results are shown in the following.

**Table 1**

| Compound A concentration (µM) | Inhibition (%) of remnant lipoprotein production |
|---|---|
| 0 | 0 |
| 3 | 25 |
| 10 | 54 |
| 30 | 77 |

**Table 2**

| Compound B concentration (µM) | Inhibition (%) of remnant lipoprotein production |
|---|---|
| 0 | 0 |
| 0.01 | 29 |
| 0.03 | 36 |
| 0.1 | 42 |
| 0.3 | 52 |
| 1 | 84 |

**Table 3**

| Compound C concentration (µM) | Inhibition (%) of remnant lipoprotein production |
|---|---|
| 0 | 0 |
| 0.1 | 21 |
| 0.3 | 34 |
| 1 | 41 |
| 3 | 93 |

**Table 4**

| Compound D concentration (µM) | Inhibition (%) of remnant lipoprotein production |
|---|---|
| 0 | 0 |
| 0.01 | 38 |
| 0.03 | 32 |
| 0.1 | 44 |
| 0.3 | 59 |
| 1 | 62 |

**Table 5**

| Monoclonal antibody concentration (µg/mL) | Inhibition (%) of remnant lipoprotein production |
|---|---|
| 0 | 0 |
| 3 | 32 |
| 10 | 49 |
| 30 | 91 |
| 100 | 84 |

### Experimental Example 2: effect on (cholesteryl ester transfer from donor lipoprotein to remnant lipoprotein

The donor lipoprotein obtained above was added to the plasma of healthy subject to prepare a [³H]CE-HDL₃-containing plasma (2,000 - 4,000 dpm/µL). Compound A, Compound B, Compound C and Compound D were dissolved in dimethyl sulfoxide (DMSO). A solution of each compound in DMSO (1 µL) or DMSO (1 µL), and the [³H]CE- HDL₃-containing plasma (100 µL) were added into a microtube. On the other hand, the monoclonal antibody was diluted with physiological saline and added into a microtube together with a diluted solution (5 µL) of the monoclonal antibody or physiological saline (5 µL), and [³H]CE-HDL₃ containing plasma (95 µL). These were incubated at 37°C or 4°C for 6 hr (Compound A) or 4 hr (Compound B, Compound C, Compound D and the monoclonal antibody). After ice-cooling, a remnant fraction was harvested using "RLP-cholesterol "JIMRO II" " (manufactured by Japan Immunoresearch Laboratories Co., Ltd.), and the radioactivity in the fraction was measured with a liquid scintillation counter. The difference in the measured values from incubation of solvent alone at 4°C and 37°C was taken as a 100% cholesteryl ester transfer activity, and the percent of decrease in the measured values of incubation at 37°C with the addition of each compound or the antibody was taken as percent inhibition of cholesteryl ester transfer.

The CETP activity was measured according to the method shown below using a part of the same plasma after incubation. After ice-cooling, plasma (80 µL) was separated and a TBS solution [80 µL, 20 mmol/L Tris; 0.15 mol/L Nacl (pH 7.4)] containing 50 mmol/L magnesium chloride and 0.1% dextran sulfate was added to each microtube and thoroughly stirred. After standing at 4°C for 30 min, the mixture was centrifuged (10,000xg, 4°C, 10 min.), and the radioactivity in the obtained supernatant (HDL fraction) was measured with a scintillation counter. The difference in the measured values from incubation of solvent alone at 4°C and 37°C was taken as a 100% CETP activity, and the percent of decrease in the measured values of incubation at 37°C with the addition of each compound or the antibody was taken as percent inhibition of CETP activity. The above results are shown in the following.

**Table 6**

| Compound A concentration (µM) | Inhibition (%) of cholesteryl ester transfer to remnant fraction | Inhibition (%) of CETP activity |
|---|---|---|
| 0 | 0 | 0 |
| 3 | 21 | 3 |
| 10 | 29 | 34 |
| 30 | 92 | 75 |

**Table 7**

| Compound B concentration (µM) | Inhibition (%) of cholesteryl ester transfer to remnant fraction | Inhibition (%) of CETP activity |
|---|---|---|
| 0 | 0 | 0 |
| 0.01 | 15 | 10 |
| 0.03 | 26 | 25 |
| 0.1 | 55 | 53 |
| 0.3 | 89 | 74 |
| 1 | 100 | 86 |

**Table 8**

| Compound C concentration (µM) | Inhibition (%) of cholesteryl ester transfer to remnant fraction | Inhibition (%) of CETP activity |
|---|---|---|
| 0 | 0 | 0 |
| 0.1 | -25 | 6 |
| 0.3 | 9 | 12 |
| 1 | 35 | 31 |
| 3 | 92 | 57 |

**Table 9**

| Compound D concentration (µM) | Inhibition (%) of cholesteryl ester transfer to remnant fraction | Inhibition (%) of CETP activity |
|---|---|---|
| 0 | 0 | 0 |
| 0.01 | 12 | 9 |
| 0.03 | 28 | 24 |
| 0.1 | 54 | 53 |
| 0.3 | 78 | 73 |
| 1 | 94 | 83 |

**Table 10**

| Monoclonal antibody concentration (µg/mL) | Inhibition (%) of cholesteryl ester transfer to remnant fraction | Inhibition (%) of CETP activity |
|---|---|---|
| 0 | 0 | 0 |
| 3 | 19 | 18 |
| 10 | 57 | 47 |
| 30 | 67 | 68 |
| 100 | 90 | 80 |

From the foregoing test results and the like, it is considered that CETP promotes remnant lipoprotein production, and a CETP inhibitor and an anti-CETP antibody can suppress production of remnant lipoprotein by inhibiting cholesteryl ester transfer from HDL. It is clear,that the remnant cholesterol is a risk factor of promotion of arteriosclerosis, and a CETP inhibitor and an anti-CETP antibody are useful for the prophylaxis or treatment of hyperlipidemia or hyper-remnant lipoproteinemia, as well as for the prophylaxis or treatment of arteriosclerotic diseases (e.g., atherosclerosis, myocardial infarction, cerebral infarction, angina pectoris, peripheral vascular disease, cardiovascular disorder, ischemia, cardiac ischemia, stroke, Ischemia-reperfusion injury, restenosis after angioplasty, vascular complication of diabetes and the like) and the like, because they can decrease remnant cholesterol in plasma.

### INDUSTRIAL APPLICABILITY

The present invention provides a prophylactic drug or a therapeutic drug of hyperlipidemia, arteriosclerosis or hyper-remnant lipoproteinemia (hyper-remnant-emia) in which a remnant lipoprotein is involved (e.g., hereditary hyper-remnant lipoproteinemia such as familial dysbetalipidemia, familial lipase deficiency, familial combined hyperlipidemia and the like; or secondary hyper-remnant lipoproteinemia that expresses subsequent to diabetic hypertriglyceridemia, hypothyroidism, nephritic syndrome and the like) by selectively inhibiting CETP.

This application is based on a patent application No. 373453/2003 filed in Japan and a provisional application No. 60/590,811 filed in U.S.A., the contents of which are hereby incorporated by preference.

## Claims

1. A dibenzylamine compound represented by the formula (IX) 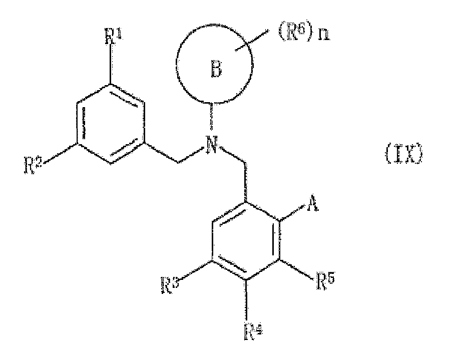 wherein
R¹ and R² are the same or different and each is a halogen atom, a nitro group, a cyano group or a C₁₋₆ alkyl group optionally substituted by halogen atom;
R³, R⁴ and R⁵ are the same or different and each is a hydrogen atom, a halogen atom, a C₁₋₆ alkyl group optionally substituted by halogen atom, a C₁₋₆ alkylthio group optionally substituted by halogen atom or a C₁₋₆ alkoxy group optionally substituted by halogen atom, or R³ and R⁴ or R⁴ and R⁵ may form, together with a carbon atom they are bonded to, a homocyclic ring optionally having substituent(s) or a heterocyclic ring optionally having substituent(s);
A is -N(R⁷) (R⁸) (wherein R⁷ and R⁸ are the same or different and each is a hydrogen atom, a C₁₋₆ alkyl group (wherein C₁₋₆ alkyl group is optionally substituted by phenyl group or -(CH₂)ₘ-COOR⁹ (wherein R⁹ is a hydrogen atom or a C₁₋₆ alkyl group and m is 0 or an integer of 1 to 5)) or a C₄₋₁₀ cycloalkylalkyl group (wherein C₄₋₁₀ cycloalkylalkyl group is optionally substituted by 1 to 3 substituents from halogen atom, nitro group, amino group, hydroxyl group, cyano group, acyl group, C₁₋₆ alkoxy group, C₁₋₆ alkyl group (wherein C₁₋₆ alkyl group is optionally substituted by hydroxyl group, C₁₋₆ alkoxy group or phosphono group), -(CH₂)_{q}-CON(R²⁰) (R²¹) (wherein R²⁰ and R²¹ are the same or different and each is hydrogen atom or C₁₋₆ alkyl group and q is 0 or an integer of 1 to 5) or -(CH₂)ᵣ-COOR¹⁰ (wherein R¹⁰ is hydrogen atom or C₁₋₆ alkyl group and r is 0 or an integer of 1 to 5)), -C(R¹¹)(R¹²)(R¹³) (wherein R¹¹, R¹² and R¹³ are the same or different and each is a hydrogen atom, a C₁₋₆ alkyl group (wherein C₁₋₆ alkyl group is optionally substituted by phenyl group or -COOR⁹ (wherein R⁹ is as defined above)) or a C₄₋₁₀ cycloalkylalkyl, group (wherein C₄₋₁₀ cycloalkylalkyl group is optionally substituted by 1 to 3 substituents from halogen atom, nitro group, amino group, hydroxyl group, cyano group, acyl group, C₁₋₆ alkoxy group, C₁₋₆ alkyl group (wherein C₁₋₆ alkyl group is optionally substituted by hydroxyl group, C₁-₆ alkoxy group or phosphono group), -(CH₂)_{q}-CON(R²⁰)(R²¹) (wherein R²⁰, R²¹ and q are as defined above) or -(CH₂)ᵣ-COOR¹⁰ (wherein R¹⁰ and r are as defined above))) or -O-C(R¹¹)(R¹²)(R¹³) (wherein R¹¹, R¹² and R¹³ are as defined above);
ring B is an aryl group or a heterocyclic residue;
_{R}⁶ is a hydrogen atom, a halogen atom, a nitro group, an amino group, a hydroxyl group, a cyano group, an acyl group, a C₁₋₆ alkoxy group, a C₂₋₆ alkenyl group or a C₁-₆ alkyl group (wherein C₁₋₆ alkyl group is optionally substituted by hydroxyl group or -COOR¹⁴ (wherein R¹⁴ is a hydrogen atom or a C₁₋₆ alkyl group)); and
n is an integer of 1 to 3
or a prodrug thereof or a pharmaceutically acceptable salt thereof, for use in the prophylaxis or treatment of a disease selected from the group consisting of hyperlipidemia, arteriosclerosis and hyper-remnant-emia.

2. The compound for use of claim 1, wherein the compound of the formula (IX) is a compound selected from a group consisting of
N-[3-(N'-cyclopentylmethyl-N'-ethylamino)-5,6,7,8-tetrahydronaphthalen-2-ylmethyl]-N-[3,5-bis(trifluoromethyl)benzyl]-(2-methyl-2H-tetrazol-5-yl)amine,
3-{[N-[3-(N'-cyclopentylmethyl-N'-ethylamino)-5,6,7,8-tetrahydronaphthalen-2-ylmethyl]-N-(2-methyl-2H-tetrazol-5-yl)amino]methyl}-5-trifluoromethylbenzonitrile,
N-[3,5-bis(trifluoromethyl)benzyl]-N-6-(N'-cyclopentylmethyl-N'-ethylamino)indan-5-ylmethyl]-(2-methyl-2H-tetrazol-5-yl)amine,
N-[3,5-bis(trifluoromethyl)benzyl]-N-[6-(N'-cyclopentylmethyl-N'-ethylamino)indan-5-ylmethyl]-(2-methyl-2H-tetrazol-5-yl)amine hydrochloride,
N-[6-(N'-cyclopentylmethyl-N'-ethylamino)indan-5-ylmethyl]-N-(2H-tetrazol-5-yl)-[3,5-bis(trifluoromethyl-)benzyl]amine,
N-[6-(N'-cyclopentylmethyl-N'-ethylamino)indan-5-ylmethyl]-N-[3,5-bis(trifluoromethyl)benzyl]-(pyrimidin-2-yl)amine hydrochloride,
N-[6-(N'-cyclopentylmethyl-N'-ethylamino)indan-5-ylmethyl]-N-[3,5-bis(trifluoromethyl)benzyl]-(5-methyl-1H-pyraxol-3-yl)amine,
5-{N-(6-{ [N'-[3,5-bis(trifluoromethyl)benzyl]-N'-(5-methyl-[1,2,4]oxadiazol-3-yl)amino]methyl}indan-5-y1)-N-ethylamino}pentanoic acid hydrochloride,
methyl trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylate,
3-{ [N-[6-(N'-cyclopentylmethyl-N'-ethylamino)indan-5-ylmethyl]-N-(2-methyl-2H-tetrazol-5-yl)amino]methyl}-5-trifluoromethylbenzonitrile,
N-[3,5-bis(trifluoromethyl)benzyl]-N-[6-(N'-cyclopentylmethyl-N'-ethylamino)indan-5-ylmethyl]-(2-ethyl-2H-tetrazol-5-yl)amine,
N-[3,5-bis(trifluoromethyl)benzyl]-N-[6-(N'-cyclopentylmethyl-N'-ethylamino)indan-5-ylmethyl]-(1-methyl-1H-[1,2,4]triazol-3-yl)amine,
3-({N-[6-(N'-cyclopentylmethyl-N'-ethylamino)indan-5-ylmethyl]-N-phenylamino}methyl)-5-trifluoromethylbenzonitrile,
3-{[N-[6-(N'-cyclopentylmethyl-N'-ethylamino)indan-5-ylmethyl]-N-(4,5-dimethylthiazol-2-yl)amino]methyl}-5-trifluoromethylbenzonitrile,
N-[3,5-bis(trifluoromethyl)benzyl]-N-[6-(N'-cyclopentylmethyl-N'-ethylamino)indan-5-ylmethyl]-(thiazol-2-yl)amine hydrochloride,
3-({N-[6-(N'-cyclopentylmethyl-N'-ethylamino)indan-5-ylmethyl]-N-(thiazol-2-yl)amino}methyl)-5-trifluoromethylbenzonitrile hydrochloride,
N-[3,5-bis(trifluoromethyl)benzyl]-N-[6-(N'-cyclopentylmethyl-N'-ethylamino)indan-5-ylmethyl]-(oxazol-2-yl)amine hydrochloride,
N-[3,5-bis(trifluoromethyl)benzyl]-N-[6-(N'-cyclopentylmethyl-N'-ethylamino)indan-5-ylmethyl]-(5-methylthiazol-2-yl)amine hydrochloride,
N-[3,5-bis(trifluoromethyl)benzyl]-N-[6-(N'-cyclopentylmethyl-N'-ethylamino)indan-5-ylmethyl]-(4-methylthiazol-2-yl)amine hydrochloride,
N-[3,5-bis(trifluoromethyl)benzyl]-N-[6-(N'-cyclopentylmethyl-N'-ethylamino)indan-5-ylmethyl]-(4,5-dimethylthiazol-2-yl)amine hydrochloride,
3-{[N-[6-(N'-cyclopentylmethyl-N'-ethylamino)indan-5-ylmethyl]-N-(5-methylthiazol-2-yl)amino]methyl}-5-trifluoromethylbenzonitrile hydrochloride,
3-{[N-[6-(N'-cyclopentylmethyl-N'-ethylamino)indan-5-ylmethyl]-N-(4-methylthiazol-2-yl)amino]methyl}-5-trifluoromethylbenzonitrile hydrochloride,
N-[3,5-bis(trifluoromethyl)benzyl]-N-[6-(N'-cyclopentylmethyl-N'-ethylamino)indan-5-ylmethyl]-(4-methyloxazol-2-yl)amine hydrochloride,
N-[3,5-bis(trifluoromethyl)benzyl]-N-[6-(N'-cyclopentylmethyl-N'-ethylamino)indan-5-ylmethyl]-(3-methylisothiazol-5-yl)amine hydrochloride,
N-[3,5-bis(trifluoromethyl)benzyl]-N-[6-(N'-cyclopentylmethyl-N'-ethylamino)indan-5-ylmethyl]-(5-methylisoxazol-3-yl)amine hydrochloride,
N-[3,5-bis(trifluoromethyl)benzyl]-N-[6-(N'-cyclopentylmethyl-N'-ethylamino)indan-5-ylmethyl]-(3-methylisoxazol-5-yl)amine hydrochloride,
N-[3,5-bis(trifluoromethyl)benzyl]-N-[6-(N'-cyclopentylmethyl-N'-ethylamino)indan-5-ylmethyl]-(1-methyl-1H-pyrazol-3-yl)amine hydrochloride,
N-[3,5-bis(trifluoromethyl)benzyl]-N-[6-(N'-cyclopentylmethyl-N'-ethylamino)indan-5-ylmethyl]-(1-methyl-1H-pyrazol-4-yl)amine hydrochloride,
N-[3,5-bis(trifluoromethyl)benzyl]-N-[6-(N'-cyclopentylmethyl-N'-ethylamino)indan-5-ylmethyl]-(5-methyl-[1,3,4]thiadiazol-2-yl)amine hydrochloride,
N-[3,5-bis(trifluoromethyl)benzyl]-N-[6-(N'-cyclopentylmethyl-N'-ethylamino)indan-5-ylmethyl]-(5-methyl-[1,3,4]oxadzazol-2-yl)amine hydrochloride,
N-[3,5-bis(trifluoromethyl)benzyl]-N-[6-(N'-oyclopentylmethyl-N'-ethylamino)indan-5-ylmethyl]-pyridin-3-ylamine hydrochloride,
N-[3,5-bis(trifluoromethyl)benzyl]-N-[6-(N'-cyclopentylmethyl-N'-ethylamino)indan-5-ylmethyl]-pyridin-2-ylamine hydrochloride,
N-[3,5-bis(trifluoromethyl)benzyl]-N-[2-(N'-cyclopentylmethyl-N'-ethylamino)-5-trifluoromethylbenzyl]-(2-methyl-2H-tetrazol-5-yl)amine hydrochloride,
3-{[N-[2-(N'-cyclopentylmethyl-N'-ethylamino)-5-trifluoromethylbenzyl]-N-(2-metl-iyl-2H-tetrazol-5-yl)amino]methyl}-5-trifluoromethylbenzonitrile hydrochloride,
methyl 5-[N-(6-{ [N'-[3,5-bis(trifluoromethyl)benzyl]-N'-(2-methyl-2H-tetrazol-5-yl)amino]methyl}indan-5-yl)-N-ethylamino]pentanoate hydrochloride,
methyl 5-[N-(6-{ [N'-(3-cyano-5-trifluoromethylbenzyl)-N'-(2-methyl-2H-tetrazol-5-yl)amino]methyl}indan-5-ylmethyl)-N-ethylamino]pentanoate hydrochloride,
methyl 5-[N-(6-{[N'-[3,5-bis(trifluoromethyl)benzyl]-N'-(1-methyl-1H-pyrazol-3-yl)amino]methyl}indan-5-yl)-N-ethylamino]pentanoate hydrochloride,
5-[N-(6-{[N'-[3,5-bis(trifluoromethyl)benzyl]-N'-(2-methyl-2H-tetrazol-5-yl)amino]methyl}indan-5-yl)-N-ethylamino]pentanoic acid hydrochloride,
5-[N-(6-{ [N'-(3-cyano-5-trifluoromethylbenzyl)-N'-(2-methyl-2H-tetrazol-5-yl)amino]methyl}indan-5-yl)-N-ethylamino]pentanoic acid hydrochloride,
5-[N-(6-{[N'-[3,5-bis(trifluoromethyl)benzyl]-N'-(3-methylisoxazol-5-yl)amino]methyl}indan-5-yl)-N-ethylamino]pentanoic acid hydrochloride,
5-[N-(6-{[N'-[3,5-bis(trifluoromethyl)benzyl]-N'-(1-methyl-1H-pyrazol-3-yl-)amino]methyl}indan-5-yl)-N-ethylamino]pentanoic acid hydrochloride,
methyl trans-4-{[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]methyl}cyclohexanecarboxylate hydrochloride,
methyl trans-4-{[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]methyl}cyclohexanecarboxylate hydrochloride,
trans-4-{ [N-(3-{ [N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]methyl}cyclohexanecarboxylic acid hydrochloride,
trans-4-{[N-(3-{[N'-(3-cyano-5-trifluoromethylbenzyl)-N'-(1-methyl-1H-pyrazol-3-yl)amⁱno]methyl}-5,6,7,8-tetrahydronaphthalen-2-yl)-N-ethylamino]methyl}cyclohexanecarboxylic acid hydrochloride,
N-[3,5-bis(trifluoromethyl)benzyl]-N-[6-(N'-cyclopentylmethyl-N'-ethylamino)indan-5-ylmethyl]-(5-methyl-[1,2,4]oxadiazol-3-yl)amine hydrochloride,
methyl trans-4-{[N-(6-{[N'-[3,5-bis(trifluoromethyl)benzyl]-N'-(1-methyl-1H-pyrazol-3-yl)amino]methyl}indan-5-yl)-N-ethylamino]methyl}cyclohexanecarboxylate hydrochloride,
trans-4-{[N-(6-{[N'-[3,5-bis(trifluoromethyl)benzyl]-N'-(1-methyl-1H-pyrazol-3-yl)amino]methyl}indan-5-y1)-N-ethylamino]methyl}cyclohexanecarboxylic acid hydrochloride,
methyl trans-4-{[N-(6-{[N'-{[N'-3-cyano-5-trifluoromethylbenzyl)-N'-(1-methyl-1H-pyrazol-3-yl)amino]methyl}indan-5-yl)-N-ethylamino]methyl}cyclohexanecarboxylate,
trans-4-{ [N-(6-{ [N'-(3-cyano-5-trifluoromethylbenzyl)-N'-(1-methyl-1H-pyrazol-3-yl)amino]methyl}indan-5-yl)-N-ethylamino]methyl}cyclohexanecarboxylic acid hydrochloride,
trans-4-{[N-(6-{[N'-[3,5-bis(trifluoromethyl)benzyl]-N'-(2-methyl-2H-tetrazol-5-yl)amino]methyl}indan-5-yl)-N-ethylamino]methyl}cyclohexanecarboxylic acid hydrochloride,
trans-4-{ [N-(6-{ [N'-(3-cyano-5-trifluoromethylbenzyl)-N'-(2-methyl-2H-tetrazol-5-yl)amino]methyl}indan-5-yl)-N-ethylamino]methyl}cyclohexanecarboxylic acid hydrochloride,
trans-4-{ [N-(6-{ [N'-[3,5-bis(trifluoromethyl)benzyl]-N'-(3-methylisoxazol-5-yl)amino]methyl}indan-5-yl)-N-ethylamino]methyl}cyclohexanecarboxylic acid hydrochloride,
trans-4-{ [N-(6-{ [N'-(3-cyano-5-trifluoromethylbenzyl)-N'-(3-methylisoxazol-5-yl)amino]methyl}indan-5-yl)-N-ethylamino]methyl}cyclohexanecarboxylic acid hydrochloride,
methyl 5-[N-(6-([N'-(3-cyano-5-trifluoromethylbenzyl)-N'-(1-methyl-1H-pyrazol-3-yl)amino]methyl}indan-5-yl)-N-ethylamino]pentanoate hydrochloride,
5-[N-(6-{ [N'-(3-cyano-5-trifluoromethylbenzyl)-N'-(3-methyl-isoxazol-5-yl)amino]methyl}indan-5-yl)-N-ethylamino]pentanoic acid hydrochloride,
5-[N-(6-{ [N'-(3-cyano-5-trifluoromethylbenzyl)-N'-(1-methyl-1H-pyrazol-3-yl)amino]methyl}indan-5-yl)-N-ethylamino]pentanoic acid hydrochloride,
5-[N-(6-{[N'-[3,5-bis(trifluoromethyl)benzyl]-N'-(1-methyl-1H-[1,2,4]triazol-3-yl)amino]methyl}indan-5-yl)-N-ethylamino]pentanoic acid hydrochloride,
trans-4-{[N-(6-{[N'-[3,5-bis(trifluoromethyl)benzyl]-N'-(1-methyl-1H-[1,2,4]triazol-3-yl)amino]methyl}indan-5-yl)-N-ethylamino]methyl}cyclohexanecarboxylic acid hydrochloride,
trans-4-{[N-(6-{[N'-(3-cyano-5-trifluoromethylbenzyl)-N'-(1-methyl-1H-[1,2,4]triazol-3-yl)amino]methyl}indan-5-yl)-N-ethylamino]methyl}cyclohexanecarboxylic acid,
N-[3,5-bis(trifluoromethyl)benzyl]-N-[6-(N'-cyclopentylmethyl-N'-ethylamino)indan-5-ylmethyl]-(3-methyl-[1,2,4]thiadiazol-5-yl)amine,
5-[N-(6-{ [N'-(3-cyano-5-trifluoromethylbenzyl)-N'-(1-methyl-1H-[1,2,4]triazol-3-yl)amino]methyl}indan-5-yl)-N-ethylamino]pentanoic acid hydrochloride,
methyl 5-[N-(3-{[N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]pentanoate hydrochloride,
methyl 5-[N-(3-{[N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]pentanoate hydrochloride,
5-[N-(3-{ [N'-[3,5-bis(trifluoromethyl)benzyl]-N'-(1-methyl-1H-pyrazol-3-yl)amino]methyl}-5,6,7,8-tetrahydronaphthalen-2-yl)-N-ethylamino]pentanoic acid hydrochloride,
5-[N-(3-{ [N'-(3-cyano-5-trifluoromethylbenzyl)-N'-(1-methyl-1H-pyrazol-3-yl)amino]methyl}-5,6,7,8-tetrahydronaphthalen-2-yl)-N-ethylamino]pentanoic acid hydrochloride,
trans-4-{[N-(3-{[N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]methyl}cyclohexanecarboxylic acid hydrochloride,
trans-4-{[N-(3-{[N'-(3-cyano-5-trifluoromethylbenzyl)-N'-(2-methyl-2H-tetrazol-5-yl)amino]methyl}-5,6,7,8-tetrahydronaphthalen-2-yl)-N-ethylamino]methyl}cyclohexanecarboxylic acid hydrochloride,
trans-4-{[N-(3-{[N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]methyl}cyclohexanecarboxylic acid hydrochloride,
trans-4-{[N-(3-{[N'(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]methyl}cyclohexanecarboxylic acid,
trans-4-{[N-(3-{[N'-[3,5-bis(trifluoromethyl)benzyl]-N'-(3-methylisoxazol-5-yl)amino]methyl}-5,6,7,8-tetrahydronaphthalen-2-yl)-N-ethylamino]methyl}cyclohexanecarboxylic acid hydrochloride,
trans-4-{ [N-(3-{ [N'-(3-cyano-5-trifluoromethylbenzyl)-N'-(3-methylisoxazol-5-yl)amino]methyl}-5,6,7,8-tetrahydronaphthalen-2-yl)-N-ethylamino]methyl}cyclohexanecarboxylic acid hydrochloride,
2-(5-{N-[3,5-bis(trifluoromethyl)benzyl]-N-[6-(N'-cyclopentylmethyl-N'-ethylamino)indan-5-ylmethyl]amino}tetrazol-2-yl)ethanol hydrochloride,
methyl 5-[N-(3-([N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]pentanoate hydrochloride,
methyl 5-[N-(3-{ [N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]pentanoate hydrochloride,
5-[N-(3-{[N'-[3,5-bis(trifluoromethyl)benzyl]-N'-(1-methyl-1H-[1,2,4]triazol-3-yl)amino]methyl}-5,6,7,8-tetrahydronaphthalen-2-yl)-N-ethylamino]pentanoic acid hydrochloride,
5-[N-(3-{ [N'-(3-cyano-5-trifluoromethylbenzyl)-N'-(1-methyl-1H-[1,2,4]triazol-3-yl)amino]methyl}-5,6,7,8-tetrahydronaphthalen-2-yl)-N-ethylamino]pentanoic acid hydrochloride,
5-[N-(3-{[N'-[3,5-bis(trifluoromethyl)benzyl]-N'-(3-methylisoxazol-5-yl)amino]methyl}-5,6,7,8-tetrahydronaphthalen-2-yl)-N-ethylamino]pentanoic acid hydrochloride,
5-[N-(3-{ [N'-(3-cyano-5-trifluoromethylbenzyl)-N'-(3-methylisoxazol-5-yl)amino]methyl}-5,6,7,8-tetrahydronaphthalen-2-yl)-N-ethylamino]pentanoic acid hydrochloride,
5-[N-(3-{[N'-[3,5-bis(trifluoromethyl)benzyl]-N'-(2-methyl-2H-tetrazol-5-yl)amino]ethyl}-5,6,7,8-tetrahydronaphthalen-2-yl)-N-ethylamino]pentanoic acid hydrochloride,
5-[N-(3-{[N'-(3-cyano-5-trifluoromethylbenzyl)-N'-(2-methyl-2H-tetrazol-5-yl)amino]methyl}-5,6,7,8-tetrahydronaphthalen-2-yl)-N-ethylamino]pentanoic acid hydrochloride,
trans-4-{[N-(6-{[N'-[3,5-bis(trifluoromethyl)benzyl]-N'-(5-methyl-[1,2,4]oxadiazol-3-yl}amino]methyl}indan-5-yl)-N-ethylamino]methyl}cyclohexanecarboxylic acid,
5-[N-(2-{[N'-[3,5-bis(trifluoromethyl)benzyl]-N'-(1-methyl-1H-pyrazol-3-yl)amino]methyl)-4-trifluoromethoxyphenyl)-N-ethylamino]pentanoic acid hydrochloride,
5-[N-(2-{[N'-[3,5-bis(trifluoromethyl)benzyl]-N'-(1-methyl-1H-pyrazol-3-yl)amino]methyl)-4-trifluoromethylphenyl)-N-ethylamino]pentanoic acid hydrochloride,
5-[N-(2-{[N'-(3-cyano-5-trifluoromethylbenzyl)-N'-(1-methyl-1H-pyrazol-3-yl)amino]methyl}-4-trifluoromethylphenyl)-N-ethylamino]pentanoic acid hydrochloride,
5-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]pentanoic acid hydrochloride,
5-[N-(6-{[N'-[3,5-bis(trifluoromethyl)benzyl]-N'-(2-ethyl-2H-tetrazol-5-yl)amino]methyl}indan-5-yl)-N-ethylamino]pentanoic acid hydrochloride,
5-{N-[6-({N'-[3,5-bis(trifluoromethyl)benzyl]-N'-[2-(2-hydroxyethyl)-2H-tetrazol-5-yl]amino}methyl)indan-5-yl]-N-ethylamino}pentanoic acid hydrochloride,
5-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]pentanoic acid hydrochloride,
5-[N-(6-([N'-[3,5-bis(trifluoromethyl)benzyl]-N'-(2-methyl-2H-tetrazol-5-yl)amino]methyl)indan-5-yl)-N-ethylamino]-2,2-dimethylpentanoic acid hydrochloride, 6-[N-(6-{[N'-(3,5-bis(trifluoromethyl)benzyl]-N'-(2-methyl-2H-tetrazol-5-yl)amino]methyl}indan-5-yl)-N-ethylamino]-hexanoic acid hydrochloride,
5-[N-(6-{[N'-[3,5-bis(trifluoromethyl)benzyl]-N'-(2-methyl-2H-tetrazol-5-yl)amino]methyl}indan-5-yl)-N-ethylamino]-3,3-dimethylpentanoic acid hydrochloride,
trans-4-{[N-(6-{[N'-[3,5-bis(trifluoromethyl)benzyl]-N'-(2-ethyl-2H-tetrazol-5-yl)amino]methyl}indan-5-yl)-N-ethylamino]methyl}cyclohexanecarboxylic acid hydrochloride,
(1-{2-[N-(6-{ [N'-[3,5-bis(trifluoromethyl)benzyl]-N'-(2-methyl-2H-tetrazol-5-yl)amino]methyl}indan-5-yl)-N-ethylamino]ethyl}cyclopentyl)acetic acid hydrochloride,
trans-4-({N-[6-({N'-[3,5-bis(trifluoromethyl)benzyl]-N'-[2-(2-hydroxyethyl)-2H-tetrazol-5-yl]amino}methyl)indan-5-yl]-N-ethylamino}methyl)cyclohexanecarboxylic acid,
trans-4-{[N-(2-{[N'-[3,5-bis(trifluoromethyl)benzyl]-N'-(2-methyl-2H-tetrazol-5-yl)amino]methyl}-4-trifluoromethylphenyl)-N-ethylamino]methyl}cyclohexanecarboxylic acid hydrochloride,
(1-{2-[N-(3-{ [N'-[3,5-bis(trifluoromethyl)benzyl]-N'-(2-methyl-2H-tetrazol-5-yl)amino]methyl}-5,6,7,8-tetrahydronaphthalen-2-yl)-N-ethylamino]ethyl}cyclopentyl)acetic acid,
trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid hydrochloride,
trans-4-{ [N-(3-{ [N'-[3,5-bis(trifluoromethyl)benzyl]-N'-(2-ethyl-2H-tetrazol-5-yl)amino]methyl}-5,6,7,8-tetrahydronaphthalen-2-yl)-N-ethylamino]methyl}cyclohexanecarboxylic acid hydrochloride,
trans-4-({N-[3-({N'-[3,5-bis(trifluoromethyl)benzyl]-N'-[2-(2-hydroxyethyl)-2H-tetrazol-5-yl]amino}methyl)-5,6,7,8-tetranydronaphthalen-2-yl]-N-ethylamino}methyl)cyclohexanecarboxylic acid hydrochloride,
1-{3-[N-(6-{ [N'-[3,5-bis(trifluoromethyl)benzyl]-N'-(2-methyl-2H-tetrazol-5-yl)amino]methyl}indan-5-yl)-N-ethylamino]propyl}cyclohexanecarboxylic acid hydrochloride,
5-[N-(6-{[N'-[3,5-bis(trifluoromethyl)benzyl]-N'-(1-methyl-1H-pyrazol-3-yl)amino]methyl}indan-5-yl)-N-ethylamino]-3,3-dimethylpentanoic acid,
5-[N-(6-{[N'-[3,5-bis(trifluoromethyl)benzyl]-N'-(5-methyl-[1,2,4]oxadiazol-3-yl)amino]methyl]indan-5-yl)-N-ethylamino]-3,3-dimethylpentanoic acid hydrochloride,
5-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]-3,3-dimethylpentanoic acid hydrochloride,
5-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]-3,3-dimethylpentanoic acid hydrochloride,
trans-4-{[N-(2-{[N'-[3,5-bis(trifluoromethyl)benzyl]-N'-(1-methyl-1H-pyrazol-3-yl)amino]methyl}-4-trifluoromethoxyphenyl)-N-ethylamino]methyl}cyclohexanecarboxylic acid hydrochloride,
5-[N-(2-{[N'-[3,5-bis(trifluoromethyl)benzyl]-N]-(1-methyl-1H-pyrazol-3-yl)amino]methyl-4-trifluoromethoxyphenyl)-N-ethylamino]-3,3-dimethylpentanoic acid hydrochloride,
5-[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]-3,3-dimethylpentanoic acid hydrochloride,
trans-4-{[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]methyl}cyclohexanecarboxylic acid hydrochloride,
6-[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]hexanoic acid hydrochloride,
trans-(4-{ [N-(6-{[N'-[3,5-bis(trifluoromethyl)benzyl]-N'-(2-methyl-2H-tetrazol-5-yl)amino]methyl}indan-5-yl)-N-ethylamino]methyl}cyclohexyl)acetic acid hydrochloride,
6-[N-(6-{[N'-[3,5-bis(trifluoromethyl)benzyl]-N'-(2-methyl-2H-tetrazol-5-yl)amino]methyl}indan-5-yl)-N-ethylamino]-4,4-dimethylhexanoic acid hydrochloride,
6-[N-(6-{[N'-[3,5-bis(trifluoromethyl)benzyl]-N'-(2-methyl-2H-tetrazol-5-yl)amino]methyl}indan-5-yl)-N-ethylamino]-3,3-dimethylhexanoic acid hydrochloride,
5-[N-(6-1[N'-[3,5-bis(trifluoromethyl)benzyl]-N'-(2-methyl-2H-tetrazol-5-yl)amino]methyl}indan-5-yl)-N-ethylamino]-4,4-dimethylpentanoic acid hydrochloride,
trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)acetic acid hydrochloride,
6-[N-(2-{[N'-[3,5-bis(trifiuoromethyl)benzyl]-N'-(2-methyl-2H-tetrazol-5-yl)amino]methyl}-4-trifluoromethoxyphenyl)-N-ethylamino]-4,4-dimethylhexanoic acid hydrochloride,
6-[N-(2-{[N'-[3,5-bis(trifluoromethyl)benzyl]-N'-(2-methyl-2H-tetrazol-5-yl)amino]methyl}-4-trifluoromethylphenyl)-N-ethylamino]-4,4-dimethylhexanoic acid hydrochloride,
trans-(4-{[N-(2-{[N'-[3,5-bis(trifluoromethyl)benzyl]-N'-(2-methyl-2H-tetrazol-5-yl)amino]methyl}-4-trifluoromethylphenyl)-N-ethylamino]methyl}cyclohexyl)acetic acid hydrochloride,
trans-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)methanol hydrochloride,
6-[N-(6-{ [N'-[3,5-bis(trifluoromethyl)benzyl]-N'-(2-methyl-2H-tetrazol-5-yl)amino]methyl}indan-5-yl)-N-ethylamino]-5,5-dimethylhexanoic acid hydrochloride,
trans-4-{[N-(2-{[N'-[3,5-"bis(trifluoromethyl)benzyl]-N'-(2-methyl-2H-tetrazol-5-yl)amino]methyl}-4-trifluoromethoxyphenyl)-N-propylamino]methyl}cyclohexanecarboxylic acid hydrochloride,
trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-isobutylamino]methyl}cyclohexanecarboxylic acid hydrochloride,
trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid amide,
trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid methylamide,
trans-4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexanecarboxylic acid dimethylamide,
trans-4-{[N-(2-{[N'-[3,5-bis(trifluoromethyl)benzyl]-N'-(4-chlorophenyl)amino]methyl}-4-trifluoromethoxyphenyl)-N-ethylamino]methyl}cyclohexanecarboxylic acid hydrochloride,
trans-4-{[N'-(2-{[N'-[3,5-bis(trifluoromethyl)benzyl]-N'-(p-tolyl)amino]methyl}-4-trifluoromethoxyphenyl)-N-ethylamino]methyl}cyclohexanecarboxylic acid hydrochloride,
trans-4-{[N-(2-{[N'-[3,5-bis(trifluoromethyl)benzyl]-N'-(m-tolyl)amino]methyl}-4-trifluoromethoxyphenyl)-N-ethylamino]methyl}cyclohexanecarboxylic acid hydrochloride,
trans-4-{[N-(2-{[N'-(3,5-dichlorobenzyl)-N'-(2-methyl-2H-tetrazol-5-yl)amino]methyl}-4-trifluoromethoxyphenyl)-N-ethylamino]methyl}cyclohexanecarboxylic acid hydrochloride,
trans-4-{[N-ethyl-N-(2-{[N'-(2-methyl-2H-tetrazol-5-yl)-N'-(3-methyl-5-trifluoromethylbenzyl)amino]methyl}-4-trifluoromethoxyphenyl)amino]methyl}cyclohexanecarboxylic acid hydrochloride,
trans-4-{[N-(2-{[N'-(3-chloro-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
trans-4-{[N-ethyl-N-(2-{[N'-(2-methyl-2H-tetrazol-5-yl)-N'-(3-nitro-5-trifluoromethylbenzyl)amino]methyl}-4-trifluoromethoxyphenyl)amino]methyl}cyclohexanecarboxylic acid hydrochloride,
trans-(4-{[N-(6-{[N'-[3,5-bis(trifluoromethyl)benzyl]-N'-(2-methyl-2H-tetrazol-5-yl)amino]methyl}-2,2-difluorobenzo[1,3]dioxol-5-yl)-N-ethylamino]methyl}cyclohexyl)methanol hydrochloride,
trans-(4-{[N-(6-{[N'-[3,5-bis(trifluoromethyl)benzyl]-N'-(2-methyl-2H-tetrazol-5-yl)amino]methyl}-2,2-difluorobenzo[1,3]dioxol-5-yl)-N-ethylamino]methyl}cyclohexyl)acetic acid hydrochloride,
trans-3-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)propionic acid hydrochloride,
trans-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)methanol hydrochloride,
trans-(4-([N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)acetic acid hydrochloride,
trans-2-(4-{[N-(7-{[N'-[3,5-bis(trifluoromethyl)benzyl]-N'-(2-methyl-2H-tetrazol-5-yl)amino]methyl}-2,2,3,3-tetrafluoro-2,3-dihydrobenzo[1,4]dioxin-6-yl)-N-ethylamino]methyl}cyclohexyl)acetamide hydrochloride,
trans-N-[3,5-bis(trifluoromethyl)benzyl]-N-{2-[N'-ethyl-N'-(4-(methoxymethyl)cyclohexylmethyl)amino]-5-trifluoromethoxybenzyl}-(2-methyl-2H-tetrazol-5-yl)amine hydrochloride,
trans-2-(4-{[N-(2-{[N'-[3,5-bis(trifluoromethyl)benzyl]-N'-(2-methyl-2H-tetrazol-5-yl)amino]methyl}-4-trifluoromethoxyphenyl)-N-ethylamino]methyl}cyclohexyl)ethanol hydrochloride,
trans-(4-{[N-(2-{[N'-[3,5-bis(trifluoromethyl)benzyl}-N'-(2-methyl-2H-tetrazol-5-yl)amino]methyl}-4-methyl-5-trifluoromethylphenyl)-N-ethylamino]methyl}cyclohexyl)methanol hydrochloride,
trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid hydrochloride,
trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)acetic acid hydrochloride,
trans-(4-{ [N-(2-{ [N'-[3,5-bis(trifluorormethyl)benzyl]-N'-(2-methyl-2H-tetrazol-5-yl)amino]methyl}-4-trifluoromethoxyphenyl)-N-ethylamino]methyl}cyclohexylmethyl)phosphonic acid,
trans-4-{[N-(2-{[N'-(3-bromo-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
trans-(4-{[N-(2-{[N'-[3,5-bis(trifluoromethyl)benzyl]-N'-(2-methyl-2H-tetrazol-5-yl)amino]methyl}-4-bromophenyl)-N-ethylamino]methyl}cyclohexyl)methanol hydrochloride,
trans-(4-{[N-(2-{[N'-[3,5-bis(trifluoromethyl)benzyl]-N'-(2-methyl-2H-tetrazol-5-yl)amino]methyl}-4-bromophenyl)-N-ethylamino]methyl}cyclohexyl)acetic acid hydrochloride,
trans-(4-{[N-(2-{[N'-[3,5-bis(trifluoromethyl)benzyl]-N'-(2-methyl-2H-tetrazol-5-yl)amino]methyl}-4-chloro-5-ethylphenyl)-N-ethylamino]methyl}cyclohexyl)methanol hydrochloride,
trans-4-{[N-(2-{[N'-[3,5-bis(trifluoromethyl)benzyl]-N'-(4-methoxyphenyl)amino]methyl}-4-trifluoromethoxyphenyl)-N-ethylamino]methyl}cyclohexanecarboxylic acid hydrochloride,
trans-(4-{ [N-(2-{ [N'-[3,5-bis(trifluoromethyl)benzyl]-N'-(2-methyl-2H-tetrazol-5-yl)amino]methyl}-5-methoxy-4-methylphenyl)-N-ethylamino]methyl}cyclohexyl)methanol hydrochloride,
trans-(4-{[N-(2-{[N'-[3,5-bis(trifluoromethyl)benzyl]-N'-(2-methyl-2H-tetrazol-5-yl)amino]methyl}-4,5-dimethylphenyl)-N-ethylamino]methyl}cyclohexyl)acetic acid hydrochloride,
trans-(4-{[N-(2-{[N'-[3,5-bis(trifluoromethyl)benzyl]-N'-(2-methyl-2H-tetrazol-5-yl)amino]methyl}-4-trifluoromethylthiophenyl)-N-ethylamino]methyl}cyclohexyl)acetic acid hydrochloride,
trans-(4-{[N-(2-{[N'-[3,5-bis(trifluoromethyl)benzyl]-N'-(2-methyl-2H-tetrazol-5-yl)amino]methyl}-4-chloro-5-ethylphenyl)-N-ethylamino]methyl}cyclohexyl)acetic acid hydrochloride,
trans-(4-{ [N-(2-{ [N'-[3,5-bis(trifluoromethyl)benzyl]-N'-(2-methyl-2H-tetrazol-5-yl)amino]methyl}-4-trifluoromethylphenyl)-N-propylamino]methyl}cyclohexyl)acetic acid hydrochloride,
trans-(4-{ [N-(2-{ [N'-[3,5-bis(trifluoromethyl)benzyl]-N'-(2-methyl-2H-tetrazol-5-yl)amino]methyl}-5-methoxy-4-methylphenyl)-N-ethylamino]methyl}cyclohexyl)acetic acid hydrochloride,
trans-4-({N-[2-{[N'-[3,5-bis(trifluoromethyl)benzyl]-N'-(2-methyl-2H-tetrazol-5-yl)amino]methyl}-4-(2,2,2-trifluoroethyl)phenyl]-N-ethylamino}methyl)cyclohexanecarboxylic acid hydrochloride,
trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-propylamino]methyl}cyclohexyl)acetic acid hydrochloride,
trans-(4-{ [N-(2-{ [N'-[3,5-bis(trifluoromethyl)benzyl]-N'-(2-methyl-2H-tetrazol-5-yl)amino]methyl}-4-trifluoromethoxyphenyl)-N-propylamino]methyl}cyclohexyl)acetic acid hydrochloride,
trans-4-{[N-(2-{[N'-[3,5-bis(trifluoromethyl)benzyl]-N'-(4-ethylphenyl)amino]methyl}-4-trifluoromethoxyphenyl)-N-ethylamino]methyl}cyclohexanecarboxylic acid hydrochloride,
trans-4-{[N-(2-{[N'-(3-cyano-5-trifluoromethylbenzyl)-N'-(2-methyl-2H-tetrazol-5-yl)amino]methyl}-4-trifluoromethoxyphenyl)-N-ethylamino]methyl}cyclohexanecarboxylic acid hydrochloride,
trans-4-{[N-(2-{[N'-[3,5-bis(trifluoromethyl)benzyl]-N'-(4-isopropenylphenyl)amino]methyl}-4-trifluoromethoxyphenyl)-N-ethylamino]methyl}cyclohexanecarboxylic acid,
trans-(4-{[N-(2-{[N'-[3,5-bis(trifluoromethyl)benzyl]-N'-(p-tolyl)amino]methyl}-4-trifluoromethoxyphenyl)-N-ethylamino]methyl}cyclohexyl)acetic acid dihydrochloride,
trans-(4-{[N-(2-{[N'-[3,5-bis(trifluoromethyl)benzyl]-N'-(5-methyl-[1,2,4]oxadiazol-3-yl)amino]methyl}-4-trifluoromethoxyphenyl)-N-ethylamino]methyl}cyclohexyl)acetic acid hydrochloride,
trans-(4-{ [N-(2-{ [N'-[3,5-bis(trifluoromethyl)benzyl]-N'-(5-methyl-[1,2,4]oxadiazol-3-yl)amino]methyl}-5-methyl-4-trifluoromethylphenyl)-N-ethylamino]methyl}cyclohexyl)acetic acid hydrochloride,
trans-(4-{[N-(2-{[N'-[3,5-bis(trifluoromethyl)benzyl]-N'-(5-methyl-[1,2,4]oxadiazol-3-yl)amino]methyl}-5-methyl-4-trifluoromethylphenyl)-N-propylamino]methyl}cyclohexyl)acetic acid hydrochloride,
trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-propylamino]methyl}cyclohexyl)acetic acid,
trans-(4-{[N-(2-{[N'-[3,5-bis(trifluoromethyl)benzyl]-N'-(2-methyl-2H-tetrazol-5-yl)amino]methyl}-4-methyl-5-trifluoromethylphenyl)-N-ethylamino]methyl}cyclohexyl)acetic acid,
trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate,
ethyl trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetate, and
trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid, a prodrug thereof or a pharmaceutically acceptable salt thereof.

3. The compound for use of claim 1 or 2, wherein the compound is trans-(4-{[N-(2-{[N'-[3,5-bis(trifluoromethyl)benzyl]-N'-(2-methyl-2H-tetrazol-5-yl)amino]methyl}-5-methyl-4-trifluoromethylphenyl)-N-ethylamino]methyl}cyclohexyl)acetic acid methanesulfonate.

4. Use of a compound as defined in any of claims 1 to 3 for the manufacture of a medicament for the prophylaxis or treatment of a disease selected from the group consisting of hyperlipidemia, arteriosclerosis and hyper-remnant-emia.
